# EUROPEAN PATENT SPECIFICATION

(11) **EP 4 486 753 B1**
(45) Date of publication and mention of the grant of the patent: **13.05.2026**
(21) Application number: 23711787.4
(22) Date of filing: 03.03.2023
(51) Int. Cl.: C07H 15/232, A61K 31/7036, A61P 31/04

(54) **BROAD SPECTRUM AMINOGLYCOSIDES AND USES THEREOF**
AMINOGLYCOSIDE MIT BREITEM SPEKTRUM UND VERWENDUNGEN DAVON
AMINOGLYCOSIDES À LARGE SPECTRE ET LEURS UTILISATIONS

(30) Priority: 04.03.2022 US 202263316731 P
(43) Date of publication of application: 08.01.2025
(73) Proprietor: Revagenix, Inc., San Francisco, California 94104-5401 (US)
(72) Inventor: LOPEZ, Michael Steven, San Francisco, California 94104-5401 (US); CIRZ, Ryan Thomas, San Francisco, California 94104-5401 (US); CALABRESE, Andrew Antony, San Francisco, California 94104-5401 (US)
(74) Representative: Murgitroyd & Company
(86) International application number: PCT/IB2023/052011
(87) International publication number: WO 2023/166488

(56) References cited:
- WO-A1-2019/046126

## Description

### CROSS-REFERENCE TO RELATED APPLICATIONS

This application claims priority to and the benefit of U.S. Provisional Patent Application No. 63/316,731, filed March 4, 2022.

### STATEMENT REGARDING FEDERALLY SPONSORED RESEARCH OR DEVELOPMENT

This invention was made with government support under Contract No. 75N93020C00018 awarded by the National Institutes of Health. The government has certain rights in this invention.

### TECHNICAL FIELD

The present disclosure relates generally to aminoglycoside compounds having antibacterial activity, and to uses thereof in treatment of medical conditions associated with pathogenic microorganisms.

### BACKGROUND

The rapid spread of antibiotic resistance has prompted a continuing search for new agents capable of antibacterial activity. Aminoglycosides (AGs) are highly potent, broad-spectrum antibiotics with many desirable properties for the treatment of life-threatening infections. AGs are used to treat a variety of diseases including, but not limited to, urinary tract infections, bloodstream infections, pneumonia and neonatal sepsis. Examples of AGs include tobramycin, gentamicin, and amikacin. AG antibiotics exert their antibacterial effects by binding to specific target sites in the bacterial ribosome. For the structurally related antibiotics neamine, ribostamycin, neomycin B, and paromomycin, the binding site has been localized to the A-site of the prokaryotic 16S ribosomal decoding region RNA (see Moazed, D.; Noller, H.F., Nature, 1987, 327, 389). Binding of AGs to this RNA target interferes with the fidelity of mRNA translation and results in miscoding and truncation, leading ultimately to bacterial cell death (see Alper et al., J. Am. Chem. Soc., 1998, 120, 1965).

Over time, resistance to AGs has emerged in the clinic and degraded our antibacterial armamentarium. AG resistance (AG-R) can be categorized mechanistically into two primary types: (1) AG-modifying enzymes (AMEs), which modify and inactive the drug; and (2) enzymes called ribosomal methyltransferases (RMTs), which modify the drug target binding pocket. At present, the most common form of AG-R amongst clinical isolates is conferred by AMEs, which are diverse and widely distributed. RMTs, while less common than AMEs in some species, are a serious threat because they modify the 16S rRNA AG binding site and prevent AG target engagement, conferring high-level resistance to all clinically available parenteral AGs. As an additional concern, AME and RMT genes are typically located on plasmids or transposons together with genes encoding resistance to other classes of antibacterial agents, which leads to multidrug resistant isolates.

Further, AGs can be toxic to the kidney (nephrotoxicity) and cochlea (ototoxicity). These are cumulative processes, with the likelihood of observing toxicity increasing with treatment duration. The nephrotoxic potential of AGs limits the dose and the length of treatment, making it challenging to achieve the systemic exposures required for efficacy against some infections. Quite often, *but not always,* the structure-activity relationships that lead to increased antibacterial potency are correlated with those that cause toxicity to human kidney cells. The origin of this toxicity is assumed to result from a combination of different factors and mechanisms such as interactions with phospholipids, inhibition of phospholipases and the formation of free radicals. WO2019/046126 A1 describes aminoglycoside compounds and their use in treating a bacterial infection in a subject, for example a Gram-negative bacterial infection.

For the foregoing reasons, while progress has been made in this field, there is a need for new chemical entities that possess antibacterial activity against highly resistant organisms and an acceptable safety profile.

### SUMMARY

The present disclosure relates to aminoglycoside compounds, including stereoisomers, tautomers, solvates, and pharmaceutically acceptable salts thereof, having antibacterial activity, and to uses thereof in, for example, treatment of medical conditions associated with a pathogenic microorganism, which are also referred to herein as "bacterial infections."

According to a first aspect there is provided a compound as claimed in claim 1.

**In** one embodiment, the compound is a compound having a structure according to Formula (I): 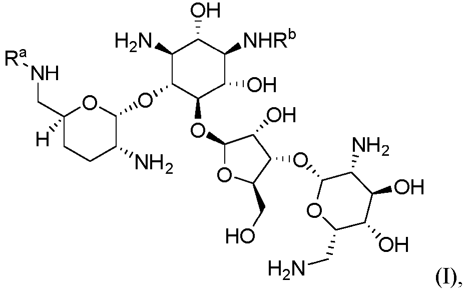 or a pharmaceutically acceptable salt or solvate thereof,
wherein:
R^{a} is hydrogen (H) or methyl; and
R^{b} has a structure according to Formula (II): 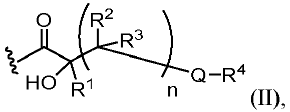 wherein:
   Q is NH, O, or S;
   n is an integer from 0 to 4;
   R¹ is hydrogen or C₁₋₃ alkyl;
   R² and R³ are each selected independently for each occurrence from the group consisting of hydrogen, alkyl, halogen, and -OH; and
   R⁴ is H, C₁₋₃ alkyl, or -C(=NH)NR^{4a}R⁴ , wherein R^{4a} and R^{4b} are each independently selected from the group consisting of H and C₁₋₃ alkyl; or
   R¹ and R⁴, together with the atoms to which they are attached, form a heterocycloalkyl ring system comprising at least one N.

In some embodiments, R^{a} is H.

In some embodiments, R^{a} is methyl (CH₃).

In some embodiments, R^{b} is selected from the group consisting of:

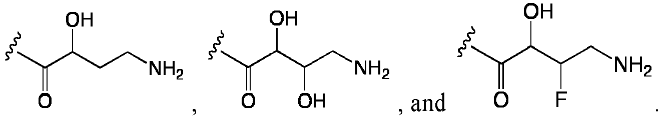

In some embodiments, R^{b} is selected from the group consisting of:

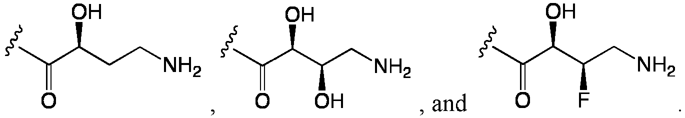

In some embodiments, the compound of Formula (I) has a structure selected from the group consisting of:

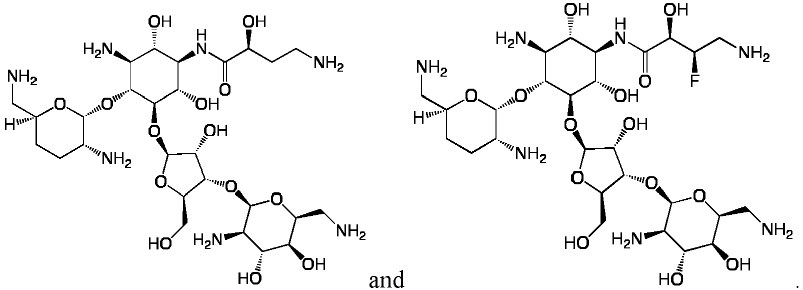

In some embodiments, the compound of Formula (I) has a structure:

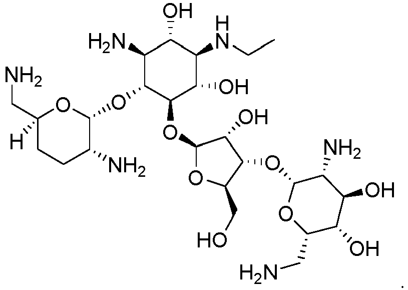

In another embodiment, the compound of the invention is a compound having a structure according to Formula (III): 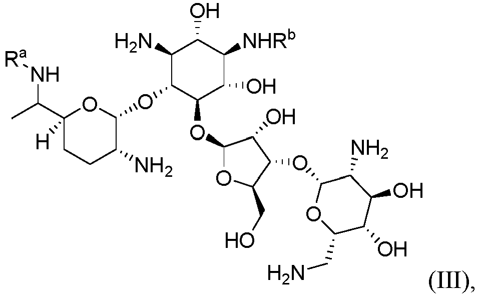 wherein:
R^{a} is H or methyl; and
R^{b} has a structure according to Formula (II): 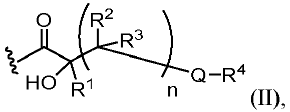 wherein:
   Q is NH, O, or S;
   n is an integer from 0 to 4;
   R¹ is hydrogen or C₁₋₃ alkyl;
   R² and R³ are each selected independently for each occurrence from the group consisting of hydrogen, alkyl, halogen, and -OH; and
   R⁴ is H, C₁₋₃ alkyl, or -C(=NH)NR^{4a}R⁴ , wherein R^{4a} and R^{4b} are each independently selected from the group consisting of hydrogen and C₁₋₃ alkyl; or
   R¹ and R⁴, together with the atoms to which they are attached, form a heterocycloalkyl ring system comprising at least one N.

In some embodiments, R^{a} is H.

In some embodiments, R^{a} is methyl.

In some embodiments, the compound of Formula (III) has a structure selected from the group consisting of:

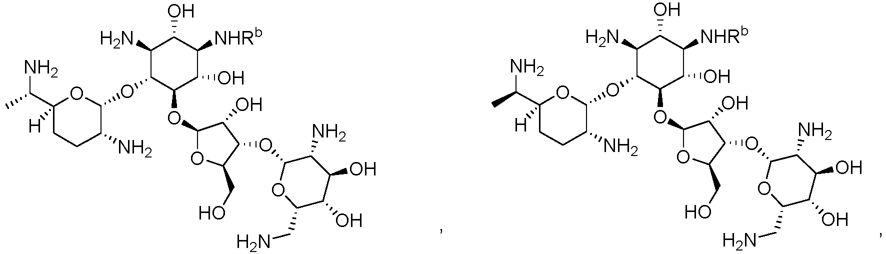

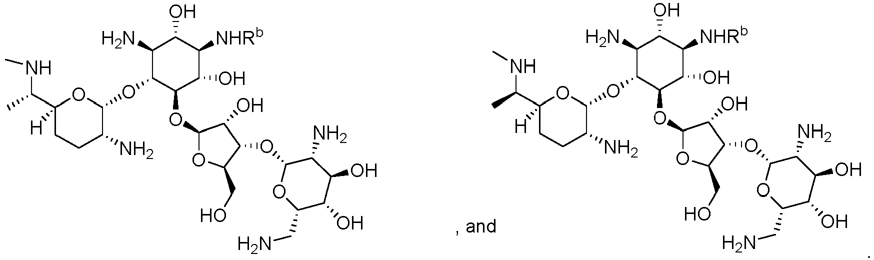

In some embodiments, R^{b} is selected from the group consisting of:

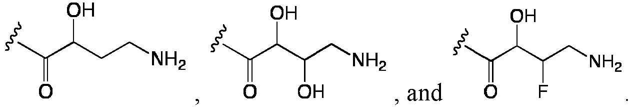

In some embodiments, R^{b} is selected from the group consisting of:

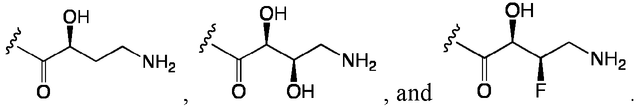

In some embodiments, the compound of Formula (III) has a structure selected from the group consisting of:

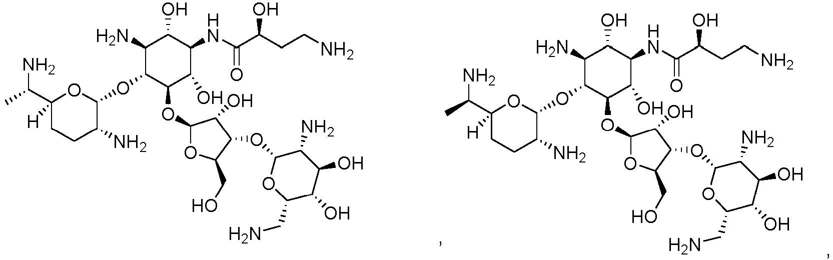

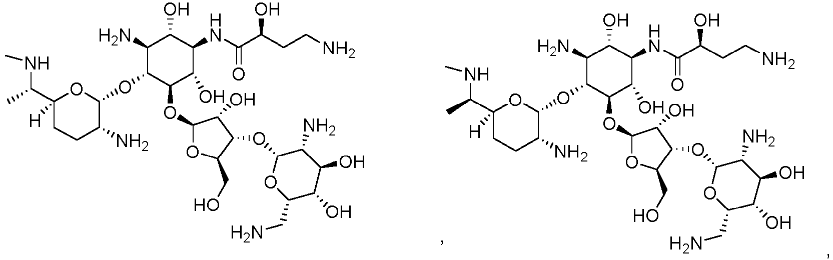

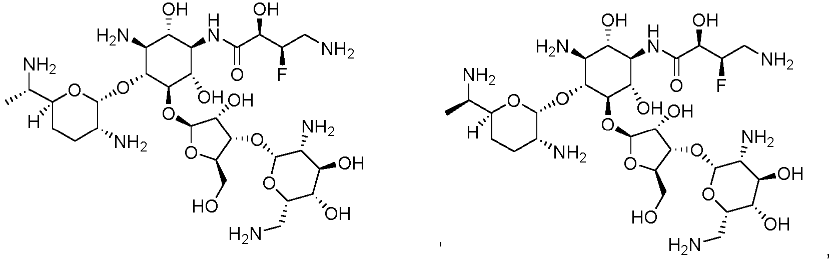

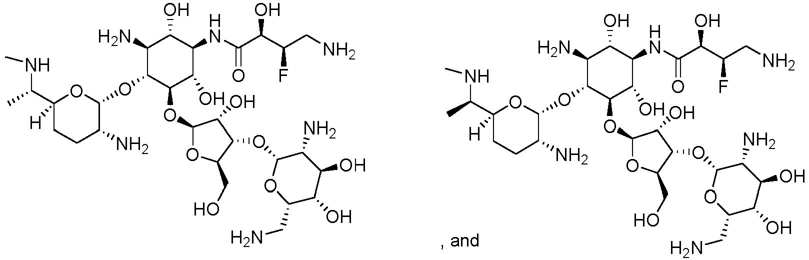

In a second aspect there is provided a pharmaceutical composition comprising a compound of Formula (I), Formula (III), or a pharmaceutically acceptable salt or solvate of either thereof and at least one pharmaceutically acceptable excipient.

In a further aspect is provided the compound of the first aspect of the invention, or a pharmaceutically acceptable salt, solvate, tautomer, or stereoisomer thereof, or the pharmaceutical composition of the second aspect of the invention, for use in treating a bacterial infection in a subject in need thereof.. In some embodiments, the treatment comprises administering to the subject a compound of Formula (I) or (III), or a pharmaceutically acceptable salt or solvate of any thereof, or a pharmaceutical composition comprising said compound.

In some embodiments, the bacterial infection is with Gram-positive, Gram-negative, aerobic, or facultative anaerobic bacteria. In some embodiments, the bacterial infection is a Gram-negative bacterial infection. In some embodiments, the bacterial infection is a Gram-positive bacterial infection.

In some embodiments, the bacterial infection is infection with a *Staphylococcus, Lactobacillus, Streptococcus, Sarcina, Escherichia, Enterobacter, Klebsiella, Pseudomonas, Acinetobacter, Mycobacterium, Proteus, Campylobacter, Citrobacter, Neisseria, Bacillus, Peptococcus, Salmonella, Shigella, Serratia, Haemophilus, Brucella, Francisella, Yersinia, Corynebacterium, Moraxella, Burkholderia* or *Enterococcus* species.

In some embodiments, the bacterial infection is infection with *Escherichia coli, Klebsiella pneumoniae, Klebsiella oxytoca, Enterobacter cloacae, Enterobacter aerogenes, Citrobacter freundii, Citrobacter koseri, Proteus mirabilis, Bacillus anthracis, Pseudomonas aeruginosa, Acinetobacter baumannii, Proteus vulgaris, Francisella tularensis, Burkholderia cepacia, Burkholderia mallei, Burkholderia pseudomallei* or *Yersinia pestis.*

In some embodiments, the bacterial infection is a Gram-negative bacterial infection. In some embodiments, the Gram-negative bacterial infection is an infection with *Pseudomonas aeruginosa, Stenotrophomonas maltophila, Burkholderia cepacia,* or *Alcaligenes xylosoxidans,*

In some embodiments, the bacterial infection is caused by multidrug resistant (MDR) bacteria.

In some embodiments, the bacterial infection is infection with an extended spectrum beta-lactamase (ESBL)-producing or carbapenem-resistant *Enterobacteriaceae.*

In some embodiments, the bacterial infection is caused by *Staphylococcus aureus.* In some embodiments, the *Staphylococcus aureus* is Methicillin-resistant *Staphylococcus aureus* (MRSA).

These and other features, aspects, and advantages of the present disclosure will be apparent from a reading of the following detailed description. The present disclosure includes any combination of two, three, four or more features or elements set forth in this disclosure, regardless of whether such features or elements are expressly combined or otherwise recited in a specific example implementation described herein. This disclosure is intended to be read holistically such that any separable features or elements of the disclosure, in any of its aspects and example implementations, should be viewed as combinable, unless the context of the disclosure clearly dictates otherwise.

It will therefore be appreciated that this Summary is provided merely for purposes of summarizing some example implementations so as to provide a basic understanding of some aspects of the disclosure. Accordingly, it will be appreciated that the above-described example implementations are merely examples and should not be construed to narrow the scope or spirit of the disclosure in any way. Other example implementations, aspects, and advantages will become apparent from the following detailed description.

### BRIEF DESCRIPTION OF THE DRAWINGS

Having thus described aspects of the disclosure in the foregoing general terms, reference will now be made to the accompanying drawings, which are not necessarily drawn to scale. The drawings are exemplary only and should not be construed as limiting the disclosure.
**FIG. 1** illustrates a non-limiting scheme for preparation of a compound of the disclosure having the structure of **Ring A1** according to one embodiment;
**FIG. 2** illustrates a non-limiting scheme for preparation of a compound of the disclosure having the structure of **Ring A2** according to one embodiment;
**FIG. 3** illustrates a non-limiting scheme for preparation of a compound of the disclosure having the structure of **Ring A3** according to one embodiment;
**FIG. 4** illustrates a non-limiting scheme for preparation of compounds of the disclosure having the structure of **Ring B** according to one embodiment;
**FIG. 5** illustrates a non-limiting scheme for preparation of a compound of the disclosure having the structure of **Ring AB1** according to one embodiment;
**FIG. 6** illustrates a non-limiting scheme for preparation of a compound of the disclosure having the structure of **Ring AB2** according to one embodiment;
**FIG. 7** illustrates a non-limiting scheme for preparation of a compound of the disclosure having the structure of **Ring AB3** according to one embodiment;
**FIG. 8** illustrates a non-limiting scheme for preparation of compounds of the disclosure having the structure of **Ring ABCD1** according to one embodiment;
**FIG. 9** illustrates a non-limiting scheme for preparation of compounds of the disclosure having the structure of **Ring ABCD2** according to one embodiment;
**FIG. 10** illustrates a non-limiting scheme for preparation of compounds of the disclosure having the structure of **Ring ABCD3** according to one embodiment;
**FIG. 11** illustrates a non-limiting scheme for preparation of compounds of the disclosure according to Formula (I) having the structure of **Ring ABCD1** according to one embodiment;
**FIG. 12** illustrates a non-limiting scheme for preparation of compounds of the disclosure according to Formula (III) having the structure of **Ring ABCD2** according to one embodiment;
**FIG. 13** illustrates a non-limiting scheme for preparation of compounds of the disclosure according to Formula (III) having the structure of **Ring ABCD3** according to one embodiment; and
**FIG. 14** illustrates a non-limiting scheme for preparation of a protected precursor of (2*R*,3*R*)-4-amino-3-fluoro-2-hydroxybutanoic acid according to one embodiment.

### DETAILED DESCRIPTION

The present disclosure will now be described more fully hereinafter with reference to example embodiments thereof. Before describing several example embodiments of the technology, it is to be understood that the technology is not limited to the details of construction or process steps set forth in the following description. The technology is capable of other embodiments and of being practiced or being carried out in various ways.

The following description sets forth numerous exemplary configurations, methods, parameters, and the like in order to provide a thorough understanding of various embodiments of the disclosure. It should be recognized, however, that such description is not intended as a limitation on the scope of the present disclosure but is instead provided as a description of exemplary embodiments.

The present disclosure is generally directed to aminoglycoside compounds, including stereoisomers, tautomers, solvates, and pharmaceutically acceptable salts thereof, and pharmaceutical compositions comprising said compounds, or stereoisomers, tautomers, solvates, and pharmaceutically acceptable salts thereof. The disclosure particularly relates to such compounds, stereoisomers, tautomers, solvates, and pharmaceutically acceptable salts thereof with antibacterial activity. Accordingly, compounds and pharmaceutical compositions of the disclosure may be useful for treating bacterial infections as well as medical conditions associated therewith. Compounds and pharmaceutical compositions of the disclosure may also be useful for treating difficult-to-treat resistance mechanisms as well as multidrug resistant (MDR) bacterial infections.

Unless defined otherwise, all technical and scientific terms used herein have the same meaning as is commonly understood by one of skill in the art to which this disclosure belongs.

### Definitions

Unless defined otherwise, all technical and scientific terms used herein have the same meaning as is commonly understood by one of skill in the art to which this invention belongs. With respect to the terms used in this disclosure, the following definitions are provided. This application will use the following terms as defined below unless the context of the text in which the term appears requires a different meaning.

The articles "a" and "an" as used in this disclosure may refer to one or more than one (i.e., to at least one) of the grammatical object of the article. By way of example, "an element" may mean one element or more than one element.

The term "and/or" as used in this disclosure may mean either "and" or "or" unless indicated otherwise.

The term "about" used throughout this specification is used to describe and account for small fluctuations. For example, the term "about" can refer to less than or equal to ±10%, or less than or equal to ±5%, such as less than or equal to ±2%, less than or equal to ±1%, less than or equal to ±0.5%, less than or equal to ±0.2%, less than or equal to ±0.1% or less than or equal to ±0.05%. All numeric values herein are modified by the term "about," whether or not explicitly indicated. A value modified by the term "about" of course includes the specific value. For instance, "about 5.0" must include 5.0.

Unless the context requires otherwise, throughout the present specification and claims, as used herein, the terms "including," "containing," and "comprising" are used in their open, non-limiting sense.

"Alkyl" refers to a straight or branched hydrocarbon chain radical consisting solely of carbon and hydrogen atoms, containing no unsaturation, and preferably having from one to fifteen carbon atoms (*i.e.,* C₁-C₁₅ alkyl). In certain embodiments, an alkyl comprises one to thirteen carbon atoms (*i.e.,* C₁-C₁₃ alkyl). In certain embodiments, an alkyl comprises one to eight carbon atoms (*i.e.,* C₁-C₈ alkyl). In other embodiments, an alkyl comprises one to five carbon atoms (*i.e.,* C₁-C₅ alkyl). In other embodiments, an alkyl comprises one to four carbon atoms (*i.e.,* C₁-C₄ alkyl). In other embodiments, an alkyl comprises one to three carbon atoms (*i.e.,* C₁-C₃ alkyl). In other embodiments, an alkyl comprises one to two carbon atoms *(i.e.,* C₁-C₂ alkyl). In some embodiments, an alkyl comprises two carbon atoms *(i.e.,* C₂ alkyl or ethyl). In other embodiments, an alkyl comprises one carbon atom (*i.e.,* C₁ alkyl). In other embodiments, an alkyl comprises five to fifteen carbon atoms (*i.e.,* C₅-C₁₅ alkyl). In other embodiments, an alkyl comprises five to eight carbon atoms (*i.e.,* C₅-C₈ alkyl). In other embodiments, an alkyl comprises two to five carbon atoms *(i.e.,* C₂-C₅ alkyl). In other embodiments, an alkyl comprises three to five carbon atoms *(i.e.,* C₃-C₅ alkyl). In certain embodiments, the alkyl group is selected from methyl, ethyl, 1-propyl (*n*-propyl), 1-methylethyl (*iso*-propyl), 1-butyl (*n*-butyl), 1-methylpropyl (*sec*-butyl), 2-methylpropyl (*iso*-butyl), 1,1-dimethylethyl (*tert*-butyl), 1-pentyl (*n*-pentyl). The alkyl is attached to the rest of the molecule by a single bond.

The term "C_{x-y}" when used in conjunction with a chemical moiety, such as alkyl, alkenyl, or alkynyl is meant to include groups that contain from x to y carbons in the chain. For example, the term "C₁₋₆alkyl" refers to substituted or unsubstituted saturated hydrocarbon groups, including straight-chain alkyl and branched-chain alkyl groups that contain from 1 to 6 carbons. The term -C_{x-y}alkylene- refers to a substituted or unsubstituted alkylene chain with from x to y carbons in the alkylene chain. For example - C₁₋₆alkylene- may be selected from methylene, ethylene, propylene, butylene, pentylene, and hexylene, any one of which is optionally substituted.

"Alkoxy" refers to a radical bonded through an oxygen atom of the formula -o-alkyl, where alkyl is an alkyl chain as defined above.

"Alkenyl" refers to a straight or branched hydrocarbon chain radical group consisting solely of carbon and hydrogen atoms, containing at least one carbon-carbon double bond, and preferably having from two to twelve carbon atoms (*i.e.,* C₂-C₁₂ alkenyl). In certain embodiments, an alkenyl comprises two to eight carbon atoms *(i.e.,* C₂-C₈ alkenyl). In certain embodiments, an alkenyl comprises two to six carbon atoms (*i.e.,* C₂-C₆ alkenyl). In other embodiments, an alkenyl comprises two to four carbon atoms *(i.e.,* C₂-C₄ alkenyl). The alkenyl is attached to the rest of the molecule by a single bond, for example, ethenyl (*i.e.,* vinyl), prop-1-enyl (*i.e.,* allyl), but-1-enyl, pent-1-enyl, penta-1,4-dienyl, and the like.

"Alkynyl" refers to a straight or branched hydrocarbon chain radical group consisting solely of carbon and hydrogen atoms, containing at least one carbon-carbon triple bond, and preferably having from two to twelve carbon atoms (*i.e.,* C₂-C₁₂ alkynyl). In certain embodiments, an alkynyl comprises two to eight carbon atoms *(i.e.,* C₂-C₈ alkynyl). In other embodiments, an alkynyl comprises two to six carbon atoms (*i.e.,* C₂-C₆ alkynyl). In other embodiments, an alkynyl comprises two to four carbon atoms *(i.e.,* C₂-C₄ alkynyl). The alkynyl is attached to the rest of the molecule by a single bond, for example, ethynyl, propynyl, butynyl, pentynyl, hexynyl, and the like.

The terms "C_{x-y}alkenyl" and "C_{x-y}alkynyl" refer to substituted or unsubstituted unsaturated aliphatic groups analogous in length and possible substitution to the alkyls described above, but that contain at least one double or triple bond, respectively. The term -C_{x-y}alkenylene- refers to a substituted or unsubstituted alkenylene chain with from x to y carbons in the alkenylene chain. For example, -C₂₋₆alkenylene- may be selected from ethenylene, propenylene, butenylene, pentenylene, and hexenylene, any one of which is optionally substituted. An alkenylene chain may have one double bond or more than one double bond in the alkenylene chain. The term -C_{x-y}alkynylene- refers to a substituted or unsubstituted alkynylene chain with from x to y carbons in the alkenylene chain. For example, -C₂₋₆alkenylene- may be selected from ethynylene, propynylene, butynylene, pentynylene, and hexynylene, any one of which is optionally substituted. An alkynylene chain may have one triple bond or more than one triple bond in the alkynylene chain.

"Alkylene" or "alkylene chain" refers to a straight or branched divalent hydrocarbon chain linking the rest of the molecule to a radical group, consisting solely of carbon and hydrogen, containing no unsaturation, and preferably having from one to twelve carbon atoms, for example, methylene, ethylene, propylene, n-butylene, and the like. The alkylene chain is attached to the rest of the molecule through a single bond and to the radical group through a single bond. The points of attachment of the alkylene chain to the rest of the molecule and to the radical group may be through any two carbons within the chain. In certain embodiments, an alkylene comprises one to ten carbon atoms (*i.e.,* C₁-C₈ alkylene). In certain embodiments, an alkylene comprises one to eight carbon atoms (*i.e.,* C₁-C₈ alkylene). In other embodiments, an alkylene comprises one to five carbon atoms (*i.e.,* C₁-C₅ alkylene). In other embodiments, an alkylene comprises one to four carbon atoms (*i.e.,* C₁-C₄ alkylene). In other embodiments, an alkylene comprises one to three carbon atoms (*i.e.,* C₁-C₃ alkylene). In other embodiments, an alkylene comprises one to two carbon atoms *(i.e.,* C₁-C₂ alkylene). In other embodiments, an alkylene comprises one carbon atom (*i.e.,* C₁ alkylene). In other embodiments, an alkylene comprises five to eight carbon atoms (*i.e.,* C₅-C₈ alkylene). In other embodiments, an alkylene comprises two to five carbon atoms *(i.e.,* C₂-C₅ alkylene). In other embodiments, an alkylene comprises three to five carbon atoms *(i.e.,* C₃-C₅ alkylene).

"Alkenylene" or "alkenylene chain" refers to a straight or branched divalent hydrocarbon chain linking the rest of the molecule to a radical group, consisting solely of carbon and hydrogen, containing at least one carbon-carbon double bond, and preferably having from two to twelve carbon atoms. The alkenylene chain is attached to the rest of the molecule through a single bond and to the radical group through a single bond. The points of attachment of the alkenylene chain to the rest of the molecule and to the radical group may be through any two carbons within the chain. In certain embodiments, an alkenylene comprises two to ten carbon atoms (*i.e.,* C₂-C₁₀ alkenylene). In certain embodiments, an alkenylene comprises two to eight carbon atoms *(i.e.,* C₂-C₈ alkenylene). In other embodiments, an alkenylene comprises two to five carbon atoms *(i.e.,* C₂-C₅ alkenylene). In other embodiments, an alkenylene comprises two to four carbon atoms (*i.e.,* C₂-C₄ alkenylene). In other embodiments, an alkenylene comprises two to three carbon atoms (*i.e.,* C₂-C₃ alkenylene). In other embodiments, an alkenylene comprises two carbon atom *(i.e.,* C₂ alkenylene). In other embodiments, an alkenylene comprises five to eight carbon atoms (*i.e.,* C₅-C₈ alkenylene). In other embodiments, an alkenylene comprises three to five carbon atoms *(i.e.,* C₃-C₅ alkenylene).

"Alkynylene" or "alkynylene chain" refers to a straight or branched divalent hydrocarbon chain linking the rest of the molecule to a radical group, consisting solely of carbon and hydrogen, containing at least one carbon-carbon triple bond, and preferably having from two to twelve carbon atoms. The alkynylene chain is attached to the rest of the molecule through a single bond and to the radical group through a single bond. The points of attachment of the alkynylene chain to the rest of the molecule and to the radical group may be through any two carbons within the chain. In certain embodiments, an alkynylene comprises two to ten carbon atoms (*i.e.,* C₂-C₁₀ alkynylene). In certain embodiments, an alkynylene comprises two to eight carbon atoms *(i.e.,* C₂-C₈ alkynylene). In other embodiments, an alkynylene comprises two to five carbon atoms *(i.e.,* C₂-C₅ alkynylene). In other embodiments, an alkynylene comprises two to four carbon atoms (*i.e.,* C₂-C₄ alkynylene). In other embodiments, an alkynylene comprises two to three carbon atoms (*i.e.,* C₂-C₃ alkynylene). In other embodiments, an alkynylene comprises two carbon atom *(i.e.,* C₂ alkynylene). In other embodiments, an alkynylene comprises five to eight carbon atoms (*i.e.,* C₅-C₈ alkynylene). In other embodiments, an alkynylene comprises three to five carbon atoms *(i.e.,* C₃-C₅ alkynylene).

"Aryl" refers to a radical derived from an aromatic monocyclic or aromatic multicyclic hydrocarbon ring system by removing a hydrogen atom from a ring carbon atom. The aromatic monocyclic or aromatic multicyclic hydrocarbon ring system contains only hydrogen and carbon and from five to eighteen carbon atoms, where at least one of the rings in the ring system is aromatic, i.e., it contains a cyclic, delocalized (4n+2) π-electron system in accordance with the Hückel theory. The ring system from which aryl groups are derived include, but are not limited to, groups such as benzene, fluorene, indane, indene, tetralin and naphthalene.

"Aralkyl" refers to a radical of the formula -R-aryl where R is an alkylene chain as defined above, for example, methylene, ethylene, and the like.

"Aralkenyl" refers to a radical of the formula -R-aryl where R is an alkenylene chain as defined above. "Aralkynyl" refers to a radical of the formula -R-aryl, where R is an alkynylene chain as defined above.

"Carbocycle" refers to a saturated, unsaturated or aromatic ring in which each atom of the ring is carbon. Carbocycle may include 3- to 10-membered monocyclic rings, 6- to 12-membered bicyclic rings, and 6- to 12-membered bridged rings. Each ring of a bicyclic carbocycle may be selected from saturated, unsaturated, and aromatic rings. In some embodiments, the carbocycle is an aryl. In some embodiments, the carbocycle is a cycloalkyl. In some embodiments, the carbocycle is a cycloalkenyl. In an exemplary embodiment, an aromatic ring, e.g., phenyl, may be fused to a saturated or unsaturated ring, e.g., cyclohexane, cyclopentane, or cyclohexene. Any combination of saturated, unsaturated and aromatic bicyclic rings, as valence permits, are included in the definition of carbocyclic. Exemplary carbocycles include cyclopentyl, cyclohexyl, cyclohexenyl, adamantyl, phenyl, indanyl, and naphthyl.

"Cycloalkyl" refers to a stable fully saturated monocyclic or polycyclic hydrocarbon radical consisting solely of carbon and hydrogen atoms, which includes fused or bridged ring systems, and preferably having from three to twelve carbon atoms. In certain embodiments, a cycloalkyl comprises three to ten carbon atoms. In other embodiments, a cycloalkyl comprises five to seven carbon atoms. The cycloalkyl may be attached to the rest of the molecule by a single bond. Examples of monocyclic cycloalkyls include, e.g., cyclopropyl, cyclobutyl, cyclopentyl, cyclohexyl, cycloheptyl, and cyclooctyl. Polycyclic cycloalkyl radicals include, for example, adamantyl, norbornyl (*i.e.,* bicyclo[2.2.1]heptanyl), norbornenyl, decalinyl, 7,7-dimethyl-bicyclo[2.2.1]heptanyl, and the like.

"Cycloalkenyl" refers to a stable unsaturated non-aromatic monocyclic or polycyclic hydrocarbon radical consisting solely of carbon and hydrogen atoms, which includes fused or bridged ring systems, preferably having from three to twelve carbon atoms and comprising at least one double bond. In certain embodiments, a cycloalkenyl comprises three to ten carbon atoms. In other embodiments, a cycloalkenyl comprises five to seven carbon atoms. The cycloalkenyl may be attached to the rest of the molecule by a single bond. Examples of monocyclic cycloalkenyls include, *e.g*., cyclopentenyl, cyclohexenyl, cycloheptenyl, and cyclooctenyl.

"Cycloalkylalkyl" refers to a radical of the formula -R-cycloalkyl where R is an alkylene chain as described above.

"Cycloalkylalkoxy" refers to a radical bonded through an oxygen atom of the formula -OR^{c}-cycloalkyl where R^{c} is an alkylene chain as described above.

"Halo" or "halogen" refers to halogen substituents such as bromo, chloro, fluoro and iodo substituents.

As used herein, the term "haloalkyl" or "haloalkane" refers to an alkyl radical, as defined above, that is substituted by one or more halogen radicals, for example, trifluoromethyl, dichloromethyl, bromomethyl, 2,2,2-trifluoroethyl, 1-fluoromethyl-2-fluoroethyl, and the like. In some embodiments, the alkyl part of the fluoroalkyl radical is optionally further substituted. Examples of halogen substituted alkanes ("haloalkanes") include halomethane (e.g., chloromethane, bromomethane, fluoromethane, iodomethane), di-and trihalomethane (e.g., trichloromethane, tribromomethane, trifluoromethane, triiodomethane), 1-haloethane, 2-haloethane, 1,2-dihaloethane, 1-halopropane, 2-halopropane, 3-halopropane, 1,2-dihalopropane, 1,3-dihalopropane, 2,3-dihalopropane, 1,2,3-trihalopropane, and any other suitable combinations of alkanes (or substituted alkanes) and halogens (e.g., Cl, Br, F, I, etc.). When an alkyl group is substituted with more than one halogen radicals, each halogen may be independently selected e.g., 1-chloro,2-fluoroethane.

"Fluoroalkyl" refers to an alkyl radical, as defined above, that is substituted by one or more fluoro radicals, for example, trifluoromethyl, difluoromethyl, fluoromethyl, 2,2,2-trifluoroethyl, 1-fluoromethyl-2-fluoroethyl, and the like.

"Heterocycle" refers to a saturated, unsaturated or aromatic ring comprising one or more heteroatoms. Exemplary heteroatoms include N, O, Si, P, B, and S atoms. Heterocycles include 3- to 10-membered monocyclic rings, 6- to 12-membered bicyclic rings, and 6- to 12-membered bridged rings. Each ring of a bicyclic heterocycle may be selected from saturated, unsaturated, and aromatic rings. In some embodiments, the heterocycle is a heteroaryl. In some embodiments, the heterocycle is a heterocycloalkyl. "Heterocyclene" refers to a divalent heterocycle linking the rest of the molecule to a radical group.

"Heterocycloalkyl" refers to a stable 3- to 12-membered non-aromatic ring radical that comprises two to twelve carbon atoms and at least one heteroatom wherein each heteroatom may be selected from N, O, Si, P, B, and S atoms. The heterocycloalkyl may be selected from monocyclic or bicyclic, and fused or bridged ring systems. The heteroatoms in the heterocycloalkyl radical are optionally oxidized. One or more nitrogen atoms, if present, are optionally quaternized. The heterocycloalkyl radical is partially or fully saturated. The heterocycloalkyl is attached to the rest of the molecule through any atom of the heterocycloalkyl, valence permitting, such as any carbon or nitrogen atoms of the heterocycloalkyl. Examples of heterocycloalkyl radicals include, but are not limited to, dioxolanyl, thienyl[1,3]dithianyl, decahydroisoquinolyl, imidazolinyl, imidazolidinyl, isothiazolidinyl, isoxazolidinyl, morpholinyl, octahydroindolyl, octahydroisoindolyl, 2-oxopiperazinyl, 2-oxopiperidinyl, 2-oxopyrrolidinyl, oxazolidinyl, piperidinyl, piperazinyl, 4-piperidonyl, pyrrolidinyl, pyrazolidinyl, quinuclidinyl, thiazolidinyl, tetrahydrofuryl, trithianyl, tetrahydropyranyl, thiomorpholinyl, thiamorpholinyl, 1-oxo-thiomorpholinyl, and 1,1-dioxo-thiomorpholinyl.

"Heterocycloalkylalkyl" refers to a radical of the formula -R-heterocycloalkyl, where R is an alkylene chain as defined above. If the heterocycloalkyl is a nitrogen-containing heterocycloalkyl, the heterocycloalkyl is optionally attached to the alkylene chain at the nitrogen atom.

"Heteroaryl" or "aromatic heterocycle" refers to a radical derived from a 3- to 12-membered aromatic ring radical that comprises one to eleven carbon atoms and at least one heteroatom wherein each heteroatom may be selected from N, O, and S. As used herein, the heteroaryl ring may be selected from monocyclic or bicyclic and fused or bridged ring systems rings wherein at least one of the rings in the ring system is aromatic, *i.e.,* it contains a cyclic, delocalized (4n+2) π-electron system in accordance with the Hückel theory. The heteroatom(s) in the heteroaryl radical may be optionally oxidized. One or more nitrogen atoms, if present, are optionally quaternized. The heteroaryl may be attached to the rest of the molecule through any atom of the heteroaryl, valence permitting, such as a carbon or nitrogen atom of the heteroaryl. Examples of heteroaryls include, but are not limited to, azepinyl, acridinyl, benzimidazolyl, benzindolyl, 1,3-benzodioxolyl, benzofuranyl, benzooxazolyl, benzo[d]thiazolyl, benzothiadiazolyl, benzo[b][1,4]dioxepinyl, benzo[b][1,4]oxazinyl, 1,4-benzodioxanyl, benzonaphthofuranyl, benzoxazolyl, benzodioxolyl, benzodioxinyl, benzopyranyl, benzopyranonyl, benzofuranyl, benzofuranonyl, benzothienyl (benzothiophenyl), benzothieno[3,2-d]pyrimidinyl, benzotriazolyl, benzo[4,6]imidazo[1,2-a]pyridinyl, carbazolyl, cinnolinyl, cyclopenta[d]pyrimidinyl, 6,7-dihydro-5H-cyclopenta[4,5]thieno[2,3-d]pyrimidinyl, 5,6-dihydrobenzo[h]quinazolinyl, 5,6-dihydrobenzo[h]cinnolinyl, 6,7-dihydro-5H-benzo[6,7]cyclohepta[1,2-c]pyridazinyl, dibenzofuranyl, dibenzothiophenyl, furanyl, furanonyl, furo[3,2-c]pyridinyl, 5,6,7,8,9,10-hexahydrocycloocta[d]pyrimidinyl, 5,6,7,8,9,10-hexahydrocycloocta[d]pyridazinyl, 5,6,7,8,9,10-hexahydrocycloocta[d]pyridinyl, isothiazolyl, imidazolyl, indazolyl, indolyl, indazolyl, isoindolyl, indolinyl, isoindolinyl, isoquinolyl, indolizinyl, isoxazolyl, 5,8-methano-5,6,7,8-tetrahydroquinazolinyl, naphthyridinyl, 1,6-naphthyridinonyl, oxadiazolyl, 2-oxoazepinyl, oxazolyl, oxiranyl, 5,6,6a,7,8,9,10,10a-octahydrobenzo[h]quinazolinyl, 1-phenyl-1H-pyrrolyl, phenazinyl, phenothiazinyl, phenoxazinyl, phthalazinyl, pteridinyl, purinyl, pyrrolyl, pyrazolyl, pyrazolo[3,4-d]pyrimidinyl, pyridinyl, pyrido[3,2-d]pyrimidinyl, pyrido[3,4-d]pyrimidinyl, pyrazinyl, pyrimidinyl, pyridazinyl, pyrrolyl, quinazolinyl, quinoxalinyl, quinolinyl, isoquinolinyl, tetrahydroquinolinyl, 5,6,7,8-tetrahydroquinazolinyl, 5,6,7,8-tetrahydrobenzo[4,5]thieno[2,3-d]pyrimidinyl, 6,7,8,9-tetrahydro-SH-cyclohepta [4,5]thieno [2,3-d]pyrimidinyl, 5,6,7,8-tetrahydropyrido[4,5-c]pyridazinyl, thiazolyl, thiadiazolyl, triazolyl, tetrazolyl, triazinyl, thieno[2,3-d]pyrimidinyl, thieno[3,2-d]pyrimidinyl, thieno[2,3-c]pridinyl, and thiophenyl (*i.e.* thienyl). An "X-membered heteroaryl" refers to the number of endocylic atoms, i.e., X, in the ring. For example, a 5-membered heteroaryl ring or 5-membered aromatic heterocycle has 5 endocyclic atoms, e.g., triazole, oxazole, thiophene, etc.

"Heteroarylalkyl" refers to a radical of the formula -R-heteroaryl, where Ris an alkylene chain as defined above. If the heteroaryl is a nitrogen-containing heteroaryl, the heteroaryl is optionally attached to the alkylene chain at the nitrogen atom.

"Isomers" are different compounds that have the same molecular formula. "Stereoisomers" are isomers that differ only in the way the atoms are arranged in space. "Enantiomers" are a pair of stereoisomers that are non-superimposable mirror images of each other. A 1:1 mixture of a pair of enantiomers is a "racemic" mixture. The term "(±)" is used to designate a racemic mixture where appropriate. "Diastereoisomers" or "diastereomers" are stereoisomers that have at least two asymmetric atoms but are not mirror images of each other. The absolute stereochemistry is specified according to the Cahn-Ingold-Prelog R-S system. When a compound is a pure enantiomer, the stereochemistry at each chiral carbon can be specified by either R or S. Resolved compounds whose absolute configuration is unknown can be designated (+) or (-) depending on the direction (dextro- or levorotatory) in which they rotate plane polarized light at the wavelength of the sodium D line. Certain compounds described herein contain one or more asymmetric centers and can thus give rise to enantiomers, diastereomers, and other stereoisomeric forms, the asymmetric centers of which can be defined, in terms of absolute stereochemistry, as (R)- or (S)-. The present chemical entities, pharmaceutical compositions and methods are meant to include all such possible stereoisomers, including racemic mixtures, optically pure forms, mixtures of diastereomers and intermediate mixtures. Optically active (R)- and (S)-isomers can be prepared using chiral synthons or chiral reagents or resolved using conventional techniques. The optical activity of a compound can be analyzed via any suitable method, including but not limited to chiral chromatography and polarimetry, and the degree of predominance of one stereoisomer over the other isomer can be determined.

In certain embodiments, the compounds of the disclosure may contain asymmetric or chiral centers, and, therefore, exist in different stereoisomeric forms. The term "stereoisomers" may refer to the set of compounds which have the same number and type of atoms and share the same bond connectivity between those atoms but differ in three-dimensional structure. The term "stereoisomer" may refer to any member of this set of compounds. For instance, a stereoisomer may be an enantiomer or a diastereomer. It is intended that all stereoisomeric forms of the compounds of the disclosure as well as mixtures thereof, including racemic mixtures, form part of the present disclosure.

When stereochemistry is not specified, certain molecules described herein include isomers, such as enantiomers and diastereomers, mixtures of enantiomers, including racemates, mixtures of diastereomers, and other mixtures thereof, to the extent they can be made by one of ordinary skill in the art by routine experimentation. In certain embodiments, the single enantiomers or diastereomers, i.e., optically active forms, can be obtained by asymmetric synthesis or by resolution of the racemates or mixtures of diastereomers. Resolution of the racemates or mixtures of diastereomers, if possible, can be accomplished, for example, by conventional methods such as crystallization in the presence of a resolving agent, or chromatography, using, for example, a chiral high-pressure liquid chromatography (HPLC) column. Furthermore, a mixture of two enantiomers enriched in one of the two can be purified to provide further optically enriched form of the major enantiomer by recrystallization and/or trituration.

In certain embodiments, the chiral centers of the present disclosure may have the S or R configuration as defined by the IUPAC 1974 Recommendations.

The term "amino" as used herein refers to -NH₂.

The term "hydroxy" or "hydroxyl" as used herein refers to -OH.

The term "oxo" as used herein refers to an "=O" group. It can also be abbreviated herein as C(O) or as C=O.

The term "substituted" refers to moieties having substituents replacing a hydrogen on one or more carbons or substitutable heteroatoms, e.g., NH, of the structure. It will be understood that "substitution" or "substituted with" includes the implicit proviso that such substitution is in accordance with permitted valence of the substituted atom and the substituent, and that the substitution results in a stable compound, *i.e.,* a compound which does not spontaneously undergo transformation such as by rearrangement, cyclization, elimination, etc. In certain embodiments, substituted refers to moieties having substituents replacing two hydrogen atoms on the same carbon atom, such as substituting the two hydrogen atoms on a single carbon with an oxo, imino or thioxo group. As used herein, the term "substituted" is contemplated to include all permissible substituents of organic compounds. In a broad aspect, the permissible substituents include acyclic and cyclic, branched and unbranched, carbocyclic and heterocyclic, aromatic and non-aromatic substituents of organic compounds. The permissible substituents can be one or more and the same or different for appropriate organic compounds. For purposes of this disclosure, the heteroatoms such as nitrogen may have hydrogen substituents and/or any permissible substituents of organic compounds described herein which satisfy the valences of the heteroatoms.

In some embodiments, substituents may include any substituents described herein, for example: halogen, hydroxy, oxo (=O), thioxo (=S), cyano (-CN), nitro (-NO₂), imino (=N-H), oximo (=N-OH), hydrazino(=N-
NH₂), -R^{b}-OR^{a}, -R^{b}-OC(O)-R^{a}, -R^{b}-OC(O)-OR^{a}, -R^{b}-OC(O)-N(R^{a})₂, -R^{b}-N(R^{a})₂, -R^{b}-C(O)R^{a}, -R-C(O)O R^{a}, -R^{b}-C(O)N(R^{a})₂, -R^{b}-O-R^{c}-C(O)N(R^{a})₂, -R^{b}-N(R^{a})C(O)OR^{a}, -R^{b}-N(R^{a})C(O)R^{a}, -R^{b}-N(R^{a})S(O)ₜR^{a} (where t is 1 or 2), -R^{b}-S(O)ₜR^{a} (where t is 1 or 2), -R^{b}-S(O)ₜOR^{a} (where t is 1 or 2), and -R^{b}-S(O)ₜN(R^{a})₂ (where t is 1 or 2); and alkyl, alkenyl, alkynyl, aryl, aralkyl, aralkenyl, aralkynyl, cycloalkyl, cycloalkylalkyl, heterocycloalkyl, heterocycloalkylalkyl, heteroaryl, and heteroarylalkyl any of which may be optionally substituted by alkyl, alkenyl, alkynyl, halogen, haloalkyl, haloalkenyl, haloalkynyl, oxo (=O), thioxo (=S), cyano (-CN), nitro (-NO₂), imino (=N-H), oximo (=N-OH), hydrazine (=N-NH₂), -R^{b}-OR^{a}, -R^{b}-OC(O)-R^{a}, -R^{b}-OC(O)-OR^{a}, -R^{b}-OC(O)-N(R^{a})₂, -R^{b}-N(R^{a})₂, -R^{b}-C(O)R^{a}, -R-C(O)O R^{a}, -R^{b}-C(O)N(R^{a})₂, -R^{b}-O-R^{c}-C(O)N(R^{a})₂, -R^{b}-N(R^{a})C(O)OR^{a}, -R^{b}-N(R^{a})C(O)R^{a}, -R^{b}-N(R^{a})S(O)ₜR^{a} (where t is 1 or 2), -R^{b}-S(O)ₜR^{a} (where t is 1 or 2), -R^{b}-S(O)ₜOR^{a} (where t is 1 or 2) and -R^{b}-S(O)ₜN(R^{a})₂ (where t is 1 or 2); wherein each R^{a} is independently selected from hydrogen, alkyl, cycloalkyl, cycloalkylalkyl, aryl, aralkyl, heterocycloalkyl, heterocycloalkylalkyl, heteroaryl, or heteroarylalkyl, wherein each R^{a}, valence permitting, may be optionally substituted with alkyl, alkenyl, alkynyl, halogen, haloalkyl, haloalkenyl, haloalkynyl, oxo (=O), thioxo (=S), cyano (-CN), nitro (-NO₂), imino (=N-H), oximo (=N-OH), hydrazine (=N-NH₂), -R^{b}-OR^{a}, -R^{b}-OC(O)-R^{a}, -R^{b}-OC(O)-OR^{a}, -R^{b}-OC(O)-N(R^{a})₂, -R^{b}-N(R^{a})₂, -R^{b}-C(O)R^{a}, -R-C(O)O R^{a}, -R^{b}-C(O)N(R^{a})₂, -R^{b}-O-R^{c}-C(O)N(R^{a})₂, -R^{b}-N(R^{a})C(O)OR^{a}, -R^{b}-N(R^{a})C(O)R^{a}, -R^{b}-N(R^{a})S(O)ₜR^{a} (where t is 1 or 2), -R^{b}-S(O)ₜR^{a} (where t is 1 or 2), -R^{b}-S(O)ₜOR^{a} (where t is 1 or 2) and -R^{b}-S(O)ₜN(R^{a})₂ (where t is 1 or 2); and wherein each R^{b} is independently selected from a direct bond or a straight or branched alkylene, alkenylene, or alkynylene chain, and each R^{c} is a straight or branched alkylene, alkenylene or alkynylene chain.

The term "radical of a compound" as used herein refers to a structure derived from a parent compound by removal of one or more atoms, e.g., hydrogen atoms. In one embodiment, a "radical of a compound" is a monovalent radical derived from the removal of one hydrogen atom from the parent compound.

It is to be understood that certain radical naming conventions can include either a mono-radical or a di-radical, depending on the context. For example, where a substituent requires two points of attachment to the rest of the molecule, it is understood that the substituent is a di-radical. For example, a substituent identified as alkyl that requires two points of attachment includes di-radicals such as -CH₂-, -CH₂CH₂-, - CH₂CH(CH₃)CH₂-, and the like. Other radical naming conventions clearly indicate that the radical is a di-radical such as "alkylene," "alkenylene," "arylene," and the like.

Wherever a substituent is depicted as a di-radical (i.e., has two points of attachment to the rest of the molecule), it is to be understood that the substituent can be attached in any directional configuration unless otherwise indicated.

A "tautomer" refers to a molecule wherein a proton shift from one atom of a molecule to another atom of the same molecule is possible. The compounds presented herein, in certain embodiments, exist as tautomers. In circumstances where tautomerization is possible, a chemical equilibrium of the tautomers will exist. The exact ratio of the tautomers depends on several factors, including physical state, temperature, solvent, and pH. Some examples of tautomeric equilibrium include:

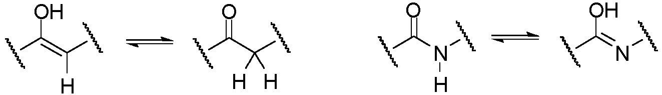

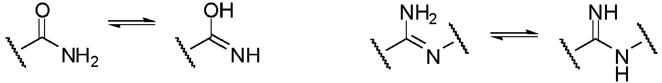

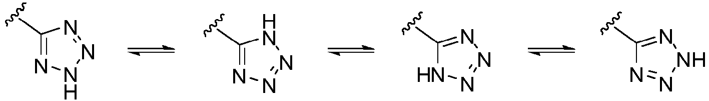

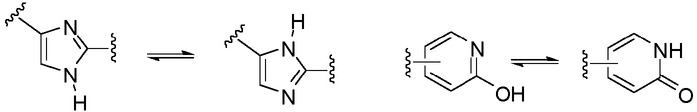

As used herein, the term "unsubstituted" may mean that the specified group bears no substituents beyond the moiety recited (e.g., where valency is satisfied by hydrogen).

"Stable compound" and "stable structure" may indicate a compound that is sufficiently robust to survive isolation to a useful degree of purity from a reaction mixture, and formulation into an efficacious therapeutic agent.

The phrases "parenteral administration" and "administered parenterally" as used herein means modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal and intrasternal injection and infusion.

The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

The phrase "pharmaceutically acceptable excipient" or "pharmaceutically acceptable carrier" as used herein means a pharmaceutically acceptable material, composition or vehicle, such as a liquid or solid filler, diluent, excipient, solvent or encapsulating material. Each carrier must be "acceptable" in the sense of being compatible with the other ingredients of the formulation and not injurious to the patient. Some examples of materials which can serve as pharmaceutically acceptable carriers include: (1) sugars, such as lactose, glucose and sucrose; (2) starches, such as corn starch and potato starch; (3) cellulose, and its derivatives, such as sodium carboxymethyl cellulose, ethyl cellulose and cellulose acetate; (4) powdered tragacanth; (5) malt; (6) gelatin; (7) talc; (8) excipients, such as cocoa butter and suppository waxes; (9) oils, such as peanut oil, cottonseed oil, safflower oil, sesame oil, olive oil, corn oil and soybean oil; (10) glycols, such as propylene glycol; (11) polyols, such as glycerin, sorbitol, mannitol and polyethylene glycol; (12) esters, such as ethyl oleate and ethyl laurate; (13) agar; (14) buffering agents, such as magnesium hydroxide and aluminum hydroxide; (15) alginic acid; (16) pyrogen-free water; (17) isotonic saline; (18) Ringer's solution; (19) ethyl alcohol; (20) phosphate buffer solutions; and (21) other non-toxic compatible substances employed in pharmaceutical formulations.

The term "carrier," as used in this disclosure, may encompass carriers, excipients, and diluents and may mean a material, composition or vehicle, such as a liquid or solid filler, diluent, excipient, solvent or encapsulating material, involved in carrying or transporting a pharmaceutical agent, such as one or more compounds, or pharmaceutically acceptable salts, solvates (e.g., hydrates), isomers (e.g., stereoisomers), and tautomers thereof, of the disclosure, from one organ, or portion of the body, to another organ, or portion of the body of a subject. Carriers should be selected on the basis of compatibility and the release profile properties of the desired dosage form. Exemplary carrier materials may include, e.g., adjuvants, binders, suspending agents, disintegration agents, filling agents, surfactants, solubilizers, stabilizers, lubricants, wetting agents, diluents, spray-dried dispersions, and the like. See, e.g., Hoover, John E., Remington's Pharmaceutical Sciences, Mack Publishing Co., Easton, Pa. 1975. Exemplary carrier materials may also include without limitation any adjuvant, excipient, glidant, sweetening agent, diluent, preservative, dye/colorant, flavor enhancer, surfactant, wetting agent, dispersing agent, suspending agent, stabilizer, isotonic agent, solvent, or emulsifier which has been approved by the United States Food and Drug Administration as being acceptable for use in humans or domestic animals.

The terms "pharmaceutically acceptable" or "pharmacologically acceptable" may refer to a material which is not biologically, or otherwise, undesirable-the material may be administered to an individual without causing any substantially undesirable biological effects or interacting in a deleterious manner with any of the components of the composition in which it is contained.

A "pharmaceutical composition" may refer to a formulation of a compound of the disclosure and a medium generally accepted in the art for the delivery of the biologically active compound to a subject, e.g., mammals or humans. Such a medium may include all pharmaceutically acceptable carriers therefor.

The terms "subject," "individual," and "patient" may be used interchangeably and refer to humans as well as non-human mammals (e.g., non-human primates, canines, equines, felines, porcines, bovines, ungulates, lagomorphs, and the like). In various embodiments, the subject can be a human (e.g., adult male, adult female, adolescent male, adolescent female, male child, female child) under the care of a physician or other health worker in a hospital, as an outpatient, or other clinical context. In certain embodiments, the subject may not be under the care or prescription of a physician or other health worker.

As used herein, the phrase "a subject in need thereof" refers to a subject, as described infra, that suffers from, or is at risk for, a pathology to be prophylactically or therapeutically treated with a compound or salt described herein.

As used herein, "agent" or "biologically active agent" refers to a biological, pharmaceutical, or chemical compound, or other moiety. Non-limiting examples include an organic or inorganic molecule, a peptide, a protein, a peptide nucleic acid (PNA), an oligonucleotide (including e.g., aptamer and polynucleotides), an antibody, an antibody derivative, antibody fragment, a vitamin derivative, a carbohydrate, a toxin, a branched chain amino acid in free amino acid form or metabolite thereof, or a chemotherapeutic compound. Various compounds can be synthesized, for example, small molecules and oligomers (e.g., oligopeptides and oligonucleotides), and synthetic organic compounds based on various core structures. In addition, various natural sources can provide compounds for screening, such as plant or animal extracts, and the like. A skilled artisan can readily recognize that there is no limit as to the structural nature of the agents.

The terms "administer", "administered", "administers" and "administering" are defined as providing a composition to a subject via a route known in the art, including but not limited to intravenous, intraarterial, oral, parenteral, buccal, topical, transdermal, rectal, intramuscular, subcutaneous, intraosseous, transmucosal, or intraperitoneal routes of administration. In certain embodiments, oral routes of administering a composition can be used. The term "effective amount" or "therapeutically effective amount" refers to that amount of a compound or salt described herein that is sufficient to effect the intended application including but not limited to disease treatment, as defined below. The therapeutically effective amount may vary depending upon the intended application (in vitro or in vivo), or the subject and disease condition being treated, e.g., the weight and age of the subject, the severity of the disease condition, the manner of administration and the like, which can readily be determined by one of ordinary skill in the art. The term can also apply to a dose that can induce a particular response in target cells, e.g., reduction of proliferation or down regulation of activity of a target protein. The specific dose can vary depending on the particular compounds chosen, the dosing regimen to be followed, whether it is administered in combination with other compounds, timing of administration, the tissue to which it is administered, and the physical delivery system in which it is carried.

As used herein, "treatment" or "treating" refers to an approach for obtaining beneficial or desired results with respect to a disease, disorder, or medical condition including, but not limited to, a therapeutic benefit and/or a prophylactic benefit. In certain embodiments, treatment or treating involves administering a compound or composition disclosed herein to a subject. A therapeutic benefit may include the eradication or amelioration of the underlying disorder being treated. Also, a therapeutic benefit may be achieved with the eradication or amelioration of one or more of the physiological symptoms associated with the underlying disorder, such as observing an improvement in the subject, notwithstanding that the subject may still be afflicted with the underlying disorder. In certain embodiments, for prophylactic benefit, the compositions are administered to a subject at risk of developing a particular disease, or to a subject reporting one or more of the physiological symptoms of a disease, even though a diagnosis of this disease may not have been made. Treating can include, for example, reducing, delaying or alleviating the severity of one or more symptoms of the disease or condition, or it can include reducing the frequency with which symptoms of a disease, defect, disorder, or adverse condition, and the like, are experienced by a patient. Treating can be used herein to refer to a method that results in some level of treatment or amelioration of the disease or condition and can contemplate a range of results directed to that end, including but not restricted to prevention of the condition entirely.

In certain embodiments, the term "prevent" or "preventing" as related to a disease or disorder may refer to a compound that, in a statistical sample, reduces the occurrence of the disorder or condition in the treated sample relative to an untreated control sample, or delays the onset or reduces the severity of one or more symptoms of the disorder or condition relative to the untreated control sample.

The term "prodrug" is intended to encompass compounds which, under physiologic conditions, are converted into the therapeutically pharmaceutical agents of the present disclosure. A common method for making a prodrug is to include one or more selected moieties which are hydrolyzed under physiologic conditions to reveal the desired molecule. In other embodiments, the prodrug is converted by an enzymatic activity of the host animal. For example, esters or carbonates (e.g., esters or carbonates of alcohols or carboxylic acids and esters of phosphonates) are preferred prodrugs of the present disclosure.

The terms "treat," "treating" or "treatment," as used herein, may include alleviating, abating or ameliorating a disease or condition symptoms, preventing additional symptoms, ameliorating or preventing the underlying causes of symptoms, inhibiting the disease or condition, e.g., arresting the development of the disease or condition, relieving the disease or condition, causing regression of the disease or condition, relieving a condition caused by the disease or condition, or stopping the symptoms of the disease or condition either prophylactically and/or therapeutically.

The terms "effective amount" or "therapeutically effective amount" when used in connection with one or more compounds or pharmaceutical compositions may refer to a sufficient amount of the one or more compounds or pharmaceutical compositions to provide the desired biological result. That result can be reduction and/or alleviation of the signs, symptoms, or causes of a disorder, or any other desired alteration of a biological system. For example, an "effective amount" for therapeutic use may be the amount of the pharmaceutical composition comprising one or more compounds, or pharmaceutically acceptable salts, solvates (e.g., hydrates), isomers (e.g., stereoisomers), and tautomers thereof, as disclosed herein required to provide a clinically significant decrease in a disorder. An appropriate "effective amount" in any individual case may be determined by one of ordinary skill in the art using routine experimentation.

In certain embodiments, the terms "disease" and "condition" may be used interchangeably or may be different in that the particular malady or condition may not have a known causative agent (so that etiology has not yet been worked out) and it is therefore not yet recognized as a disease but only as an undesirable condition or syndrome, wherein a more or less specific set of symptoms have been identified by clinicians.

The terms "antibacterial agent," "antibiotic," or "antibacterial compound" are used interchangeably herein and may refer to agents or compounds that have either bactericidal or bacteriostatic activity. The term "inhibiting the growth" may indicate that the rate of increase in the numbers of a population of a particular bacterium is reduced. Thus, the term may include situations in which the bacterial population increases but at a reduced rate, as well as situations where the growth of the population is stopped, as well as situations where the numbers of the bacteria in the population are reduced or the population even eliminated. The activity of antibacterial agents or compounds is not necessarily limited to bacteria but may also encompass general antimicrobial activity against parasites or fungi or general antiviral activity against viruses.

### Compounds of the Disclosure

Generally, the present disclosure provides aminoglycoside compounds which may be characterized as having antibacterial activity.

In one aspect is provided a compound having a structure according to Formula (I): 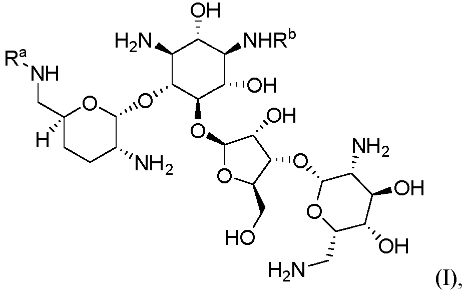
or a pharmaceutically acceptable salt or solvate thereof;
wherein:
   R^{a} is hydrogen (H) or methyl; and
   R^{b} has a structure according to Formula (II): 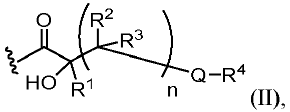 wherein:
      Q is NH, O, or S;
      n is an integer from 0 to 4;
      R¹ is hydrogen or C₁₋₃ alkyl;
      R² and R³ are each selected independently for each occurrence from the group consisting of hydrogen, alkyl, halogen, and -OH; and
   R⁴ is H, C₁₋₃ alkyl, or -C(=NH)NR^{4a}R^{4b}, wherein R^{4a} and R^{4b} are each independently selected from the group consisting of H and C₁₋₃ alkyl; or
      R¹ and R⁴, together with the atoms to which they are attached, form a heterocycloalkyl ring system comprising at least one N.

In another aspect is provided a compound having a structure according to Formula (III): 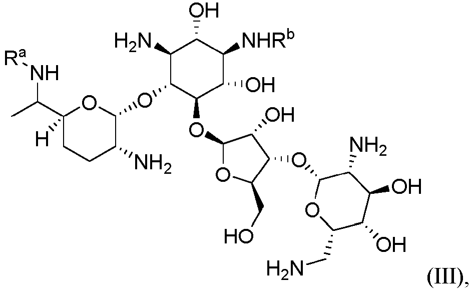
or a pharmaceutically acceptable salt or solvate thereof;
wherein:
   R^{a} is H or methyl; and
   R^{b} has a structure according to Formula (II): 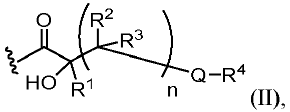 wherein:
      Q is NH, O, or S;
      n is an integer from 0 to 4;
      R¹ is hydrogen or C₁₋₃ alkyl;
      R² and R³ are each selected independently for each occurrence from the group consisting of hydrogen, alkyl, halogen, and -OH; and
      R⁴ is H, C₁₋₃ alkyl, or -C(=NH)NR^{4a}R⁴ , wherein R^{4a} and R^{4b} are each independently selected from the group consisting of hydrogen and C₁₋₃ alkyl; or
      R¹ and R⁴, together with the atoms to which they are attached, form a heterocycloalkyl ring system comprising at least one N.

One of skill in the art will recognize that compounds of the disclosure according to Formula (I) and Formula (III) each share a common hexahydropyranyl ring system: 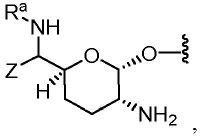 where Z is H (Formula (I)) or methyl (Formula (III)).

Without wishing to be bound by any particular theory, it is believed that the specific hexahydropyranyl ring system of the compounds according to Formula (I) and Formula (III) may impart favorable antibacterial activity to compounds comprising such a ring system. Compounds of Formula (I) and Formula (III) further comprise additional optionally substituted saturated carbocyclic or heterocyclic ring systems (e.g., three-ring systems, such that the compounds of Formula (I) and Formula (III) each comprise four connected rings including the hexahydropyranyl ring shown), together comprising an aminoglycoside core. Compounds of Formula (I) and Formula (III) may be referred to herein as "ABCD compounds" in reference to the four-ring system (aminoglycoside) comprising the compounds, where the A, B, C, and D rings are as identified below:

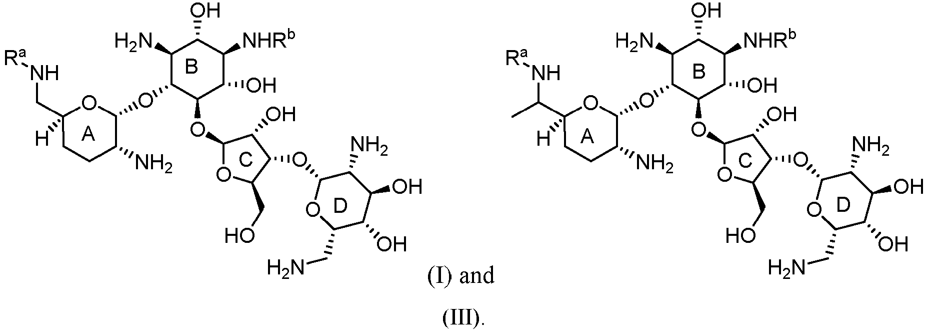

Conventional numbering is applied herein to such ring systems. For simplicity, the conventional numbering is illustrated below in reference to a simplified, non-limiting ABCD ring scaffold (ABCD', i.e., having many of the substituents removed relative to Formula (I) and Formula (III) for ease of visualization):

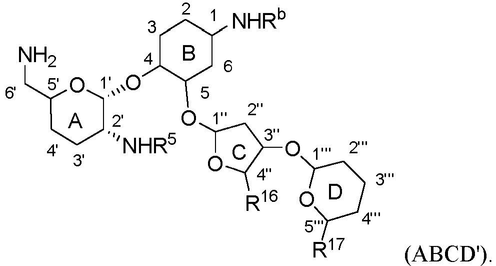

The structure of the ABCD' ring scaffold may alternatively be depicted in a three-dimensional format as shown below: 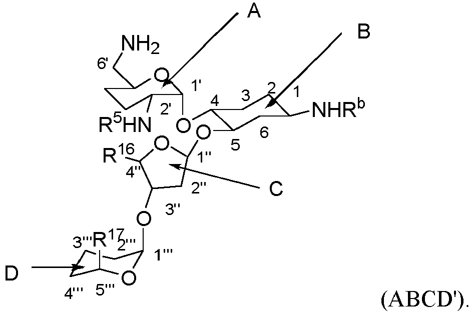 wherein:

In some embodiments is provided a compound having a structure according to Formula (I), 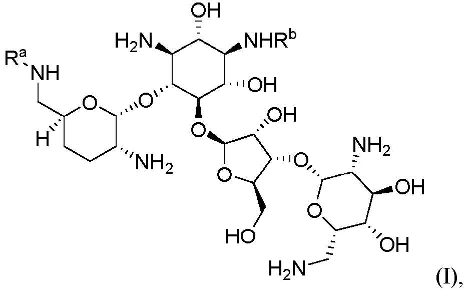 wherein:
R^{a} is hydrogen (H) or methyl; and
R^{b} has a structure according to Formula (II): 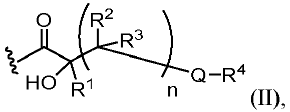 wherein:
   Q is NH, O, or S;
   n is an integer from 0 to 4;
   R¹ is hydrogen or C₁₋₃ alkyl;
   R² and R³ are each selected independently for each occurrence from the group consisting of hydrogen, alkyl, halogen, and -OH; and
   R⁴ is H, C₁₋₃ alkyl, or -C(=NH)NR^{4a}R⁴ , wherein R^{4a} and R^{4b} are each independently selected from the group consisting of H and C₁₋₃ alkyl; or
   R¹ and R⁴, together with the atoms to which they are attached, form a heterocycloalkyl ring system comprising at least one N.

In some embodiments, R^{a} is H.

In some embodiments, R^{a} is methyl (CH₃).

In some embodiments, R^{b} is C₁₋₆ alkyl, such as methyl, ethyl, propyl, butyl (including isobutyl and sec-butyl), pentyl (including all potential isomers thereof), or hexyl (including all potential isomers thereof).

R^{b} has a structure according to Formula (II) as defined herein above.

In some embodiments, with reference to Formula (II), Q is NH; n is an integer from 1 to 3; R¹ is hydrogen; R² and R³ are each selected independently for each occurrence from the group consisting of hydrogen, alkyl, halogen, and -OH; and R⁴ is H. In some embodiments, n is 2.

In some embodiments, R^{b} is 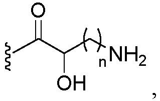 wherein n is 1, 2, or 3.

In some embodiments, R^{b} is selected from the group consisting of:

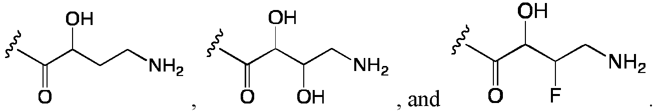

In some embodiments, R^{b} is selected from the group consisting of:

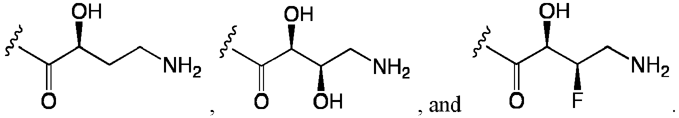

In some embodiments, the compound of Formula (I) has a structure selected from the group consisting of:

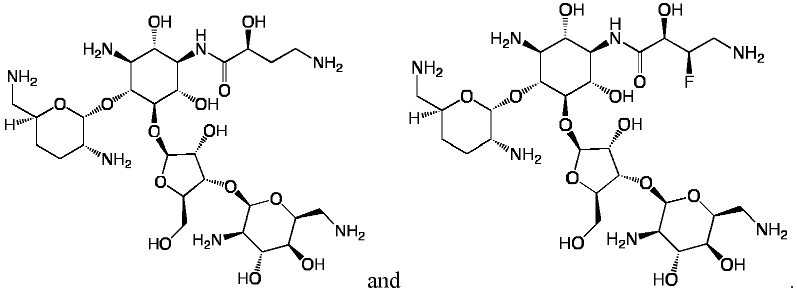

In some embodiments, the compound of Formula (I) has a structure:

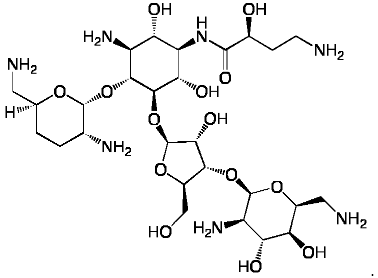

In some embodiments, the compound of Formula (I) has a structure:

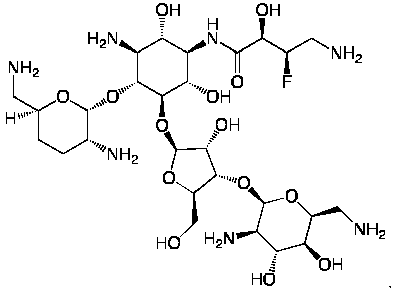

In some embodiments, the compound of Formula (I) has a structure selected from the group consisting of:

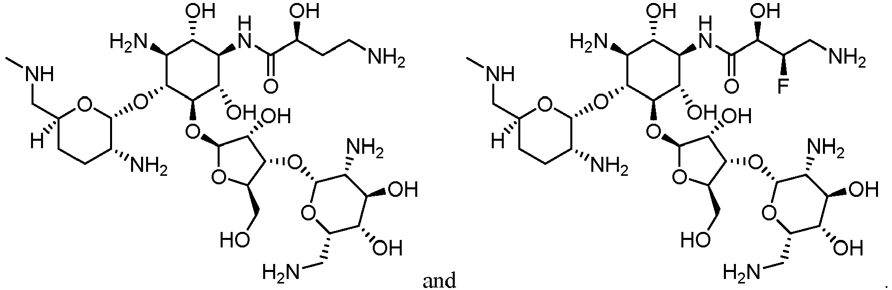

In some embodiments, the compound of Formula (I) has a structure:

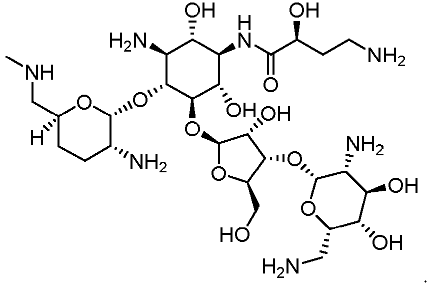

In some embodiments, the compound of Formula (I) has a structure:

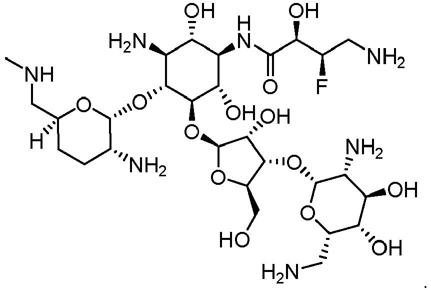

In another embodiment is provided a compound having a structure according to Formula (III): 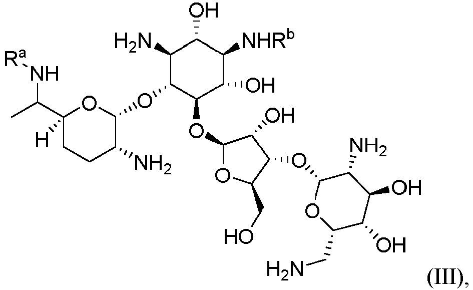 wherein:
R^{a} is H or methyl; and
R^{b} has a structure according to Formula (II): 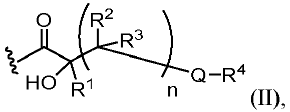 wherein:
   Q is NH, O, or S;
   n is an integer from 0 to 4;
   R¹ is hydrogen or C₁₋₃ alkyl;
   R² and R³ are each selected independently for each occurrence from the group consisting of hydrogen, alkyl, halogen, and -OH; and
   R⁴ is H, C₁₋₃ alkyl, or -C(=NH)NR^{4a}R⁴ , wherein R^{4a} and R^{4b} are each independently selected from the group consisting of hydrogen and C₁₋₃ alkyl; or
   R¹ and R⁴, together with the atoms to which they are attached, form a heterocycloalkyl ring system comprising at least one N.

In some embodiments, R^{a} is H.

In some embodiments, R^{a} is methyl.

In some embodiments, the compound of Formula (III) has a structure selected from the group consisting of:

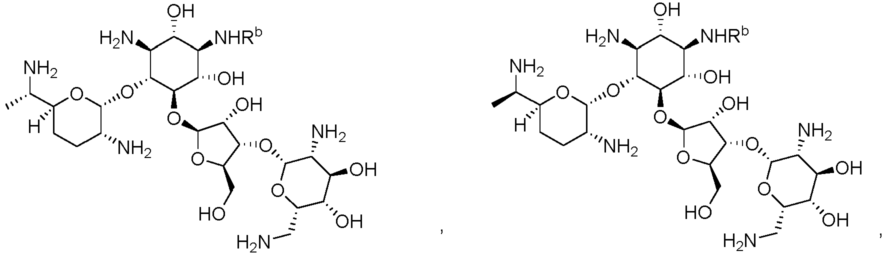

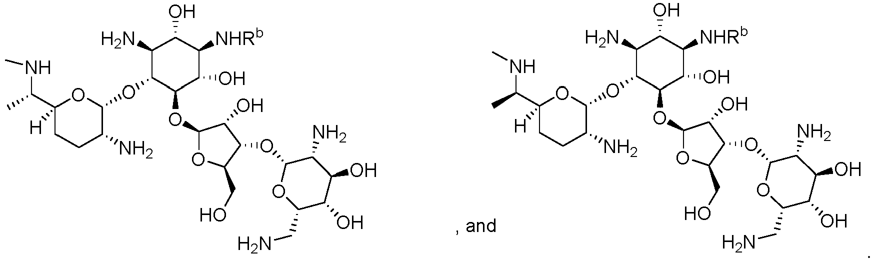

R^{b} has a structure according to Formula (II) as defined herein above.

In some embodiments, with reference to Formula (II), Q is NH; n is an integer from 1 to 3; R¹ is hydrogen; R² and R³ are each selected independently for each occurrence from the group consisting of hydrogen, alkyl, halogen, and -OH; and R⁴ is H. In some embodiments, n is 2.

In some embodiments, R^{b} is 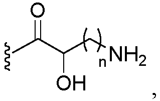 wherein n is 1, 2, or 3.

In some embodiments, R^{b} is selected from the group consisting of:

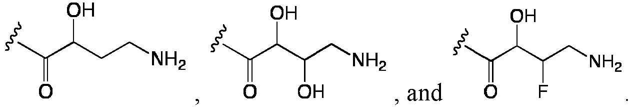

In some embodiments, R^{b} is selected from the group consisting of:

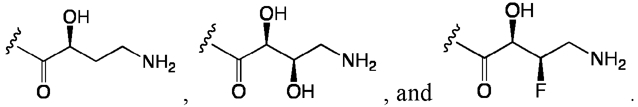

In some embodiments, the compound of Formula (III) has a structure selected from the group consisting of:

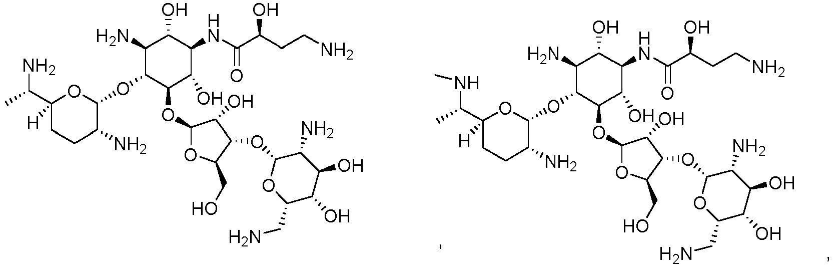

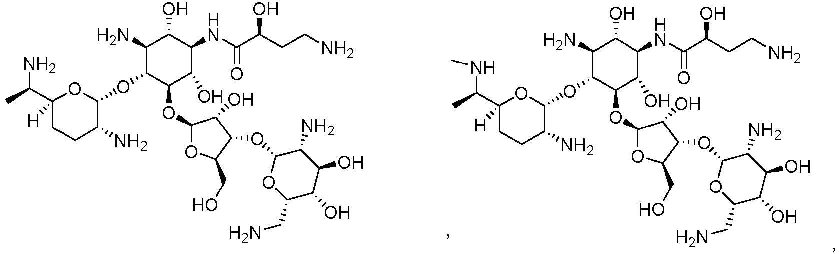

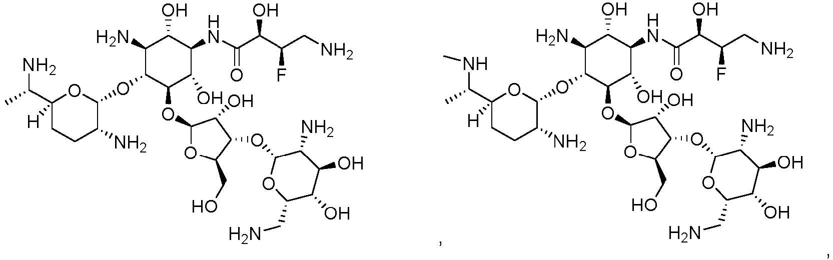

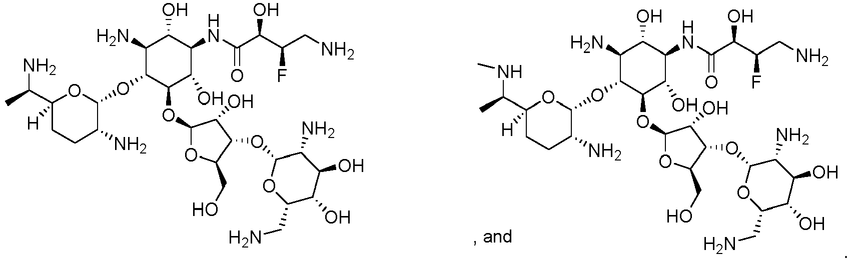

In some embodiments, the compound of Formula (III) has a structure:

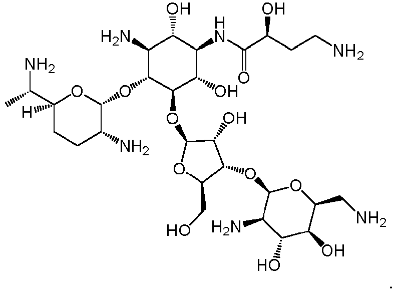

In some embodiments, the compound of Formula (III) has a structure:

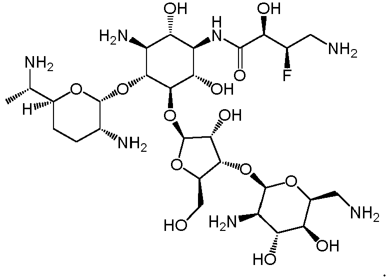

In some embodiments, the compound of Formula (III) has a structure:

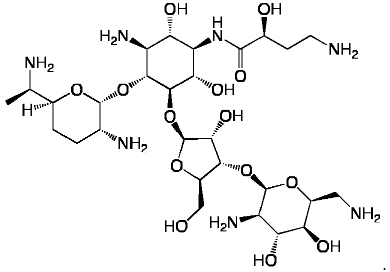

In some embodiments, the compound of Formula (III) has a structure:

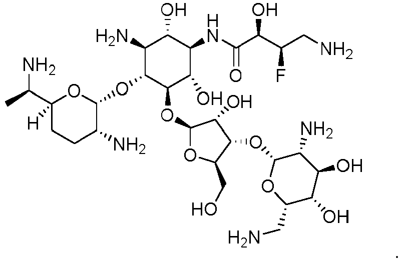

In some embodiments, the compound of Formula (III) has a structure:

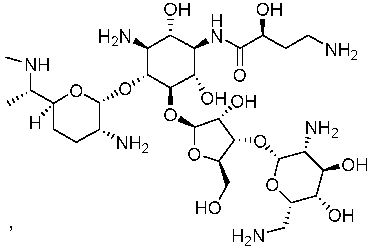

In some embodiments, the compound of Formula (III) has a structure:

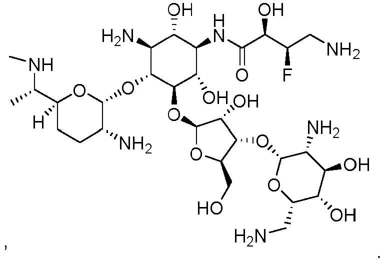

In some embodiments, the compound of Formula (III) has a structure:

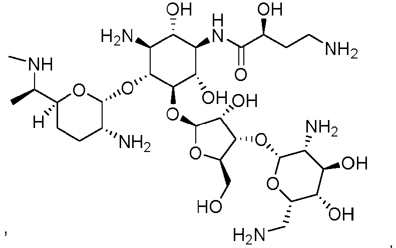

In some embodiments, the compound of Formula (III) has a structure:

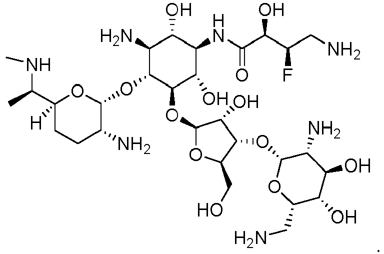

### Isotopes and Isotopically Labeled Compounds

The compounds described herein may exhibit their natural isotopic abundance, or one or more of the atoms may be artificially enriched in a particular isotope having the same atomic number, but an atomic mass or mass number different from the atomic mass or mass number predominantly found in nature. All isotopic variations of the compounds of the present disclosure, whether radioactive or not, are encompassed within the scope of the present disclosure. Accordingly, reference to a certain element is meant to include all isotopes of that element. For example, if an R group is defined to include hydrogen or H, it also includes isotopes thereof. For example, hydrogen has three naturally occurring isotopes, denoted ¹H (protium), ²H (deuterium), and ³H (tritium). Protium is the most abundant isotope of hydrogen in nature. Enriching for deuterium may afford certain therapeutic advantages, such as increased *in vivo* half-life and/or exposure, or may provide a compound useful for investigating *in vivo* routes of drug elimination and metabolism. Isotopically enriched compounds may be prepared by conventional techniques well known to those skilled in the art.

The compounds described herein further include all pharmaceutically acceptable isotopically labeled compounds. An "isotopically" or "radio-labeled" compound may be a compound where one or more atoms are replaced or substituted by an atom having an atomic mass or mass number different from the atomic mass or mass number typically found in nature (i.e., naturally occurring). For example, in some embodiments, in the compounds described herein hydrogen atoms are replaced or substituted by one or more deuterium or tritium.

Certain isotopically labeled compounds of this disclosure, for example, those incorporating a radioactive isotope, may be useful in drug and/or substrate tissue distribution studies. The radioactive isotopes tritium, i.e., ³H, and carbon 14, i.e., ¹⁴C, may be particularly useful for this purpose in view of their ease of incorporation and ready means of detection. Substitution with heavier isotopes such as deuterium, i.e., ²H, may afford certain therapeutic advantages resulting from greater metabolic stability, for example, increased *in vivo* half-life or reduced dosage requirements, and hence may be preferred in some circumstances. In some embodiments, the compound comprises at least one deuterium atom. For example, one or more hydrogen atoms in a compound of the present disclosure can be replaced or substituted by deuterium. In some embodiments, the compound comprises two or more deuterium atoms. In some embodiments, the compound comprises 1, 2, 3, 4, 5, 6, 7, 8, 9, 10, 11 or 12 deuterium atoms. Suitable isotopes that may be incorporated in compounds described herein include but are not limited to ²H (also written as D for deuterium), ³H (also written as T for tritium), ¹¹C, ¹³C, ¹⁴C, ¹³N, ¹⁵N, ¹⁵O, ¹⁷O, ¹⁸O, ¹⁸F, ³⁵S, ³⁶Cl, ⁸²Br, ⁷⁵Br, ⁷⁶Br, ⁷⁷Br, ¹²³I, ¹²⁴I, ¹²⁵I, and ¹³¹I. Substitution with positron emitting isotopes, such as ¹¹C, ¹⁸F, ¹⁵O*,* and ¹³N, can be useful in Positron Emission Topography (PET) studies.

Isotopically labelled versions of the compounds disclosed herein can generally be prepared by following procedures analogous to those disclosed in the Schemes and/or in the Examples herein, by substituting an appropriate isotopically labelled reagent for a non-isotopically labelled reagent.

Also disclosedherein are the *in vivo* metabolic products of the disclosed compounds. Such products may result from, for example, the oxidation, reduction, hydrolysis, amidation, esterification, and the like of the administered compound, primarily due to enzymatic processes. Accordingly, disclosed herein are compounds produced by a process comprising administering a compound of this disclosure to a subject, e.g., a mammal, for a period of time sufficient to yield a metabolic product thereof. Such products are typically identified by administering a radiolabeled compound of the disclosure in a detectable dose to subject, such as rat, mouse, guinea pig, monkey, or to human, allowing sufficient time for metabolism to occur, and isolating its conversion products from the urine, blood or other biological samples.

### Salts and Solvates

The present disclosure further provides pharmaceutically acceptable salts, solvates (e.g., hydrates), and combinations thereof of any of the compounds as disclosed herein. The use of the terms "salt," "hydrate," "solvate," and the like, is intended to equally apply to the salt, hydrate, or solvate of enantiomers, diastereomers, isomers, stereoisomers, rotamers, tautomers, positional isomers, or racemates of the disclosed compounds.

The term "salt" or "pharmaceutically acceptable salt" refers to salts derived from a variety of organic and inorganic counter ions well known in the art. Pharmaceutically acceptable acid addition salts can be formed with inorganic acids and organic acids. Inorganic acids from which salts can be derived include, for example, hydrochloric acid, hydrobromic acid, sulfuric acid, nitric acid, phosphoric acid, and the like. Organic acids from which salts can be derived include, for example, acetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, methanesulfonic acid, ethanesulfonic acid, *p-*toluenesulfonic acid, salicylic acid, and the like. Pharmaceutically acceptable base addition salts can be formed with inorganic and organic bases. Inorganic bases from which salts can be derived include, for example, sodium, potassium, lithium, ammonium, calcium, magnesium, iron, zinc, copper, manganese, aluminum, and the like. Organic bases from which salts can be derived include, for example, primary, secondary, and tertiary amines, substituted amines including naturally occurring substituted amines, cyclic amines, basic ion exchange resins, and the like, specifically such as isopropylamine, trimethylamine, diethylamine, triethylamine, tripropylamine, and ethanolamine. In some embodiments, the pharmaceutically acceptable base addition salt is chosen from ammonium, potassium, sodium, calcium, and magnesium salts. In some embodiments, the "pharmaceutically acceptable salts" may include, e.g., water-soluble and water-insoluble salts, such as the acetate, amsonate (4,4-diaminostilbene-2,2-disulfonate), benzenesulfonate, benzonate, bicarbonate, bisulfate, bitartrate, borate, bromide, butyrate, calcium, calcium edetate, camsylate, carbonate, chloride, citrate, clavulariate, dihydrochloride, edetate, edisylate, estolate, esylate, fiunarate, gluceptate, gluconate, glutamate, glycollylarsanilate, hexafluorophosphate, hexylresorcinate, hydrabamine, hydrobromide, hydrochloride, hydroxynaphthoate, iodide, sethionate, lactate, lactobionate, laurate, magnesium, malate, maleate, mandelate, mesylate, methylbromide, methylnitrate, methylsulfate, mucate, napsylate, nitrate, N-methylglucamine ammonium salt, 3-hydroxy-2-naphthoate, oleate, oxalate, palmitate, pamoate, 1,1-methene-bis-2-hydroxy-3-naphthoate, einbonate, pantothenate, phosphate/diphosphate, picrate, polygalacturonate, propionate, p-toluenesulfonate, salicylate, stearate, subacetate, succinate, sulfate, sulfosalicylate, suramate, tannate, tartrate, teoclate, tosylate, triethiodide, and valerate salts.

Compounds of the present invention also include crystalline and amorphous forms of those compounds, pharmaceutically acceptable salts, and active metabolites of these compounds having the same type of activity, including, for example, polymorphs, pseudopolymorphs, solvates, hydrates, unsolvated polymorphs (including anhydrates), conformational polymorphs, and amorphous forms of the compounds, as well as mixtures thereof.

Compounds of the disclosure, including their stereoisomers and tautomers, as well as salts of any thereof, may exist as solvates. Often crystallizations produce a solvate of the compound of the disclosure. As used herein, the term "solvate" may refer to an aggregate that comprises one or more molecules of a compound of the disclosure with one or more molecules of solvent. The solvent may be water, in which case the solvate may be a hydrate. Alternatively, the solvent may be an organic solvent. Thus, the compounds and salts of the present disclosure may exist as a hydrate, including a monohydrate, dihydrate, hemihydrate, sesquihydrate, trihydrate, tetrahydrate and the like, as well as the corresponding solvated forms. The compound of the disclosure may be true solvates, while in other cases the compound of the disclosure may merely retain adventitious water or be a mixture of water plus some adventitious solvent.

### Preparation of Compounds

Compounds of the present disclosure having an ABCD ring system as described herein above (e.g., compounds of Formula (I) and Formula (III)) may be prepared by methods known in the art of organic synthesis as set forth in part by the synthetic schemes in the following description, examples, and Figures in conjunction with the guidance provided herein. In the schemes described below and provided in the Figures, it is understood that protecting groups for sensitive or reactive groups may be employed where necessary in accordance with general principles or chemistry in accordance with the guidance provided herein. Protecting groups may be manipulated according to standard methods of organic synthesis (T. W. Greene and P. G. M. Wuts, "Protective Groups in Organic Synthesis," Third edition, Wiley, New York 1999). These groups may be removed at a convenient stage of the compound synthesis using methods that are readily apparent to those skilled in the art based on the detailed teaching provided herein. The selection processes, as well as the reaction conditions and order of their execution, shall be consistent with the present disclosure.

Generally, the methods of preparing compounds of the present disclosure having an ABCD ring system comprise combinations of reactions and conditions that can provide certain novel intermediate compounds. General Scheme 1 illustrates a representative, non-limiting strategy for preparation of compounds having an ABCD ring system (e.g., compounds of Formula (I) and Formula (III)).

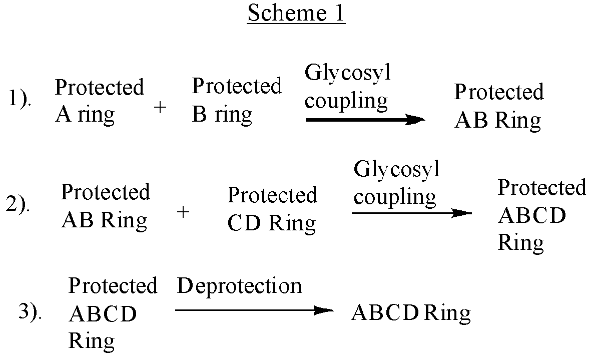

Each aspect of the preparative methods according to Scheme I is discussed further herein below. The reactions in the Scheme 1 can be performed through chemical reactions using standard synthetic chemistry procedures and practices optionally using reagents such as catalysts including, but not limited to, Pd reagents, chiral ligands, and enzymes.

### Preparation of A-Ring Compounds

An exemplary preparation of compounds of the disclosure having the ABCD ring system (e.g., compounds of Formula (I) and Formula (III)) according to Scheme 1 utilizes an A-Ring compound. In some embodiments, the A-Ring compound has the general structure (A): 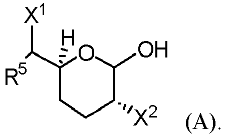 wherein:
X¹ is an amine, an amine precursor, or a protected amino group;
X² is an amine, an amine precursor, or a protected amino group; and
R⁵ is hydrogen (H) or methyl (CH₃).

In some embodiments, R⁵ is H.

In some embodiments, R⁵ is CH₃.

In some embodiments, X¹ is azide (N₃). In some embodiments, X¹ is -N(Bn)(Cbz). In some embodiments, X¹ is -N(CH₃)(Cbz).

In some embodiments, X² is azide (N₃).

In some embodiments, the A-Ring compound has the structure of Formula A1 (i.e., **Ring A1**):

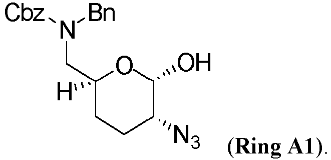

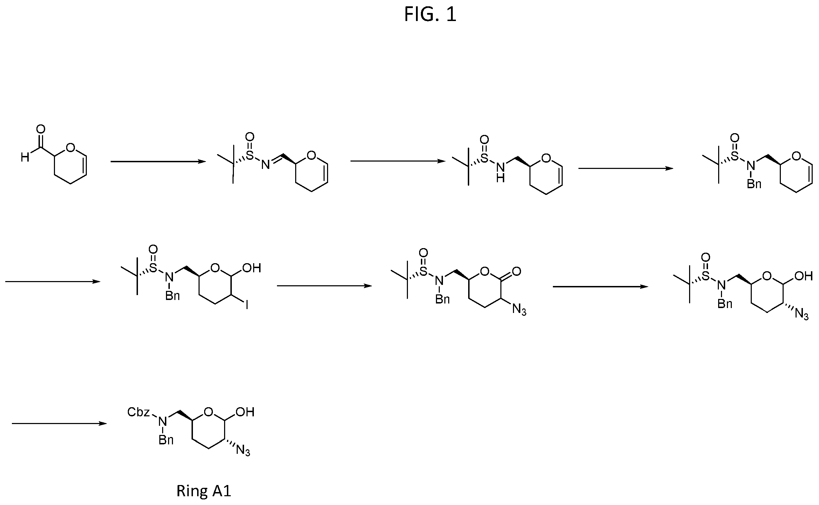

An exemplary scheme for the preparation of **Ring A1** is illustrated in **FIG. 1****,** and is discussed in greater detail herein. Generally, **Ring A1** may be prepared starting from 2-formyl-3,4-dihydro-2H-pyran using combinations of condensation, reduction, oxidation, alkylation, acylation, deprotection, and substitution reactions.

With reference to **FIG. 1****,** in some embodiments, 2-formyl-3,4-dihydro-2H-pyran is converted to a chiral sulfinylimine with, e.g., (S)-2-methylpropane-2-sulfinamide in the presence of a Lewis acid dehydrating agent, such as copper sulfate. The sulfinylimine may then be reduced to the corresponding sulfinamide with a suitable reducing agent, for example, sodium borohydride. In some embodiments, the resulting sulfinamide nitrogen is then protected with a suitable protecting group, e.g., benzyl. In some embodiments, the tetrahydropyran is converted to the corresponding halo lactol by reaction with, for example, bromine or iodine under aqueous conditions. In some embodiments, the hydroxy group of the halo lactol is oxidized to form the corresponding lactone using a suitable oxidant, such as a hexavalent chromium-based reagent. In some embodiments, an azide group is introduced by substitution of the halo (e.g., iodo) group with a source of azide ions. In some embodiments, the lactone carbonyl is reduced to form the corresponding lactol with a suitable reducing agent, such as an aluminum hydride reagent. The sulfinamide chiral auxiliary may then be cleaved, for example, under acidic conditions, and the resulting amino group protected with a suitable protecting group, for example, as the benzyl carbamate, to provide

### Ring A1.

In some embodiments, the A-Ring compound has the structure of Formula A2 (i.e., **Ring A2**):

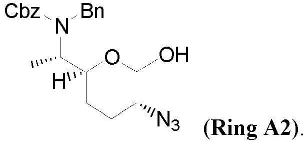

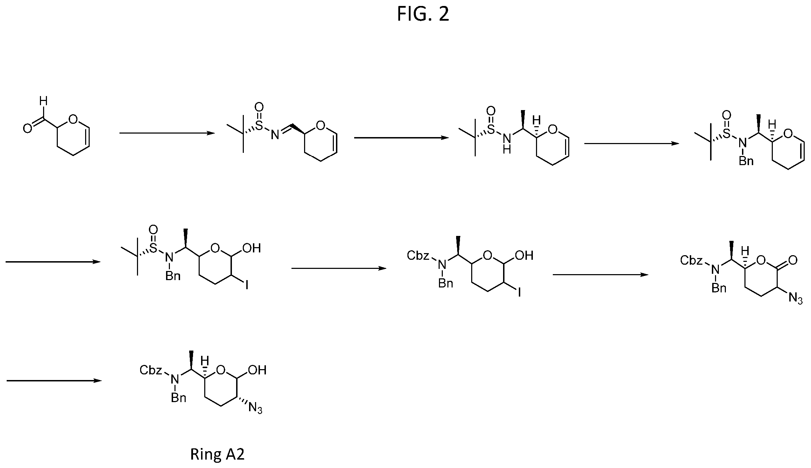

An exemplary scheme for the preparation of **Ring A2** is illustrated in **FIG. 2****.** Referring to **FIG. 2****,** in some embodiments, the aldehyde is converted to a chiral sulfinylimine with, e.g., (S)-2-methylpropane-2-sulfinamide in the presence of a Lewis acid dehydrating agent, such as copper sulfate. The sulfinylimine may then be treated with a suitable organometallic reagent, such as methyl magnesium bromide, to give the chiral sulfinamide. In some embodiments, the resulting sulfinamide nitrogen is then protected with a suitable protecting group, e.g., benzyl. In some embodiments, the tetrahydropyran is converted to the corresponding halo lactol by reaction with, for example, bromine or iodine under aqueous conditions. In some embodiments, the hydroxy group of the halo lactol is oxidized to form the corresponding lactone using a suitable oxidant, such as a hexavalent chromium-based reagent. In some embodiments, an azide group is introduced by substitution of the halo (e.g., iodo) group with a source of azide ions. In some embodiments, the lactone carbonyl is reduced to form the corresponding lactol with a suitable reducing agent, such as an aluminum hydride reagent. The sulfinamide chiral auxiliary may then be cleaved, for example, under acidic conditions, and the resulting amino group protected with a suitable protecting group, for example, as the benzyl carbamate, to provide **Ring A2.**

In some embodiments, the A-Ring compound has the structure of Formula A3 (i.e., **Ring A3**):

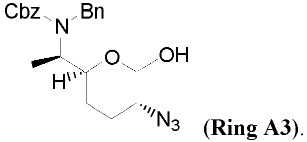

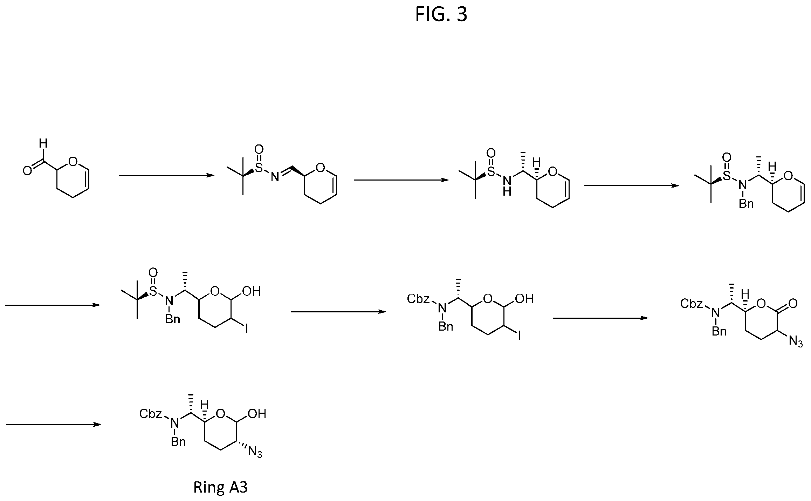

**Ring A3** may be prepared according to the general scheme discussed with respect to **FIG. 2****,** but using the R-antipode of 2-methylpropane-2-sulfinamide as depicted in **FIG. 3****.**

In some embodiments, the A-Ring compound has the structure of Formula A4 **(Ring A4):**

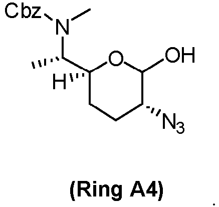

In some embodiments, the A-Ring compound has the structure of Formula A5 **(Ring A5):**

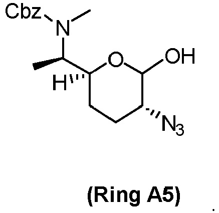

**Ring A4** and **Ring A5** may be prepared according to the general schemes for **Ring A2** and **Ring A3** respectively, discussed with respect to **FIGs. 2** and **3****,** but replacing the benzyl protecting group with methyl.

### Preparation of B-Ring Compounds

Preparation of compounds of the disclosure having the ABCD ring system (e.g., compounds of Formula (I) and Formula (III)) according to Scheme 1 utilizes a B-Ring compound. In some embodiments, the B-Ring compound has the general structure: 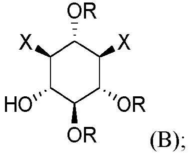 wherein:
X is an amine, an amine precursor, or a protected amino group; and
R is a protecting group.

In some embodiments, X is NH(Cbz) and R is Ac. Accordingly, in some embodiments, the B-Ring compound has the structure of Formula B1 (i.e., **Ring B1**):

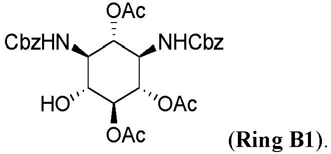

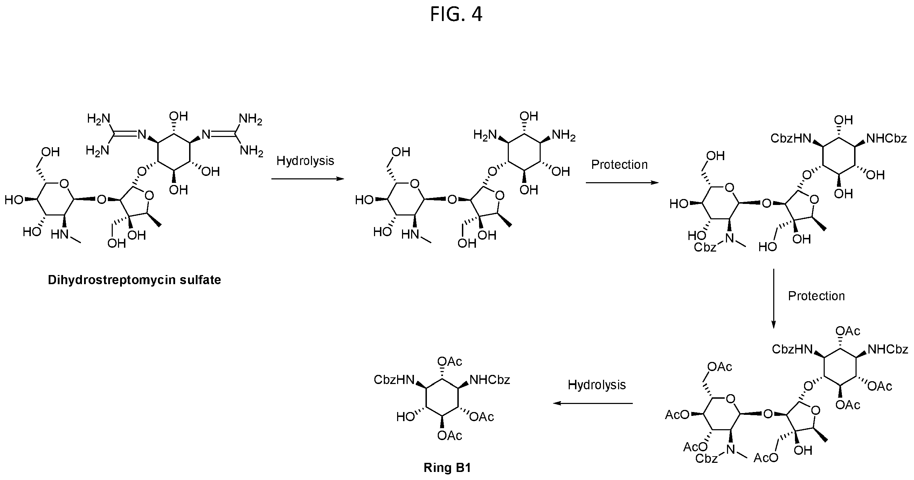

An exemplary method for the preparation of **Ring B1** is illustrated in **FIG. 4****.** Referring to **FIG. 4****,** generally, **Ring B1** may be prepared starting from dihydrostreptomycin by hydrolysis of the guanidine groups, N-protection, O-protection, and hydrolysis of the glycosyl linkage. Procedures for the preparation of such compounds are reported in, for example, International Patent Application Publication Nos. WO2019194858 and WO2019046126, both to Achaogen, Inc.

### Preparation of AB-Ring Compounds

An exemplary preparation of compounds of the disclosure having the ABCD ring system (e.g., compounds of Formula (I) and Formula (III)) according to Scheme 1 utilizes an AB-Ring compound. Generally, and with reference to Scheme 1, an A-Ring and a B-Ring compound are joined via a glycosidic linkage to form the AB-Ring compound. Generally, the reaction is performed under to conditions to allow for chemical glycosylation with coupling of a glycosyl donor and a glycosyl acceptor. For example, a hydroxyl group on a B-ring compound may serve as a glycosyl donor, reacting with an A-ring compound having a suitable leaving group to act as a glycosyl acceptor.

As a non-limiting example, the anomeric hydroxyl group of the A-Ring compound is converted to a suitable leaving group capable of reacting with a hydroxyl group on a B-Ring compound to form an interglycosidic linkage and allowed to react with the free hydroxyl group on the B-Ring compound, in a suitable solvent, to form said linkage. Examples of suitable leaving groups include, but are not limited to, halides, thioalkyl groups, thioaryl groups, imidates, acetate, phosphate, O-pentenyl groups, mesylate, tosylate, and the like. In one embodiment, the A-Ring compound is treated with an activating agent such as trichloroacetonitrile to form an imidate, and the B-Ring compound is added, followed by addition of an additional activating agent, such as a Lewis acid. In some embodiments, the Lewis acid is trimethylsilyl triflate, trimethylaluminum, or boron trifluoride etherate.

The structure of an AB-Ring compound may vary. In some embodiments, the AB-Ring compound has the general structure: 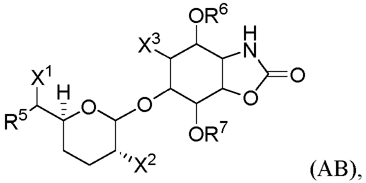 wherein:
X¹, X², and X³ are independently selected for each occasion from an amine, an amine precursor, and a protected amino group;
R⁵ is H or CH₃; and
R⁶ and R⁷ are independently selected for each occasion from H, Bn, and Piv.

In some embodiments, R⁵ is H.

In some embodiments, R⁵ is CH₃.

In some embodiments, X¹ is azide (N₃). In some embodiments, X¹ is -NH(Cbz). In some embodiments, X¹ is -N(Bn)(Cbz). In some embodiments, X¹ is -N(CH₃)(Cbz).

In some embodiments, X² is azide (N₃). In some embodiments, X² is -NH(Cbz).

In some embodiments, X³ is azide (N₃). In some embodiments, X³ is -NH(Cbz).

In some embodiments, R⁶ is benzyl. In some embodiments, R⁶ is pivaloyl.

In some embodiments, R⁷ is H.

In some embodiments, the AB-Ring compound has a structure selected from the group consisting of:

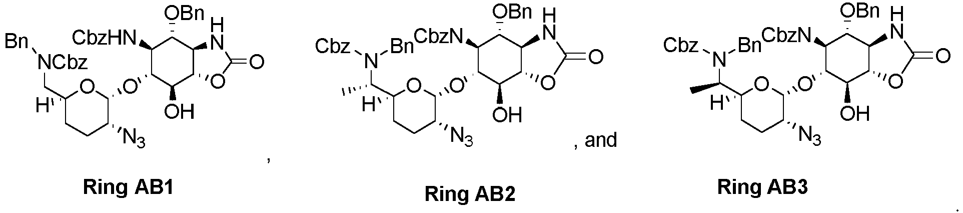

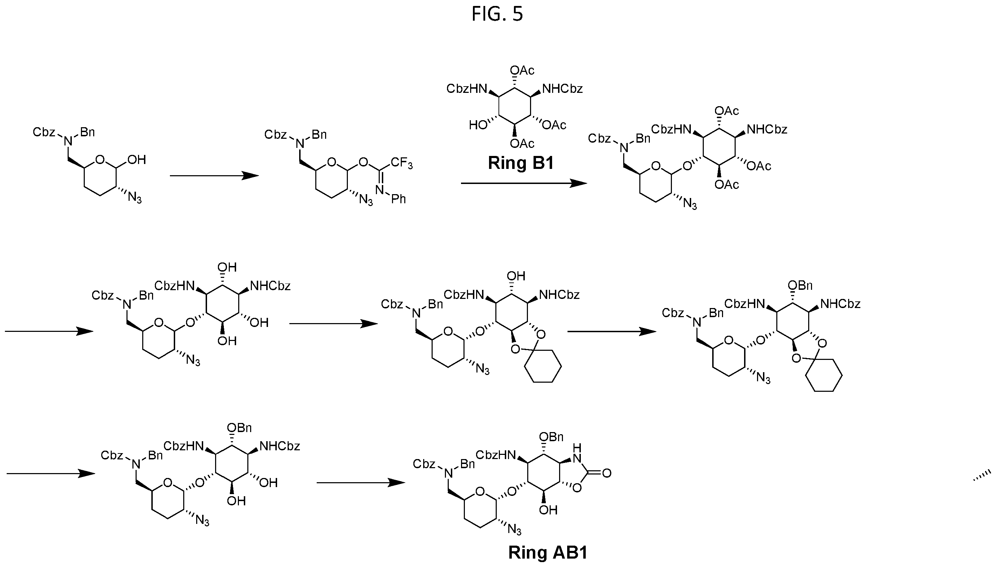

In some embodiments, the AB-Ring compound is **Ring AB1.** An exemplary scheme for the preparation of **Ring AB1** is illustrated in **FIG. 5****.** In one embodiment, with reference to **FIG. 5****, Ring A1** is activated by, for example, treatment with an activating reagent such as trichloroacetonitrile or 2,2,2-trifluoro-N-phenyl-acetimidoyl chloride, followed by addition of **Ring B1** and a Lewis acid such as bismuth triflate to provide the glycosylated product, from which the acetate protecting groups are then removed with, e.g., sodium methoxide or methanol in the presence of carbonate base. The resulting 1,2-diol functionality is then protected, for example, by conversion to a ketal. One suitable ketal is that formed from reaction of the diol with cyclohexanone or cyclohexanone dimethyl ketal. The remaining B-ring hydroxy group may then be protected, for example, as a benzyl ether. The ketal protecting group may then be removed to provide the 1,2-diol. To provide the desired single free hydroxyl group of **Ring AB1,** the adjacent amino and hydroxyl groups are protected. One suitable protection strategy is incorporation of the amino and hydroxyl groups into a oxazolidinone. One suitable means of forming the oxazolidinone is treatment with a strong, non-nucleophilic base such as sodium hydride, forming the oxazolidinone with expulsion of benzyl alcohol.

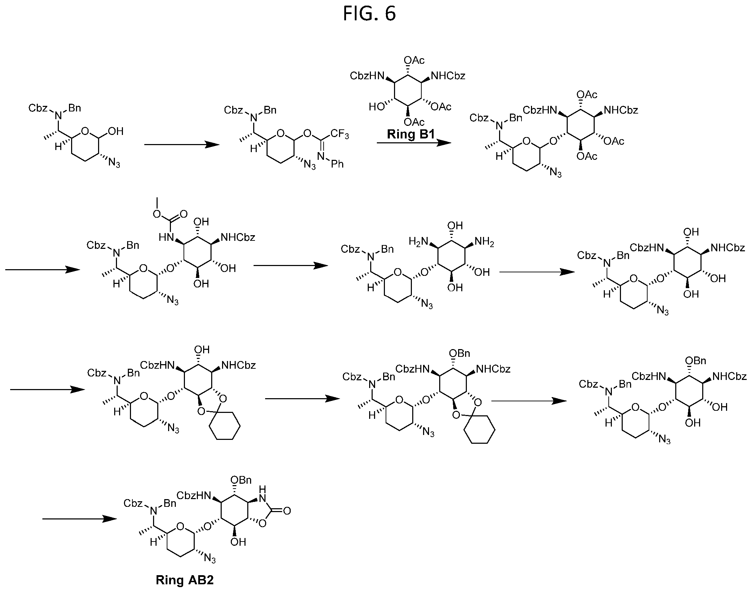

In some embodiments, the AB-Ring compound is **Ring AB2.** An exemplary scheme for the preparation of **Ring AB2** is illustrated in **FIG. 6****.** The scheme utilizes the same general strategy as that provided in **FIG. 5** but replacing **Ring A1** with **Ring A2.**

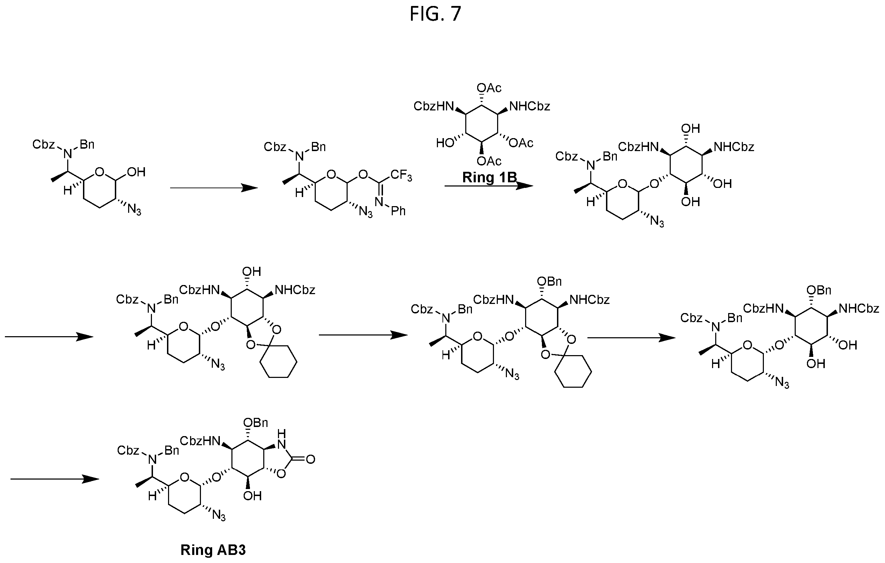

In some embodiments, the AB-Ring compound is **Ring AB3.** An exemplary scheme for the preparation of Ring **AB3** is illustrated in **FIG. 7****.** The scheme utilizes the same general strategy as that provided in **FIG. 5****,** replacing **Ring A1** with **Ring A3.**

Additional AB ring compounds may be similarly prepared using, for example, alternative A ring compounds (e.g., **Ring A4** or **Ring A5**), alternative B ring compounds, or both, and altering protecting group strategies as necessary.

### Preparation of CD-Ring Compounds

An exemplary preparation of compounds of the disclosure having the ABCD ring system (e.g., compounds of Formula (I) and Formula (III)) according to Scheme 1 utilizes a CD-Ring compound. In some embodiments, the CD-Ring compound is **Ring CD1:** 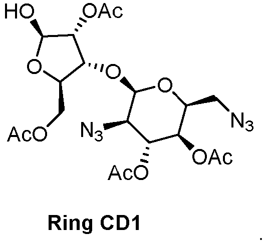 **Ring CD1** may be prepared according to the procedure reported in, for example, International Patent Application Publication No. WO2019/194858 to Achaogen, Inc..

In some embodiments, the CD-Ring compound is **Ring CD2:** 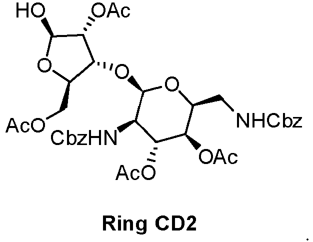 **Ring CD2** may be prepared from neomycin B according to the procedure reported in, for example, International Patent Application Publication No. WO2019/194858 to Achaogen, Inc.

### Preparation of ABCD Ring Compounds

Preparation of compounds having the ABCD ring system according to Scheme 1 (i.e., compounds of Formula (I) and Formula (III)) generally, and with reference to Scheme 1, comprises joining an AB-Ring and a CD-Ring compound, each as described herein above, via a glycosidic linkage. As a non-limiting example, the anomeric hydroxyl group of the CD-Ring compound is converted to a suitable leaving group (acceptor) capable of reacting with a hydroxyl group (donor) on an AB-Ring compound to form an interglycosidic linkage and allowed to react with the free hydroxyl group on the AB-Ring compound, in a suitable solvent, to form said linkage. Examples of suitable leaving groups include, but are not limited to, halides, thioalkyl groups, thioaryl groups, imidates, acetate, phosphate, O-pentenyl groups, mesylate, tosylate, and the like. In one embodiment, the CD-Ring compound is treated with an activating agent such as trichloroacetonitrile to form an imidate, and the AB-Ring compound is added, followed by addition of an additional activating agent, such as a Lewis acid. In some embodiments, the Lewis acid is trimethylsilyl triflate, trimethylaluminum, or boron trifluoride etherate.

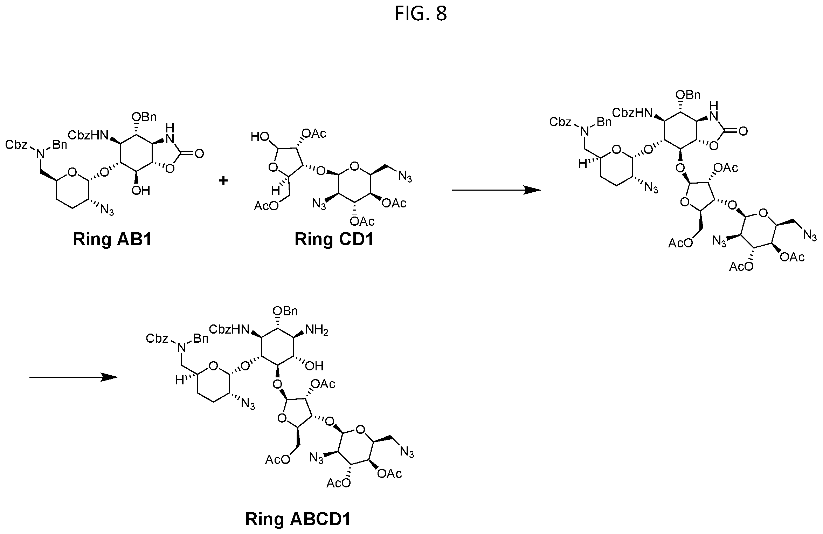

In some embodiments, the AB-Ring compound is **Ring AB1, Ring AB2,** or **Ring AB3.** In some embodiments, the CD-Ring compound is **Ring CD1.** Exemplary schemes for the preparation of **Ring ABCD1, Ring ABCD2,** and **Ring ABCD3** are illustrated in **FIGS. 8****,** **9****,** and **10****,** respectively.

In one embodiment, **Ring ABCD1** (i.e., a precursor to compounds of Formula (I)), may be prepared according to the scheme illustrated in **FIG. 8****.** With reference to **FIG. 8****, Ring CD1** is activated by, for example, treatment with an activating agent such as trichloroacetonitrile, followed by addition of **Ring AB1** and a Lewis acid to provide a compound having the ABCD ring system. In some embodiments, the Lewis acid is boron trifluoride etherate. Following the glycosylation reaction, the protected amino and hydroxyl groups, joined as an oxazolidinone, are liberated by hydrolysis under basic conditions to provide **Ring ABCD1.**

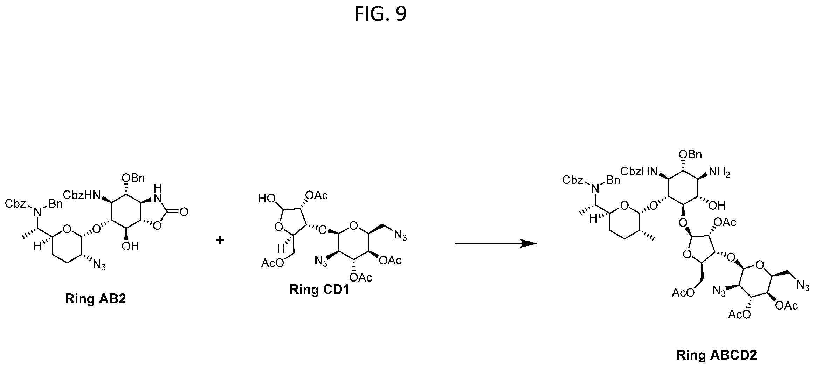

In one embodiment, **Ring ABCD2** (i.e. a precursor to compounds of Formula (III)) may be prepared according to the scheme illustrated in **FIG. 9****.** With reference to **FIG. 9****, Ring CD1** is activated with, for example, treatment with trichloroacetonitrile, followed by addition of **Ring AB2** and a Lewis acid to provide a compound having the ABCD ring system. In some embodiments, the Lewis acid is boron trifluoride etherate. Following the glycosylation reaction, the protected amino and hydroxyl groups, joined as an oxazolidinone, are liberated by hydrolysis under basic conditions to provide **Ring ABCD2.**

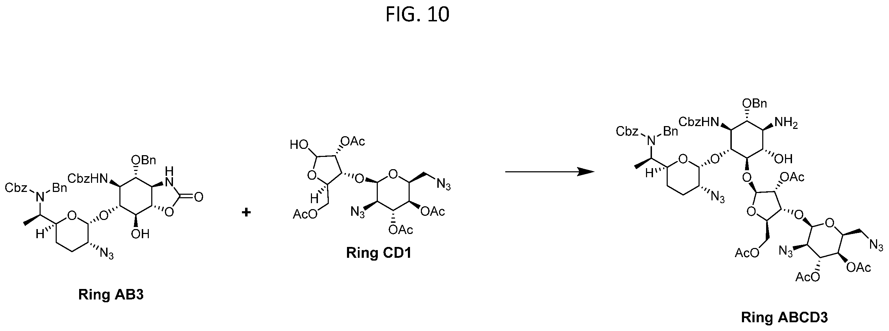

In one embodiment, **Ring ABCD3** (i.e., a precursor to compounds of Formula (III)) may be prepared according to the scheme illustrated in **FIG. 10****.** The scheme utilizes the same transformations described above with respect to **FIG. 9** but replacing **Ring AB2** with **Ring AB3.**

Further ABCD ring compounds may be prepared using schemes similar to those described herein above, and substituting the appropriate AB-Ring and CD-Ring compounds while adjusting the protecting group strategy accordingly. One of skill in the art will recognize the protecting group strategy adjustments to be made to accommodate the various combinations of potential AB-Ring and CD-Ring compounds contemplated within the generic Formulae disclosed herein (e.g., (I) and (III)).

### Introduction of Substituent R^{b}

In any of the foregoing embodiments, the ABCD compound (i.e., a compound of Formula (I) or Formula (III)) bears a substituent R^{b}. R^{b} has a structure according to Formula (II).

In some embodiments, the ABCD compound is a compound of Formula (I) or Formula (III)) and bears a substituent R^{b} wherein R^{b} has a structure according to Formula (II): 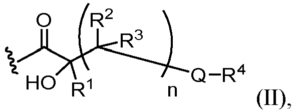 wherein:
Q is NH, O, or S;
n is an integer from 0 to 4;
R' is hydrogen or C₁₋₃ alkyl;
R² and R³ are each selected independently for each occurrence from the group consisting of hydrogen, alkyl, halogen, and -OH; and
R⁴ is H, C₁₋₃ alkyl, or -C(=NH)NR^{4a}R^{4b}, wherein R^{4a} and R^{4b} are each independently selected from the group consisting of hydrogen and C₁₋₃ alkyl; or
R¹ and R⁴, together with the atoms to which they are attached, form a heterocycloalkyl ring system comprising at least one N.

Accordingly, preparation of ABCD compounds of Formula (I) or Formula (III) in various embodiments can comprise reacting a suitable protected precursor of R^{b} with the free NH group of the corresponding ABCD ring compound to provide an ABCD ring compound with substituent R^{c}, which after additional steps as indicated, provides an ABCD ring compound of Formula (I) or Formula (III) having the desired substituent R^{b}.

Referring to the structure of Formula (II), in some embodiments, Q is NH; R¹ is hydrogen; R² and R³ are each selected independently for each occurrence from the group consisting of hydrogen, alkyl, halogen, and -OH; and R⁴ is H. Accordingly, in some embodiments, the substituent R^{c} has a structure according to Formula (IIa): 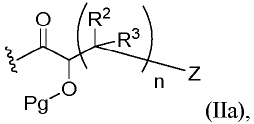 wherein:
Pg is a protecting group;
Z is an amine precursor or a protected amino group; and
the wavy line indicates a point of attachment to the amine of the B-ring.

In some embodiments, Z is azide (N₃). In some embodiments, Z is NHCBz.

In some embodiments, Pg is benzyl. In some embodiments, Pg is para-methoxybenzyl (PMB).

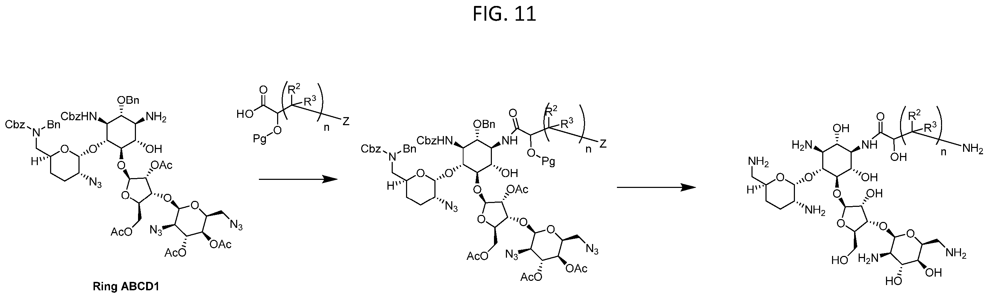

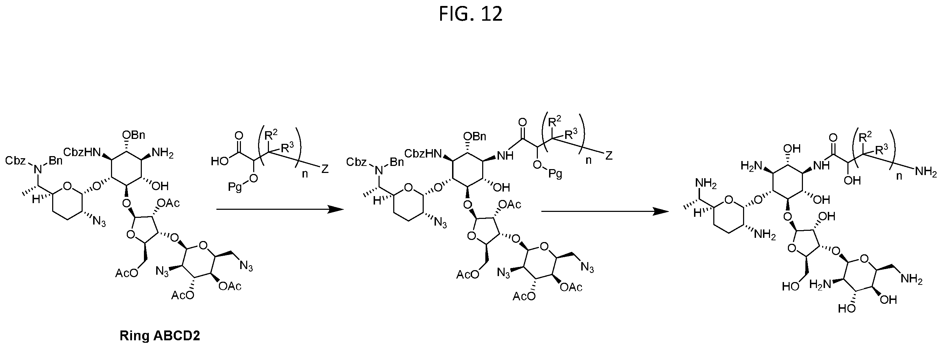

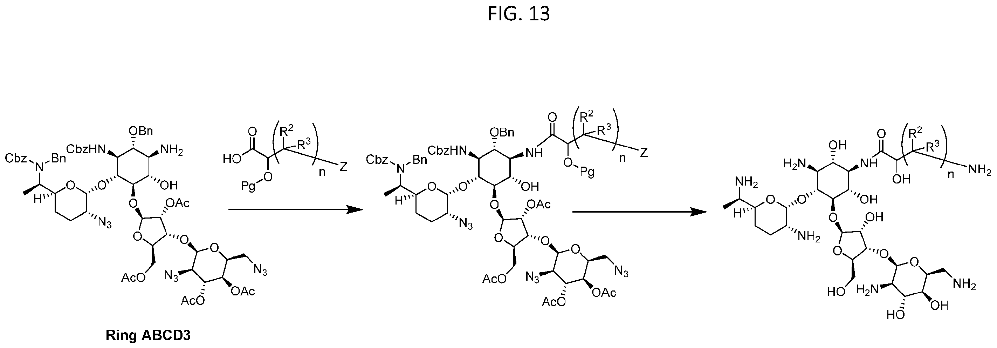

In the foregoing embodiments, one of skill in the art will recognize a suitable reactant for introduction of the substituent R^{c}, such as the corresponding carboxylic acid or an activated version thereof (i.e., wherein the wavy line in the compound of Formula (IIa) denotes an OH group or leaving group). Generally, introduction of substituent R^{c} is performed by coupling of the ABCD ring compound with the corresponding carboxylic acid of R^{c}. Further, one of skill will recognize that the final global deprotection (e.g., by hydrogenolysis) of the ABCD ring compound will also remove all benzylic protecting groups, and/or reduce the azide to an amine of the substituent R^{c}, providing the corresponding substituent R^{b} of the final ABCD ring compound of Formula (I) or Formula (III) as illustrated in FIGs. 11-13.

In some embodiments, R^{b} has a structure selected from the group consisting of: 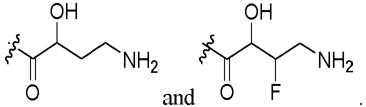 Accordingly, in some embodiments, the substituent R^{c} has a structure according to Formula (IIa), wherein n is 2; and R² and R³ are each selected independently for each occurrence from the group consisting of hydrogen and F. In such embodiments, substituent R^{c} has a structure selected from the group consisting of:

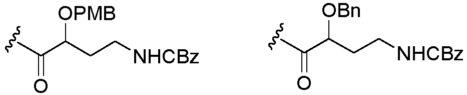

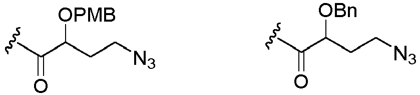

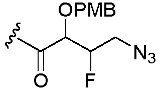

In some embodiments, R^{b} is selected from the group consisting of: 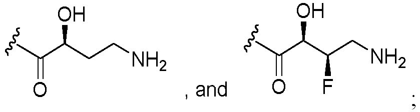 and
R^{c} is one of:

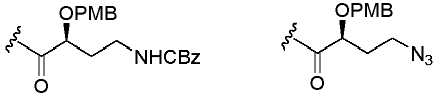

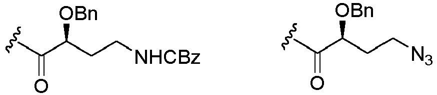

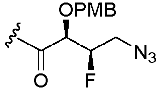

In some embodiments, the substituent R^{b} has a structure: 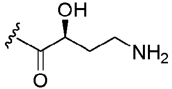 and the corresponding reactant is (S)-2-(benzyloxy)-4-((2-oxo-2-phenyl-1λ-ethyl)amino)butanoic acid, having the structure: 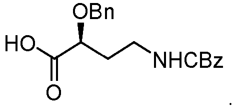 Both the illustrated reactant and the corresponding substituent Rb are referred to herein as "HABA" in reference to the compounds' hydroxy-aminobutyric acid moiety.

In some embodiments, the substituent R^{b} has a structure: 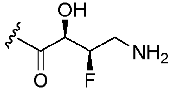 and the corresponding reactant is (2R,3R)-4-azido-3-fluoro-2-((4-methoxybenzyl)oxy)butanoic acid, having the structure: 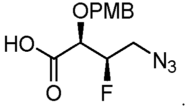 Both the illustrated reactant and the corresponding substituent Rb are referred to herein as "F-HABA" in reference to the compounds' fluoro-hydroxy-aminobutyric acid moiety

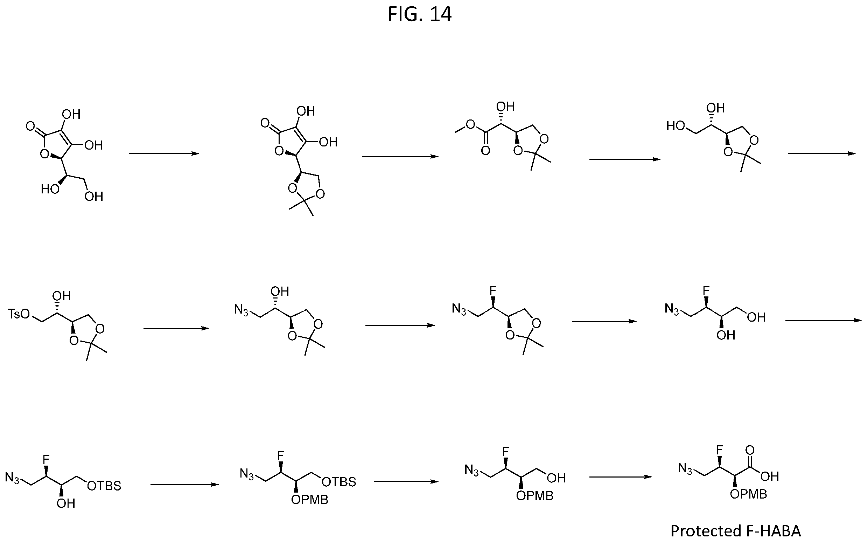

In one embodiment, the (2*R*,3*R*)-4-azido-3-fluoro-2-((4-methoxybenzyl)oxy)butanoic acid (F-HABA) may be prepared according to the scheme illustrated in **FIG. 14****.** With reference to **FIG. 14****,** D-isoascorbic acid is protected as a ketal, and oxidatively cleaved to provide the α-hydroxy ester. The ester is then reduced to the alcohol, and the primary hydroxyl group converted to an azide. The secondary hydroxyl group is then converted to a fluoro group with stereochemical inversion. The ketal is removed, and the two hydroxyl groups differentially protected. For example, the primary hydroxyl group is protected as a silyl ether, and the secondary hydroxyl as a benzylic ether (e.g., para-methoxybenzyl). Cleavage of the silyl ether and oxidation of the primary hydroxymethyl group to the corresponding carboxylic acid then provides the desired (2*R*,3*R*)-4-azido-3-fluoro-2-((4-methoxybenzyl)oxy)butanoic acid.

### Pharmaceutical Formulations and Compositions

The compounds described herein can be considered useful as pharmaceutical compositions for administration to a subject in need thereof. Pharmaceutical compositions can comprise at least the compounds or salts described herein and one or more pharmaceutically acceptable carriers, diluents, excipients, stabilizers, dispersing agents, suspending agents, and/or thickening agents.

Pharmaceutical compositions can be formulated using one or more physiologically acceptable carriers comprising excipients and auxiliaries. Formulation can be modified depending upon the route of administration chosen. Pharmaceutical compositions comprising a compound or salt as described herein can be manufactured, for example, by lyophilizing the compound or salt, mixing, dissolving, emulsifying, encapsulating or entrapping the compound or salts. The pharmaceutical compositions can also include the compounds or salts, described herein in a free-base form or pharmaceutically acceptable salt form.

Pharmaceutical compositions described herein can comprise at least one active ingredient (e.g., a compound or salts). The active ingredients can be entrapped in microcapsules prepared, for example, by coacervation techniques or by interfacial polymerization (e.g., hydroxymethylcellulose or gelatin microcapsules and poly-(methylmethacylate) microcapsules, respectively), in colloidal drug-delivery systems (e.g., liposomes, albumin microspheres, microemulsions, nanoparticles and nanocapsules) or in macroemulsions.

Pharmaceutical compositions as described herein often further can comprise more than one active compound (e.g., a compound or salt and other agents) as necessary for the particular indication being treated. The active compounds can have complementary activities that do not adversely affect each other. For example, the composition can also comprise a chemotherapeutic agent, cytotoxic agent, cytokine, growth-inhibitory agent, anti-hormonal agent, anti-angiogenic agent, and/or cardioprotectant. Such molecules can be present in combination in amounts that are effective for the purpose intended.

The compositions and formulations can be sterilized. Sterilization can be accomplished by filtration through sterile filtration.

The compositions described herein can be formulated for administration as an injection. Non-limiting examples of formulations for injection can include a sterile suspension, solution or emulsion in oily or aqueous vehicles. Suitable oily vehicles can include, but are not limited to, lipophilic solvents or vehicles such as fatty oils or synthetic fatty acid esters, or liposomes. Aqueous injection suspensions can contain substances which increase the viscosity of the suspension. The suspension can also contain suitable stabilizers. Injections can be formulated for bolus injection or continuous infusion. Alternatively, the compositions described herein can be lyophilized or in powder form for reconstitution with a suitable vehicle, e.g., sterile pyrogen-free water, before use.

For parenteral administration, the compounds or salts can be formulated in a unit dosage injectable form (e.g., use letter solution, suspension, emulsion) in association with a pharmaceutically acceptable parenteral vehicle. Such vehicles can be inherently non-toxic, and non-therapeutic. Vehicles can be water, saline, Ringer's solution, dextrose solution, and 5% human serum albumin. Non-aqueous vehicles such as fixed oils and ethyl oleate can also be used. Liposomes can be used as carriers. The vehicle can contain minor amounts of additives such as substances that enhance isotonicity and chemical stability (e.g., buffers and preservatives).

Sustained-release preparations can also be prepared. Examples of sustained-release preparations can include semipermeable matrices of solid hydrophobic polymers that can contain the compound or salt, and these matrices can be in the form of shaped articles (e.g., films or microcapsules). Examples of sustained-release matrices can include polyesters, hydrogels (e.g., poly(2-hydroxyethyl-methacrylate), or poly (vinyl alcohol)), polylactides, copolymers of L-glutamic acid and y ethyl-L-glutamate, nondegradable ethylene-vinyl acetate, degradable lactic acid-glycolic acid copolymers such as the LUPRON DEPOTM (i.e., injectable microspheres composed of lactic acid-glycolic acid copolymer and leuprolide acetate), and poly-D-( -)-3-hydroxybutyric acid.

Pharmaceutical formulations described herein can be prepared for storage by mixing a compound or salt with a pharmaceutically acceptable carrier, excipient, and/or a stabilizer. This formulation can be a lyophilized formulation or an aqueous solution. Acceptable carriers, excipients, and/or stabilizers can be nontoxic to recipients at the dosages and concentrations used. Acceptable carriers, excipients, and/or stabilizers can include buffers such as phosphate, citrate, and other organic acids; antioxidants including ascorbic acid and methionine; preservatives, polypeptides; proteins, such as serum albumin or gelatin; hydrophilic polymers; amino acids; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes; and/or non-ionic surfactants or polyethylene glycol.

In some embodiments the drug delivery composition may be incorporated into a system comprising a substrate that carries the composition to the administration site or delivery site or treatment site. The substrate may remain with the composition upon administration (or upon delivery of the composition) and for any amount of time or indefinitely thereafter or be removed upon administration (or upon delivery of the composition) leaving the composition at the administration site or delivery site or treatment site.

The compounds of Formula (I), or Formula (III) can be administered in the "native" form or, if desired, in the form of salts, esters, amides, prodrugs, clathrates, derivatives, and the like, provided the salt, ester, amide, prodrug, clathrate, or derivative is pharmacologically suitable, e.g., effective in treatment of a pathology and/or various symptoms thereof, e.g., as described herein. Salts, esters, amides, clathrates, prodrugs and other derivatives of the compounds of Formula (I), or Formula (III) can be prepared using standard procedures known to those skilled in the art of synthetic organic chemistry and described, for example, by March (1992) Advanced Organic Chemistry; Reactions, Mechanisms and Structure, 4th Ed. N.Y. Wiley-Interscience, and as described above.

For example, a pharmaceutically acceptable salt can be prepared for any of the compounds of Formula (I), or Formula (III), described herein having a functionality capable of forming a salt. A pharmaceutically acceptable salt is any salt that retains the activity of the parent compound and does not impart any deleterious or untoward effect on the subject to which it is administered and in the context in which it is administered.

In various embodiments pharmaceutically acceptable salts may be derived from organic or inorganic bases. The salt may be a mono or polyvalent ion. Of particular interest are the inorganic ions, lithium, sodium, potassium, calcium, and magnesium. Organic salts may be made with amines, particularly ammonium salts such as mono-, di- and trialkyl amines or ethanol amines. Salts may also be formed with caffeine, tromethamine and similar molecules.

Methods of formulating pharmaceutically compounds of Formula (I), or Formula (III) as salts, esters, amide, prodrugs, and the like are well known to those of skill in the art. For example, salts can be prepared from the free base using conventional methodology that typically involves reaction with a suitable acid. Generally, the base form of the drug is dissolved in a polar organic solvent such as methanol or ethanol and the acid is added thereto. The resulting salt either precipitates or can be brought out of solution by addition of a less polar solvent. Suitable acids for preparing acid addition salts include, but are not limited to both organic acids, e.g., acetic acid, propionic acid, glycolic acid, pyruvic acid, oxalic acid, malic acid, malonic acid, succinic acid, maleic acid, fumaric acid, tartaric acid, citric acid, benzoic acid, cinnamic acid, mandelic acid, methanesulfonic acid, ethanesulfonic acid, p-toluenesulfonic acid, salicylic acid, and the like, as well as inorganic acids, e.g., hydrochloric acid, hydrobromic acid, sulfuric acid, nitric acid, phosphoric acid, and the like. An acid addition salt can be reconverted to the free base by treatment with a suitable base. Certain particularly preferred acid addition salts of the compounds of Formula (I) or Formula (III) herein include halide salts, such as may be prepared using hydrochloric or hydrobromic acids. Conversely, preparation of basic salts of the compounds of Formula (I), or Formula (III) of this disclosure are prepared in a similar manner using a pharmaceutically acceptable base such as sodium hydroxide, potassium hydroxide, ammonium hydroxide, calcium hydroxide, trimethylamine, or the like. Particularly preferred basic salts include alkali metal salts, e.g., the sodium salt, and copper salts.

For the preparation of salt forms of basic drugs, the pKa of the counterion is preferably at least about 2 pH units lower than the pKa of the drug. Similarly, for the preparation of salt forms of acidic drugs, the pKa of the counterion is preferably at least about 2 pH units higher than the pKa of the drug. This permits the counterion to bring the solution's pH to a level lower than the pHmax to reach the salt plateau, at which the solubility of salt prevails over the solubility of free acid or base. The generalized rule of difference in pKa units of the ionizable group in the active pharmaceutical ingredient (API) and in the acid or base is meant to make the proton transfer energetically favorable. When the pKa of the API and counterion are not significantly different, a solid complex may form but may rapidly disproportionate (i.e., break down into the individual entities of drug and counterion) in an aqueous environment.

Preferably, the counterion is a pharmaceutically acceptable counterion. Suitable anionic salt forms include, but are not limited to acetate, benzenesulfonate, benzoate, benzylate, bicarbonate, bitartrate, bitartrate, bromide, calcium edetate, camsylateh, carbonate, chloride, citrate, dihydrochloride, edetate, edisylate, estolate, esylate, fumarate, gluceptate, gluconate, glutamate, glycollylarsanilate, hexylresorcinate, hydrabamine, hydrobromide, hydrochloride, hydroxynaphthoate, iodide, isethionatei, lactate, lactobionate, malate, maleate, mandelate, mesylate , methylbromide, methylnitrate, methylsulfate, mucate, napsylate, nitrate, pamoate (embonate), pantothenate, phosphate and diphosphate, polygalacturonate, salicylate and disalicylate, stearate, subacetate, succinate, sulfate, tannate, tartrate, teoclate, tosylate, triethiodide, valerate, and the like, while suitable cationic salt forms include, but are not limited to aluminum, benzathine, calcium, ethylene diamine, lysine, magnesium, meglumine, potassium, procaine, sodium, tromethamine, zinc, and the like. Suitable cationic salt forms include, but are not limited to Benzathine, chloroprocaine, choline, diethanolamine, ethylenediamine, meglumine, procaine, aluminum, calcium, lithium, magnesium, potassium, sodium, zinc, and the like.

Preparation of esters typically involves functionalization of hydroxyl and/or carboxyl groups that are present within the molecular structure of the compounds of Formula (I) or Formula (III). In certain embodiments, the esters are typically acyl-substituted derivatives of free alcohol groups, i.e., moieties that are derived from carboxylic acids of the formula RCOOH where R is alkyl, and preferably is lower alkyl. Esters can be reconverted to the free acids, if desired, by using conventional hydrogenolysis or hydrolysis procedures.

Amides can also be prepared using techniques known to those skilled in the art or described in the pertinent literature. For example, amides may be prepared from esters, using suitable amine reactants, or they may be prepared from an anhydride or an acid chloride by reaction with ammonia or a lower alkyl amine.

In various embodiments, the compounds of Formula (I) or Formula (III), described herein are useful for parenteral administration, topical administration, oral administration, nasal administration (or otherwise inhaled), rectal administration, or local administration, such as by aerosol or transdermally, for prophylactic and/or therapeutic treatment of one or more of the pathologies/indications described herein (e.g., pathologies characterized by excess amyloid plaque formation and/or deposition or undesired amyloid or pre-amyloid processing).

The compounds of Formula (I) or Formula (III), described herein can also be combined with a pharmaceutically acceptable carrier (excipient) to form a pharmacological composition. Pharmaceutically acceptable carriers can contain one or more physiologically acceptable compound(s) that act, for example, to stabilize the composition or to increase or decrease the absorption of the active agent(s). Physiologically acceptable compounds can include, for example, carbohydrates, such as glucose, sucrose, or dextrans, antioxidants, such as ascorbic acid or glutathione, chelating agents, low molecular weight proteins, protection and uptake enhancers such as lipids, compositions that reduce the clearance or hydrolysis of the active agents, or excipients or other stabilizers and/or buffers.

Other physiologically acceptable compounds, particularly of use in the preparation of tablets, capsules, gel caps, and the like include, but are not limited to binders, diluent/fillers, disintegrants, lubricants, suspending agents, and the like.

In certain embodiments, to manufacture an oral dosage form (e.g., a tablet), an excipient (e.g., lactose, sucrose, starch, mannitol, etc.), an optional disintegrator (e.g. calcium carbonate, carboxymethylcellulose calcium, sodium starch glycollate, crospovidone etc.), a binder (e.g. alpha-starch, gum arabic, microcrystalline cellulose, carboxymethylcellulose, polyvinylpyrrolidone, hydroxypropylcellulose, cyclodextrin, etc.), and an optional lubricant (e.g., talc, magnesium stearate, polyethylene glycol 6000, etc.), for instance, are added to the active component or components (e.g. compound represented by the structure of Formula I and the like)) and the resulting composition is compressed. Where necessary the compressed product is coated, e.g., using known methods for masking the taste or for enteric dissolution or sustained release. Suitable coating materials include, but are not limited to ethyl-cellulose, hydroxymethylcellulose, POLYOX^{®} polyyethylene glycol, cellulose acetate phthalate, hydroxypropylmethylcellulose phthalate, and Eudragit (Rohm & Haas, Germany; methacrylic-acrylic copolymer).

Other physiologically acceptable compounds include wetting agents, emulsifying agents, dispersing agents or preservatives that are particularly useful for preventing the growth or action of microorganisms. Various preservatives are well known and include, for example, phenol and ascorbic acid. One skilled in the art would appreciate that the choice of pharmaceutically acceptable carrier(s), including a physiologically acceptable compound depends, for example, on the route of administration of the active agent(s) and on the particular physio-chemical characteristics of the active agent(s). In certain embodiments the excipients are sterile and generally free of undesirable matter. These compositions can be sterilized by conventional, well-known sterilization techniques. For various oral dosage form excipients such as tablets and capsules sterility is not required. The USP/NF standard is usually sufficient.

The pharmaceutical compositions can be administered in a variety of unit dosage forms depending upon the method of administration. Suitable unit dosage forms, include, but are not limited to powders, tablets, pills, capsules, lozenges, suppositories, patches, nasal sprays, injectibles, implantable sustained-release formulations, mucoadherent films, topical varnishes, lipid complexes, etc.

Pharmaceutical compositions comprising the compounds of Formula (I) or Formula (III) can be manufactured by means of conventional mixing, dissolving, granulating, dragee-making, levigating, emulsifying, encapsulating, entrapping or lyophilizing processes. Pharmaceutical compositions can be formulated in a conventional manner using one or more physiologically acceptable carriers, diluents, excipients or auxiliaries that facilitate processing of the active agent(s) into preparations that can be used pharmaceutically. Proper formulation is dependent upon the route of administration chosen.

In certain embodiments, the active agents described herein (e.g., compounds of Formula (I) or Formula (III)) and compositions comprising said compounds) are formulated for oral administration. For oral administration, suitable formulations can be readily formulated by combining the active agent(s) with pharmaceutically acceptable carriers suitable for oral delivery well known in the art. Such carriers enable the active agent(s) described herein to be formulated as tablets, pills, dragees, caplets, lozenges, gelcaps, capsules, liquids, gels, syrups, slurries, suspensions and the like, for oral ingestion by a patient to be treated. For oral solid formulations such as, for example, powders, capsules and tablets, suitable excipients can include fillers such as sugars (e.g., lactose, sucrose, mannitol and sorbitol), cellulose preparations (e.g., maize starch, wheat starch, rice starch, potato starch, gelatin, gum tragacanth, methyl cellulose, hydroxypropylmethyl-cellulose, sodium carboxymethylcellulose), synthetic polymers (e.g., polyvinylpyrrolidone (PVP)), granulating agents; and binding agents. If desired, disintegrating agents may be added, such as the cross-linked polyvinylpyrrolidone, agar, or alginic acid or a salt thereof such as sodium alginate. If desired, solid dosage forms may be sugar-coated or enteric-coated using standard techniques. The preparation of enteric-coated particles is disclosed for example in U.S. Pat. Nos. 4,786,505 and 4,853,230.

For administration by inhalation, the active agent(s) are conveniently delivered in the form of an aerosol spray from pressurized packs or a nebulizer, with the use of a suitable propellant, e.g., dichlorodifluoromethane, trichlorofluoromethane, dichlorotetrafluoroethane, carbon dioxide or other suitable gas. In the case of a pressurized aerosol the dosage unit can be determined by providing a valve to deliver a metered amount. Capsules and cartridges of e.g., gelatin for use in an inhaler or insufflator may be formulated containing a powder mix of the compound and a suitable powder base such as lactose or starch.

In various embodiments the active agent(s) can be formulated in rectal or vaginal compositions such as suppositories or retention enemas, e.g., containing conventional suppository bases such as cocoa butter or other glycerides. Methods of formulating active agents for rectal or vaginal delivery are well known to those of skill in the art (see, e.g., Allen (2007) Suppositories, Pharmaceutical Press) and typically involve combining the active agents with a suitable base (e.g., hydrophilic (PEG), lipophilic materials such as cocoa butter or Witepsol W45), amphiphilic materials such as Suppocire AP and polyglycolized glyceride, and the like). The base is selected and compounded for a desired melting/delivery profile.

For topical administration the compounds of Formula (I) or Formula (III) can be formulated as solutions, gels, ointments, creams, suspensions, and the like as are well-known in the art.

In certain embodiments the compounds of Formula (I) or Formula (III) described herein are formulated for systemic administration (e.g., as an injectable) in accordance with standard methods well known to those of skill in the art. Systemic formulations include, but are not limited to, those designed for administration by injection, e.g. subcutaneous, intravenous, intramuscular, intrathecal or intraperitoneal injection, as well as those designed for transdermal, transmucosal oral or pulmonary administration. For injection, the active agents described herein can be formulated in aqueous solutions, preferably in physiologically compatible buffers such as Hank's solution, Ringer's solution, or physiological saline buffer and/or in certain emulsion formulations. The solution(s) can contain formulatory agents such as suspending, stabilizing and/or dispersing agents. In certain embodiments the active agent(s) can be provided in powder form for constitution with a suitable vehicle, e.g., sterile pyrogen-free water, before use. For transmucosal administration, and/or for blood/brain barrier passage, penetrants appropriate to the barrier to be permeated can be used in the formulation. Such penetrants are generally known in the art. Injectable formulations and inhalable formulations are generally provided as a sterile or substantially sterile formulation.

**In** addition to the formulations described previously, the active agent(s) may also be formulated as a depot preparation. Such long-acting formulations can be administered by implantation (for example subcutaneously or intramuscularly) or by intramuscular injection. Thus, for example, the active agent(s) may be formulated with suitable polymeric or hydrophobic materials (for example as an emulsion in an acceptable oil) or ion exchange resins, or as sparingly soluble derivatives, for example, as a sparingly soluble salt.

**In** certain embodiments, the active agent(s) described herein can also be delivered through the skin using conventional transdermal drug delivery systems, i.e., transdermal "patches" wherein the active agent(s) are typically contained within a laminated structure that serves as a drug delivery device to be affixed to the skin. In such a structure, the drug composition is typically contained in a layer, or "reservoir," underlying an upper backing layer. It will be appreciated that the term "reservoir" in this context refers to a quantity of "active ingredient(s)" that is ultimately available for delivery to the surface of the skin. Thus, for example, the "reservoir" may include the active ingredient(s) in an adhesive on a backing layer of the patch, or in any of a variety of different matrix formulations known to those of skill in the art. The patch may contain a single reservoir, or it may contain multiple reservoirs.

**In** one illustrative embodiment, the reservoir comprises a polymeric matrix of a pharmaceutically acceptable contact adhesive material that serves to affix the system to the skin during drug delivery. Examples of suitable skin contact adhesive materials include, but are not limited to polyethylenes, polysiloxanes, polyisobutylenes, polyacrylates, polyurethanes, and the like. Alternatively, the drug-containing reservoir and skin contact adhesive are present as separate and distinct layers, with the adhesive underlying the reservoir which, in this case, may be either a polymeric matrix as described above, or it may be a liquid or hydrogel reservoir, or may take some other form. The backing layer in these laminates, which serves as the upper surface of the device, preferably functions as a primary structural element of the "patch" and provides the device with much of its flexibility. The material selected for the backing layer is preferably substantially impermeable to the active agent(s) and any other materials that are present.

Alternatively, other pharmaceutical delivery systems can be employed. For example, liposomes, emulsions, and microemulsions/nanoemulsions are well known examples of delivery vehicles that may be used to protect and deliver pharmaceutically active compounds. Certain organic solvents such as dimethylsulfoxide also can be employed, although usually at the cost of greater toxicity.

In certain embodiments the compounds of Formula (I) or Formula (III) as described herein are formulated in a nanoemulsion. Nanoemulsions include, but are not limited to, oil in water (O/W) nanoemulsions, and water in oil (W/O) nanoemulsions. Nanoemulsions can be defined as emulsions with mean droplet diameters ranging from about 20 to about 1000 nm. Usually, the average droplet size is between about 20 nm or 50 nm and about 500 nm. The terms sub-micron emulsion (SME) and mini-emulsion are used as synonyms.

Illustrative oil in water (O/W) nanoemulsions include, but are not limited to: Surfactant micelles -- micelles composed of small molecules surfactants or detergents (e.g., SDS/PBS/2-propanol); Polymer micelles -- micelles composed of polymer, copolymer, or block copolymer surfactants (e.g., Pluronic L64/PBS/2-propanol); Blended micelles -- micelles in which there is more than one surfactant component or in which one of the liquid phases (generally an alcohol or fatty acid compound) participates in the formation of the micelle (e.g., octanoic acid/PBS/EtOH); Integral micelles -- blended micelles in which the active agent(s) serve as an auxiliary surfactant, forming an integral part of the micelle; and Pickering (solid phase) emulsions -- emulsions in which the active agent(s) are associated with the exterior of a solid nanoparticle (e.g., polystyrene nanoparticles/PBS/no oil phase).

Illustrative water in oil (W/O) nanoemulsions include, but are not limited to: Surfactant micelles -- micelles composed of small molecules surfactants or detergents (e.g., dioctyl sulfosuccinate/PBS/2-propanol, isopropylmyristate/PBS/2-propanol, etc.); Polymer micelles --micelles composed of polymer, copolymer, or block copolymer surfactants (e.g., PLURONIC^{®} L121/PBS/2-propanol); Blended micelles --micelles in which there is more than one surfactant component or in which one of the liquid phases (generally an alcohol or fatty acid compound) participates in the formation of the micelle (e.g., capric/caprylic diglyceride/PBS/EtOH); Integral micelles --blended micelles in which the active agent(s) serve as an auxiliary surfactant, forming an integral part of the micelle (e.g., active agent/PBS/polypropylene glycol); and Pickering (solid phase) emulsions --emulsions in which the active agent(s) are associated with the exterior of a solid nanoparticle (e.g., chitosan nanoparticles/no aqueous phase/mineral oil).

As indicated above, in certain embodiments the nanoemulsions comprise one or more surfactants or detergents. In some embodiments the surfactant is a non-anionic detergent (e.g., a polysorbate surfactant, a polyoxyethylene ether, etc.). Surfactants that find use in the present invention include but are not limited to surfactants such as the TWEEN^{®}, TRITON^{®}, and TYLOXAPOL^{®} families of compounds.

In certain embodiments the emulsions further comprise one or more cationic halogen containing compounds, including but not limited to, cetylpyridinium chloride. In still further embodiments, the compositions further comprise one or more compounds that increase the interaction ("interaction enhancers") of the composition with microorganisms (e.g., chelating agents like ethylenediaminetetraacetic acid, or ethylenebis(oxyethylenenitrilo)tetraacetic acid in a buffer).

In some embodiments, the nanoemulsion further comprises an emulsifying agent to aid in the formation of the emulsion. Emulsifying agents include compounds that aggregate at the oil/water interface to form a kind of continuous membrane that prevents direct contact between two adjacent droplets. Certain embodiments of the present invention feature oil-in-water emulsion compositions that may readily be diluted with water to a desired concentration without impairing their anti-pathogenic properties.

In addition to discrete oil droplets dispersed in an aqueous phase, certain oil-in-water emulsions can also contain other lipid structures, such as small lipid vesicles (e.g., lipid spheres that often consist of several substantially concentric lipid bilayers separated from each other by layers of aqueous phase), micelles (e.g., amphiphilic molecules in small clusters of 50-200 molecules arranged so that the polar head groups face outward toward the aqueous phase and the apolar tails are sequestered inward away from the aqueous phase), or lamellar phases (lipid dispersions in which each particle consists of parallel amphiphilic bilayers separated by thin films of water).

These lipid structures are formed as a result of hydrophobic forces that drive apolar residues (e.g., long hydrocarbon chains) away from water. The above lipid preparations can generally be described as surfactant lipid preparations (SLPs). SLPs are minimally toxic to mucous membranes and are believed to be metabolized within the small intestine (see e.g., Hamouda et al., (1998) J. Infect. Disease 180: 1939).

In certain embodiments the emulsion comprises a discontinuous oil phase distributed in an aqueous phase, a first component comprising an alcohol and/or glycerol, and a second component comprising a surfactant or a halogen-containing compound. The aqueous phase can comprise any type of aqueous phase including, but not limited to, water (e.g., deionized water, distilled water, tap water) and solutions (e.g., phosphate buffered saline solution, or other buffer systems). The oil phase can comprise any type of oil including, but not limited to, plant oils (e.g., soybean oil, avocado oil, flaxseed oil, coconut oil, cottonseed oil, squalene oil, olive oil, canola oil, corn oil, rapeseed oil, safflower oil, and sunflower oil), animal oils (e.g., fish oil), flavor oil, water insoluble vitamins, mineral oil, and motor oil. In certain embodiments, the oil phase comprises 30-90 vol % of the oil-in-water emulsion (i.e., constitutes 30-90% of the total volume of the final emulsion), more preferably 50-80%. The formulations need not be limited to particular surfactants, however in certain embodiments, the surfactant is a polysorbate surfactant (e.g., TWEEN 20^{®}, TWEEN 40^{®}, TWEEN 60^{®}, and TWEEN 80^{®}), a phenoxypolyethoxyethanol (e.g., TRITON^{®} X-100, X-301, X-165, X-102, and X-200, and TYLOXAPOL^{®}), or sodium dodecyl sulfate, and the like.

In certain embodiments a halogen-containing component is present. The nature of the halogen-containing compound, in some preferred embodiments the halogen-containing compound comprises a chloride salt (e.g., NaCl, KCl, etc.), a cetylpyridinium halide, a cetyltrimethylammonium halide, a cetyldimethylethylammonium halide, a cetyldimethylbenzylammonium halide, a cetyltributylphosphonium halide, dodecyltrimethylammonium halides, tetradecyltrimethylammonium halides, cetylpyridinium chloride, cetyltrimethylammonium chloride, cetylbenzyldimethylammonium chloride, cetylpyridinium bromide, cetyltrimethylammonium bromide, cetyldimethylethylammonium bromide, cetyltributylphosphonium bromide, dodecyltrimethylammonium bromide, tetradecyltrimethylammonium bromide, and the like.

In certain embodiments the emulsion comprises a quaternary ammonium compound. Quaternary ammonium compounds include, but are not limited to, N-alkyldimethyl benzyl ammonium saccharinate, 1,3,5-triazine-1,3,5(2H,4H,6H)-triethanol; 1-decanaminium, N-decyl-N,N-dimethyl-, chloride (or) Didecyl dimethyl ammonium chloride; 2-(2-(p-(diisobuyl)cresosxy)ethoxy)ethyl dimethyl benzyl ammonium chloride; 2-(2-(p-(diisobutyl)phenoxy)ethoxy)ethyl dimethyl benzyl ammonium chloride; alkyl 1 or 3 benzyl-1-(2-hydroxethyl)-2-imidazolinium chloride; alkyl bis(2-hydroxyethyl)benzyl ammonium chloride; alkyl dimethyl benzyl ammonium chloride; alkyl dimethyl 3,4-dichlorobenzyl ammonium chloride (100% C12); alkyl dimethyl 3,4-dichlorobenzyl ammonium chloride (50% C14, 40% C12, 10% C16); alkyl dimethyl 3,4-dichlorobenzyl ammonium chloride (55% C14, 23% C12, 20% C16); alkyl dimethyl benzyl ammonium chloride; alkyl dimethyl benzyl ammonium chloride (100% C14); alkyl dimethyl benzyl ammonium chloride (100% C16); alkyl dimethyl benzyl ammonium chloride (41% C14, 28% C12); alkyl dimethyl benzyl ammonium chloride (47% C12, 18% C14); alkyl dimethyl benzyl ammonium chloride (55% C16, 20% C14); alkyl dimethyl benzyl ammonium chloride (58% C14, 28% C16); alkyl dimethyl benzyl ammonium chloride (60% C14, 25% C12); alkyl dimethyl benzyl ammonium chloride (61% C11, 23% C14); alkyl dimethyl benzyl ammonium chloride (61% C12, 23% C14); alkyl dimethyl benzyl ammonium chloride (65% C12, 25% C14); alkyl dimethyl benzyl ammonium chloride (67% C12, 24% C14); alkyl dimethyl benzyl ammonium chloride (67% C12, 25% C14); alkyl dimethyl benzyl ammonium chloride (90% C14, 5% C12); alkyl dimethyl benzyl ammonium chloride (93% C14, 4% C12); alkyl dimethyl benzyl ammonium chloride (95% C16, 5% C18); alkyl dimethyl benzyl ammonium chloride (and) didecyl dimethyl ammonium chloride; alkyl dimethyl benzyl ammonium chloride (as in fatty acids); alkyl dimethyl benzyl ammonium chloride (C12-C16); alkyl dimethyl benzyl ammonium chloride (C12-C18); alkyl dimethyl benzyl and dialkyl dimethyl ammonium chloride; alkyl dimethyl dimethybenzyl ammonium chloride; alkyl dimethyl ethyl ammonium bromide (90% C14, 5% C16, 5% C12); alkyl dimethyl ethyl ammonium bromide (mixed alkyl and alkenyl groups as in the fatty acids of soybean oil); alkyl dimethyl ethylbenzyl ammonium chloride; alkyl dimethyl ethylbenzyl ammonium chloride (60% C14); alkyl dimethyl isoproylbenzyl ammonium chloride (50% C12, 30% C14, 17% C16, 3% C18); alkyl trimethyl ammonium chloride (58% C18, 40% C16, 1% C14, 1% C12); alkyl trimethyl ammonium chloride (90% C18, 10% C16); alkyldimethyl(ethylbenzyl) ammonium chloride (C12-18); Di-(C8-10)-alkyl dimethyl ammonium chlorides; dialkyl dimethyl ammonium chloride; dialkyl dimethyl ammonium chloride; dialkyl dimethyl ammonium chloride; dialkyl methyl benzyl ammonium chloride; didecyl dimethyl ammonium chloride; diisodecyl dimethyl ammonium chloride; dioctyl dimethyl ammonium chloride; dodecyl bis(2-hydroxyethyl) octyl hydrogen ammonium chloride; dodecyl dimethyl benzyl ammonium chloride; dodecylcarbamoyl methyl dimethyl benzyl ammonium chloride; heptadecyl hydroxyethylimidazolinium chloride; hexahydro-1,3,5-thris(2-hydroxyethyl)-s-triazine; myristalkonium chloride (and) Quaternium 14; N,N-dimethyl-2-hydroxypropylammonium chloride polymer; n-alkyl dimethyl benzyl ammonium chloride; n-alkyl dimethyl ethylbenzyl ammonium chloride; n-tetradecyl dimethyl benzyl ammonium chloride monohydrate; octyl decyl dimethyl ammonium chloride; octyl dodecyl dimethyl ammonium chloride; octyphenoxyethoxyethyl dimethyl benzyl ammonium chloride; oxydiethylenebis (alkyl dimethyl ammonium chloride); quaternary ammonium compounds, dicoco alkyldimethyl, chloride; trimethoxysily propyl dimethyl octadecyl ammonium chloride; trimethoxysilyl quats, trimethyl dodecylbenzyl ammonium chloride; n-dodecyl dimethyl ethylbenzyl ammonium chloride; n-hexadecyl dimethyl benzyl ammonium chloride; n-tetradecyl dimethyl benzyl ammonium chloride; n-tetradecyl dimethyl ethylbenzyl ammonium chloride; and n-octadecyl dimethyl benzyl ammonium chloride.

Nanoemulsion formulations and methods of making such are well known to those of skill in the art and described for example in U.S. Patent Nos: 7,476,393, 7,468,402, 7,314,624, 6,998,426, 6,902,737, 6,689,371, 6,541,018, 6,464,990, 6,461,625, 6,419,946, 6,413,527, 6,375,960, 6,335,022, 6,274,150, 6,120,778, 6,039,936, 5,925,341, 5,753,241, 5,698,219, an d5,152,923 and in Fanun et al. (2009) Microemulsions: Properties and Applications (Surfactant Science), CRC Press, Boca Raton FL.

In certain embodiments, one or more active agents described herein can be provided as a "concentrate", e.g., in a storage container (e.g., in a premeasured volume) ready for dilution, or in a soluble capsule ready for addition to a volume of water, alcohol, hydrogen peroxide, or other diluent.

In certain embodiments, the compounds of Formula (I) or Formula (III) described herein, are formulated as inclusion complexes. While not limited to cyclodextrin inclusion complexes, it is noted that cyclodextrin is the agent most frequently used to form pharmaceutical inclusion complexes. Cyclodextrins (CD) are cyclic oligomers of glucose, that typically contain 6, 7, or 8 glucose monomers joined by α-1,4 linkages. These oligomers are commonly called α-CD, β-CD, and γ-CD, respectively. Higher oligomers containing up to 12 glucose monomers are known and contemplated to in the formulations described herein. Functionalized cyclodextrin inclusion complexes are also contemplated. Illustrative, but non-limiting functionalized cyclodextrins include, but are not limited to sulfonates, sulfonates and sulfinates, or disulfonates of hydroxybutenyl cyclodextrin; sulfonates, sulfonates and sulfinates, or disulfonates of mixed ethers of cyclodextrins where at least one of the ether substituents is hydroxybutenyl cyclodextrin. Illustrative cyclodextrins include a polysaccharide ether which comprises at least one 2-hydroxybutenyl substituent, wherein the at least one hydroxybutenyl substituent is sulfonated and sulfinated, or disulfonated, and an alkylpolyglycoside ether which comprises at least one 2-hydroxybutenyl substituent, wherein the at least one hydroxybutenyl substituent is sulfonated and sulfinated, or disulfonated. In various embodiments inclusion complexes formed between sulfonated hydroxybutenyl cyclodextrins and one or more of the active agent(s) described herein are contemplated. Methods of preparing cyclodextrins, and cyclodextrin inclusion complexes are found for example in U.S. Patent Publication No: 2004/0054164 and the references cited therein and in U.S. Patent Publication No: 2011/0218173 and the references cited therein.

In certain embodiments the compounds of Formula (I) or Formula (III) described herein can also be administered using medical devices known in the art. For example, in one embodiment, a pharmaceutical composition of the invention can be administered with a needleless hypodermic injection device, such as the devices disclosed in U.S. Pat. No. 5,399,163; U.S. Pat. No. 5,383,851; U.S. Pat. No. 5,312,335; U.S. Pat. No. 5,064,413; U.S. Pat. No. 4,941,880; U.S. Pat. No. 4,790,824; or U.S. Pat. No. 4,596,556. Examples of well-known implants and modules useful for such deliver include, but are not limited to U.S. Pat. No. 4,487,603, which discloses an implantable micro-infusion pump for dispensing medication at a controlled rate; U.S. Pat. No. 4,486,194, which discloses a therapeutic device for administering medicaments through the skin; U.S. Pat. No. 4,447,233, which discloses a medication infusion pump for delivering medication at a precise infusion rate; U.S. Pat. No. 4,447,224, which discloses a variable flow implantable infusion apparatus for continuous drug delivery; U.S. Pat. No. 4,439,196, which discloses an osmotic drug delivery system having multi-chamber compartments; and U.S. Pat. No. 4,475,196, which discloses an osmotic drug delivery system. In a specific embodiment a compound of Formula (I) or Formula (III) may be administered using a drug-eluting stent, for example one corresponding to those described in WO 01/87263 and related publications or those described by Perin (Perin, E.C., 2005). Many other such implants, delivery systems, and modules are known to those skilled in the art.

The dosage to be administered of a compound of Formula (I) or Formula (III) as described herein will vary according to the particular compound, the disease involved, the subject, and the nature and severity of the disease and the physical condition of the subject, and the selected route of administration. The appropriate dosage can be readily determined by a person skilled in the art.

In certain embodiments the compositions may contain from 0.1%, e.g., from 0.1-70%, or from 5-60%, or from 10-30%, of one or more compounds of Formula (I) or Formula (III) depending on the method of administration.

It will be recognized by one of skill in the art that the optimal quantity and spacing of individual dosages of a compound of Formula (I) or Formula (III) as described herein will be determined by the nature and extent of the condition being treated, the form, route and site of administration, and the age and condition of the particular subject being treated, and that a physician will ultimately determine appropriate dosages to be used. This dosage may be repeated as often as appropriate. If side effects develop the amount and/or frequency of the dosage can be altered or reduced, in accordance with normal clinical practice.

In some embodiments, pharmaceutically acceptable salts may also include both acid and base addition salts. In some embodiments, "Pharmaceutically acceptable acid addition salt" may refer to those salts which retain the biological effectiveness and properties of the free bases, which are not biologically or otherwise undesirable, and which may be formed with inorganic acids such as, but are not limited to, hydrochloric acid, hydrobromic acid, sulfuric acid, nitric acid, phosphoric acid and the like, and organic acids such as, but not limited to, acetic acid, 2,2-dichloroacetic acid, adipic acid, alginic acid, ascorbic acid, aspartic acid, benzenesulfonic acid, benzoic acid, 4-acetamidobenzoic acid, camphoric acid, camphor-10-sulfonic acid, capric acid, caproic acid, caprylic acid, carbonic acid, cinnamic acid, citric acid, cyclamic acid, dodecylsulfuric acid, ethane-1,2-disulfonic acid, ethanesulfonic acid, 2-hydroxyethanesulfonic acid, formic acid, fumaric acid, galactaric acid, gentisic acid, glucoheptonic acid, gluconic acid, glucuronic acid, glutamic acid, glutaric acid, 2-oxo-glutaric acid, glycerophosphoric acid, glycolic acid, hippuric acid, isobutyric acid, lactic acid, lactobionic acid, lauric acid, maleic acid, malic acid, malonic acid, mandelic acid, methanesulfonic acid, mucic acid, naphthalene-1,5-disulfonic acid, naphthalene-2-sulfonic acid, 1-hydroxy-2-naphthoic acid, nicotinic acid, oleic acid, orotic acid, oxalic acid, palmitic acid, pamoic acid, propionic acid, pyroglutamic acid, pyruvic acid, salicylic acid, 4-aminosalicylic acid, sebacic acid, stearic acid, succinic acid, tartaric acid, thiocyanic acid, p-toluenesulfonic acid, trifluoroacetic acid, undecylenic acid, and the like.

In some embodiments, "pharmaceutically acceptable base addition salt" may refer to those salts that retain the biological effectiveness and properties of the free acids, which are not biologically or otherwise undesirable. These salts may be prepared from addition of an inorganic base or an organic base to the free acid. Salts derived from inorganic bases may include, but are not limited to, the sodium, potassium, lithium, ammonium, calcium, magnesium, iron, zinc, copper, manganese, aluminum salts and the like. For example, inorganic salts may include, but are not limited to, ammonium, sodium, potassium, calcium, and magnesium salts. Salts derived from organic bases may include, but are not limited to, salts of primary, secondary, and tertiary amines, substituted amines including naturally occurring substituted amines, cyclic amines and basic ion exchange resins, such as ammonia, isopropylamine, trimethylamine, diethylamine, triethylamine, tripropylamine, diethanolamine, ethanolamine, deanol, 2-dimethylaminoethanol, 2-diethylaminoethanol, dicyclohexylamine, lysine, arginine, histidine, caffeine, procaine, hydrabamine, choline, betaine, benethamine, benzathine, ethylenediamine, glucosamine, methylglucamine, theobromine, triethanolamine, tromethamine, purines, piperazine, piperidine, N-ethylpiperidine, polyamine resins and the like.

For the purposes of administration, the compounds of the present disclosure may be administered as a raw chemical or may be formulated as pharmaceutical compositions. Pharmaceutical compositions of the present disclosure comprise one or more compounds of Formula (I) or Formula (III), or pharmaceutically acceptable salts, solvates, stereoisomers, or tautomers of any of these, and a pharmaceutically acceptable carrier. The one or more compounds of Formula (I) or Formula (III), or pharmaceutically acceptable salts, solvates, stereoisomers, or tautomers of any of these, are present in the composition in an amount that is effective to treat a particular disease or condition of interest - for example, in an amount sufficient to treat a bacterial infection, and generally with acceptable toxicity to the subject. In some embodiments, the one or more compounds of Formula (I) or Formula (III), or pharmaceutically acceptable salts, solvates, stereoisomers, or tautomers of any of these, are present in the composition in an amount that is effective to treat a particular disease or condition of interest. The antibacterial activity of compounds of Formula (I) or Formula (III), or pharmaceutically acceptable salts, solvates, stereoisomers, or tautomers of any of these, can be determined by one skilled in the art, for example, as described in the Examples herein. Appropriate concentrations and dosages can be readily determined by one skilled in the art.

Administration of one or more compounds of the disclosure, such as compounds of Formula (I) or Formula (III), or pharmaceutically acceptable salts, solvates, stereoisomers, or tautomers of any of these, in pure form or in an appropriate pharmaceutical composition can be carried out via any of the accepted modes of administration of agents for serving similar utilities. In some embodiments, the one or more compounds of the disclosure administered are compounds of Formula (I) or Formula (III), or pharmaceutically acceptable salts, solvates, stereoisomers, or tautomers of any of the foregoing, in pure form or in an appropriate pharmaceutical composition.

The pharmaceutical compositions of the disclosure can be prepared by combining one or more compounds of the disclosure, or pharmaceutically acceptable salts, solvates, stereoisomers, or tautomers thereof, with an appropriate pharmaceutically acceptable carrier, and may be formulated into preparations in solid, semi solid, liquid or gaseous forms, such as tablets, capsules, powders, granules, ointments, solutions, suppositories, injections, inhalants, gels, microspheres, and aerosols. Typical routes of administering such pharmaceutical compositions may include, without limitation, oral, topical, transdermal, inhalation, parenteral, sublingual, buccal, rectal, vaginal, and intranasal. The term parenteral as used herein may include subcutaneous injections, intravenous, intramuscular, intrasternal injection or infusion techniques. Pharmaceutical compositions of the disclosure can be formulated so as to allow the active ingredients contained therein to be bioavailable upon administration of the composition to a subject. Compositions that will be administered to a subject or patient take the form of one or more dosage units, where for example, a tablet may be a single dosage unit, and a container of one or more compounds of the disclosure, or pharmaceutically acceptable salts, solvates, stereoisomers, or tautomers thereof, in aerosol form may hold a plurality of dosage units. Actual methods of preparing such dosage forms are known, or will be apparent, to those skilled in this art; for example, see Remington: The Science and Practice of Pharmacy, 20th Edition (Philadelphia College of Pharmacy and Science, 2000).

A pharmaceutical composition of the disclosure may be in the form of a solid or liquid. In one aspect, the carrier(s) are particulate, so that the compositions are, for example, in tablet or powder form. The carrier(s) may be liquid, with the compositions being, for example, an oral syrup, injectable liquid or an aerosol, which may be useful in, for example, inhalatory administration.

When intended for oral administration, pharmaceutical compositions of the present disclosure typically are either solid or liquid form, where semi solid, semi liquid, suspension and gel forms may be included within the forms considered herein as either solid or liquid.

As a solid composition for oral administration, the pharmaceutical compositions may be formulated into a powder, granule, compressed tablet, pill, capsule, chewing gum, wafer or the like form. Such a solid composition will typically contain one or more inert diluents or edible carriers. In addition, one or more of the following may be present: binders such as carboxymethylcellulose, ethyl cellulose, microcrystalline cellulose, gum tragacanth or gelatin; excipients such as starch, lactose or dextrins; disintegrating agents such as alginic acid, sodium alginate, Primojel^{®}, corn starch and the like; lubricants such as magnesium stearate or Sterotex^{®}; glidants such as colloidal silicon dioxide; sweetening agents such as sucrose or saccharin; a flavoring agent such as peppermint, methyl salicylate or orange flavoring; and a coloring agent.

When the pharmaceutical composition is in the form of a capsule, for example, a gelatin capsule, it may contain, in addition to materials disclosed herein, a liquid carrier such as polyethylene glycol or oil.

Pharmaceutical compositions of the disclosure may be in the form of a liquid, for example, an elixir, syrup, solution, emulsion or suspension. The liquid may be for oral administration or for delivery by injection, as two examples. When intended for oral administration, pharmaceutical compositions of the disclosure typically contain, in addition to one or more compounds of the disclosure, or pharmaceutically acceptable salts, solvates, stereoisomers, or tautomers thereof, one or more of a sweetening agent, preservatives, dye/colorant and flavor enhancer. In a composition intended to be administered by injection, one or more of a surfactant, preservative, wetting agent, dispersing agent, suspending agent, buffer, stabilizer and isotonic agent may be included.

Liquid pharmaceutical compositions of the disclosure, whether they be solutions, suspensions or other like form, may include one or more of the following adjuvants: sterile diluents such as water for injection, saline solution, physiological saline, Ringer's solution, isotonic sodium chloride, fixed oils such as synthetic mono or diglycerides which may serve as the solvent or suspending medium, polyethylene glycols, glycerin, propylene glycol or other solvents; antibacterial agents such as benzyl alcohol or methyl paraben; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. Parenteral preparations can be enclosed in ampoules, disposable syringes or multiple dose vials made of glass or plastic. In some embodiments, the adjuvant is physiological saline. In some embodiments, the injectable pharmaceutical composition is sterile.

A liquid pharmaceutical composition of the disclosure intended for either parenteral or oral administration should contain an amount of one or more compounds of the disclosure, or pharmaceutically acceptable salts, solvates, stereoisomers, or tautomers thereof, such that a suitable dosage will be obtained.

Pharmaceutical compositions of the disclosure may be intended for topical administration, in which case the carrier may suitably comprise a solution, emulsion, ointment or gel base. The base, for example, may comprise one or more of the following: petrolatum, lanolin, polyethylene glycols, bee wax, mineral oil, diluents such as water and alcohol, and emulsifiers and stabilizers. Thickening agents may be present in a pharmaceutical composition for topical administration. If intended for transdermal administration, the composition may include a transdermal patch or iontophoresis device.

Pharmaceutical compositions of the disclosure may be intended for rectal administration, in the form, for example, of a suppository, which will melt in the rectum and release the drug. Compositions for rectal administration may contain an oleaginous base as a suitable nonirritating excipient. Such bases may include, without limitation, lanolin, cocoa butter and polyethylene glycol.

Pharmaceutical compositions of the disclosure may include various materials, which modify the physical form of a solid or liquid dosage unit. For example, the compositions may include materials that form a coating shell around the active ingredients. The materials that form the coating shell are typically inert, and may be selected from, for example, sugar, shellac, and other enteric coating agents. Alternatively, the active ingredients may be encased in a gelatin capsule.

Pharmaceutical compositions of the disclosure in solid or liquid form may include an agent that binds to the one or more compounds of the disclosure, or pharmaceutically acceptable salts, solvates, stereoisomers, or tautomers thereof, and thereby assists in the delivery of the one or more compounds, or pharmaceutically acceptable salts, solvates, stereoisomers, or tautomers thereof. Suitable agents that may act in this capacity may include a monoclonal or polyclonal antibody, a protein or a liposome.

Pharmaceutical compositions of the disclosure may be prepared in dosage units that can be administered as an aerosol. The term aerosol is used to denote a variety of systems ranging from those of colloidal nature to systems consisting of pressurized packages. Delivery may be by a liquefied or compressed gas or by a suitable pump system that dispenses the active ingredients. Aerosols of one or more compounds of the disclosure, or pharmaceutically acceptable salts, solvates, stereoisomers, or tautomers thereof, may be delivered in single phase, bi phasic, or tri phasic systems in order to deliver the active ingredient(s). Delivery of the aerosol may include the necessary container, activators, valves, subcontainers, and the like, which together may form a kit. One skilled in the art, without undue experimentation may determine preferred aerosols.

The pharmaceutical compositions of the disclosure may be prepared by methodology well known in the pharmaceutical art. For example, a pharmaceutical composition intended to be administered by injection can be prepared by combining one or more compounds of the disclosure, or pharmaceutically acceptable salts, solvates, stereoisomers, or tautomers thereof, with sterile, distilled water to form a solution. A surfactant may be added to facilitate the formation of a homogeneous solution or suspension. Surfactants are compounds that non-covalently interact with the one or more compounds of the disclosure, or pharmaceutically acceptable salts, solvates, stereoisomers, or tautomers thereof, to facilitate dissolution or homogeneous suspension of the one or more compounds or the disclosure, or pharmaceutically acceptable salts, solvates, stereoisomers, or tautomers thereof, in the aqueous delivery system.

**In** some embodiments, pharmaceutical compositions of the disclosure may be enriched to provide predominantly one enantiomer of a compound described herein. An enantiomerically enriched mixture may comprise, for example, at least 60 mol percent of one enantiomer, or at least 75, at least 80, at least 85, at least 90, at least 95, at least 96, at least 97, at least 98, at least 99, at least 99.5 or even 100 mol percent. In some embodiments, the compositions described herein enriched in one enantiomer may be substantially free of the other enantiomer, wherein substantially free may mean that the substance in question makes up less than 10%, or less than 5%, or less than 4%, or less than 3%, or less than 2%, or less than 1% as compared to the amount of the other enantiomer, e.g., in the pharmaceutical composition or compound mixture. For example, if a pharmaceutical composition or compound mixture contains 98 grams of a first enantiomer and 2 grams of a second enantiomer, it would be said to contain 98 mol percent of the first enantiomer and only 2 mol percent of the second enantiomer.

In some embodiments, the pharmaceutical compositions of the disclosure may be enriched to provide predominantly one diastereomer of a compound disclosed herein. A diastereomerically enriched mixture may comprise, for example, at least 60 mol percent of one diastereomer, or at least 75, at least 80, at least 85, at least 90, at least 95, at least 96, at least 97, at least 98, at least 99, at least 99.5, or even 100 mol percent. In some embodiments, the compositions described herein enriched in one diastereomer may be substantially free of other diastereomers, wherein substantially free may mean that the substance in question makes up less than 10%, or less than 5%, or less than 4%, or less than 3%, or less than 2%, or less than 1% as compared to the amount of other diastereomers, e.g., in the pharmaceutical composition or compound mixture.

Diastereomeric mixtures can be separated into their individual diastereomers on the basis of their physical chemical differences by methods well known to those skilled in the art, such as, for example, by chromatography and/or fractional crystallization. Enantiomers may be separated by converting the enantiomeric mixture into a diastereomeric mixture by reaction with an appropriate optically active compound (e.g., chiral auxiliary such as a chiral alcohol or Mosher's acid chloride), separating the diastereomers and converting (e.g., hydrolyzing) the individual diastereomers to the corresponding pure enantiomers. Enantiomers can also be separated by use of a chiral HPLC column. Also, some of the compounds of the disclosure may be atropisomers or rotameric forms and are considered as part of this disclosure.

### Therapeutic Applications

The compounds, compositions, and methods of the present disclosure can be useful for a plurality of different subjects including, but not limited to, a mammal, a human, a non-human mammal, a domesticated animal (e.g., laboratory animals, household pets, or livestock), a non-domesticated animal (e.g., wildlife), a dog, a cat, a rodent, a mouse, a hamster, a cow, a bird, a chicken, a fish, a pig, a horse, a goat, a sheep, a rabbit, or any combination thereof.

The compounds and compositions described herein can be useful as therapeutics, for example, a treatment that can be administered to a subject in need thereof. A therapeutic effect of the present disclosure can be obtained in a subject by reduction, suppression, remission, or eradication of a disease state, including, but not limited to, a symptom thereof. A therapeutic effect in a subject having a disease or condition, or pre-disposed to have or is beginning to have the disease or condition, can be obtained by a reduction, a suppression, a prevention, a remission, or an eradication of the condition or disease, or precondition or pre-disease state.

In practicing the methods described herein, therapeutically effective amounts of the compositions described herein can be administered to a subject in need thereof, often for treating and/or preventing a condition or progression thereof. A pharmaceutical composition can affect the physiology of the subject, such as the immune system, an inflammatory response, or other physiologic affect. A therapeutically effective amount can vary depending on the severity of the disease, the age and relative health of the subject, the potency of the compounds used, and other factors.

Treat and/or treating can refer to any indicia of success in the treatment or amelioration of the disease or condition. Treating can include, for example, reducing, delaying or alleviating the severity of one or more symptoms of the disease or condition, or it can include reducing the frequency with which symptoms of a disease, defect, disorder, or adverse condition, and the like, are experienced by a patient. Treat can be used herein to refer to a method that results in some level of treatment or amelioration of the disease or condition and can contemplate a range of results directed to that end, including but not restricted to prevention of the condition entirely.

Prevent, preventing and the like can refer to the prevention of the disease or condition, e.g., tumor formation, in the patient. For example, if an individual at risk of developing a tumor or other form of cancer is treated with the methods of the present disclosure and does not later develop the tumor or other form of cancer, then the disease has been prevented, at least over a period of time, in that individual. Preventing can also refer to preventing re-occurrence of a disease or condition in a patient that has previously been treated for the disease or condition, e.g., by preventing relapse.

A therapeutically effective amount can be the amount of a composition or an active component thereof sufficient to provide a beneficial effect or to otherwise reduce a detrimental non-beneficial event to the individual to whom the composition is administered. A therapeutically effective dose can be a dose that produces one or more desired or desirable (e.g., beneficial) effects for which it is administered, such administration occurring one or more times over a given period of time. An exact dose can depend on the purpose of the treatment and can be ascertainable by one skilled in the art using known techniques.

### Methods of Treating Bacterial Infection

This disclosure describes methods of and uses for treating a bacterial infection comprising administering to a subject in need thereof a therapeutically effective amount of one or more compounds of the disclosure, or pharmaceutically acceptable salts, solvates, stereoisomers, or tautomers thereof, or a therapeutically effective amount of a pharmaceutical composition comprising one or more compounds of the disclosure, or pharmaceutically acceptable salts, solvates, stereoisomers, or tautomers thereof.

Compounds, or pharmaceutically acceptable salts, solvates, stereoisomers, or tautomers thereof, and pharmaceutical compositions of the present disclosure (e.g., compounds of Formula (I) or Formula (III)) may possess antibacterial activity against a wide spectrum of gram-positive and gram-negative bacteria. Representative susceptible organisms may include those gram-positive, gram-negative, aerobic, and facultative anaerobic organisms whose growth can be inhibited by the compounds of the disclosure, including, but not limited to, *Staphylococcus, Lactobacillus, Streptococcus, Sarcina, Escherichia, Enterobacter, Klebsiella, Pseudomonas, Acinetobacter, Mycobacterium, Proteus, Campylobacter, Citrobacter, Neisseria, Bacillus, Peptococcus, Salmonella, Shigella, Serratia, Haemophilus, Brucella, Francisella, Yersinia, Corynebacterium, Moraxella, Enterococcus, Burkholderia* and other organisms.

Accordingly, compounds, or pharmaceutically acceptable salts, solvates, stereoisomers, or tautomers thereof, and pharmaceutical compositions comprising the compounds or pharmaceutically acceptable salts, solvates, stereoisomers, or tautomers thereof, as disclosed herein can be used, for example, for treating bacterial infections and for treating conditions caused by the bacterial infection. Bacterial infections susceptible to treatment according to the present disclosure may include primary infections and co-infections caused by a species of bacteria and one or more additional infectious agents such as, for example, bacteria, virus, parasite and fungi.

**In** one aspect is provided a the compound of the first aspect of the invention, or a pharmaceutically acceptable salt, solvate, tautomer, or stereoisomer thereof, or the pharmaceutical composition of the second aspect of the invention, for use in treating a bacterial infection in a subject in need thereof..

**In** some embodiments, the bacterial infection is an infection with a *Staphylococcus sp., Lactobacillus sp., Streptococcus sp., Sarcina sp., Escherichia sp., Enterobacter sp., Klebsiella sp., Pseudomonas sp., Acinetobacter sp., Mycobacterium sp., Proteus sp., Campylobacter sp., Citrobacter sp., Neisseria sp., Bacillus sp., Peptococcus sp., Salmonella sp., Shigella sp., Serratia sp., Haemophilus sp., Brucella sp., Francisella sp., Yersinia sp., Corynebacterium sp., Moraxella sp., Burkholderia sp.,* or *Enterococcus sp.*

**In** some embodiments, the bacterial infection is with Gram-positive, Gram-negative, aerobic, or facultative anaerobic bacteria.

**In** some embodiments, the bacterial infection is with Gram-positive bacteria. Examples of Gram-positive bacteria include, but are not limited to, *Staphylococcus sp., Lactobacillus sp., Streptococcus sp., Sarcina sp., Bacillus sp., Peptococcus sp., Corynebacterium sp.*, and *Enterococcus sp.* In some embodiments, the Gram-positive bacterial infection is an infection with a *Staphylococcus sp., Lactobacillus sp., Streptococcus sp., Sarcina sp., Bacillus sp., Peptococcus sp., Corynebacterium sp.*, or *Enterococcus sp.* In some embodiments, the bacterial infection is caused by *Staphylococcus aureus.* In some embodiments, the bacterial infection is caused by Methicillin-resistant *Staphylococcus aureus* (MRSA).

**In** some embodiments, the bacterial infection is with Gram-negative bacteria. Examples of Gram-negative bacteria include, but are not limited to, *Escherichia sp., Enterobacter sp., Klebsiella sp., Pseudomonas sp., Acinetobacter sp., Proteus sp., Campylobacter sp., Citrobacter sp., Neisseria sp., Salmonella sp., Shigella sp., Serratia sp., Haemophilus sp., Brucella sp., Francisella sp., Yersinia sp., Burkholderia sp.*, and *Moraxella sp.*

In some embodiments, the bacterial infection is caused by fermentative or non-fermentative Gram-negative bacteria. Examples of fermentative or non-fermentative Gram-negative bacteria include, but are not limited to, *Pseudomonas aeruginosa; Stenotrophomonas maltophila; Burkholderia cepacia; Alcaligenes xylosoxidans; Enterobacteriaceae; Haemophilus; Franciscellaceae (e.g., Francisella tularensis); Neisseria* species; and *Enterobacteriaceae*, such as *Serratia, Proteus, Klebsiella, Enterobacter, Citrobacter, Salmonella, Providencia, Yersinia (e.g., Yersinia pestis), Morganella, Cedecea, Edwardsiella species, Acinetobacter*, and *Escherichia coli.*

In some embodiments, the Gram-negative bacterial infection is an infection with an *Escherichia sp., Enterobacter sp., Klebsiella sp., Pseudomonas sp., Acinetobacter sp., Proteus sp., Campylobacter sp., Citrobacter sp., Neisseria sp., Salmonella sp., Shigella sp., Serratia sp., Haemophilus sp., Brucella sp., Francisella sp., Yersinia sp., Burkholderia sp.*, or *Moraxella sp.* In some embodiments, the Gram-negative bacterial infection is an infection with an *Enterobacteriaceae, Haemophilus, Franciscellaceae,* or *Neisseria* species. In some embodiments, the bacterial infection is caused by an *Enterobacteriaceae.* In some embodiments, the bacterial infection is caused by an extended spectrum beta-lactamase (ESBL)-producing or carbapenem-resistant *Enterobacteriaceae.*

In some embodiments, the Gram-negative bacterial infection is an infection with *Escherichia coli, Klebsiella pneumoniae, Klebsiella oxytoca, Enterobacter cloacae, Enterobacter aerogenes, Citrobacter freundii, Citrobacter koseri, Proteus mirabilis, Bacillus anthracis, P. aeruginosa, Acinetobacter baumannii, Proteus vulgaris, Francisella tularensis, Burkholderia cepacia, Burkholderia mallei, Burkholderia pseudomallei* or *Yersinia pestis.* In some embodiments, the Gram-negative bacterial infection is an infection with *Pseudomonas aeruginosa, Stenotrophomonas maltophila, Burkholderia cepacia*, or *Alcaligenes xylosoxidans*,

In some embodiments, the bacterial infection is caused by multidrug resistant (MDR) bacteria. In some embodiments, the bacterial infection is caused by a multidrug-resistant biothreat pathogen, including those defined as such by the United States Centers for Disease Control and Prevention's Category A/B/C bioterrorism agent designation. In certain such embodiments, the biothreat pathogen is Gram-negative bacteria.

In some embodiments, one or more compounds of Formula (I) or Formula (III), or pharmaceutically acceptable salts, solvates, stereoisomers, or tautomers thereof, or pharmaceutical compositions of the disclosure may be useful in treating diseases or conditions caused by a bacterial infection as disclosed herein, including, but not limited to, skin infections; pneumonia; nosocomial pneumonia; sepsis; respiratory tract infections (e.g., lower respiratory tract or upper respiratory tract infections); lung infection in cystic fibrosis patients; acute exacerbation of chronic bronchitis; community acquired pneumonia; nosocomial pneumonia (including ventilator-associated pneumonia (VAP)); hospital or community acquired infections caused by Gram-negative bacteria; diseases of the upper airways; diffuse panbronchiolitis; tonsillitis; pharyngitis; acute sinusitis and otitis including mastoiditis; urinary tract and genital infections, for example, complicated urinary tract infections, cystitis, resistant or relapsing cystitis, urethritis, pyelonephritis, acute pyelonephritis, endometritis, prostatitis, salpingitis, and epididymitis; ocular infections such as conjunctivitis, corneal ulcer, iridocyclitis and post-operative infection in radial keratotomy surgery patients; blood infections, for example septicemia or bacteremia; infections of the skin and soft tissues, for example infective dermatitis, infected wounds, infected burns, phlegmon, folliculitis and impetigo; bone and joint infections such as osteomyelitis and septic arthritis; gastrointestinal infections, for example dysentery, enteritis, colitis, necrotizing enterocolitis and anorectal infections; intraabdominal infections, including, but not limited to, complicated intraabdominal infections, typhoid fever, infectious diarrhea, peritonitis with appendicitis, pelviperitonitis, and intra-abdominal abscesses; infections in the oral region, for example infections after dental operations; other infections, for example, melioidosis, infectious endocarditis, sexually transmitted diseases caused by Gram-negative or Gram-positive bacteria, hepatic abscesses, cholecystitis, cholangitis, mastitis as well as meningitis, and infections of the nervous systems.

In some embodiments, the compounds of the invention are for use in the treatment of infections selected from the group consisting of complicated intraabdominal infections, complicated urinary tract infections, resistant or relapsing cystitis, acute pyelonephritis, and bacteremia.

In a further aspect, the disclosure provides for use of one or more compounds of Formula (I) or Formula (III), or pharmaceutically acceptable salts, solvates, stereoisomers, or tautomers thereof, to treat a bacterial infection as described herein in a subject in need thereof.

In still a further aspect, provided herein is one or more compounds of Formula (I) or Formula (III), or pharmaceutically acceptable salts, solvates, stereoisomers, or tautomers thereof, for use in a method of treating a bacterial infection in a subject in need thereof.

In another aspect, the disclosure provides for one or more compounds of Formula (I) or Formula (III), or pharmaceutically acceptable salts, solvates, stereoisomers, or tautomers of any of the foregoing, for use as a medicament.

In yet a further aspect, provided herein is the use of one or more compounds of Formula (I) or Formula (III), or pharmaceutically acceptable salts, solvates, stereoisomers, or tautomers thereof, in the manufacture of a medicament for treating a bacterial infection in a subject in need thereof.

In still a further aspect, provided herein is one or more pharmaceutical compositions of the disclosure for use in a method of treating a bacterial infection in a subject in need thereof.

In a still further aspect, provided herein is the use of one or more pharmaceutical compositions of the disclosure in the manufacture of a medicament for treating a bacterial infection in a subject in need thereof.

In any of the foregoing methods and uses as disclosed herein, the subject may be a mammal. In some embodiments, the subject is a mammal. In some embodiments, the mammal is a human.

### Administration

One or more compounds of Formula (I) or Formula (III), or pharmaceutically acceptable salts, solvates, stereoisomers, or tautomers of any of these, or compositions of any thereof, may be administered in a therapeutically effective amount, which will vary depending upon a variety of factors including: the activity of the specific compound employed; the metabolic stability and length of action of the compound; the age, body weight, general health, sex, and diet of the subject; the mode and time of administration; the rate of excretion; the drug combination; the severity of the particular disorder or condition; and the subject undergoing therapy.

Administration of one or more compounds of the disclosure, such as compounds of Formula (I) or Formula (III), or pharmaceutically acceptable salts, solvates, stereoisomers, or tautomers of any of these, in pure form or in an appropriate pharmaceutical composition can be carried out via any of the accepted modes of administration of agents for serving similar utilities. In some embodiments, the one or more compounds of the disclosure administered are compounds of Formula (I) or Formula (III), or pharmaceutically acceptable salts, solvates, stereoisomers, or tautomers of any of the foregoing, in pure form or in an appropriate pharmaceutical composition.

Pharmaceutical compositions can be used in the methods described herein and can be administered to a subject in need thereof using a technique known to one of ordinary skill in the art which can be suitable as a therapy for the disease or condition affecting the subject. One of ordinary skill in the art would understand that the amount, duration and frequency of administration of a pharmaceutical composition described herein to a subject in need thereof depends on several factors including, for example but not limited to, the health of the subject, the specific disease or condition of the patient, the grade or level of a specific disease or condition of the patient, the additional therapeutics the subject is being or has been administered, and the like.

The methods and compositions described herein can be for administration to a subject in need thereof. Often, administration of the compositions described herein can include routes of administration, non-limiting examples of administration routes include oral, intravenous, intraarterial, subcutaneous, subdural, intramuscular, intracranial, intrasternal, intratumoral, or intraperitoneally. Additionally, a pharmaceutical composition can be administered to a subject by additional routes of administration, for example, by inhalation, dermal, intranasal, or intrathecal administration.

Compositions of the present disclosure can be administered to a subject in need thereof in a first administration, and in one or more additional administrations. The one or more additional administrations can be administered to the subject in need thereof minutes, hours, days, weeks or months following the first administration. Any one of the additional administrations can be administered to the subject in need thereof less than 21 days, or less than 14 days, less than 10 days, less than 7 days, less than 4 days or less than 1 day after the first administration. The one or more administrations can occur more than once per day, more than once per week or more than once per month. The administrations can be weekly, biweekly (every two weeks), every three weeks, monthly or bimonthly.

One or more compounds of Formula (I) or Formula (III), or pharmaceutically acceptable salts, solvates, stereoisomers, or tautomers of any of these, may also be administered simultaneously with, prior to, or after administration of one or more other therapeutic agents. Such combination therapy may include administration of a single pharmaceutical dosage formulation that contains one or more compounds of the disclosure, or pharmaceutically acceptable salts, solvates, stereoisomers, or tautomers thereof, and one or more additional active agents, as well as administration of one or more compounds of the disclosure, or pharmaceutically acceptable salts, solvates, stereoisomers, or tautomers thereof, and each active agent in its own separate pharmaceutical dosage formulation. For example, one or more compounds of the disclosure, or pharmaceutically acceptable salts, solvates, stereoisomers, or tautomers thereof, and the other active agent can be administered to the subject together in a single oral dosage composition such as a tablet or capsule, or each agent administered in separate oral dosage formulations. Where separate dosage formulations are used, the one or more compounds of the disclosure, or pharmaceutically acceptable salts, solvates, stereoisomers, or tautomers thereof, and one or more additional active agents can be administered at essentially the same time, e.g., concurrently, or at separately staggered times, e.g., sequentially; combination therapy is understood to include all these regimens.

### Kits

In some aspects, the present disclosure provides a kit comprising a compound as disclosed herein, or a pharmaceutically acceptable salt, solvate, stereoisomer, or tautomer thereof, or a composition comprising said compound or pharmaceutically acceptable salt, solvate, stereoisomer, or tautomer thereof, and instructions.

In some embodiments, the present disclosure provides a kit comprising a pharmaceutical composition as disclosed herein and instructions.

In some embodiments, the instructions comprise instructions for administering the compound, salt or pharmaceutical composition as disclosed to a subject in need thereof. In some embodiments, the kit comprises a compound, salt or pharmaceutical composition disclosed herein, packaged in a low moisture vapor transmission container with a desiccant. Optionally, a label is on or associated with the container. For example, a label is on a container when letters, numbers or other characters forming the label are attached, molded or etched on the container itself, and a label is associated with a container when it is present within a receptacle or carrier, such as a box, that also holds the container, e.g., as a package insert. In addition, a label may be used to indicate that the contents are to be used for a specific therapeutic application. In some embodiments, the label includes directions for use of the contents, such as in the methods described herein. In some embodiments, a compound, salt or pharmaceutical composition disclosed herein is presented in a pack or container that contains one or more unit dosage forms comprising the compound, salt or pharmaceutical composition disclosed herein. The pack may contain metal or plastic foil, such as a blister pack. The pack or container may be accompanied by instructions for administration of the unit dosage form. In some embodiments, the pack or container is accompanied with a notice in a form prescribed by a governmental agency regulating the manufacture, use or sale of pharmaceuticals, which notice is reflective of approval by the agency of the form of the drug for human or veterinary administration. Such notice, for example, may be the labeling approved by the U.S. Food and Drug Administration for prescription of drugs, or the approved product insert. In some embodiments, compositions comprising the compound, salt or pharmaceutical composition disclosed herein are prepared, placed in an appropriate container, and labeled for treatment of an indicated condition.

A "kit" as used herein may include a container for containing at least one pharmaceutical composition or compound of the disclosure and may also include divided containers such as a divided bottle or a divided foil packet. The container can be in any conventional shape or form as known in the art that is made of a pharmaceutically acceptable material, for example a paper or cardboard box, a glass or plastic bottle or jar, a resealable bag (for example, to hold a "refill" of tablets for placement into a different container), or a blister pack with individual doses for pressing out of the pack according to a therapeutic schedule. The container employed can depend on the exact dosage form involved, for example a conventional cardboard box would not generally be used to hold a liquid suspension. It is feasible that more than one container can be used together in a single package to market a single dosage form. For example, tablets may be contained in a bottle that is in turn contained within a box.

An example of such a kit is a so-called blister pack. Blister packs are well known in the packaging industry and are being widely used for the packaging of pharmaceutical unit dosage forms (tablets, capsules, and the like). Blister packs generally consist of a sheet of relatively stiff material covered with a foil of a transparent plastic material. During the packaging process, recesses are formed in the plastic foil. The recesses have the size and shape of individual tablets or capsules to be packed or may have the size and shape to accommodate multiple tablets and/or capsules to be packed. Next, the tablets or capsules are placed in the recesses accordingly and the sheet of relatively stiff material is sealed against the plastic foil at the face of the foil that is opposite from the direction in which the recesses were formed. As a result, the tablets or capsules are individually sealed or collectively sealed, as desired, in the recesses between the plastic foil and the sheet. In some embodiments, the strength of the sheet is such that the tablets or capsules can be removed from the blister pack by manually applying pressure on the recesses whereby an opening is formed in the sheet at the place of the recess. The tablet or capsule may then be removed via said opening.

Another specific embodiment of a kit is a dispenser designed to dispense the daily doses one at a time in the order of their intended use. In some embodiments, the dispenser is equipped with a memory-aid, so as to further facilitate compliance with the regimen. An example of such a memory-aid is a mechanical counter, that indicates the number of daily doses that has been dispensed. Another example of such a memory-aid is a battery-powered micro-chip memory coupled with a liquid crystal readout, or audible reminder signal that, for example, reads out the date that the last daily dose has been taken and/or reminds one when the next dose is to be taken.

The kits of the present disclosure may also include, in addition to one or more compounds or pharmaceutical compositions of the present disclosure, one or more additional pharmaceutically active compounds or pharmaceutical compositions. For example, the additional compound may be a second antibacterial compound or the additional pharmaceutical composition may comprise a second antibacterial compound. The additional compounds or pharmaceutical compositions may be administered in the same dosage form as the one or more compounds or pharmaceutical compositions of the present disclosure or in a different dosage form. Likewise, the additional compounds or pharmaceutical compositions can be administered at the same time as the one or more compounds or pharmaceutical compositions of the present disclosure or at different times.

In some embodiments, the kit of the present disclosure has a notice in the form prescribed by a governmental agency regulating the manufacture, use or sale of pharmaceuticals or biological products, which notice reflects (a) approval by the agency of manufacture, use or sale for human administration, (b) directions for use, or both.

Although the technology herein has been described with reference to particular embodiments, it is to be understood that these embodiments are merely illustrative of the principles and applications of the present technology. It will be apparent to those skilled in the art that various modifications and variations can be made to the method and apparatus of the present technology without departing from the spirit and scope of the technology. Thus, it is intended that the present technology include modifications and variations that are within the scope of the appended claims and their equivalents. Accordingly, the disclosure is not limited except as by the appended claims.

Reference throughout this specification to "one embodiment" or "an embodiment" means that a particular feature, structure or characteristic described in connection with the embodiment is included in at least one embodiment of the present disclosure. Thus, the appearances of the phrases "in one embodiment" or "in an embodiment" in various places throughout this specification are not necessarily all referring to the same embodiment. Furthermore, the particular features, structures, or characteristics may be combined in any suitable manner in one or more embodiments. Any ranges cited herein are inclusive.

While preferred embodiments of the present invention have been shown and described herein, it will be obvious to those skilled in the art that such embodiments are provided by way of example only. Numerous variations, changes, and substitutions will now occur to those skilled in the art without departing from the invention. It should be understood that various alternatives to the embodiments of the invention described herein may be employed in practicing the invention. It is intended that the following claims define the scope of the invention and that methods and structures within the scope of these claims and their equivalents be covered thereby.

Aspects of the present technology are more fully illustrated with reference to the following examples. Before describing several exemplary embodiments of the technology, it is to be understood that the technology is not limited to the details of construction or process steps set forth in the following description. The technology is capable of other embodiments and of being practiced or being carried out in various ways. The following examples are set forth to illustrate certain aspects of the present technology and are not to be construed as limiting thereof. It is understood that one skilled in the art may be able to make these compounds by similar methods or by combining other methods known to one skilled in the art. It is also understood that one skilled in the art would be able to make, in a similar manner as described below, further compounds within the scope of the present disclosure by using appropriate starting materials and modifying the synthetic route as needed. In general, starting materials and reagents can be obtained from commercial vendors or synthesized according to sources known to those skilled in the art or prepared as described herein.

Reagent/reactant names given are as named on the commercial bottle or as generated by IUPAC conventions, ChemDraw 19.0 (CAMBRIDGESOFT^{®}; PerkinElmer). Compounds designated as salts (e.g., hydrochloride, acetate, sulfate) may include more than one molar equivalent of the acid.

### EXAMPLES

### ABBREVIATIONS

The following abbreviations are used herein.
- Ac: Acetate or acetyl
- Ac₂O: Acetic anhydride
- AcOH: Glacial acetic acid
- AIBN: Azobisisobutyronitrile
- BF₃·OEt₂: Boron trifluoride etherate
- Bn: Benzyl
- BnBr: Benzyl bromide
- Boc: *t*-Butoxycarbonyl
- BSA: Bovine serum albumin
- Bu₃SnH: Tri-n-butyltin hydride
- Cbz: Carboxybenzyl
- CCl₃CN: Trichloroacetonitrile
- CsF: Cesium fluoride
- d: Doublet
- DAST: Diaminosulfur trifluoride
- DCM: Dichloromethane (methylene chloride)
- dd: Doublet of doublets
- DIAD: Diisopropyl azodicarboxylate
- DMAP: N,N-Dimethylaminopyridine
- DMEM: Dulbecco's Modified Eagle Medium
- DMF: *N,N*-Dimethylformamide
- DMSO: Dimethyl sulfoxide
- DMTMM chloride: 4-(4,6-Dimethoxy-1,3,5-triazin-2-yl)-4-methyl-morpholinium chloride
- DPPA: Diphenylphosphoryl azide
- equiv: Equivalent(s)
- EtOAc: Ethyl acetate
- Et₂O: Diethyl ether
- EtOH: Ethanol
- g: Gram(s)
- h: Hour(s)
- HPLC: High-pressure liquid chromatography
- LAH: Lithium aluminum hydride
- LC/MS: Liquid chromatography/mass spectrometry
- LiOH: Lithium hydroxide
- m: Multiplet
- M: Molar
- MeCN: Acetonitrile
- MeOH: Methyl alcohol
- min: Minute(s)
- mmol: Millimole
- MPLC: Medium pressure liquid chromatography
- MS: Mass spectrometry
- *n-*: Normal (nonbranched)
- N: Normal
- NaH: Sodium hydride
- NaN₃: Sodium azide
- NaOMe: Sodium methoxide
- NBS: N-bromosuccinimide
- NH₄OAc: Ammonium acetate
- NMR: Nuclear magnetic resonance
- Pd(OH)₂: Palladium hydroxide
- pH: -log[H⁺]
- PhI(OAc)₂: Phenyl iodonium acetate
- Piv: Pivaloyl (trimethylacetyl)
- PMB: para-methoxybenzyl
- PMB-Cl: para-methoxybenzyl chloride
- ppm: Parts per million
- psi: Pounds per square inch
- PTSA: para-toluenesulfonic acid
- RP-HPLC: Reverse-phase high-pressure liquid chromatography
- Rₜ: Retention time
- rt: Room temperature
- s: Singlet
- t: Triplet
- *t-*: Tertiary
- *t*-BuOH: *t*-butanol
- TBAI: Tetra-*n*-Butylammonium iodide
- TBSCl: *t*-Butyldimethylsilyl chloride
- TEA: Triethylamine
- TfN₃: Trifluoromethanesulfonyl azide
- TFA: Trifluoroacetic acid
- THF: Tetrahydrofuran
- TMS: Trimethylsilyl
- TMSOTf: Trimethylsilyl trifluormethanesulfonate
- TolSH: p-Thiocresol
- TsCl: para-toluenesulfonyl chloride
- USP: United States Pharmacopeia
- wt%: Weight percent

### Analytical Methods

Analytical data is included within the procedures below. Unless otherwise stated, all¹H NMR data were collected on a Bruker Ultra Shield 500 MHz/54 mm instrument (BZH 43/500/70B, D221/54-3209) and chemical shifts are quoted in parts per million (ppm).

### Example 1. Synthesis of (benzyl (((2S,5R)-5-azido-6-hydroxytetrahydro-2H-pyran-2-yl)methyl)(benzyl)carbamate (Ring A1).

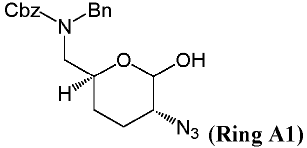

### Step A. (S)-N-((E)-((S)-3,4-dihydro-2H-pyran-2-yl)methylene)-2-methylpropane-2-sulfinamide

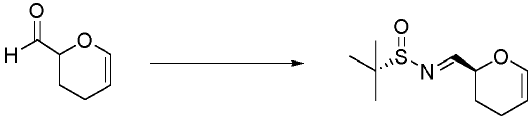

(S)-(+)-2-Methyl-2-propanesulfinamide (50 g, 412 mmol) and CuSO₄ (131.7 g, 825 mmol) were mixed in DCM (600.0 mL). A solution of 2-formyl-3,4-dihydro-2H-pyran (48.6 g, 433 mmol, Synthonix) in DCM (100.0 mL) was added over 20 min. The mixture was stirred at room temperature for 18 h, and then filtered through diatomaceous earth and rinsed with DCM. The filtrate was evaporated under reduced pressure. The material was purified on silica gel (5 x 330 g, dry loading) by MPLC using 0- 20% Et₂O in hexane as eluent to provide the title compound (23.74 g, 27%, second eluting diastereomer). ¹HNMR (400 MHz, CDCl₃) δ 8.08 (d, J = 3.0 Hz, 1H), 6.47 (dt, J = 6.3, 1.6 Hz, 1H), 4.81 - 4.72 (m, 2H), 2.19 - 1.94 (m, 4H), 1.24 (s, 9H).

### Step B. (R)-N-(((S)-3,4-dihydro-2H-pyran-2-yl)methyl)-2-methylpropane-2-sulfinamide

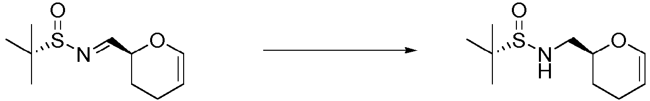

Sodium borohydride (101 mg, 2.66 mmol) was added to a solution of (NE)-N-[[(2S)-3,4-dihydro-2H-pyran-2-yl]methylene]-2-methyl-propane-2-sulfinamide (572 mg, 2.66 mmol) in ethanol (10.0 mL) at 0°C. The ice bath was removed and the reaction was kept stirring for another 30 min. The reaction was cooled to 0°C and sat. NH₄Cl (20.0 mL) was added (*nota bene*: gas evolution). The EtOH was evaporated under reduced pressure and the residue was extracted with EtOAc (30.0 mL). The layers were separated and the organic phase was successively washed with water (10.0 mL) and brine (10.0 mL), dried (Na₂SO₄), filtered and concentrated under reduced pressure to provide the title compound as a solid (547 mg, 95%). LCMS m/z: ES⁺ [M+H]⁺: 218.20; (A05) retention time = 2.04 m. This material was used in the following steps without further purifications.

### Step C. (R)-N-Benzyl-N-[[(2S)-3,4-dihydro-2H-pyran-2-yl]methyl]-2-methvyl-propane-2-sulfinamide

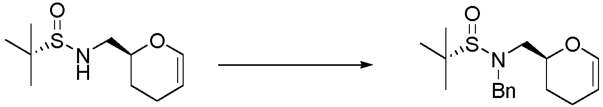

Sodium hydride (60%, 116 mg, 2.89 mmol) was added to a mixture of (R)-N-[[(2S)-3,4-dihydro-2H-pyran-2-yl]methyl]-2-methyl-propane-2-sulfinamide (547 mg, 2.52 mmol) and BnBr (448 µL, 3.78 mmol) in DMF (1.5 mL) at 0°C. The mixture was stirred at room temperature for 1 h, then cooled to 0°C followed by addition of water (10.0 mL). The aqueous layer was extracted with EtOAc (25.0 mL) and the organic layer was successively washed with water (10.0 mL) and brine (5.0 mL), dried (Na₂SO₄), filtered and concentrated under reduced pressure. The crude product was purified by silica gel chromatography (24 g cartridge) with EtOAc and hexanes (0-40%) to produce the title compound as an oil (768 mg, 99%). LCMS m/z: ES⁺ [M+H]⁺: 308.18; (A05) retention time = 2.57 m.

### Step D. (R)-N-Benzyl-N-[(1R)-1-[(2S)-6-hydroxy-5-iodo-tetrahydropyran-2-yl]ethyl]-2-methyl-propane-2-sulfinamide

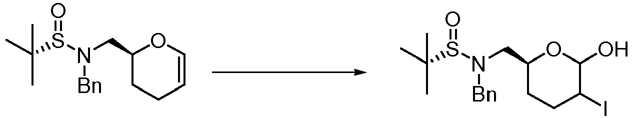

Iodine (272 mg, 1.07 mmol) was added to a suspension of (R)-N-benzyl-N-[(1R)-1-[(2S)-3,4-dihydro-2H-pyran-2-yl]ethyl]-2-methyl-propane-2-sulfinamide (320 mg, 1.04 mmol) and NaHCO₃ (262 mg, 3.12 mmol) in 1:1 H₂O/ACN (8.0 mL) at ambient temperature. After 30 min, the MeCN was evaporated and the residue was partitioned in between EtOAc (20.0 mL) and 1:1 sat. NaHCO₃/sat. Na₂S₂O₃ (10.0 mL). The organic layer was washed with water (5.0 mL) and brine (5.0 mL), dried (Na₂SO₄), filtered and concentrated under reduced pressure to provide the title compound as a foam (4 diastereomers, 450 mg, 96%). LCMS m/z: ES⁺ [M+H]⁺: 452.06; (A05) retention time = 2.30-2.50 m. This material was used in the following reactions without further purifications.

### Step E. (R)-N-[[(2S)-5-Azido-6-oxo-tetrahydropyran-2-yl]methyl]-N-benzyl-2-methyl-propane-2-sulfinamide

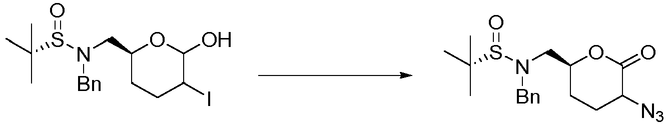

Molecular sieves (4 Å, 500 mg) and pyridinium dichromate (750 mg, 1.99 mmol) were added to a solution of benzyl (R)-N-benzyl-N-[[(2S)-6-hydroxy-5-iodo-tetrahydropyran-2-yl]methyl]-2-methylpropane-2-sulfinamide (450 mg, 997 µmol) in dry DCM (10.0 mL) under N₂ at ambient temperature. After 24 h, the solution was concentrated and the crude product was partitioned in between EtOAc (30.0 mL) and water (15.0 mL). The organic layer was washed with water (2 x 10.0 mL) and brine (10.0 mL), dried (Na₂SO₄) and concentrated. The crude product was dissolved in dry DMF (1.5 mL) under N₂ and NaN₃ (130 mg, 1.99 mmol) was added. After 1 h, the solution was partitioned in between EtOAc (30.0 mL) and water (15.0 mL). The organic layer was washed with water (2 x 10.0 mL) and brine (10.0 mL), dried (Na₂SO₄) and concentrated to provide the title compound as an oil (235 mg, 64%). LCMS m/z: ES⁺ [M+H]⁺: 365.17; (A05) retention time = 2.37 m. This material was used in the next reactions without further purifications.

### Step F. (R)-N-[[(2S,5R)-5-Azido-6-hydroxy-tetrahydropyran-2-yl]methyl]-N-benzyl-2-methylpropane-2-sulfinamide

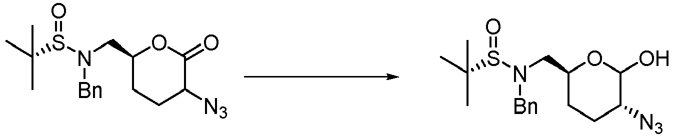

A solution of DIBAL-H in toluene (1 M, 1.03 mL, 1.03 mmol) was dropwise added to a solution of (R)-N-[[(2S)-5-azido-6-oxo-tetrahydropyran-2-yl]methyl]-N-benzyl-2-methyl-propane-2-sulfinamide (235 mg, 645 µmol) in dry DCM (8.0 mL) at -78°C under N₂. After 100 min, sat. potassium sodium tartrate (15.0 mL) was added to the solution slowly, followed by the addition of water (15.0 mL). The mixture was allowed to warm to room temperature and vigorously stirred for 16 h. The mixture was extracted with DCM (10.0 + 2 x 5.0 mL). The combined organic layers were dried (Na₂SO₄), filtered and concentrated under reduced pressure. The crude was purified by silica gel chromatography (12 g cartridge) with EtOAc and hexanes (10%-50%) to provide the title compound as an oil (diastereomers, 195 mg, 74%). LCMS m/z: ES⁺ [M+H]⁺: 367.19; (A05) retention time = 2.28-2.40 min.

### Step G. Benzyl N-[[(2S,5R)-5-azido-6-hydroxytetrahydropyran-2-yl]methyl]-N-benzyl-carbamate (Ring A1)

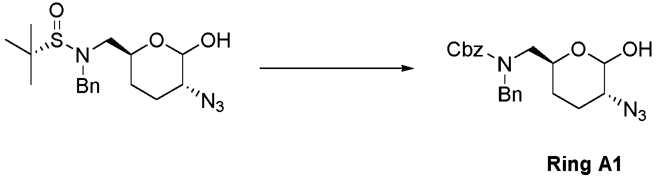

Aqueous HCl (1.0 M, 2.66 mL, 2.66 mmol) was dropwise added to a solution of (R)-N-[[(2S)-5-azido-6-hydroxy-tetrahydropyran-2-yl]methyl]-N-benzyl-2-methyl-propane-2-sulfinamide (195 mg, 532 µmol) in dioxane (2.7 mL) with vigorous stirring. After 1 h, solid Na₂CO₃ (451 mg, 4.26 mmol) was added. After another 5 min, CbzCl (106 µL, 745 mmol) was added dropwise. After another 1 h, dioxane was evaporated and the residue was partitioned in between EtOAc (20.0 mL) and H₂O (20.0 mL). The organic phase was washed with brine (10.0 mL), dried (Na₂SO₄), filtered and concentrated under reduced pressure. The crude was purified by C18 reverse phase chromatography (80 g cartridge) with ACN and 0.1% aq. formic acid (40-70%) to produce the title compound (mixture of 4 diastereomers) as an oil (167 mg, 79%). UPLC m/z: ES⁺ [M-OH]⁺: 379.21; (CSH 5 to 100% ACN/ammonium formate pH 4) retention time = 2.12, 2.15, 2.20, and 2.22 m. A portion (40 mg) of the material was purified by prep-HPLC (CSH C18 ACN/ammonium formate 50-70%) to provide the α-azido as a film (20 mg, 50% recovery). UPLC m/z: (CSH 5 to 100% ACN/ammonium formate pH 4) retention time = 2.15 and 2.22 min.

### Example 2. Synthesis of benzyl ((1S)-1-((2S,5R)-5-azido-6-hydroxytetrahydro-2H-pyran-2-yl)ethyl)(benzyl)carbamate (Ring A2).

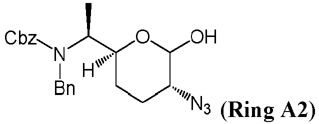

### Step A. ((S)-N-((E)-((S)-3,4-dihydro-2H-pyran-2-yl)methylene)-2-methylpropane-2-sulfinamide

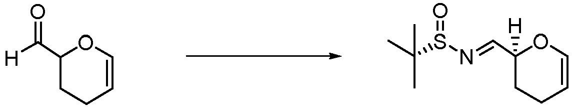

(S)-(+)-2-Methyl-2-propanesulfinamide (50 g, 412 mmol) and CuSO₄ (131.7 g, 825 mmol) were mixed in DCM (600.0 mL). A solution of 2-formyl-3,4-dihydro-2H-pyran (48.6 g, 433 mmol, Synthonix) in DCM (100.0 mL) was added over 20 min. The mixture was stirred at room temperature for 18 h, and then filtered through diatomaceous earth and rinsed with DCM. The filtrate was evaporated under reduced pressure. The material was purified on silica gel (5 x 330 g, dry loading) by MPLC using 0- 20% Et₂O in hexane as eluent to provide the title compound (23.74 g, 27%, second eluting diastereomer). ¹HNMR (400 MHz, CDCl₃) δ 8.08 (d, J = 3.0 Hz, 1H), 6.47 (dt, J = 6.3, 1.6 Hz, 1H), 4.81 - 4.72 (m, 2H), 2.19 - 1.94 (m, 4H), 1.24 (s, 9H).

### Step B. (S)-N-((S)-1-((S)-3,4-dihydro-2H-pyran-2-yl)ethyl)-2-methylpropane-2-sulfinamide

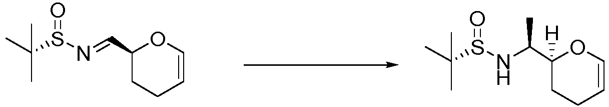

(S)-N-((E)-((S)-3,4-dihydro-2H-pyran-2-yl)methylene)-2-methylpropane-2-sulfinamide (7.30 g, 33.9 mmol) was dissolved in dry DCM (100 mL) and the solution was cooled to -78°C. MeMgBr (3.0 M in Et₂O, 22.6 mL, 67.8 mmol) was added dropwise to the mixture and stirring was continued at low temperature for 2 hours, before allowing the mixture to warm to room temperature. Stirring was continued at room temperature for 1 hour. Concentrated aqueous NH₄Cl (50 mL) was added, and the mixture was extracted with DCM (3 x 50 mL). The combined organic layers were dried (MgSO₄), and concentrated *in vacuo* to afford a crude oil containing a mixture of diastereomers. The material was purified using silica gel chromatography (120 g cartridge) with hexanes and ethyl acetate (0-100%) to afford the title compound as an oil (4.40 g, 56%). LCMS m/z: ES⁺ [M+H]⁺: 232.16.

### Step C. (S)-N-benzyl-N-((S)-1-((S)-3,4-dihydro-2H-pyran-2-yl)ethyl)-2-methylpropane-2-sulfinamide

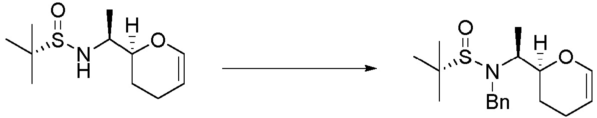

NaH (60% dispersion, 913 mg, 22.8 mmol) was added to a solution of (S)-N-((S)-1-((S)-3,4-dihydro-2H-pyran-2-yl)ethyl)-2-methylpropane-2-sulfinamide (4.40 g, 19.0 mmol) and benzyl bromide (3.39 mL, 28.5 mmol) in DMF (100 mL) at 0 °C. The mixture was stirred at this temperature for 2 hours, then brine (100 mL) was added at 0 °C. The aqueous layer was extracted with Et₂O (3 x 50.0 mL). The combined organic layers were dried (MgSO₄) and concentrated *in vacuo* to afford a residue that was purified using silica gel chromatography (120 g cartridge) using hexanes and ethyl acetate (0-100%). The pure fractions were concentrated *in vacuo* to afford the title compound as an oil (3.50 g, 57%). LCMS m/z: ES⁺ 322.14.

¹HNMR (500 MHz, DMSO) δ 7.45 (d, J = 7.1 Hz, 2H), 7.35 - 7.29 (m, 2H), 7.23 (t, J = 7.3 Hz, 1H), 6.38 (d, J = 6.1 Hz, 1H), 4.65 (ddd, J = 6.2, 4.3, 1.4 Hz, 1H), 4.35 (d, J = 16.5 Hz, 1H), 3.91 (t, J = 11.4 Hz, 1H), 3.80 (td, J = 9.2, 2.3 Hz, 1H), 3.22 - 3.11 (m, 1H), 1.97 - 1.92 (m, 1H), 1.89 - 1.78 (m, 2H), 1.53 - 1.42 (m, 1H), 1.19 (t, J = 3.4 Hz, 3H), 1.10 (s, 8H).

### Step D. (S)-N-benzyl-N-((1S)-1-((2S)-6-hydroxy-5-iodotetrahydro-2H-pyran-2-yl)ethyl)-2-methylpropane-2-sulfinamide

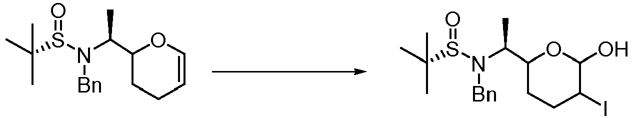

(S)-N-benzyl-N-((S)-1-((S)-3,4-dihydro-2H-pyran-2-yl)ethyl)-2-methylpropane-2-sulfinamide (4.40 g, 13.7 mmol) and NaHCO₃ (3.45 g, 41.1 mmol) were dissolved in H₂O (75.0 mL) and ACN (75.0 mL). I₂ (3.82 g, 15.1 mmol) was added portion-wise over 10 minutes at 0 °C. The cooling bath was removed, and stirring was continued at room temperature over 90 minutes. The reaction mixture was quenched with a saturated aqueous solution of Na₂S₂O₃ (30.0 mL) and extracted with EtOAc (3 x 20.0 mL). The combined organic layers were dried (MgSO₄) and concentrated *in vacuo* to afford the title compound as a solid (4.70 g, 74%). LCMS m/z: ES⁺ [M+H]⁺: 466.04.

### Step E. Benzyl benzyl((1S)-1-((2S)-6-hydroxy-5-iodotetrahydro-2H-pyran-2-yl)ethyl)carbamate

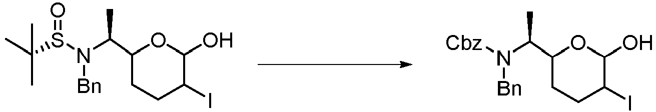

(S)-N-benzyl-N-((1S)-1-((2S)-6-hydroxy-5-iodotetrahydro-2H-pyran-2-yl)ethyl)-2-methylpropane-2-sulfinamide (4.70 g, 10.1 mmol) was dissolved in 1,4-dioxane (150 mL), and an aqueous solution of 1 N HCl (25.2 mL, 25.2 mmol) was added. Stirring was continued at room temperature for 20 minutes, before solid Na₂CO₃ (8.56 g, 80.8 mmol) was added to the mixture. Stirring was continued for 20 minutes, and then CbzCl (1.72 mL, 12.1 mmol) was added dropwise. Stirring was continued for 2 hours. Water (200 mL) was added and the mixture was extracted with EtOAc (3 x 50 mL). The combined organic layers were dried (MgSO₄) and concentrated *in vacuo* to give a residue that was purified using silica gel chromatography (80 g cartridge) using hexanes and ethyl acetate (0-100%). The pure fractions were concentrated *in vacuo* to afford the title compound as an oil (3.70 g, 74%). LCMS m/z: ES⁺ [M+H]⁺: 518.04.

### Step F. Benzyl ((1S)-1-((2S)-5-azido-6-oxotetrahydro-2H-pyran-2-yl)ethyl)(benzyl)carbamate

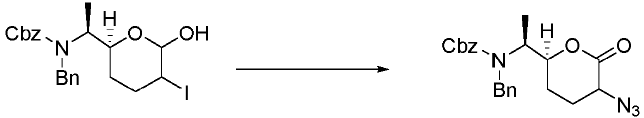

Benzyl benzyl((1S)-1-((2S)-6-hydroxy-5-iodotetrahydro-2H-pyran-2-yl)ethyl)carbamate (3.70 g, 7.47 mmol) was dissolved in DCM (350 mL). 4 Å molecular sieves (1.00 g) were suspended in the mixture, and PDC (8.43 g, 22.4 mmol) was added. Stirring was continued over 24 hours at room temperature, then filtered through a pad of diatomaceous earth using ethyl acetate, and concentrated *in vacuo* to afford crude benzyl benzyl((1S)-1-((2S)-5-iodo-6-oxotetrahydro-2H-pyran-2-yl)ethyl)carbamate as an oil. The crude product was dissolved in DMF (50.0 mL). NaN₃ (971 mg, 14.9 mmol) was added as a solid, and stirring was continued for 2 hours at room temperature. The solvent was removed *in vacuo,* and the residue was dissolved in EtOAc (100 mL) and washed with water (3 x 25 mL). The organic layer was dried (MgSO₄) and concentrated *in vacuo* to afford an oil that was purified using silica gel chromatography (80 g cartridge) with ethyl acetate and hexanes (0-100%). The pure fractions were combined and concentrated *in vacuo* to afford the title compound as an oil (1.40 g, 46%). LCMS m/z: ES⁺ [M+Na]⁺: 431.13; calc: 431.18.

### Step G. Benzyl ((1S)-1-((2S,5R)-5-azido-6-hydroxytetrahydro-2H-pyran-2-yl)ethyl)(benzyl)carbamate (Ring A2)

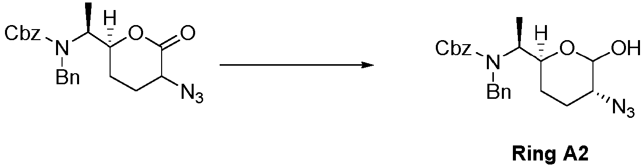

Benzyl ((1S)-1-((2S)-5-azido-6-oxotetrahydro-2H-pyran-2-yl)ethyl)(benzyl)carbamate (1.40 g, 3.43 mmol) was dissolved in dry DCM (75.0 mL) and the mixture was cooled to -78 °C. A solution of DIBAL-H (1.0 M in toluene, 6.86 mL, 6.86 mmol) was added to the mixture, and stirring was continued at low temperature for 1 hour. EtOH (2.00 mL) was added dropwise to the cold reaction mixture, which was then poured into a saturated aqueous solution of Rochelle's salt (200 mL). The mixture was stirred vigorously for 1 hour, then extracted with EtOAc (3 x 50.0 mL). The combined organic layers were dried (MgSO₄) and concentrated in vacuo to afford a residue that was purified using silica gel chromatography (80 g cartridge) with hexanes and ethyl acetate (0-70%). The pure fractions were collected and concentrated *in vacuo* to afford the title compound as a mixture of diastereomers (800 mg, 57%). LCMS m/z: ES⁺ [M+Na]⁺: 433.16; calc: 433.19.

### Example 3. Synthesis of Benzyl N-[(1R)-1-[(2S,5R)-5-azido-6-hydroxy-tetrahydropyran-2-yl]ethyl]-N-benzyl-carbamate (Ring A3).

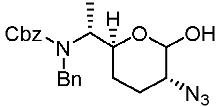

### Step A. (R)-N-((E)-((S)-3,4-dihydro-2H-pyran-2-yl)methylene)-2-methylpropane-2-sulfinamide

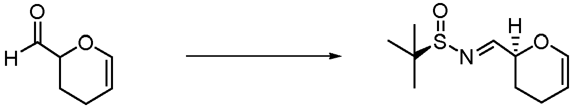

(R)-(+)-2-Methyl-2-propanesulfinamide (81.1 g, 669 mmol) and CuSO₄ (213.5 g, 1.34 mol) were mixed in DCM (700 mL). A solution of 2-formyl-3,4-dihydro-2H-pyran (75 g, 669 mmol) in DCM (100 mL) was added over 20 min. The mixture was stirred at room temperature for 72 h. The mixture was filtered through diatomaceous earth and rinsed with DCM. The filtrate was evaporated under reduced pressure. The material was purified on silica gel (5 x 330 g, dry loading) by MPLC using 0-10% Et₂O in hexane to provide the title compound (28 g, 19%) as a solid. ¹H NMR (400 MHz, CDCl₃) δ 8.07 (d, J = 3.1 Hz, 1H), 6.40 (d, J = 5.8 Hz, 1H), 4.76 - 4.68 (m, 1H), 4.68 - 4.63 (m, 1H), 2.13 - 1.83 (m, 4H), 1.18 (s, 9H).

### Step B. N-[(1R)-1-[(2S)-3,4-Dihydro-2H-pyran-2-yl]ethyl]-2-methyl-propane-2-sulfinamide

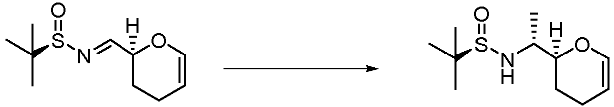

MeMgBr (3.0 M in Et₂O, 22.0 mL, 66.0 mmol) was added to a solution of (R)-N-((E)-((S)-3,4-dihydro-2H-pyran-2-yl)methylene)-2-methylpropane-2-sulfinamide (7.10 g, 33.0 mmol) in dry DCM (110 mL) at -40 °C under N₂. After 2 h, the reaction was warmed to room temperature within 1 h. After another 14 h, sat. NH₄Cl (160 mL) was added dropwise (nota bene: gas evolution). Two phases were separated and the aqueous phase was extracted with DCM (3 x 20.0 mL). The combined organic layers were dried (Na₂SO₄), filtered and concentrated under reduced pressure. The residue was purified by silica gel chromatography (220 g cartridge) with EtOAc and hexanes (20-45%) to provide the title compound as a solid (6.00 g, 79%). ¹H NMR (500 MHz, CDCl₃) δ 6.37 (d, J = 6.1 Hz, 1H), 4.75 - 4.61 (m, 1H), 3.93 (ddd, J = 11.2, 3.3, 2.0 Hz, 1H), 3.69 (d, J = 6.1 Hz, 1H), 3.56 (pd, J = 6.7, 3.5 Hz, 1H), 2.16 - 2.06 (m, 1H), 1.99 (dtt, J = 17.1, 5.6, 1.6 Hz, 1H), 1.81 (ddd, J = 13.3, 6.6, 1.8 Hz, 1H), 1.66 (dtd, J = 13.3, 11.4, 5.8 Hz, 1H), 1.22 (s, 9H), 1.18 (d, J = 6.7 Hz, 3H).

### Step C. (R)-N-Benzyl-N-[(1R)-1-[(2S)-3,4-dihydro-2H-pyran-2-yl]ethyl]-2-methyl-propane-2-sulfinamide

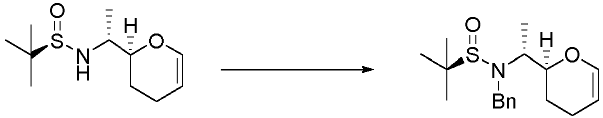

NaH (60%, 187 mg, 4.89 mmol) was added to a mixture of (R)-N-[(1R)-1-[(2S)-3,4-dihydro-2H-pyran- 2-yl]ethyl]-2-methyl-propane-2-sulfinamide (1.03 g, 4.44 mmol) and BnBr (0.79 mL, 6.67 mmol) in DMF (60 mL) at 0°C. The mixture was stirred at room temperature for 1 h, then brine (250 mL) was added at 0 °C. The aqueous layer was extracted with Et₂O (3 x 80 mL). The combined organic layer was dried over MgSO₄ and concentrated under reduced pressure. The material was purified on silica gel (40 g, dry loading) by MPLC using hexane to 60% EtOAc in hexane to provide the title compound (1.2 g, 84%) as an oil. ¹H NMR (400 MHz, cdcl3) δ 7.37 (d, J = 7.5 Hz, 2H), 7.32 (t, J = 7.4 Hz, 2H), 7.26 (d, J = 3.8 Hz, 1H), 6.32 (d, J = 6.2 Hz, 1H), 4.64 (s, 1H), 4.39 (d, J = 15.3 Hz, 1H), 4.14 (d, J = 15.2 Hz,1H), 3.66 - 3.57 (m, 1H), 3.32 - 3.20 (m, 1H), 1.98 - 1.80 (m, 3H), 1.54 - 1.43 (m, 1H), 1.37 (d, J = 6.8 Hz, 3H), 1.19 (s, 9H).

### Step D. (R)-N-Benzyl-N-[(1R)-1-[(2S)-6-hydroxy-5-iodo-tetrahydropyran-2-yl]ethyl]-2-methyl-propane-2-sulfinamide

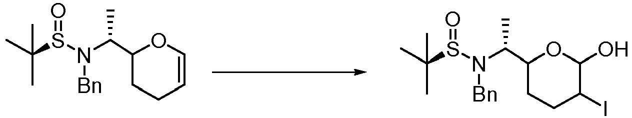

I₂ (947 mg, 3.73 mmol) was added portionwise to a suspension of (R)-N-benzyl-N-[(1R)-1-[(2S)-3,4- dihydro-2H-pyran-2-yl]ethyl]-2-methyl-propane-2-sulfinamide (1.2 g, 3.73 mmol) and NaHCO₃ (941 mg, 11.2 mmol) in ACN (50 mL) and H₂O (50 mL) at 0 °C. The mixture was stirred at room temperature for 1 h, then a saturated aqueous solution of Na₂S₂O₃ (10 mL) was added. The aqueous layer was extracted with EtOAc (3x 150 mL). The combined organic layer was washed with brine, dried over MgSO₄, filtered and concentrated under reduced pressure to provide the title compound (1.6 g, 92%) as a solid. M+H⁺: 466.1.

### Step E. Benzyl N-benzyl-N-[(1R)-1-[(2S)-6-hydroxy-5-iodo-tetrahydropyran-2-yl]ethyl]carbamate

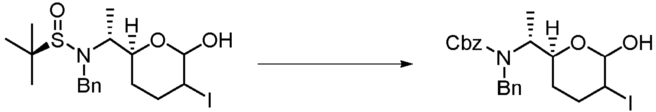

1N HCl (6.45 mL, 6.45 mmol) was added to (R)-N-benzyl-N-[(1R)-1- [(2S)-6- hydroxy-5-iodo-tetrahydropyran-2-yl]ethyl]-2-methyl-propane-2-sulfinamide (1.2 g, 2.58 mmol) in dioxane (40 mL). The mixture was stirred at room temperature for 20 min. then Na₂CO₃ (2.19 g, 20.6 mmol) was added. After 20 min, CbzCl (0.51 mL, 3.61 mmol) was added dropwise. The mixture was stirred at room temperature for 2 h. Water (200 mL) was added. The separated aqueous layer was extracted with EtOAc (3 x 150 mL). The combined organic layer was washed with brine, dried over MgSO₄ and concentrated under reduced pressure. The material was purified on silical gel (80 g, dry loading) by MPLC using hexane to 80% EtOAc to provide the title compound (1.1 g, 86%) as a solid. M+H⁺: 496.0.

### Step F. Benzyl N-[(1R)-1-[(2S)-5-azido-6-oxo-tetrahydropyran-2-yl]ethyl]-N-benzyl-carbamate

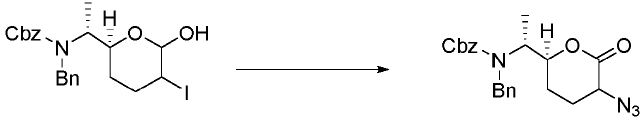

Dess-Martin Periodinane (1.88 g, 4.44 mmol) was added to a solution of benzyl N-benzyl-N-[(1R)-1- [(2S)-6-hydroxy-5-iodo-tetrahydropyran-2-yl]ethyl]carbamate (1.10 g, 2.22 mmol) in DCM (100 mL) at 0 °C. The mixture was stirred at room temperature for 5 h. Water (100 mL) was added following by a saturated aqueous solution of Na₂S₂O₃. The separated aqueous layer was extract with DCM (2 x 50 mL). The combined organic layer was washed with saturated aqueous NaHCO₃ (2 x 100 mL), brine (100 mL), dried over MgSO₄ and concentrated under reduced pressure. The residue was taken in anhydrous DMF (75 mL) and NaN₃ (217 mg, 3.33 mmol) was added. The mixture was stirred at room temperature for 15 min, then brine (300 mL) was added. The aqueous layer was extracted with Et₂O (3 x 100 mL). The combined organic layers were dried over MgSO₄ and concentrated under reduced pressure. The material was purified on silica gel (40 g, dry loading) by MPLC using hexane to EtOAc to provide the title compound (800 mg, 88%) as an oil. M+H⁺: 409.3.

### Step G. Benzyl N-[(1R)-1-[(2S,5R)-5-azido-6-hydroxy-tetrahydropyran-2-yl]ethyl]-N-benzyl-carbamate (Ring A3)

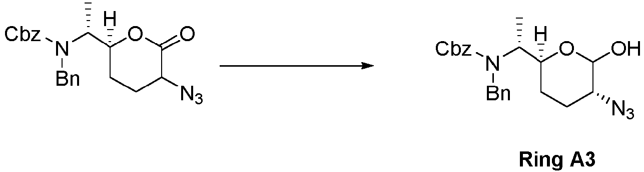

A solution of DIBAL-H (1 M in toluene, 11.8 mL, 11.8 mmol) was added dropwise to a solution of benzyl N-[(1R)-1- [(2S)-5-azido-6-oxo-tetrahydropyran-2-yl]ethyl]-N-benzyl-carbamate (800 mg, 1.96 mmol) in DCM (60 mL) at -78 °C. After 1 h at -78 °C, EtOH (0.5 mL) was added dropwise. The mixture was poured into a saturated aqueous solution of Rochelle's salt (300 mL). The mixture was vigorously stirred for 1 h. The separated aqueous layer was extracted with DCM (2 x 75 mL). The combined organic layer was washed with brine, dried over MgSO₄ and concentrated under reduced pressure. The residue was purified on silica gel (40 g, dry loading) by MPLC using hexane to 60% EtOAc in hexane to provide the title compound (363 mg, 41%) as an oil. M+H⁺: 411.2.

### Example 4. Synthesis of (1R,2R,3S,4R,5S,6S)-3,5-bis(((benzyloxy)carbonyl)amino)-6-hydroxycyclohexane-1,2,4-triyl triacetate (Ring B1).

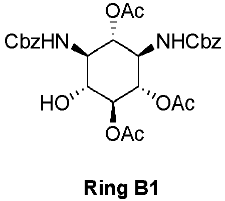

### Step A: Synthesis of (1S,2R,3S,4S,5R,6S)-3,5-diamino-6-(((2R,3R,4R,5S)-3-(((2R,3S,4S,5R,6S)-4,5-dihydroxy-6-(hydroxymethyl)-3-(methylamino)tetrahydro-2H-pyran-2-yl)oxy)-4-hydroxy-4-(hydroxymethyl)-5-methyltetrahydrofuran-2-yl)oxy)cyclohexane-1,2,4-triol

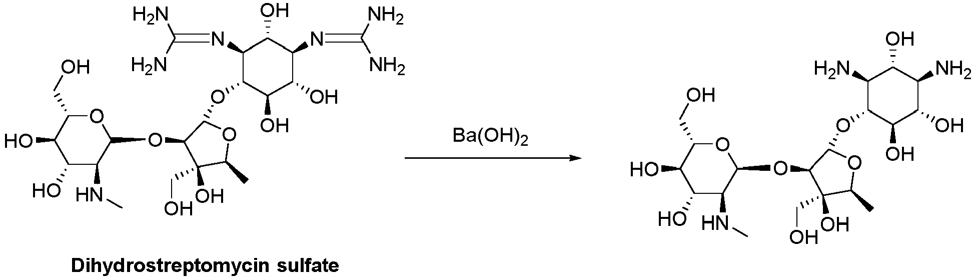

Dihydrostreptomycin sulfate (50 g), Ba(OH)₂-8H₂O (4 equivalents) and water (500 mL) were combined and heated under reflux for 2 days. After cooling to room temperature, CO₂ was bubbled through the reaction until it became slightly acidic (pH 6-7). The resulting mixture was filtered through diatomaceous earth, washing twice with water (75 mL). The aqueous layer was concentrated *in vacuo* and the aqueous solution used directly without further purification.

### Step B: Synthesis of dibenzyl ((1S,2S,3R,4S,5S,6R)-4-(((2R,3R,4R,5S)-3-(((2R,3S,4S,5R,6S)-3-(((benzyloxy)carbonyl)(methyl)amino)-4,5-dihydroxy-6-(hydroxymethyl)tetrahydro-2H-pyran-2-yl)oxy)-4-hydroxy-4-(hydroxymethyl)-5-methyltetrahydrofuran-2-yl)oxy)-2,5,6-trihydroxycyclohexane-1,3-diyl)dicarbamate

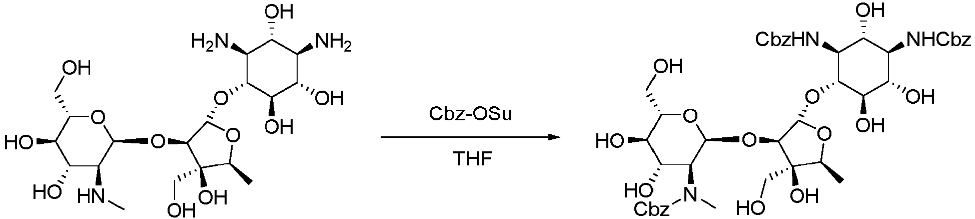

To the crude compound from Step A was added Cbz-OSu (57.7 g, 3.4 eq) in THF (110 mL) over ~1h at room temperature. The reaction was stirred at room temperature for 2 days. Saturated aqueous sodium bicarbonate was added (50 mL) and the product extracted into EtOAc (2x75 mL) and washed with brine (50 mL). The combined organics were dried over sodium sulfate and the solvent removed *in vacuo.* The crude product was dissolved in 2-butanone (80 ml) and dripped slowly into MTBE (750 mL). The precipitated solids were collected by filtration and dried in a vacuum oven at 50°C to afford the titled product (52.1 g) as an off-white solid (72% purity by absorbance at 210 nm).

### Step C: Synthesis of (1R,2R,3S,4R,5R,6S)-6-(((2R,3R,4R,5S)-4-(acetoxymethyl)-3-(((2R,3S,4S,5R,6S)-4,5-diacetoxy-6-(acetoxymethyl)-3-(((benzyloxy)carbonyl)(methyl)amino)tetrahydro-2H-pyran-2-yl)oxy)-4-hydroxy-5-methyltetrahydrofuran-2-yl)oxy)-3,5-bis(((benzyloxy)carbonyl)amino)cyclohexane-1,2,4-triyl triacetate

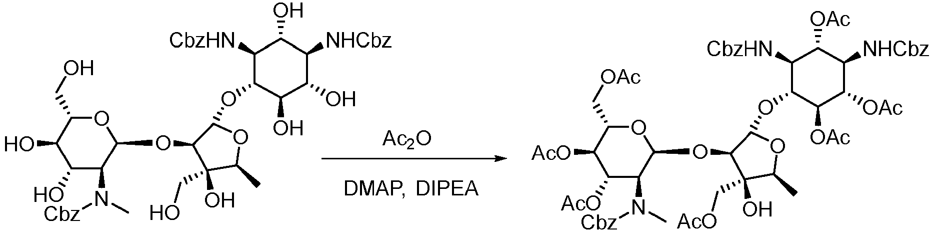

The crude compound from Step B (52.1 g) was dissolved in EtOAc (210 mL) and DMAP (721 mg, 0.1 eq) added at room temperature. DIPEA (103 mL) and Ac₂O (51 mL) in EtOAc (80 mL) was added to the reaction mixture dropwise over 1 h at room temperature. The reaction was stirred at room temperature overnight. The reaction was transferred to a separatory funnel with EtOAc (50mL, 0.9V) and washed with water (50 mL), sodium bicarbonate solution (2x50 mL), and brine (50 mL). The organics were dried over sodium sulfate and filtered to afford the titled products as a cream-colored foam (70 g; 63% AP at 210 nm).

### Step D: Synthesis of (1R,2R,3S,4R,5S,6S)-3,5-bis(((benzyloxy)carbonyl)amino)-6-hydroxycyclohexane-1,2,4-triyl triacetate (Ring B1)

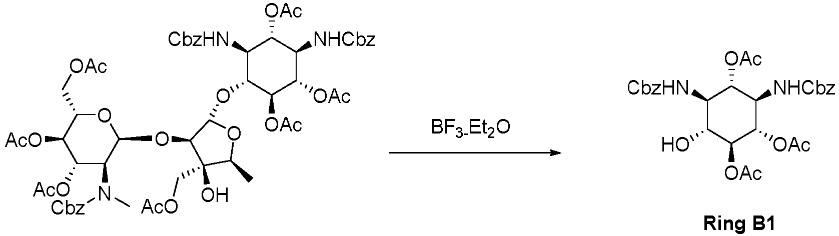

The crude product of Step C (53 g) was added to DCM (420mL), dodecanethiol (29.4 mL, 2.1 eq), and BF₃·Et₂O (16.3 mL, 2.2 eq). The reaction was heated under reflux for 2 hours. The reaction was washed with aqueous sodium bicarbonate, water, and brine (20 mL each). The organic portion was concentrated *in vacuo* and the resulting solid was stirred vigorously with MeCN/hexane (1:2, 210 mL) to afford the titled product. The crude product was triturated with heptane (300 mL) to afford the titled product (23.9 g, 71% yield).

### Example 5. Synthesis of (2R,3R)-4-azido-3-fluoro-2-((4-methoxybenzyl)oxy)butanoic acid (F-HABA).

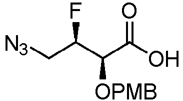

### Step A: Synthesis of methyl (R)-2-((R)-2,2-dimethyl-1,3-dioxolan-4-yl)-2-hydroxyacetate.

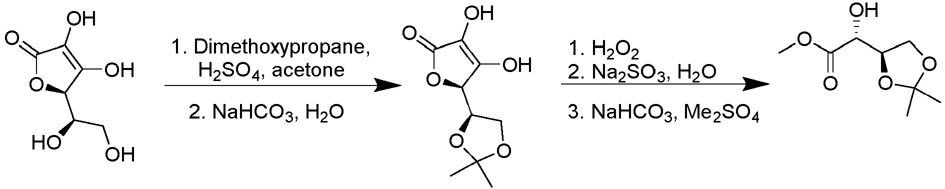

To a solution of D-isoascorbic acid (100 g, 0.568 mol, 1.0 eq.) and 2,2-dimethoxypropane (76.8 mL, 0.624 mol, 1.1 eq) in dry acetone (500 mL) was slowly added concentrated H₂SO₄ (0.6 mL, 11.2 mmol) and the mixture was stirred for 5 h at 0°C. To the reaction mixture was slowly added NaHCO₃ (142.8 g, 1.7 mol, 3.0 eq.) and H₂O (400 mL). Acetone was removed by rotary evaporator under reduced pressure. To the residue was slowly added 30% H₂O₂ (128.4 mL, 1.132 mol, 2.0 eq.) at 0°C using a dropping funnel. After stirring the mixture for 3 h, Na₂SO₃ (14.28 g, 113.2 mmol, 0.2 eq.) was added and the mixture was stirred for 30 min. Sodium bicarbonate (192.8 g, 2.28 mol, 4.0 eq.) and dimethyl sulfate (225.6 mL, 2.36 mol, 4.0 eq.) were added and the mixture was stirred for 5 h at 50°C. The reaction mixture was filtered and the aqueous layer was extracted with CH₂Cl₂ (800 mL × 2). The organic layers were combined and dried over anhydrous MgSO₄, filtered, and the filtrate was concentrated under reduced pressure. The residue was purified by flash column chromatography, eluting with petroleum ether/ethyl acetate (2:1, v/v) to give methyl (*R*)-2-((*R*)-2,2-dimethyl-1,3-dioxolan-4-yl)-2-hydroxyacetate (70 g, 64.8%) as colorless oil. ¹H NMR (400 MHz, CDCl₃) δ 4.35-4.14 (m, 2H), 4.01-3.90 (m, 2H), 3.75 (s, 3H), 3.03 (d, *J* = 6.4 Hz, 1H), 1.36 (s, 3H), 1.29 (s, 3H).

### Step B: Synthesis of (S)-1-((R)-2,2-dimethyl-1,3-dioxolan-4-yl)ethane-1,2-diol.

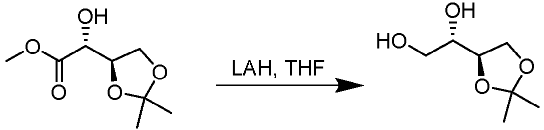

To a suspension of LAH (16.59 g, 0.44 mol, 1.2 eq.) in dry THF (600 mL) was added dropwise a solution of methyl (*R*)-2-((*R*)-2,2-dimethyl-1,3-dioxolan-4-yl)-2-hydroxyacetate (70 g, 0.36 mol, 1.0 eq) in THF (200 mL) under N₂. The resulting mixture was stirred for 3 hours at room temperature, then H₂O (20 mL) was added in dropwise, followed by 15% aq. NaOH (20 mL) and H₂O (60 mL). The reaction mixture was filtered and concentrated, and the residue purified by flash column chromatography, eluting with petroleum ether/ethyl acetate (1: 1) to give (S)-1-((R)-2,2-dimethyl-1,3-dioxolan-4-yl)ethane-1,2-diol (45 g, yield 75.4%). ¹H NMR (400 MHz, DMSO-*d*₆) δ 4.21 (br, s, 2 H), 3.79-3.95 (m, 3 H), 3.29-3.50 (m, 3H), 1.30 (s, 3 H), 1.25 (s, 3 H).

### Step C: Synthesis of (S)-2-((R)-2,2-dimethyl-1,3-dioxolan-4-yl)-2-hydroxyethyl 4-methylbenzenesulfonate.

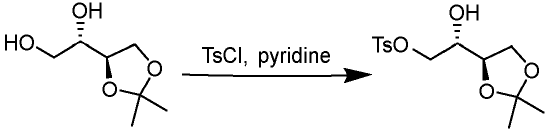

To a solution of (*S*)-1-((*R*)-2,2-dimethyl-1,3-dioxolan-4-yl)ethane-1,2-diol (45 g, 277.8 mmol, 1.0 eq.) in dry pyridine (500 mL) was added TsCl (52.96 g, 277.8 mmol, 1.0 eq) portion-wise over 1h at 0°C. The resulting mixture was stirred overnight at room temperature, then the solvent was removed. The residue was dissolved in EA (1000 mL), washed with sat'd. aq. NH₄Cl (500 mL × 2), dried over MgSO₄, filtered, and concentrated. The residue was purified by flash column chromatography, eluting with petroleum ether/ethyl acetate (4: 1) to give (*S*)-2-((*R*)-2,2-dimethyl-1,3-dioxolan-4-yl)-2-hydroxyethyl 4-methylbenzenesulfonate (59 g, yield 75.9%). ¹H NMR (400 MHz, DMSO) δ 7.79 (d, *J =* 8.2 Hz, 2H), 7.49 (d, *J =* 8.1 Hz, 2H), 5.53 (d, *J* = 5.9 Hz, 1H), 4.11 - 4.03 (m, 1H), 3.98 - 3.85 (m, 2H), 3.80 (td, *J =* 10.0, 4.9 Hz, 2H), 3.56 - 3.44 (m, 1H), 2.42 (s, 3H), 1.21 (s, 3H), 1.19 (s, 3H).

### Step D: Synthesis of (S)-2-azido-1-((R)-2,2-dimethyl-1,3-dioxolan-4-yl)ethan-1-ol.

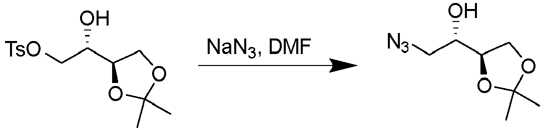

To a solution of (*S*)-2-((*R*)-2,2-dimethyl-1,3-dioxolan-4-yl)-2-hydroxyethyl 4-methylbenzenesulfonate (59 g, 186.6 mmol, 1.0 eq.) in dry DMF (500 mL) was added NaN₃ (36.38 g, 559.7 mmol, 3.0 eq) at r.t. The mixture was warmed to 80°C and stirred overnight. The resulting mixture was poured into sat'd. aq. Na₂CO₃ (2000 mL) and extracted with ethyl acetate (1000 mL × 2). The combined the organic phases were washed with sat'd. aq. NH₄Cl (1000 mL × 2), dried over MgSO₄, filtered and concentrated. The residue was purified by flash column chromatography, eluting with petroleum ether/ethyl acetate (8:1) to give (S)-2-azido-1-((R)-2,2-dimethyl-1,3-dioxolan-4-yl)ethan-1-ol (34 g, yield 97.4%). ¹H NMR (400 MHz, DMSO) δ 5.54 (d, *J =* 5.8 Hz, 1H), 3.99 (dd, *J =* 7.7, 5.9 Hz, 1H), 3.92 - 3.79 (m, 2H), 3.53 (ddd, *J =* 8.3, 6.5, 2.8 Hz, 1H), 3.32 (m, 1H), 3.22 (dd, *J =* 12.8, 6.5 Hz, 1H), 1.30 (s, 3H), 1.25 (s, 3H).

### Step E: Synthesis of (R)-4-((R)-2-azido-1-fluoroethyl)-2,2-dimethyl-1,3-dioxolane.

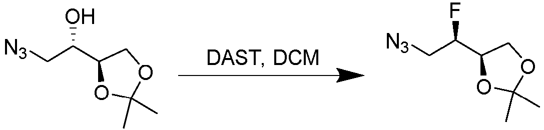

To a solution of (*S*)-2-azido-1-((*R*)-2,2-dimethyl-1,3-dioxolan-4-yl)ethan-1-ol (34 g, 0.18 mol, 1.0 eq) in dry DCM (170 mL) was added dropwise a solution of DAST (73 mL, 0.54 mol, 3.0 eq.) at -20°C, then the reaction mixture was warmed to r.t. and stirred overnight. The resulting mixture was slowly poured into sat'd. aq. NaHCO3 (1500 mL) and extracted with DCM (500 mL × 2), the combined organic phases dried over MgSO₄, filtered, and concentrated. The residue was purified by flash column chromatography, eluting with petroleum ether/ethyl acetate (30:1) to give (R)-4-((R)-2-azido-1-fluoroethyl)-2,2-dimethyl-1,3-dioxolane (3.4 g, yield 9.9%). ¹H NMR (400 MHz, CDCl₃) δ 4.57 (ddt, *J =* 48.0, 7.4, 3.6 Hz, 1H), 4.24 (dtd, *J =* 21.4, 6.7, 3.6 Hz, 1H), 4.14 - 4.05 (m, 1H), 3.94 (dd, *J =* 8.4, 6.5 Hz, 1H), 3.63 (ddd, *J =* 17.0, 13.5, 7.5 Hz, 1H), 3.44 (ddd, *J =* 27.4, 13.5, 3.6 Hz, 1H), 1.44 (s, 3H), 1.38 (s, 3H). ¹⁹F NMR (400 MHz, CDCl₃) δ 198.91 (s, 1 F).

### Step F: Synthesis of (2R,3R)-4-azido-3-fluorobutane-1,2-diol.

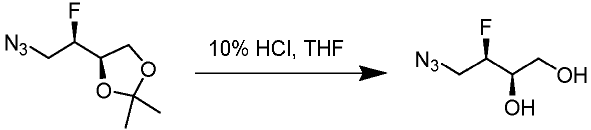

To a solution of (*R*)-4-((*R*)-2-azido-1-fluoroethyl)-2,2-dimethyl-1,3-dioxolane (3.4 g, 18.0 mmol, 1.0 eq.) in THF (30 mL) was added dropwise 10% HCl (4 mL) at 0 °C. The mixture was allowed to warm to r.t. and stirred overnight. The organic solvent was removed, and the residue was basified with sat'd. aq. Na₂CO₃ to a pH=9, extracted with EA (40 mL × 4), dried over MgSO₄, filtered, and concentrated. The residue was purified by flash column chromatography, eluting with petroleum ether/ethyl acetate (1:1) to give (2R,3R)-4-azido-3-fluorobutane-1,2-diol (2.5 g, yield: 93.3%). ¹H NMR (400 MHz, DMSO) δ 5.17 (d, *J =* 5.9 Hz, 1H), 4.86 (t, *J =* 5.5 Hz, 1H), 4.74 (ddt, *J =* 48.8, 8.4, 2.9 Hz, 1H), 3.79 - 3.44 (m, 5H). ¹⁹F NMR (400 MHz, DMSO-*d*₆) δ 202.16 (s, 1 F).

### Step G: Synthesis of (2R,3R)-4-azido-1-((tert-butyldimethylsilyl)oxy)-3-fluorobutan-2-ol.

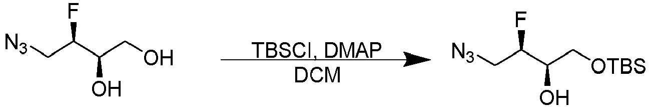

To a solution of (2*R*,3*R*)-4-azido-3-fluorobutane-1,2-diol (2.5 g, 16.7 mmol, 1.0 eq.) in DCM (50 mL) was added DMAP (4.51 g, 36.9 mmol, 2.2 eq.), followed by TBS-Cl (2.77 g, 18.5 mmol, 1.1 eq) in portions at 0°C. The reaction mixture was allowed to warm to r.t. and stirred for 3 days. The organic solvent was evaporated and the residue purified by flash chromatography, eluting with petroleum ether/ethyl acetate (10:1) to give (2*R*,3*R*)-4-azido-1-((tert-butyldimethylsilyl)oxy)-3-fluorobutan-2-ol (2.6 g, yield: 59.1%). ¹H NMR (400 MHz, DMSO) δ 5.17 (d, *J =* 5.7 Hz, 1H), 4.71 - 4.50 (m, 1H), 3.73 - 3.37 (m, 5H), 0.82 (s, 9H), 0.00 (s, 6H). ¹⁹F NMR (400 MHz, DMSO-*d*₆) δ 203.48 (s, 1 F).

### Step H: Synthesis of ((2R,3R)-4-azido-3-fluoro-2-((4-methoxybenzyl)oxy)butoxy)(tert-butyl)dimethylsilane.

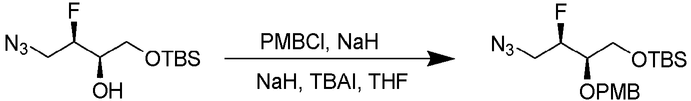

To a solution of (2*R*,3*R*)-4-azido-1-((tert-butyldimethylsilyl)oxy)-3-fluorobutan-2-ol (2.6 g, 9.88 mmol, 1.0 eq.) in THF (250 mL) was added NaH (474 mg, 11.86 mmol, 1.2 eq. 60%) in portions at 0°C. After stirring at 0°C for 30 mins, TBAI (3.64 g, 9.88 mmol, 1.0 eq.) was added, followed by dropwise addition of PMB-Cl (2.0 mL, 14.73 mmol, 1.5 eq.) at 0°C. The reaction was then warmed to r.t. and allowed to stir overnight. The mixture was poured into sat'd. aq. NH₄Cl, and extracted with EA (50 mL × 3). The extracts were dried over MgSO₄, filtered, and concentrated. The residue was purified by flash chromatography, eluting with petroleum ether/ethyl acetate (40:1) to give ((2*R*,3*R*)-4-azido-3-fluoro-2-((4-methoxybenzyl)oxy)butoxy)(tert-butyl)dimethylsilane (2.5 g, yield: 66.1%). ¹H NMR (400 MHz, Dioxane) δ 8.31 (d, *J* = 8.5 Hz, 2H), 7.95 (d, *J =* 8.6 Hz, 2H), 5.90 - 5.69 (m, 1H), 5.58 (dd, *J=* 40.1, 11.2 Hz, 2H), 4.79 (s, 3H), 4.78 - 4.48 (m, 5H), 1.91 (s, 9H), 1.09 (s, 6H). ¹⁹F NMR (400 MHz, DMSO-*d*₆) δ 199.15 (s, 1 F).

### Step I: Synthesis of (2R,3R)-4-azido-3-fluoro-2-((4-methoxybenzyl)oxy)butan-1-ol.

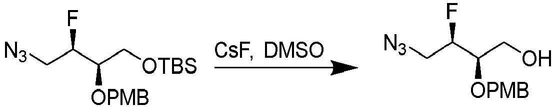

To a solution of ((2R,3R)-4-azido-3-fluoro-2-((4-methoxybenzyl)oxy)butoxy)(tert-butyl)dimethylsilane (2.5 g, 6.48 mmol, 1.0 eq.) in DMSO (15 mL) was added CsF (2.98 g, 19.44 mmol, 3.0 eq.), followed by 5 drops of MeOH. The mixture was stirred overnight at r.t., then poured into sat'd NaCl (40 mL), extracted with EA (40 mL × 3), the extracts dried over MgSO₄, filtered, and concentrated. The residue was purified by flash chromatography, eluting with petroleum ether/ethyl acetate (4:1) to give (2R,3R)-4-azido-3-fluoro-2-((4-methoxybenzyl)oxy)butan-1-ol (1 g, yield: 56.8%). ¹H NMR (400 MHz, DMSO) δ 7.35 - 7.20 (m, 2H), 7.01 - 6.83 (m, 2H), 5.76 (s, 3H), 4.93 (t, *J=* 5.3 Hz, 1H), 4.74 (ddd, *J =* 25.8, 8.0, 5.6 Hz, 1H), 4.52 (dd, *J =* 49.2, 11.3 Hz, 2H), 3.74 (s, 3H), 3.69 (ddd, *J =* 18.8, 13.9, 8.1 Hz, 1H), 3.58 - 3.42 (m, 4H). ¹⁹F NMR (400 MHz, DMSO-*d*₆) δ 199.45 (s, 1 F).

### Step J: Synthesis of (2R,3R)-4-azido-3-fluoro-2-((4-methoxybenzyl)oxy)butanoic acid (F-HABA).

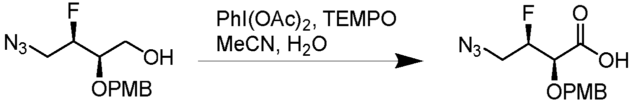

To a solution of (2*R*,3*R*)-4-azido-3-fluoro-2-((4-methoxybenzyl)oxy)butan-1-ol (1 g, 3.76 mmol, 1.0 eq.) in ACN/H₂O (10 mL/10 mL) was added PhI(OAc)₂ (2.99 g, 9.41 mmol, 2.5 eq.), followed by TEMPO (292 mg, 1.88 mmol, 0.5 eq.). The mixture was stirred overnight at r.t. Sodium hydroxide (2N, 10 mL) was added, and the mixture extracted with PE (20 mL × 4) and the extracts discarded. The water phase was acidified with 2N HCl to a pH of 3, and extracted with EtOAc (30 mL × 4). The extracts were dried over Na₂SO₄ and concentrated. The residue was purified by flash chromatography, eluting with DCM/MeOH (20:1, v/v) to give (2R,3R)-4-azido-3-fluoro-2-((4-methoxybenzyl)oxy)butanoic acid (500 mg, yield: 47.5%). ¹H NMR (400 MHz, DMSO) δ 7.30 (d, *J =* 8.7 Hz, 2H), 6.93 (d, *J =* 8.7 Hz, 2H), 5.11 - 4.86 (m, 1H), 4.52 (dd, *J =* 119.7, 11.0 Hz, 2H), 4.19 (dd, *J =* 31.5, 2.6 Hz, 1H), 3.76 (s, 3H), 3.75 - 3.65 (m, 2H). ¹⁹F NMR (400 MHz, DMSO-*d*₆) δ 196.81 (s, 1 F)

### Example 6. Synthesis of (2R,3R)-4-amino-N-((1R,2S,3S,4R,5R,6S)-3-amino-5-(((2S,3R,4S,5R)-4-(((2R,3R,4R,5S,6S)-3-amino-6-(aminomethyl)-4,5-dihydroxytetrahydro-2H-pyran-2-yl)oxy)-3-hydroxy-5-(hydroxymethyl)tetrahydrofuran-2-yl)oxy)-4-(((2R,3R,6S)-3-amino-6-(aminomethyl)tetrahydro-2H-pyran-2-yl)oxy)-2,6-dihydroxycyclohexyl)-3-fluoro-2-hydroxybutanamide (Compound 1).

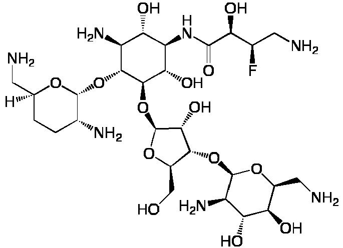

### Step A: Synthesis of (3R,6S)-3-azido-6-((benzyl((benzyloxy)carbonyl)amino)methyl) tetrahydro-2H-pyran-2-yl (E)-2,2,2-trifluoro-N-phenylacetimidate

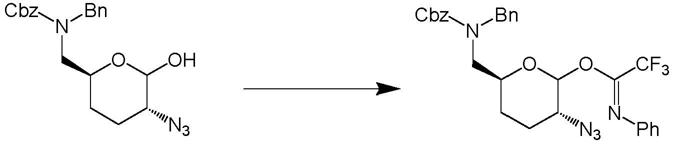

A mixture of benzyl N-[[(2S,5R)-5-azido-6-hydroxy-tetrahydropyran-2-yl]methyl]-N-benzyl-carbamate (Example 1; 1.1 g, 2.50 mmol), Cs₂CO₃ (2.44 g, 7.49 mmol) and (1E)-2,2,2-trifluoro-N-phenyl-acetimidoyl chloride (1.2 mL, 7.49 mmol) in DCM (20 mL) was stirred at rt for 18 h. The mixture was filtered, rinsed with DCM and concentrated under reduced pressure. The material was purified by silica gel chromatography (80 g cartridge) using a gradient of 0 to 30% EtOAc in hexane to provide the title product (1.2 g, 85%) as a syrup. m/z (ESI) [M+Na]⁺ 590.3, HPLC (A05) tR=3.02 min.

### Step B: Synthesis of (1S,2S,3R,4S,5S,6R)-6-(((3R,6S)-3-azido-6-((benzyl((benzyloxy)carbonyl)amino)methyl)tetrahydro-2H-pyran-2-yl)oxy)-3,5-bis(((benzyloxy)carbonyl)amino)cyclohexane-1,2,4-triyl triacetate

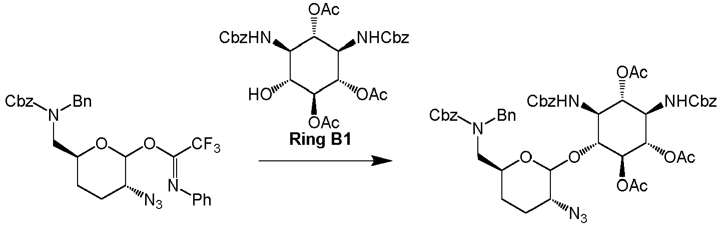

[(3R,6S)-3-azido-6-[[benzyl(benzyloxycarbonyl)amino]methyl]tetrahydropyran-2-yl] (1E)-2,2,2-trifluoro-N-phenyl-ethanimidate (1.2 g, 2.12 mmol) and [(1S,2R,3S,4S,5R,6R)-3,4-diacetoxy-2,6-bis(benzyloxycarbonylamino)-5-hydroxy-cyclohexyl]acetate (**Ring B1**; Example 4; 1.816 g, 3.17 mmol) were co-evaporated with anhydrous toluene (2 x 5 mL). The mixture was dried under reduced pressure for 30 min. The residue was taken in anhydrous DCM (100 mL) and then transferred by cannula to a RBF charged with pre-activated 3Å and 4AÅ sieves. The mixture was stirred at rt for 20 min, then cooled to -25°C. Bi(OTf)₃ (1.526 g, 2.32 mmol) was added in one portion and the mixture was stirred - 25°C for 20 min, and at rt for 2 h. The mixture was diluted by saturated aqueous NH₄Cl solution (50 mL) and DCM (100 mL), then filtered (diatomaceous earth). The separated aqueous layer was extracted with DCM (3 x 50 mL). The combined organic layers were washed brine (20 mL), dried (MgSO₄), filtered and concentrated under reduced pressure. The material was purified by reversed phase chromatography using a gradient of 5 to 100% ammonium formate (10 mM, pH 4) in MeCN, and lyophilized to provide the title product (1.22 g, 59%) as an anomeric mixture (α/β=~2.7:1). m/z (ESI) [M+H]⁺: 951.5, HPLC tR= 2.82 min.

### Step C: Synthesis of dibenzyl ((1R,2R,3S,4R,5R,6S-4-(((3R,6S)-3-azido-6-((benzyl((benzyloxy)carbonyl)amino)methyl)tetrahydro-2H-pyran-2-yl)oxy)-2,5,6-trihydroxycyclohexane-1,3-diyl)dicarbamate

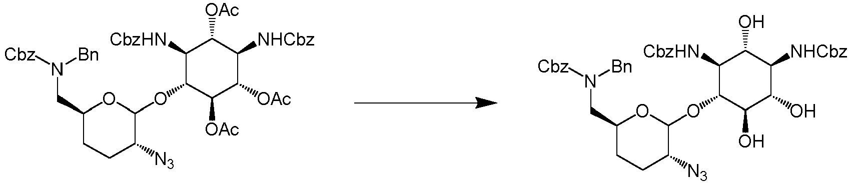

To a suspension of [(1S,2R,3S,4S,5R,6S)-3,4-diacetoxy-5-[(3R,6S)-3-azido-6-[[benzyl (benzyloxycarbonyl)amino]methyl]tetrahydropyran-2-yl]oxy-2,6-bis(benzyloxycarbonyl-amino) cyclohexyl] acetate (1.22 g, 1.28 mmol) in MeOH (30 mL) was added K₂CO₃ (887 mg, 6.41 mmol). The suspension was stirred at rt for 80 min, then filtered. The filtrate was concentrated under reduced pressure (bath temperature <30 °C) to give a residue. Water (15 mL) was added to form a precipitate, which was sonicated, filtered and washed with water (50 mL). The solid was collected, dissolved in DCM/MeOH (50 mL, 10:1), dried (MgSO₄), filtered and concentrated under reduced pressure to provide title compound (1.06 g, crude, *quant.*) as a solid. *m*/*z* (ESI) [M+H]⁺; 825.4, HPLC A(05) tR=2.63 min.

### Step D: Synthesis of dibenzyl ((3aS,4R,5S,6S,7R,7aS)-4-(((2R,3R,6S)-3-azido-6-((benzyl((benzyloxy)carbonyl)amino)methyl)tetrahydro-2H-pyran-2-yl)oxy)-6-hydroxyhexahydrospiro[benzo[d][1,3]dioxole-2,1'-cyclohexane]-5,7-diyl)dicarbamate

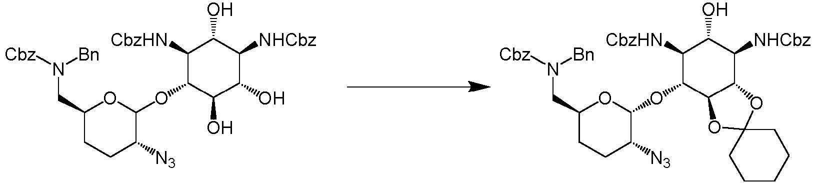

A mixture of benzyl N-[[(2S,5R)-5-azido-6-[(1R,2S,3R,4R,5S,6R)-2,4-bis(benzyloxycarbonylamino)-3,5,6-trihydroxy-cyclohexoxy]tetrahydropyran-2-yl]methyl]-N-benzyl-carbamate (1 g, 1.21 mmol), 1,1-dimethoxycyclohexane (1.11 mL, 7.27 mmol) and PPTS (122 mg, 0.485 mmol, dried on V10 via high boiling method before using) in dry DME (30 mL) was stirred at 90 °C for 2 h. The mixture was cooled, then concentrated under reduced pressure. The material was purified by reversed phase column on ACCQ using 65-87% Ammonium formate pH 4 in MeCN to provide the title compound as a solid (633 mg, 58%, α anomer), β anomer (250 mg, 23%). m/z: ES+ [M+H]⁺ 905.1; LCMS (A05); tR = 2.99 min (α anomer).

### Step E: Synthesis of dibenzyl ((3aS,4R,5S,6S,7R,7aS)-4-(((2R,3R,6S)-3-azido-6-((benzyl((benzyloxy)carbonyl)amino)methyl)tetrahydro-2H-pyran-2-yl)oxy)-6-(benzyloxy)hexahydrospiro[benzo[d][1,3]dioxole-2,1'-cyclohexane]-5,7-diyl)dicarbamate

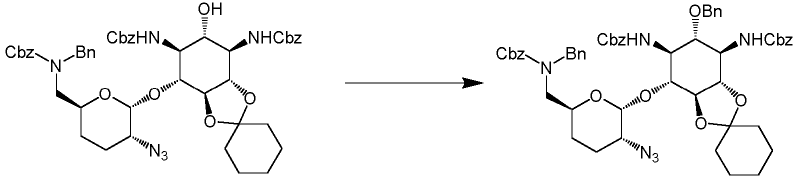

NaH (60% oil dispersion, 64.5 mg, 1.62 mmol) was added to a mixture of dibenzyl ((3a*S*,4*R*,5*S*,6*S*,7*R*,7a*S*)-4-(((2*R*,3*R*,6*S*)-3-azido-6-((benzyl((benzyloxy)carbonyl)amino)methyl) tetrahydro-2*H*-pyran-2-yl)oxy)-6-hydroxyhexahydrospiro[benzo[*d*][1,3]dioxole-2,1'-cyclohexane]-5,7-diyl)dicarbamate (630 mg, 0.644 mmol) and BnBr (0.94 mL, 7.85 mmol) in THF (20 mL) at 0 °C under nitrogen. The mixture was stirred at 0 °C for 3 h, then AcOH (0.1 mL, 1.29 mmol) and EtOAc (30 mL) were added. The mixture was concentrated under reduced pressure. The residue was purified by silica gel chromatography (40 g cartridge) using a gradient of 0 to 50% EtOAc in hexanes to provide the title compound as a solid (630 mg, 91%). m/z: ES+ [M+H]⁺ 995.5; LCMS (A05); tR = 3.13 min.

### Step F: Synthesis of dibenzyl ((1S,2R,3S,4R,5R,6S)-4-(((2R,3R,6S)-3-azido-6-((benzyl((benzyloxy)carbonyl)amino)methyl)tetrahydro-2H-pyran-2-yl)oxy)-2-(benzyloxy)-5,6-dihydroxycyclohexane-1,3-diyl)dicarbamate

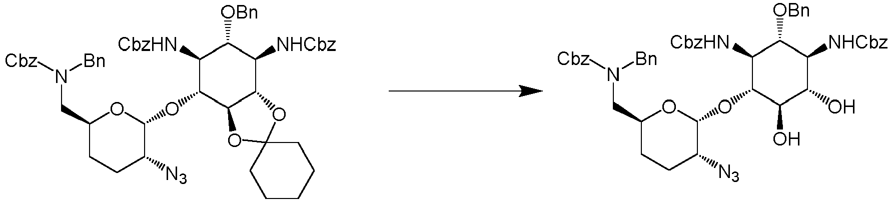

**A** solution of dibenzyl ((3aS,4R,5S,6S,7R,7aS)-4-(((2R,3R,6S)-3-azido-6-((benzyl((benzyloxy)carbonyl)amino)methyl)tetrahydro-2H-pyran-2-yl)oxy)-6-(benzyloxy)hexahydrospiro[benzo[d][1,3]dioxole-2,1'-cyclohexane]-5,7-diyl)dicarbamate (630 mg, 0.633 mmol) in THF (12 mL), water (3.5 mL) and AcOH (8 mL) was heated at 80 °C for 9 h, then cooled to rt. The mixture was diluted by slow addition of a saturated aqueous solution NaHCO₃ (~50 mL) at 0 °C and stirred for 20 min. The separated aqueous layer was extracted with EtOAc (3 x 50 mL). The combined organic layer was washed with saturated aqueous solution NaHCO₃ (3 x 10 mL), water (3 x10 mL), brine (2 x 10 mL), dried (MgSO₄), filtered and concentrated under reduced pressure to provide the title product (570 mg, 98%) as off-white solid. This material was used directly without further purification. m/z (ESI) [M+H]⁺: 915.4, HPLC (A05) tR = 2.85 min.

### Step G: Synthesis of benzyl (((2S,5R,6R)-5-azido-6-(((3aR,4S,5S,6R,7S,7aS)-4-(benzyloxy)-5-(((benzyloxy)carbonyl)amino)-7-hydroxy-2-oxooctahydrobenzo[d]oxazol-6-yl)oxy)tetrahydro-2H-pyran-2-yl)methyl)(benzyl)carbamate (Ring AB1)

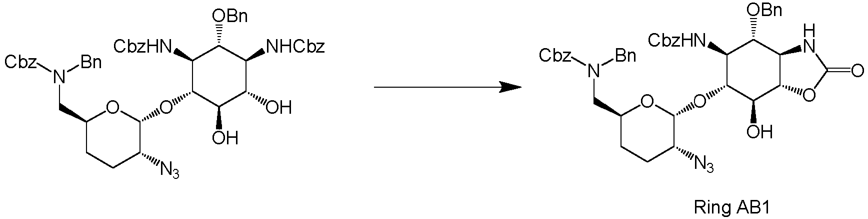

Sodium hydride (60% oil dispersion, 125 mg, 3.11 mmol,) was added to a solution of dibenzyl ((1S,2R,3S,4R,5R,6S)-4-(((2R,3R,6S)-3-azido-6-((benzyl((benzyloxy)carbonyl)amino)methyl)tetrahydro-2H-pyran-2-yl)oxy)-2-(benzyloxy)-5,6-dihydroxycyclohexane-1,3-diyl)dicarbamate (570 mg, 0.623 mmol) in dry DMF (9 mL) at 0 °C. The mixture was stirred at 0 °C for 3 h, then diluted by AcOH (0.28 mL, 4.83 mmol), and concentrated under reduced pressure. The material was purified on silica gel (40 g) by MPLC using a gradient of 0 to 100% EtOAc in hexane to provide title compound (440 mg, 88% yield) as a solid. m/z (ESI) [M+H]⁺: 807.4, HPLC (A05) tR = 2.77 min.

### Step H: Synthesis of (2S,3R,4R,5R,6R)-6-(((2R,3R,4R,5S)-4-acetoxy-2-(acetoxymethyl)-5-(((3aR,4S,5S,6R,7S,7aS)-6-(((2R,3R,6S)-3-azido-6-((benzyl((benzyloxy)carbonyl)amino)methyl)tetrahydro-2H-pyran-2-yl)oxy)-4-(benzyloxy)-5-(((benzyloxy)carbonyl)amino)-2-oxooctahydrobenzo[d]oxazol-7-yl)oxy)tetrahydrofuran-3-yl)oxy)-5-azido-2-(azidomethyl)tetrahydro-2H-pyran-3,4-diyl diacetate

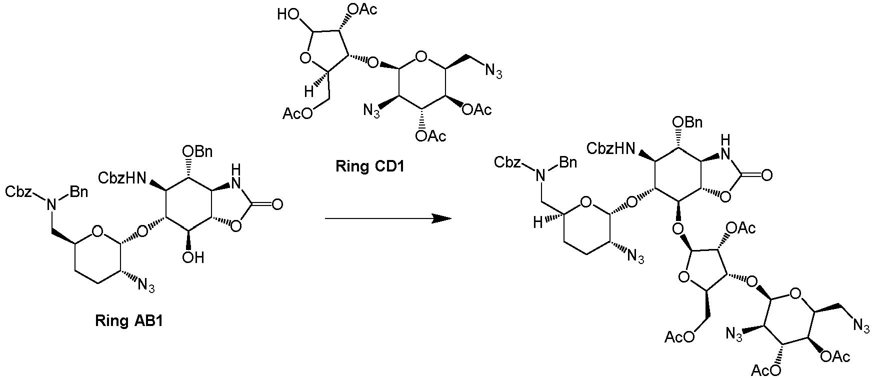

To a mixture of [(2R,3R,4R)-4-acetoxy-3-[(2R,3R,4R,5R,6S)-4,5-diacetoxy-3-azido-6-(azido methyl)tetrahydropyran-2-yl]oxy-5-hydroxy-tetrahydrofuran-2-yl]methyl acetate (**Ring CD1**, International Patent Application Publication No. WO2019194858; 1157 mg, 2.18 mmol) and K₂CO₃ (452.4 mg, 3.272 mmol) in dry DCM (15 mL) was added CCl₃CN (0.328 mL, 3.27 mmol). The mixture was stirred at rt for 18 h, then filtered on diatomaceous earth, rinsed with CH₂Cl₂, and concentrated under reduced pressure. Benzyl N-[[(2S,5R,6R)-6-[[(3aR,4S,5S,6R,7S,7aS)-4-benzyloxy-5-(benzyloxycarbonylamino)-7-hydroxy-2-oxo-3a,4,5,6,7,7a-hexahydro-3H-1,3-benzoxazol-6-yl]oxy]-5-azido-tetrahydropyran-2-yl]methyl]-N-benzyl-carbamate (**Ring AB1**; 440 mg, 0.545 mmol) in dry DCM (30 mL) was added to the previous crude material, then transferred to a RBF charged with pre-activated 3Åand 4ÅA sieves. The mixture was cooled to -78 °C, then BF₃°OEt₂ (1.02 mL, 8.23 mmol) was added dropwise at -78 °C. Upon completion of the addition, the dry ice bath was removed immediately. The mixture was stirred at rt for 1 h, then diluted by a saturated aqueous solution of NaHCO₃ (15 mL). The mixture was filtered through diatomaceous earth and the solid was rinsed with DCM (200 mL) The separated aqueous layer was extracted with DCM (3 x 20 mL). The combined organic layers were washed with brine (15 mL), dried (MgSO₄), filtered and concentrated under reduced pressure. The material was purified by reversed phase column (ACCQ) using a gradient of 64%-85% MeCN in Ammonium formate (10 mM, pH 4) to provide the title product (455 mg, 63%). m/z (ESI) [M+H]⁺: 1319.5, HPLC (A05) tR=2.95 min.

### Step I: Synthesis of (2S,3R,4R,5R,6R)-6-(((2R,3R,4R,5S)-4-acetoxy-2-(acetoxymethyl)-5-((1R,2S,3S,4R,5S,6R)-3-amino-6-(((2R,3R,6S)-3-azido-6-((benzyl((benzyloxy)carbonyl)amino)methyl)tetrahydro-2H-pyran-2-yl)oxy)-4-(benzyloxy)-5-(((benzyloxy)carbonyl)amino)-2-hydroxycyclohexyl)oxy)tetrahydrofuran-3-yl)oxy)-5-azido-2-(azidomethyl)tetrahydro-2H-pyran-3,4-diyl diacetate

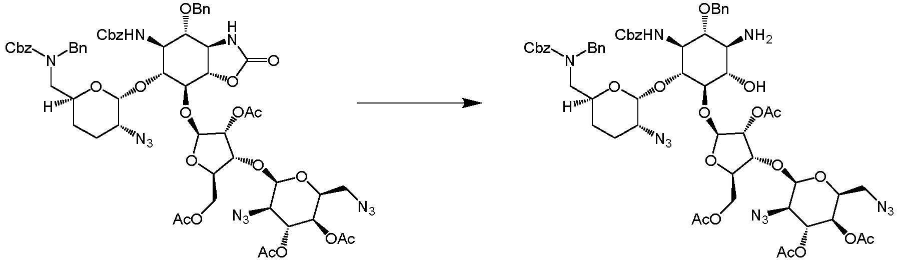

LiOH·H₂O (434 mg, 10.35 mmol) was added to a solution of [(2R,3R,4R,5S)-5-[[(3aR,4S,5S,6R,7S,7aS)-6-[(2R,3R,6S)-3-azido-6 [[benzyl(benzyloxycarbonyl)amino]methyl]-tetrahydropyran-2-yl]oxy-4-benzyloxy-5-(benzyloxycarbonylamino)-2-oxo-3a,4,5,6,7,7a-hexahydro-3H-1,3-benzoxazol-7-yl]oxy]-4-acetoxy-3-[(2R,3R,4R,5R,6S)-4,5-diacetoxy-3-azido-6-(azidomethyl)tetrahydropyran-2-yl]oxy-tetrahydrofuran-2-yl]methyl acetate (455 mg, 0.345 mmol) in 1,4-dioxane (12 mL) and water (6 mL). The mixture was stirred at 40 °C for 18 h, then cooled to rt and diluted by EtOAc (60 mL). The separated aqueous layer was extracted with EtOAc (3 x 20 mL). The combined organic layers were washed with water (3 x 5 mL), brine (10 mL), dried (MgSO₄), filtered, concentrated under reduced pressure to provide the title compound (382 mg, crude, 98%) as an off-white solid. The material was used directly without further purification. m/z (ESI) [M+H]⁺: 1125.5, HPLC (A05) tR=2.43 min.

### Step J: Synthesis of (2S,3R,4R,5R,6R)-6-(((2R,3R,4R,5S)-4-acetoxy-2-(acetoxymethyl)-5-(((1R,2S,3S,4R,5S,6R)-3-((2R,3R)-4-azido-3-fluoro-2-((4-methoxybenzyl)oxy)butanamido)-6-(((2R,3R,6S)-3-azido-6-((benzyl((benzyloxy)carbonyl)amino)methyl)tetrahydro-2H-pyran-2-yl)oxy)-4-(benzyloxy)-5-(((benzyloxy)carbonyl)amino)-2-hydroxycyclohexyl)oxy)tetrahydrofuran-3-yl)oxy)-5-azido-2-(azidomethyl)tetrahydro-2H-pyran-3,4-diyl diacetate

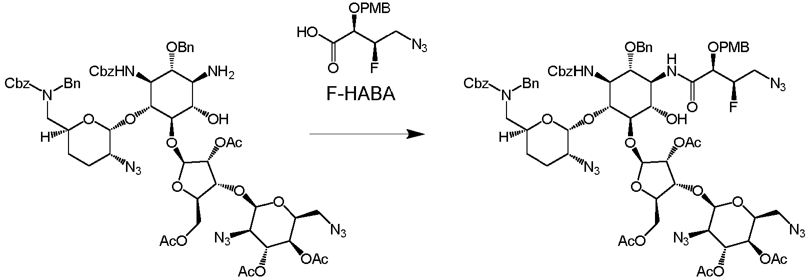

To a solution of benzyl N-[[(2S,5R,6R)-6-[(1R,2R,3S,4S,5R,6S)-4-amino-2-[(2S,3R,4S,5R)-4-[(2R,3R,4R,5S,6S)-3-azido-6-(azidomethyl)-4,5-dihydroxy-tetrahydropyran-2-yl]oxy-3-hydroxy-5-(hydroxymethyl)tetrahydrofuran-2-yl]oxy-5-benzyloxy-6-(benzyloxycarbonylamino)-3-hydroxy-cyclohexoxy]-5-azido-tetrahydropyran-2-yl]methyl]-N-benzyl-carbamate (180 mg, 0.160 mmol) and (2R,3R)-4-azido-3-fluoro-2-[(4-methoxyphenyl)methoxy]butanoic acid (F-HABA, Example 5; 77.7 mg, 0.274 mmol) in anhydrous THF (12 mL) was added DMTMM chloride (77.7 mg, 0.281 mmol). The mixture was stirred at rt for 18 h, then diluted by a saturated aqueous NH₄Cl solution (5 mL) and DCM (30 mL). The separated aqueous layer was extracted with DCM (3 x 10 mL). The combined organic layers were washed with brine (5 mL), dried (MgSO₄), filtered and concentrated under reduced pressure. The material was purified by reversed phase column using a gradient of 10 to 100% MeCN in 10 mM Ammonium formate pH 3.8 and lyophilized to provide title compound (187 mg, 84%) as a white solid. m/z (ESI) [M+H]⁺: 1390.6, HPLC A(05) tR=2.85 min.

### Step K: Synthesis of (2R,3R)-4-amino-N-((1R,2S,3S,4R,5R,6S)-3-amino-5-(((2S,3R,4S,5R)-4-(((2R,3R,4R,5S,6S)-3-amino-6-(aminomethyl)-4,5-dihydroxytetrahydro-2H-pyran-2-yl)oxy)-3-hydroxy-5-(hydroxymethyl)tetrahydrofuran-2-yl)oxy)-4-(((2R,3R,6S)-3-amino-6-(aminomethyl)tetrahydro-2H-pyran-2-yl)oxy)-2,6-dihydroxycyclohexyl)-3-fluoro-2-hydroxybutanamide (Compound 1).

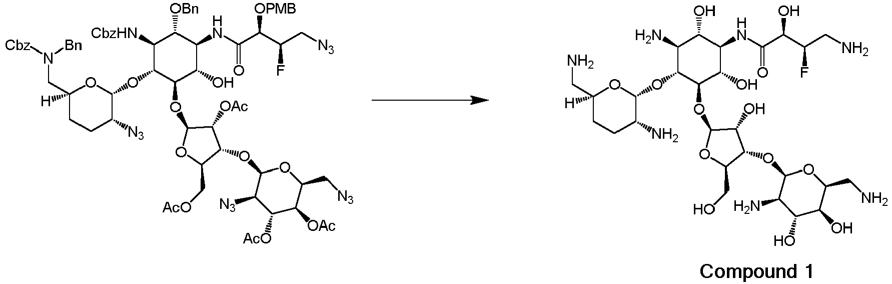

A solution of benzyl N-[[(2S,5R,6R)-5-azido-6-[(1R,2R,3S,4S,5R,6S)-2-[(2S,3R,4S,5R)-4-[(2R,3R,4R,5S,6S)-3-azido-6-(azidomethyl)-4,5-dihydroxy-tetrahydropyran-2-yl]oxy-3-hydroxy-5-(hydroxymethyl)tetrahydrofuran-2-yl]oxy-4-[[(2R,3R)-4-azido-3-fluoro-2-[(4-methoxyphenyl)methoxy]butanoyl]amino]-5-benzyloxy-6-(benzyloxycarbonylamino)-3-hydroxy-cyclohexoxy]tetrahydropyran-2-yl]methyl]-N-benzyl-carbamate (187 mg, 134 µmol) in MeOH (6 mL) was added to a suspension of Pd(OH)₂ (20% wet, 652 mg, 0.93 mmol) in a mixture of AcOH (4.8 mL) and water (2 mL). The mixture was degassed by 3 x vacuum-H₂ cycle, then bubbled with hydrogen for 10 min. The mixture was stirred under hydrogen atmosphere (1 atm) for 3 h, then degassed with N₂ for 5 min. The suspension was filtered on 0.40 µM PVDF filter which was washed with MeOH (5 x 8 mL) and water (5 x 10 mL), and the volatiles were evaporated under reduced pressure (rotavapor bath temperature <35 °C). The material was purified by prep-HPLC using a gradient of 5 to 15% MeCN in 10 mM NH₄HCO₃ pH 10 and lyophilized to give a solid (100 mg). H₂SO₄ (0.05 M in H₂O) was added dropwise to a solution of the above solid in H₂O (3 mL) at 0 °C until pH 4, then lyophilized. The solid was dissolved in water (2 mL), and MeOH (10 mL) was added to give precipitate. This precipitation was filtered through PVDF syringe filter and washed with MeOH (2 x 5 mL). The mother liquor was discarded and the solid was dissolved with water (5 mL), and the solution was lyophilized to give the title product **Compound 1** (67.2 mg, 49%) as an off-white solid. ¹H NMR (400 MHz, D₂O) δ 5.77 (d, J = 3.5 Hz, 1H), 5.24 (d, J = 1.9 Hz, 1H), 5.22 - 5.02 (m, 2H), 4.47 - 4.35 (m, 1H), 4.33 - 4.24 (m, 2H), 4.21 - 4.15 (m, 1H), 4.14 - 3.94 (m, 4H), 3.89 - 3.74 (m, 3H), 3.72 - 3.55 (m, 3H), 3.55 - 3.47 (m, 1H), 3.47 - 3.04 (m, 8H), 2.91 (dd, J = 13.4, 8.2 Hz, 1H), 2.01 - 1.69 (m, 3H), 1.44 (t, J = 12.6 Hz, 1H). Note: 26 exchangeable protons are not observed.

### Example 7. Synthesis of (S)-4-amino-N-((1R,2S,3S,4R,5R,6S)-3-amino-5-(((2S,3R,4S,5R)-4-(((2R,3R,4R,5S,6S)-3-amino-6-(aminomethyl)-4,5-dihydroxytetrahydro-2H-pyran-2-yl)oxy)-3-hydroxy-5-(hydroxymethyl)tetrahydrofuran-2-yl)oxy)-4-(((2R,3R,6S)-3-amino-6-(aminomethyl)tetrahydro-2H-pyran-2-yl)oxy)-2,6-dihydroxycyclohexyl)-2-hydroxybutanamide (Compound 2).

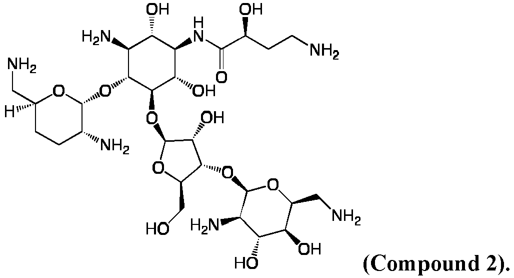

### Step A: Synthesis of (2S,3R,4R,5R,6R)-6-(((2R,3R,4R,5S)-4-acetoxy-2-(acetoxymethyl)-5-(((1R,2R,3S,4R,5S,6S)-2-(((2R,3R,6S)-3-azido-6-((benzyl((benzyloxy)carbonyl)amino)methyl)tetrahydro-2H-pyran-2-yl)oxy)-4-(benzyloxy)-5-((S)-2-(benzyloxy)-4-(((benzyloxy)carbonyl)amino)butanamido)-3-(((benzyloxy)carbonyl)amino)-6-hydroxycyclohexyl)oxy)tetrahydrofuran-3-yl)oxy)-5-azido-2-(azidomethyl)tetrahydro-2H-pyran-3,4-diyl diacetate

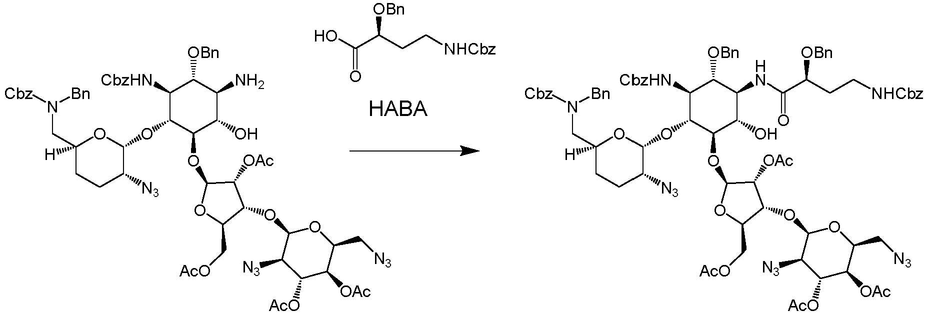

To a solution of (2*S*,3*R*,4*R*,5*R*,6*R*)-6-(((2*R*,3*R*,4*R*,5*S*)-4-acetoxy-2-(acetoxymethyl)-5-(((1*R*,2*S*,3*S*,4*R*,5*S*,6*R*)-3-amino-6-(((2*R*,3*R*,6*S*)-3-azido-6-((benzyl((benzyloxy)carbonyl)amino)methyl)tetrahydro-2*H*-pyran-2-yl)oxy)-4-(benzyloxy)-5-(((benzyloxy)carbonyl)amino)-2-hydroxycyclohexyl)oxy)tetrahydrofuran-3-yl)oxy)-5-azido-2-(azidomethyl)tetrahydro-2*H*-pyran-3,4-diyl diacetate (Example 6, Step I; 220 mg, 0.196 mmol), (2S)-2-benzyloxy-4-(benzyloxycarbonylamino)butanoic acid (HABA; 101 mg, 0.293 mmol), and DIPEA (0.10 mL, 0.587 mmol) in anhydrous DMF (4 mL) was added PyBOP (122 mg, 0.235 mmol) at 0°C. The mixture was stirred at rt for 1.5 h, then poured into water (5 mL) and EtOAc (5 mL). The separated aqueous layer was extracted with EtOAc (3 x 5 mL). The combined organic layers were washed with brine (5 mL), dried (MgSO₄), filtered and concentrated under reduced pressure. The material was purified by reversed phase column (ACCQ) using ACN and ammonium formate (10 mM, pH 3.8) and lyophilized to provide the titled compound (142 mg, 50%) as a white solid. M+H+: 1451.7

### Step B: Synthesis of (S)-4-amino-N-((1R,2S,3S,4R,5R,6S)-3-amino-5-(((2S,3R,4S,5R)-4-(((2R,3R,4R,5S,6S)-3-amino-6-(aminomethyl)-4,5-dihydroxytetrahydro-2H-pyran-2-yl)oxy)-3-hydroxy-5-(hydroxymethyl)tetrahydrofuran-2-yl)oxy)-4-(((2R,3R,6S)-3-amino-6-(aminomethyl)tetrahydro-2H-pyran-2-yl)oxy)-2,6-dihydroxycyclohexyl)-2-hydroxybutanamide (Compound 2).

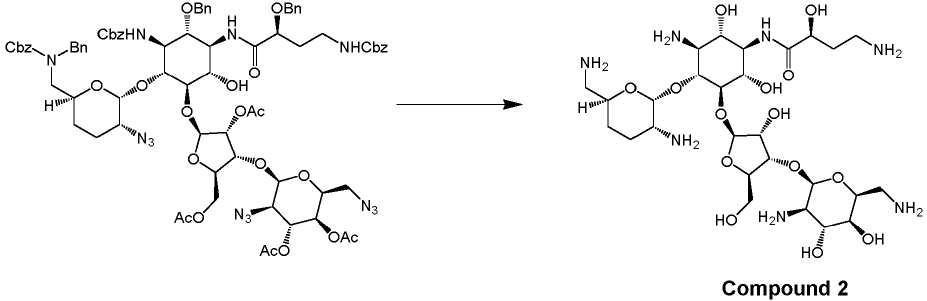

A solution of (2S,3R,4R,5R,6R)-6-(((2R,3R,4R,5S)-4-acetoxy-2-(acetoxymethyl)-5-(((1R,2R,3S,4R,5S,6S)-2-(((2R,3R,6S)-3-azido-6-((benzyl((benzyloxy)carbonyl)amino)methyl)tetrahydro-2H-pyran-2-yl)oxy)-4-(benzyloxy)-5-((S)-2-(benzyloxy)-4-(((benzyloxy)carbonyl)amino)butanamido)-3-(((benzyloxy)carbonyl)amino)-6-hydroxycyclohexyl)oxy)tetrahydrofuran-3-yl)oxy)-5-azido-2-(azidomethyl)tetrahydro-2H-pyran-3,4-diyl diacetate (142 mg, 0.10 mmol) in MeOH (6.0 mL) was added to a suspension of Pd(OH)₂ (474 mg, 0.67 mmol, 20% wet) in a mixture of AcOH (5.0 mL) and water (2.0 mL). The mixture was bubbled with hydrogen for 10 min. The mixture was stirred under hydrogen atmosphere (1 atm) for 3 h, then degassed with N₂ for 5 min. The suspension was filtered on 0.22 µM PVDF filter syringe which was washed with MeOH (3 x 3 mL) and water (2 x 2 mL). The volatiles were evaporated under reduced pressure (temperature < 35 °C). The material was purified by filtration using 5% MeCN in 10 mM NH₄HCO₃ (pH 10) and lyophilized to give a solid. H₂SO₄ (0.05 M in H₂O) was added dropwise to a solution of the above solid in H₂O (3 mL) at 0 °C until pH 4-5, then lyophilized. The solid was dissolved in water (2 mL), then MeOH (10 mL) was added to give a precipitate. The solid was filtered through 0.22 uM PVDF syringe filter, rinsed with MeOH (2 x 5 mL), then washed with MeOH (5 mL). The solid was dissolved in water (10 mL) and lyophilized to give the title **Compound 2** (58 mg, 60%) as a white solid. ¹H NMR (400 MHz, D₂O) δ 5.96 (d, J = 3.6 Hz, 1H), 5.44 (s, 1H), 5.33 (s, 1H), 4.62 - 4.54 (m, 1H), 4.50 - 4.45 (m, 1H), 4.45 - 4.35 (m, 2H), 4.30 - 4.12 (m, 4H), 4.03 - 3.90 (m, 3H), 3.88 - 3.76 (m, 3H), 3.69 (t, J = 9.8 Hz, 1H), 3.65 - 3.55 (m, 2H), 3.51 - 3.40 (m, 2H), 3.39 - 3.25 (m, 2H), 3.25 - 3.16 (m, 2H), 3.11 (dd, J = 13.5, 8.2 Hz, 1H), 2.30 - 2.17 (m, 1H), 2.14 - 2.02 (m, 3H), 1.97 (d, J = 14.7 Hz, 1H), 1.68 - 1.58 (m, 1H).

### Example 8. Synthesis of (2R,3R)-4-amino-N-((1R,2S,3S,4R,5R,6S)-3-amino-4-(((2R,3R,6S)-3-amino-6-((S)-1-aminoethyl)tetrahydro-2H-pyran-2-yl)oxy)-5-(((2S,3R,4S,5R)-4-(((2R,3R,4R,5S,6S)-3-amino-6-(aminomethyl)-4,5-dihydroxytetrahydro-2H-pyran-2-yl)oxy)-3-hydroxy-5-(hydroxymethyl)tetrahydrofuran-2-yl)oxv)-2,6-dihydroxycyclohexyl)-3-fluoro-2-hydroxybutanamide (Compound 3).

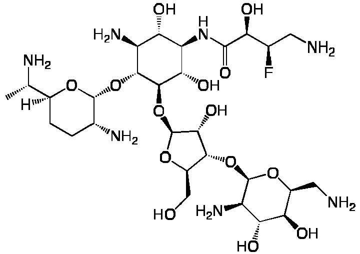

### Step A: Synthesis of (3R,6S)-3-azido-6-((S)-1-(benzyl((benzyloxy)carbonyl)amino)ethyl)tetrahydro-2H-pyran-2-yl (E)-2,2,2-trifluoro-N-phenylacetimidate

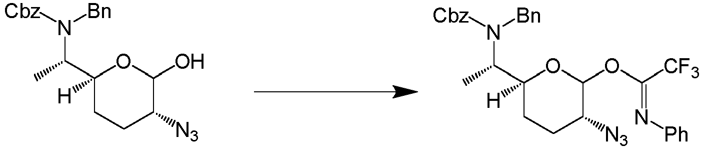

Cesium carbonate (23.5 g, 72.2 mmol) was added to a solution of 2,2,2-trifluoro-N-phenyl-acetimidoyl chloride (10.4 mL, 64.9 mmol) and benzyl N-[(1S)-1-[(2S,5R)-5-azido-6-hydroxytetrahydropyran-2-yl]ethyl]-N-benzyl-carbamate (**Ring A2**; Example 2, Step G; 14.8 g, 36.1 mmol) in DCM (336 mL) at 23 °C under nitrogen. The mixture was stirred at 23 °C for 45 min, filtered through a pad of diatomaceous earth, rinsed with DCM (200 mL) and concentrated. The residue was purified by silica gel chromatography (330 g high-capacity cartridge) with hexane and EtOAc (0-20%) to provide the title compound as a solid (21 g, quant.). m/z: ES+ no ionization; LCMS (A05) tR = 3.14 min.

### Step B: Synthesis of (1S,2S,3R,4S,5S,6R)-6-(((3R,6S)-3-azido-6-((S)-1-(benzyl((benzyloxy)carbonyl)amino)ethyl)tetrahydro-2H-pyran-2-yl)oxy)-3,5-bis(((benzyloxy)carbonyl)amino)cyclohexane-1,2,4-triyl triacetate

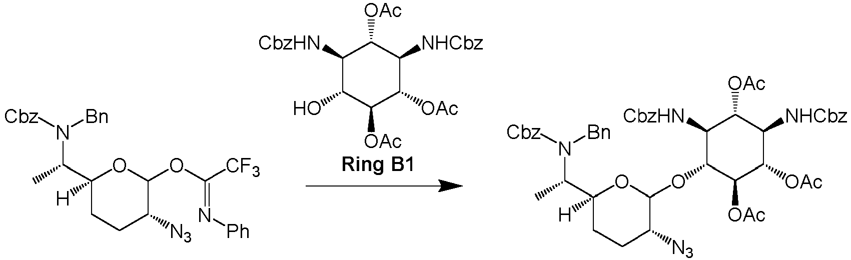

[(3R,6S)-3-azido-6-[(1S)-1-[benzyl(benzyloxycarbonyl)amino]ethyl]tetrahydropyran-2-yl] (1E)-2,2,2-trifluoro-N-phenyl-ethanimidate 2 (21.5 g, 37 mmol) and [(1S,2R,3S,4S,5R,6R)-3,4-diacetoxy-2,6-bis(benzyloxycarbonylamino)-5-hydroxy-cyclohexyl] acetate (**Ring B1**; 31.7 g, 55.5 mmol) was co-evaporated with anhydrous toluene (2 x 45 mL). The mixture was dried under reduced pressure for 30 min. The residue was taken in anhydrous DCM (550 mL) and Et₂O (250 mL), then transferred via a syringe to a RBF charged with pre-activated 4A sieves. The mixture was stirred at rt for 30 min, then Bi(OTf)₃ (26.7 g, 40.7 mmol) was added in one portion at 0 °C. The mixture was stirred at rt for 3 h, then diluted with saturated aq NH₄Cl solution (500 mL), and DCM (400 mL). The suspension was filtered through a pad of diatomaceous earth and the layers partitioned. The separated aqueous layer was extracted with DCM (3 x 300 mL). The combined organic layers were washed brine (500 mL), dried (MgSO₄), filtered and concentrated under reduced pressure to give crude 3 (56 g crude) as β/α = 1:9 anomeric mixture. The material was used without further purification for the next step. m/z: ES+ [M+H]⁺ 965.3; LCMS (A05); tR = 3.00 min (β anomer), 3.06 min (α anomer).

### Step C: Synthesis of benzyl N-[(1S)-1-[(2S,5R,6R)-5-azido-6-[(1R,2S,3R,4R,5S,6R)-2,4-bis(benzyloxycarbonylamino)-3,5,6-trihydroxy-cyclohexoxy]tetrahydropyran-2-yl]ethyl]-N-benzyl-carbamate

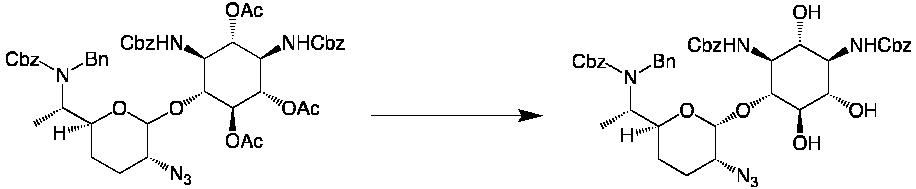

Sodium methoxide (4.60M in MeOH, 10 mL, 46.4 mmol,) was added to a solution of [(1S,2S,3R,4S,5S,6R)-2-acetoxy-3-[(3R,6S)-3-azido-6-[(1S)-1-[benzyl(benzyloxycarbonyl)amino]ethyl]tetrahydropyran-2-yl]oxy-4,6-bis(benzyloxycarbonylamino)-5-hydroxy-cyclohexyl] acetate (56 g, 58 mmol, crude) in MeOH (375 mL) at room temperature. The mixture was stirred at rt for 1 h, diluted with AcOH (10 mL) until pH ~5, then concentrated under reduced pressure. The residue was purified by preparative HPLC (X-bridge C18, 50 x 250 mm, 10 µM) using 58-72% ACN in ammonium formate (10 mM, pH 3.8). The fractions were pooled and concentrated under reduced to remove ACN. The aqueous residue was extracted with EtOAc (3 x 200 mL). The combined organic phase was washed with brine (2 x 100 mL), dried (MgSO₄), filtered, and concentrated under reduced pressure to provide the title compound (17.5 g, 59% over 3-steps) as a solid. *m*/*z* (ESI) [M+H]⁺; 839.3, HPLC A(05) tR=2.80 min.

### Step D: Synthesis of benzyl N-[(1S)-1-[(2S,5R,6R)-6-[(3aS,4R,5S,6S,7R,7aS)-5,7-bis(benzyloxycarbonyl amino)-6-hydroxy-spiro[3a,4,5,6,7,7a-hexahydro-1,3-benzodioxole-2,1'-cyclohexane]-4-yl]oxy-5-azido-tetrahydropyran-2-yl]ethyl]-N-benzyl-carbamate

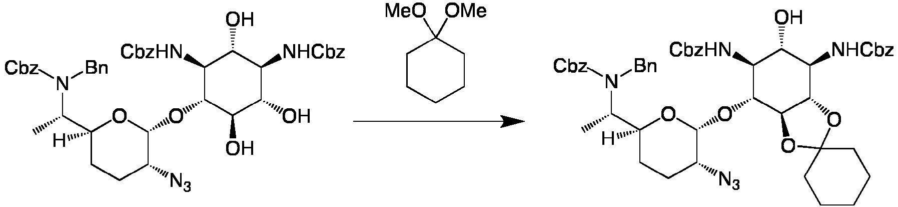

To a solution of benzyl N-[(1S)-1-[(2S,5R,6R)-5-azido-6-[(1R,2S,3R,4R,5S,6R)-2,4-bis(benzyloxycarbonylamino)-3,5,6-trihydroxy-cyclohexoxy]tetrahydropyran-2-yl]ethyl]-N-benzyl-carbamate (17.5 g, 20.9 mmol) and 1,1-dimethoxycyclohexane (26.9 mL, 177 mmol) in DME (337 mL) was added dried PPTS (2.17 g, 8.62 mmol). The mixture was stirred at 90 °C under nitrogen for 3 h. The mixture was cooled to rt and concentrated under reduced pressure. The residue was purified on silica gel (350 g, HC 25 uM) using EtOAc and hexanes (0-100%) to provide the title compound (17.5 g, 91%) as a white solid. m/z: ES+, [M+H]⁺ 919.4; LCMS (A05); tR = 3.12 min.

### Step E: Synthesis of benzyl N-[(1S)-1-[(2S,5R,6R)-6-[(3aS,4R,5S,6S,7R,7aS)-6-benzyloxy-5,7-bis(benzyloxycarbonylamino)spiro[3a,4,5,6,7,7a-hexahydro-1,3-benzodioxole-2,1'-cyclohexane]-4-yl]oxy-5-azido-tetrahydropyran-2-yl]ethyl]-N-benzyl-carbamate

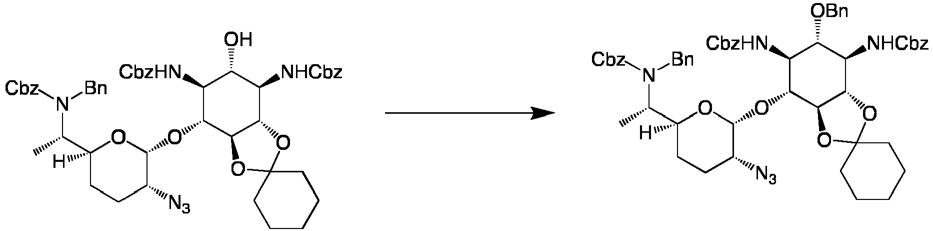

Sodium hydride (60% dispersion in mineral oil, 1.85 g, 46.3 mmol) was added to a mixture of benzyl N-[(1S)-1-[(2S,5R,6R)-6-[(3aS,4R,5S,6S,7R,7aS)-5,7-bis(benzyloxycarbonylamino)-6-hydroxy-spiro[3a,4,5,6,7,7a-hexahydro-1,3-benzodioxole-2,1'-cyclohexane]-4-yl]oxy-5-azido-tetrahydropyran-2-yl]ethyl]-N-benzyl-carbamate (18.5 g, 20.1 mmol) and benzyl bromide (26.8 mL, 226 mmol) in THF (500 mL) at 0 °C under nitrogen. The mixture was stirred at rt for 6h (less than 50% conversion) and transferred to a 30 °C oil bath and stirred for 5h (full conversion), then AcOH (2.27 mL, 39.7 mmol) was added, followed by silica gel and EtOAc (150 mL). The mixture was concentrated and purified by silica gel chromatography (350 g cartridge, 25 uM) using a gradient of 0-100% EtOAc in hexanes. The mixed fractions were pooled, concentrated, and re-purified by silica gel chromatography (350 g cartridge) using a gradient of 0-50% EtOAc in hexanes to provide the title compound (16.8 g, 83%) as a solid. m/z: ES+ [M+H]⁺ 1009.4; LCMS (A05); tR = 3.23 min.

### Step F: Synthesis of dibenzyl ((3aS,4R,5S,6S,7R,7aS)-4-(((2R,3R,6S)-3-azido-6-((S)-1-(benzyl((benzyloxy)carbonyl)amino)ethyl)tetrahydro-2H-pyran-2-yl)oxy)-6-hydroxyhexahydrospiro[benzo[d][1,3]dioxole-2,1'-cyclohexane]-5,7-diyl)dicarbamate

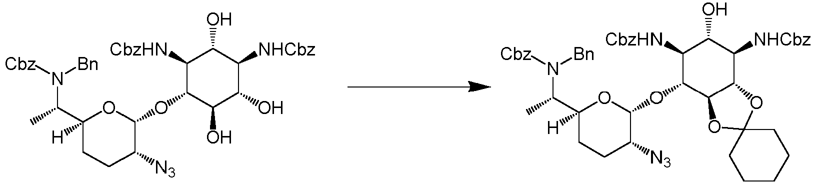

To a solution of benzyl N-[(1S)-1-[(2S,5R,6R)-5-azido-6-[(1R,2S,3R,4R,5S,6R)-2,4-bis(benzyloxycarbonylamino)-3,5,6-trihydroxy-cyclohexoxy]tetrahydropyran-2-yl]ethyl]-N-benzyl-carbamate (17.5 g, 20.9 mmol) and 1,1-dimethoxycyclohexane (26.9 mL, 177 mmol) in DME (337 mL) was added dried PPTS (2.17 g, 8.62 mmol). The mixture was stirred at 90 °C under nitrogen for 3 h. The mixture was cooled to rt and concentrated under reduced pressure. The residue was purified on silica gel (350 g, HC 25 uM) using EtOAc and hexanes (0-100%) to provide the title compound (17.5 g, 91%) as a white solid. m/z: ES+, [M+H]⁺ 919.4; LCMS (A05); tR = 3.12 min.

### Step G: Synthesis of dibenzyl ((1S,2R,3S,4R,5R,6S)-4-(((2R,3R,6S)-3-azido-6-((S)-1-(benzyl((benzyloxy)carbonyl)amino)ethyl)tetrahydro-2H-pyran-2-yl)oxy)-2-(benzyloxy)-5,6-dihydroxycyclohexane-1,3-diyl)dicarbamate

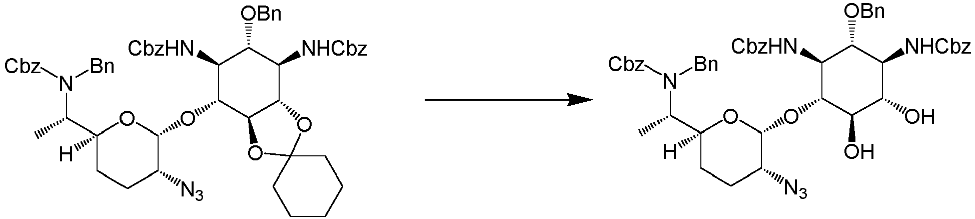

**A** solution of benzyl N-[(1S)-1-[(2S,5R,6R)-6-[(3aS,4R,5S,6S,7R,7aS)-6-benzyloxy-5,7-bis(benzyloxycarbonylamino)spiro[3a,4,5,6,7,7a-hexahydro-1,3-benzodioxole-2,1'-cyclohexane] -4-yl]oxy-5-azido-tetrahydropyran-2-yl]ethyl]-N-benzyl-carbamate (16.8 g, 16.6 mmol) in THF (180 mL), water (60 mL) and AcOH (150 mL) was heated at 80 °C for 12 h, then cooled to rt. The mixture was slowly added to water (2 L). The resultant precipitation was filtered and the solid was washed with water (3 x 100 mL), collected, and then dissolved in EtOAc (500 mL). This solution was washed with sat aq. NaHCO₃ (100 mL), and water (3 x 100 mL). The aqueous was extracted with EtOAc (3 x 50 mL). The combined organic phase was washed with brine (2 x 50 mL), dried (MgSO₄), filtered and concentrated under reduced pressure to provide the title product (14.8 g, 96%) as a solid. This solid was used directly without further purification. m/z (ESI) [2M+NH₄]⁺: 1875.3, HPLC (A05) tR = 2.99 min.

### Step H: Synthesis of benzyl ((S)-1-((2S,SR,6R)-5-azido-6-(((3aR,4S,5S,6R,7S,7aS)-4-(benzyloxy)-5-(((benzyloxy)carbonyl)amino)-7-hydroxy-2-oxooctahydrobenzo[d]oxazol-6-yl)oxy)tetrahydro-2H-pyran-2-yl)ethyl)(benzyl)carbamate (Ring AB2)

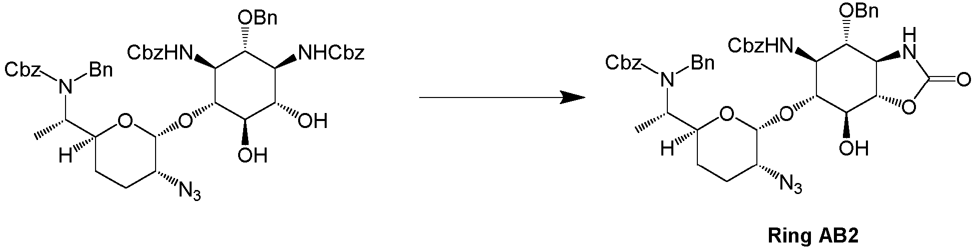

Sodium hydride (60% oil dispersion, 3.19 g, 79.7 mmol,) was added to a solution of benzyl N-[(1S)-1-[(2S,5R,6R)-5-azido-6-[(1R,2S,3R,4S,5S,6R)-3-benzyloxy-2,4-bis(benzyloxycarbonylamino)-5,6-dihydroxy-cyclohexoxy]tetrahydropyran-2-yl]ethyl]-N-benzyl-carbamate (14.8 g, 15.9 mmol) in dry DMF (150 mL) at 0 °C. The mixture was stirred at 0 °C for 1h30, diluted by AcOH (7.8 mL, 135 mmol), and then slowly poured into stirring water (1 L). The precipitation was filtered and the solid was washed with water (3 x 200 mL) and lyophilized to afford the title product (13.8 g, >99 %) as a solid. The solid was further purified by silica gel column using a gradient of 0-100% EtOAc in hexanes to provide the title product (11 g, 84%) as a solid. m/z (ESI) [M+H]⁺: 821.3, HPLC (A05) tR = 2.90 min.

### Step I: Synthesis of [(2R,3R,4R,5S)-5-[[(3aR,4S,5S,6R,7S,7aS)-6-[(2R,3R,6S)-3-azido-6-[(1S)-1-[benzyl (benzyloxycarbonyl)amino]ethyl]tetrahydropyran-2-yl]oxy-4-benzyloxy-5-(benzyloxy carbonylamino)-2-oxo-3a,4,5,6,7,7a-hexahydro-3H-1,3-benzoxazol-7-yl]oxy]-4-acetoxy-3-[(2R,3R,4R,5R,6S)-4,5-diacetoxy-3-azido-6-(azidomethyl)tetrahydropyran-2-yl] oxy-tetrahydrofuran-2-yl]methyl acetate

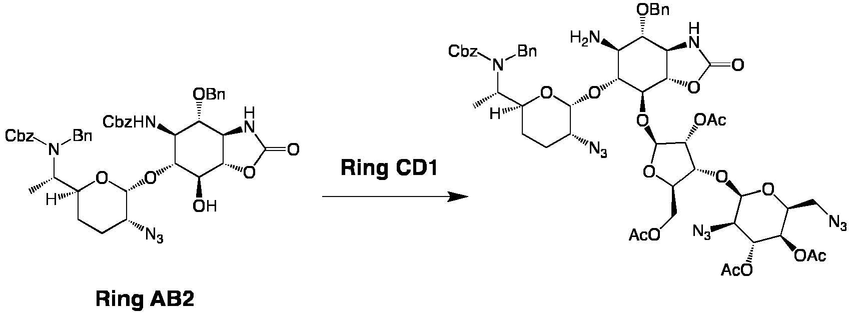

### Activation

K₂CO₃ (2.73 g, 19.7 mmol) was added to a mixture of [(2R,3R,4R)-4-acetoxy-3-[(2R,3R,4R,5R,6S)-4,5-diacetoxy-3-azido-6-(azidomethyl)tetrahydropyran-2-yl]oxy-5-hydroxy-tetrahydrofuran-2-yl]methyl acetate **Ring CD-1** (6.9 g, 13 mmol) and CCl₃CN (2 mL, 19.7 mmol) in dry DCM (300 mL). The mixture was stirred at rt for 18h, then filtered on Celite, rinsed with DCM, and concentrated under reduced pressure to give a syrup.

### Glycosylation

A mixture of this syrup and benzyl N-[(1S)-1-[(2S,5R,6R)-6-[[(3aR,4S,5S,6R,7S,7aS)-4-benzyloxy-5-(benzyloxycarbonylamino)-7-hydroxy-2-oxo-3a,4,5,6,7,7a-hexahydro-3H-1,3-benzoxazol-6-yl]oxy]-5-azido-tetrahydropyran-2-yl]ethyl]-N-benzyl-carbamate (2.7 g, 3.29 mmol) was co-evaporated with dry toluene (12 mL) under reduced pressure, dissolved in dry DCM (300 mL), then transferred to a RBF charged with pre-activated 4A sieves. The mixture was cooled to -78 °C, then BF₃·OEt₂ (6.12 mL, 49.6 mmol) was added slowly over 5 mins at -78 °C. Upon completion of the addition, the dry ice bath was removed immediately. The mixture was stirred at rt for 2 h and diluted by a saturated aqueous solution of NaHCO₃ (150 mL) and DCM (300 mL). The mixture was filtered through Celite, and the cake was washed with DCM (3 x 150 mL). The mixture was partitioned, and the aqueous was extracted with DCM (3 x 50 mL). The combined organic layers were washed with brine (2 x 100 mL), dried (MgSO₄), filtered and concentrated under reduced pressure. The material was purified by preparative HPLC (X-bridge C18, 50 x 250 mm, 10 µM) using 72-86% ACN in ammonium formate (10 mM, pH3.8). The product fractions were pooled, and ACN was removed under reduced pressure. The aqueous layer was extracted with EtOAc (3 x 150 mL). The combined organic layers were washed with brine (2 x 50 mL), dried (MgSO₄), filtered, concentrated under reduced pressure to provide the title compound [(2R,3R,4R,5S)-5-[[(3aR,4S,5S,6R,7S,7aS)-6-[(2R,3R,6S)-3-azido-6-[(1S)-1-[benzyl (benzyloxycarbonyl)amino]ethyl]tetrahydropyran-2-yl]oxy-4-benzyloxy-5-(benzyloxy carbonylamino)-2-oxo-3a,4,5,6,7,7a-hexahydro-3H-1,3-benzoxazol-7-yl]oxy]-4-acetoxy-3-[(2R,3R,4R,5R,6S)-4,5-diacetoxy-3-azido-6-(azidomethyl)tetrahydropyran-2-yl]oxy-tetrahydrofuran-2-yl]methyl acetate (4 g, 91%). m/z (ESI) [M+H]⁺: 1334.2, HPLC (A05) tR= 3.04 min.

### Step J: Synthesis of benzyl N-[(1S)-1-[(2S,SR,6R)-6-[(1R,2R,3S,4S,SR,6S)-4-amino-2-[(2S,3R,4S,5R)-4-[(2R,3R,4R,5S,6S)-3-azido-6-(azidomethyl)-4,5-dihydroxy-tetrahydropyran-2-yl]oxy-3-hydroxy-5-(hydroxymethyl)tetrahydrofuran-2-yl] oxy-5-benzyloxy-6-(benzyloxycarbonylamino)-3-hydroxy-cyclohexoxy]-5-azido-tetrahydropyran-2-yl]ethyl]-N-benzyl-carbamate

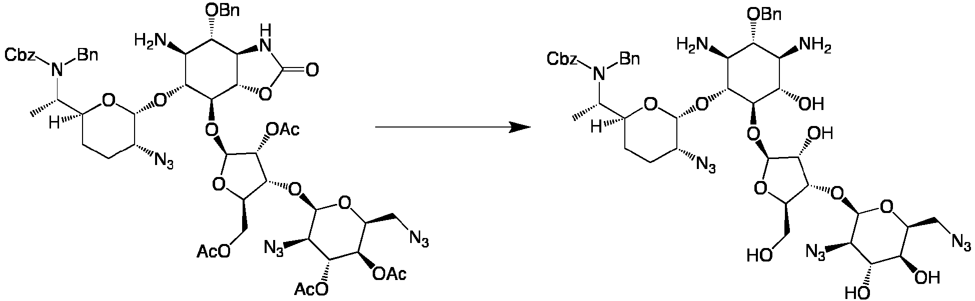

To a solution of [(2R,3R,4R,5S)-5-[[(3aR,4S,5S,6R,7S,7aS)-6-[(2R,3R,6S)-3-azido-6-[(1S)-1-[benzyl(benzyloxycarbonyl)amino]ethyl]tetrahydropyran-2-yl]oxy-4-benzyloxy-5-(benzyloxycarbonylamino)-2-oxo-3a,4,5,6,7,7a-hexahydro-3H-1,3-benzoxazol-7-yl]oxy]-4-acetoxy-3-[(2R,3R,4R,5R,6S)-4,5-diacetoxy-3-azido-6-(azidomethyl)tetrahydropyran-2-yl]oxy-tetrahydrofuran-2-yl]methyl acetate (4.0 g, 3.00 mmol) in dioxane (100 mL) and water (100 mL) was added LiOH·H₂O (3.78 g, 90.0 mmol). The mixture was stirred at 40 °C for 18 h, cooled to rt, diluted by EtOAc (300 mL) and H₂O (50 mL), and then partitioned. The separated aqueous layer was extracted with EtOAc (3 x 100 mL). The combined organic layers were dried (MgSO₄), filtered and concentrated under reduced pressure to provide the title compound (3.40 g, 99%) as a white solid. m/z (ESI) [M+H]⁺: 1139.4, HPLC A(05) tR = 2.54 min.

### Step K: Synthesis of (2S,3R,4R,5R,6R)-6-(((2R,3R,4R,5S)-4-acetoxy-2-(acetoxymethyl)-5-(((1R,2S,3S,4R,5S,6R)-3-((2R,3R)-4-azido-3-fluoro-2-((4-methoxybenzyl)oxy)butanamido)-6-(((2R,3R,6S)-3-azido-6-((S)-1-(benzyl((benzyloxy)carbonyl)amino)ethyl)tetrahydro-2H-pyran-2-yl)oxy)-4-(benzyloxy)-5-(((benzyloxy)carbonyl)amino)-2-hydroxycyclohexyl)oxy)tetrahydrofuran-3-yl)oxy)-5-azido-2-(azidomethyl)tetrahydro-2H-pyran-3,4-diyl diacetate

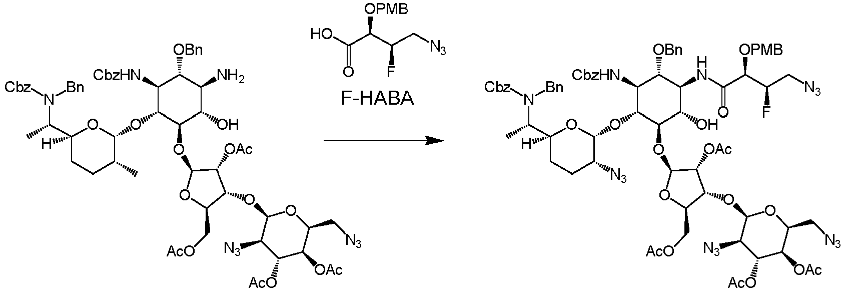

To a solution of benzyl N-[(1S)-1-[(2S,5R,6R)-6-[(1R,2R,3S,4S,5R,6S)-4-amino-2-[(2S,3R, 4S,5R)-4-[(2R,3R,4R,5S,6S)-3-azido-6-(azidomethyl)-4,5-dihydroxy-tetrahydropyran-2-yl]oxy-3-hydroxy-5-(hydroxymethyl)tetrahydrofuran-2-yl]oxy-5-benzyloxy-6-(benzyloxycarbonylamino)-3-hydroxy-cyclohexoxy]-5-azido-tetrahydropyran-2-yl]ethyl]-N-benzyl-carbamate (3.2 g, 2.81 mmol) and (2R,3R)-4-azido-3-fluoro-2-[(4-methoxyphenyl)methoxy]butanoic acid (1.37 g, 4.82 mmol) in anhydrous THF (150 mL) was added DMTMM chloride (1.37 g, 4.93 mmol). The mixture was stirred at rt for 18 h, then diluted by a saturated aqueous NH₄Cl solution (100 mL) and EtOAc (300 mL). The separated aqueous layer was extracted with EtOAc (50 mL x 3). The combined organic layers were washed with brine (2 x 50 mL), dried (MgSO₄), filtered and concentrated under reduced pressure. The material was purified by reversed phase preparative HPLC (X-bridge C18, 50 x 250 mm, 10 µM) using a gradient of 70-85% ACN in ammonium formate (10 mM, pH 3.8). The product fractions were pooled and concentrated under reduced to remove ACN. The aqueous was extracted in EtOAc (3 x 150 mL). The combined organic phases were washed with brine (2 x 50 mL), dried (MgSO₄), filtered, concentrated under reduced pressure to provide the title compound (2.7 g, 68%) as a solid. m/z (ESI) [M+H]⁺: 1404.4, HPLC A(05) tR= 2.96 min.

### Step L: Synthesis of (2R,3R)-4-amino-N-((1R,2S,3S,4R,5R,6S)-3-amino-4-(((2R,3R,6S)-3-amino-6-((S)-1-aminoethyl)tetrahydro-2H-pyran-2-yl)oxy)-5-(((2S,3R,4S,5R)-4-(((2R,3R,4R,5S,6S)-3-amino-6-(aminomethyl)-4,5-dihydroxytetrahydro-2H-pyran-2-yl)oxy)-3-hydroxy-5-(hydroxymethyl)tetrahydrofuran-2-yl)oxy)-2,6-dihydroxycyclohexyl)-3-fluoro-2-hydroxybutanamide (Compound 3)

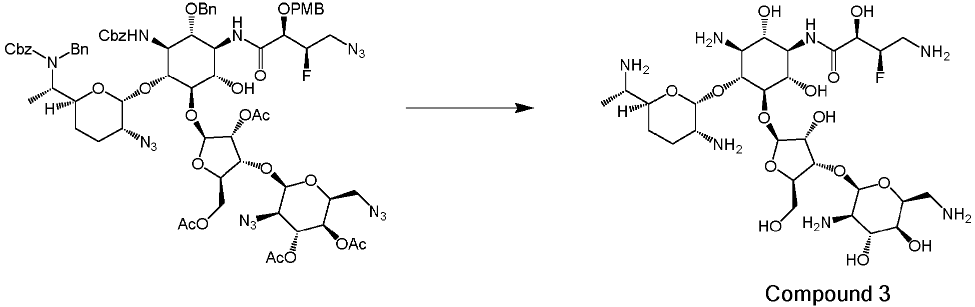

A mixture of MeOH (152 mL), AcOH (127 mL) and water (51 mL) was transferred under N₂ via syringe to a RBF charged with benzyl N-[(1S)-1-[(2S,SR,6R)-5-azido-6-[(1R,2R,3S,4S,SR,6S)-2-[(2S,3R,4S,5R)-4-[(2R,3R,4R,5S,6S)-3-azido-6-(azidomethyl)-4,5-dihydroxy-tetrahydropyran-2-yl]oxy-3-hydroxy-5-(hydroxymethyl)tetrahydrofuran-2-yl]oxy-4-[[(2R,3R)-4-azido-3-fluoro-2-[(4-methoxyphenyl)methoxy]butanoyl]amino]-5-benzyloxy-6-(benzyloxycarbonylamino)-3-hydroxy-cyclohexoxy]tetrahydropyran-2-yl]ethyl]-N-benzyl-carbamate (2.8 g, 1.99 mmol) in and Pd(OH)₂ (20% wet. 8.4 g, 12 mmol). The mixture was degassed by 3x vacuum-H₂ cycle, then bubbled with hydrogen (1 atm) for 4h, then degassed with N₂ for 15 min. The suspension was filtered on Celite, which was washed with MeOH (200 mL), water (200 mL). The volatiles were evaporated under reduced pressure (rotavapor bath temperature <35 °C). The material was purified by preparative HPLC using 5-15% of MeCN in 10 mM ammonium bicarbonate (pH 10), then lyophilized to give a solid. The solid was dissolved in water (50 mL), then Ammonium sulfate (3 eq, 790 mg, 5.98 mmol) was added. The mixture was stirred at rt for 5 min, then filtered with 0.40 uM syringe filter and added dropwise to a MeOH (1.5 L) under vigorous stirring. The suspension was filter on Fine frit and rinsed with MeOH (50 mL). The mother liquor was discarded and the solid was dissolved in water (40 mL) and lyophilized to provide title compound (1100 mg, 54%) as a solid. Compound 3 (1.1 g, 53 %) as a white solid. ¹H NMR (400 MHz, D₂O) δ 5.87 (d, *J =* 3.6 Hz, 1H), 5.29 (s, 1H), 5.25 - 5.05 (m, 2H), 4.47 - 4.17 (m, 4H), 4.18 - 3.97 (m, 3H), 3.88 - 3.78 (m, 3H), 3.77 - 3.68 (m, 3H), 3.62 (dd, *J =* 12.5, 5.6 Hz, 1H), 3.54 (t, *J =* 9.9 Hz, 1H), 3.49 - 3.44 (m, 1H), 3.44 - 3.16 (m, 7H), 1.99 - 1.82 (m, 3H), 1.49 - 1.34 (m, 1H), 1.19 (d, *J* = 6.7 Hz, 3H). Note: 20 exchangeable protons were not observed.

### Example 9. Synthesis of (2R,3R)-4-amino-N-((1R,2S,3S,4R,5R,6S)-3-amino-4-(((2R,3R,6S)-3-amino-6-((R)-1-aminoethyl)tetrahydro-2H-pyran-2-yl)oxy)-5-(((2S,3R,4S,5R)-4-(((2R,3R,4R,5S,6S)-3-amino-6-(aminomethyl)-4,5-dihydroxytetrahydro-2H-pyran-2-yl)oxy)-3-hydroxy-5-(hydroxymethyl)tetrahydrofuran-2-yl)oxy)-2,6-dihydroxycyclohexyl)-3-fluoro-2-hydroxybutanamide (Compound 4).

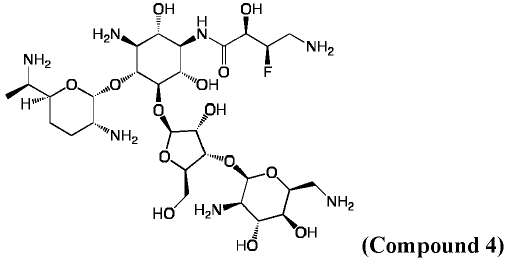

### Step A: Synthesis of (3R,6S)-3-azido-6-((R)-1-(benzyl((benzyloxy)carbonyl)amino)ethyl)tetrahydro-2H-pyran-2-yl (E)-2,2,2-trifluoro-N-phenylacetimidate

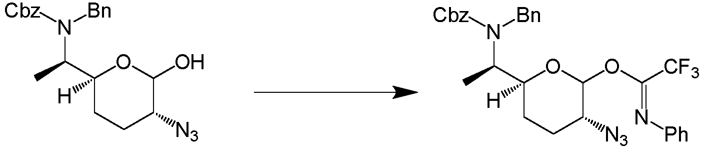

To a mixture of benzyl N-[(1R)-1-[(2S,5R)-5-azido-6-hydroxy-tetrahydropyran-2-yl]ethyl]-N-benzyl-carbamate **(Ring A3,** Example 3; 1.30 g, 2.85 mmol) and Cs₂CO₃ (1.86 g, 5.70 mmol) in DCM (10.0 mL) was added 2,2,2-Trifluoro-N-phenylacetimidoyl chloride (1.19 g, 5.70 mmol). The mixture was stirred at rt for 2 h. The mixture was filtered, the solid was rinsed with DCM and the solvent was concentrated under reduced pressure. The residue was purified on silica gel (dry loading) using a gradient of 0 to 35% EtOAc in hexane to provide title compound (1.20 g, 73%) as an oil. MS: no ionization.

### Step B: Synthesis of dibenzyl ((1R,2R,3S,4R,5R,6S)-4-(((3R,6S)-3-azido-6-((R)-1-(benzyl((benzyloxy)carbonyl)amino)ethyl)tetrahydro-2H-pyran-2-yl)oxy)-2,5,6-trihydroxycyclohexane-1,3-diyl)dicarbamate

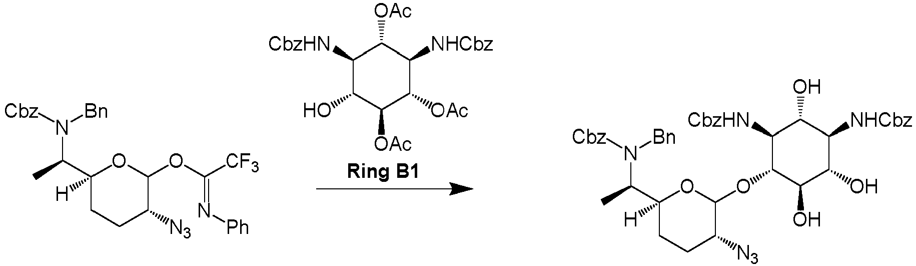

[[[(2R,3R,6S)-3-azido-6-[(1R)-1-[benzyl(benzyloxycarbonyl)amino]ethyl]tetrahydropyran-2-yl] (1E)-2,2,2-trifluoro-N-phenyl-ethanimidate (2.47 g, 4.25 mmol) and [(1S,2R,3S,4S,5R,6R)-3,4-diacetoxy-2,6-bis(benzyloxycarbonylamino)-5-hydroxy-cyclohexyl] acetate **(Ring B1,** 3.28 g, 5.73 mmol) were co-evaporated with anhydrous toluene (2 x 3 mL). The mixture was dried under reduced pressure for 30 min. The residue was taken in anhydrous DCM (100 mL) and then transferred via a syringe to an RBF charged with pre-activated 3Å and 4Å sieves. Et₂O (60 mL) was added. The mixture was stirred at rt for 20 min, then cooled to 0 °C. Bi(OTf)₃ (3.07 g, 4.67 mmol) was added in one portion and the mixture was stirred at rt for 4h. A saturated aqueous solution of NH₄Cl (100 mL) was added. The mixture was filtered on diatomaceous earth. The separated aqueous layer was extracted with DCM. The combined organic layers were dried (MgSO₄), filtered and concentrated under reduced pressure. The residue was purified on silica gel using a gradient of 0 to 100% EtOAc in hexanes. The solid was dissolved in MeOH (100 mL), and K₂CO₃ was added (1.52 g, 11.0 mmol). The mixture was stirred at rt for 2 h. The mixture was concentrated under reduced pressure (rotary evaporator water bath did not exceed 35 °C). Water (100 mL) and EtOAc (100 mL) was added. The separated aqueous layer was extracted with EtOAc. The combined organic layers were dried (MgSO₄), filtered and concentrated under reduced pressure to provide the title compound (2.30 g, 99%) as a white solid. *m*/*z* (ESI) [M+H]⁺; 839.3.

### Step C: Synthesis of dibenzyl ((3aS,4R,5S,6S,7R,7aS)-4-(((2R,3R,6S)-3-azido-6-((R)-1-(benzyl((benzyloxy)carbonyl)amino)ethyl)tetrahydro-2H-pyran-2-yl)oxy)-6-hydroxyhexahydrospiro[benzo[d][1,3]dioxole-2,1'-cyclohexane]-5,7-diyl)dicarbamate

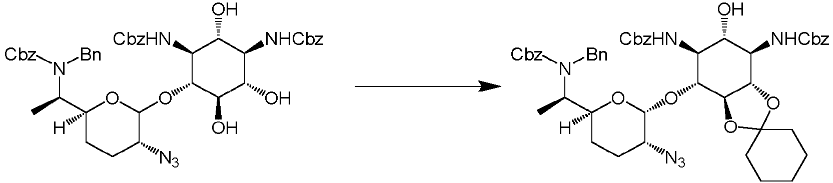

To a mixture of benzyl N-[(1R)-1-[(2S,5R,6R)-5-azido-6-[(1R,2S,3R,4R,5S,6R)-2,4-bis(benzyloxycarbonylamino)-3,5,6-trihydroxy-cyclohexoxy]tetrahydropyran-2-yl]ethyl]-N-benzyl-carbamate (2.30 g, 2.60 mmol), 1,1-dimethoxycyclohexane (2.38 mL, 15.6 mmol) and PPTS (262 mg, 1.01 mmol, dried with V10) in dry DME (111 mL) was stirred at 90 °C for 30 min. The mixture was cooled to rt and concentrated under reduced pressure. The material was purified by ACCQ using 10 mM Ammonium formate pH 3.8 and MeCN. The product was then diluted in EtOAc and washed with brine. The organic layer was dried (MgSO₄), filtered and concentrated under reduced pressure to provide (1.40 g, 59%). *m*/*z* (ESI) [M+H]⁺; 919.4.

### Step D: Synthesis of dibenzyl ((3aS,4R,5S,6S,7R,7aS)-4-(((2R,3R,6S)-3-azido-6-((R)-1-(benzyl((benzyloxy)carbonyl)amino)ethyl)tetrahydro-2H-pyran-2-yl)oxy)-6-(benzyloxy)hexahydrospiro[benzo[d][1,3]dioxole-2,1'-cyclohexane]-5,7-diyl)dicarbamate

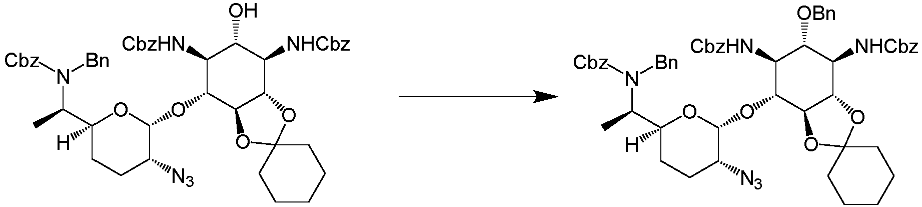

Sodium hydride (60% dispersion in mineral oil, 141 mg, 3.51 mmol) was added to a mixture of dibenzyl ((3aS,4R,5S,6S,7R,7aS)-4-(((2R,3R,6S)-3-azido-6-((R)-1-(benzyl((benzyloxy)carbonyl)amino)ethyl)tetrahydro-2H-pyran-2-yl)oxy)-6-hydroxyhexahydrospiro[benzo[d][1,3]dioxole-2,1'-cyclohexane]-5,7-diyl)dicarbamate (1.40 g, 1.52 mmol) and BnBr (2.03 mL, 17.1 mmol) in THF (75 mL) at 0 °C under nitrogen. The mixture was stirred at 0 °C for 4h, then AcOH (0.24 mL, 4.13 mL) was added. The mixture was concentrated under reduced. The residue was purified by silica gel chromatography (120 g cartridge) using a gradient of 0 to 60% EtOAc in hexanes to provide the title compound (1.43 g, 93%) as a solid. *m*/*z* (ESI) [M+H]⁺; 1009.5.

### Step E: Synthesis of dibenzyl ((1S,2R,3S,4R,5R,6S)-4-(((2R,3R,6S)-3-azido-6-((R)-1-(benzyl((benzyloxy)carbonyl)amino)ethyl)tetrahydro-2H-pyran-2-yl)oxy)-2-(benzyloxy)-5,6-dihydroxycyclohexane-1,3-diyl)dicarbamate

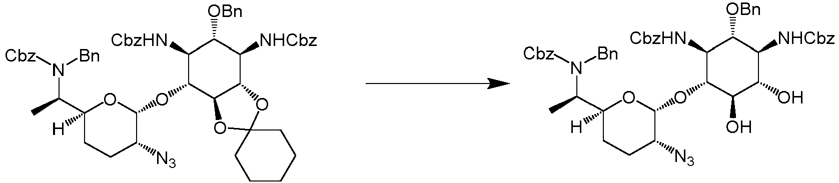

**A** mixture of dibenzyl ((3aS,4R,5S,6S,7R,7aS)-4-(((2R,3R,6S)-3-azido-6-((R)-1-(benzyl((benzyloxy)carbonyl)amino)ethyl)tetrahydro-2H-pyran-2-yl)oxy)-6-(benzyloxy)hexahydrospiro[benzo[d][1,3]dioxole-2,1'-cyclohexane]-5,7-diyl)dicarbamate (1.44 g, 1.43 mmol) in AcOH (18 mL), Water (8.0 mL) and THF (27 mL) was heated at 80 °C for 24 h. The mixture was cooled to rt and the THF was removed under reduced pressure. Water (100 mL) was added a suspension was observed. The suspension was filtered on medium frit. The solid was rinsed with water and dried under reduced pressure to provide the title compound (1.21 g, 91%). m/z: ES+ [M+H]⁺ 929.6.

### Step F: Synthesis of benzyl ((R)-1-((2S,SR,6R)-5-azido-6-(((3aR,4S,5S,6R,7S,7aS)-4-(benzyloxy)-5-(((benzyloxy)carbonyl)amino)-7-hydroxy-2-oxooctahydrobenzo[d]oxazol-6-yl)oxy)tetrahydro-2H-pyran-2-yl)ethyl)(benzyl)carbamate (Ring AB3)

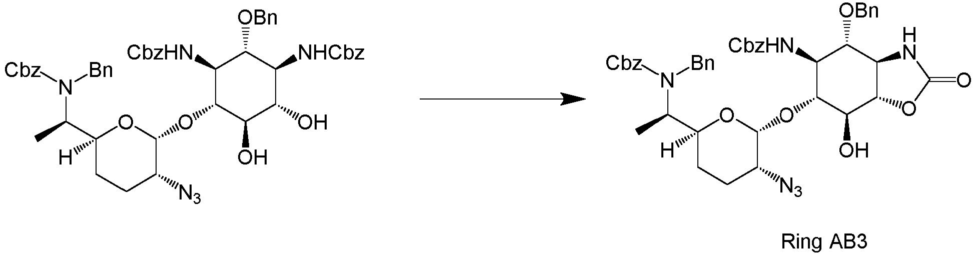

NaH (210 mg, 5.25 mmol, 60% oil dispersion) was added to a solution of dibenzyl ((1S,2R,3S,4R,5R,6S)-4-(((2R,3R,6S)-3-azido-6-((R)-1-(benzyl((benzyloxy)carbonyl)amino)ethyl)tetrahydro-2H-pyran-2-yl)oxy)-2-(benzyloxy)-5,6-dihydroxycyclohexane-1,3-diyl)dicarbamate (1.22 g, 1.31 mmol) in dry DMF (20 mL) at 0 °C. The mixture was stirred at 0 °C for 3 h, then AcOH (0.64 mL, 11.2 mmol) was added. The solution was poured into water (200 mL). The suspension was filtered, and the solid was rinsed with water (20 mL), dried under reduced pressure to provide the title compound (830 mg, 77%) as a white solid. m/z (ESI) [M+H]⁺: 821.3.

### Step G: Synthesis of benzyl ((1R)-1-((2S,5R)-5-azido-6-(((3aR,4S,5S,6R,7S,7aS)-4-(benzyloxy)-5-(((benzyloxy)carbonyl)amino)-7-hydroxy-2-oxooctahydrobenzo[d]oxazol-6-yl)oxy)tetrahydro-2H-pyran-2-yl)ethyl)(benzyl)carbamate

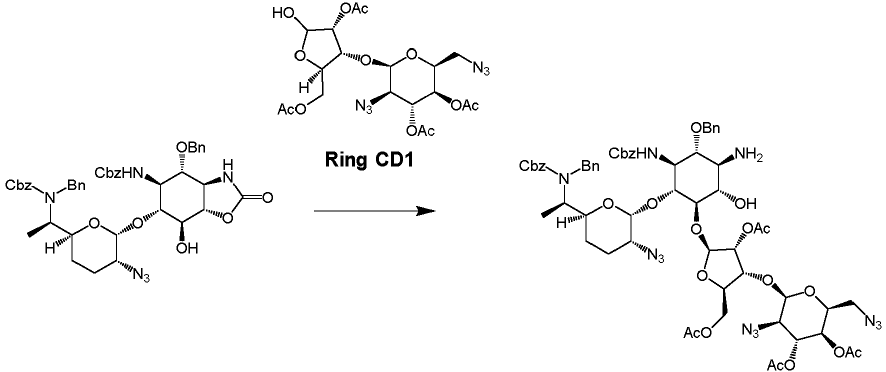

To a mixture of [(2R,3R,4R)-4-acetoxy-3-[(2R,3R,4R,5R,6S)-4,5-diacetoxy-3-azido-6-(azidomethyl)tetrahydropyran-2-yl]oxy-5-hydroxy-tetrahydrofuran-2-yl]methyl acetate **(Ring CD1,** International Patent Application Publication No. WO2019194858, 961 mg, 1.81 mmol) and K₂CO₃ (429 mg, 3.11 mmol) in dry DCM (8 mL) was added CCl₃CN (0.31 mL, 3.11 mmol). The mixture was stirred at rt for 18 h, then filtered on diatomaceous earth, rinsed with DCM and concentrated under reduced pressure. Benzyl ((R)-1-((2S,5R,6R)-5-azido-6-(((3aR,4S,5S,6R,7S,7aS)-4-(benzyloxy)-5-(((benzyloxy)carbonyl)amino)-7-hydroxy-2-oxooctahydrobenzo[d]oxazol-6-yl)oxy)tetrahydro-2H-pyran-2-yl)ethyl)(benzyl)carbamate **(Ring AB3;** 425 mg, 0.518 mmol) in dry DCM (8 mL) was added to the previous crude material, then transferred to a RBF charged with pre-activated 3A and 4A sieves. The mixture was cooled to -78 °C, then BF₃-OEt₂ (0.96 mL, 7.81 mmol) was added dropwise at -78 °C. Upon completion of the addition, the dry ice bath was removed immediately. The mixture was stirred at rt for 1 h, diluted by a saturated aqueous solution of NaHCO₃ (10 mL), filtered through diatomaceous earth, and the filter cake was washed with DCM (20 mL). The separated aqueous layer was extracted with DCM (3 x 5 mL). The combined organic layer was washed with brine (15 mL), dried (MgSO₄), filtered and concentrated under reduced pressure. The residue was taken in 1,4-dioxane (4.0 mL) and water (4.0 mL), then LiOH-H₂O (652 mg, 15.5 mmol) was added, and the mixture was stirred at 40 °C for 18 h. The mixture was cooled to rt, then 1,4-dioxane was removed under reduced pressure, then EtOAc (10 mL) was added. The separated aqueous layer was extracted with EtOAc (3 x 10 mL). The combined organic layers were dried (MgSO₄), filtered and concentrated under reduced pressure. The residue was purified by ACCQ prep using a gradient MeCN in 10 mM ammonium bicarbonate pH 10 to provide the title compound (209 mg, 35% over 2 steps) as a solid. m/z (ESI) [M+H]⁺: 1139.5

### Step H: Synthesis of 2S,3R,4R,5R,6R)-6-(((2R,3R,4R,5S)-4-acetoxy-2-(acetoxymethyl)-5-(((1R,2S,3S,4R,S5,6R)-3-((2R,3R)-4-azido-3-fluoro-2-((4-methoxybenzyl)oxy)butanamido)-6-(((2R,3R,6S)-3-azido-6-((R)-1-(benzyl((benzyloxy)carbonyl)amino)ethyl)tetrahydro-2H-pyran-2-yl)oxy)-4-(benzyloxy)-5-(((benzyloxy)carbonyl)amino)-2-hydroxycyclohexyl)oxy)tetrahydrofuran-3-yl)oxy)-5-azido-2-(azidomethyl)tetrahydro-2H-pyran-3,4-diyl diacetate

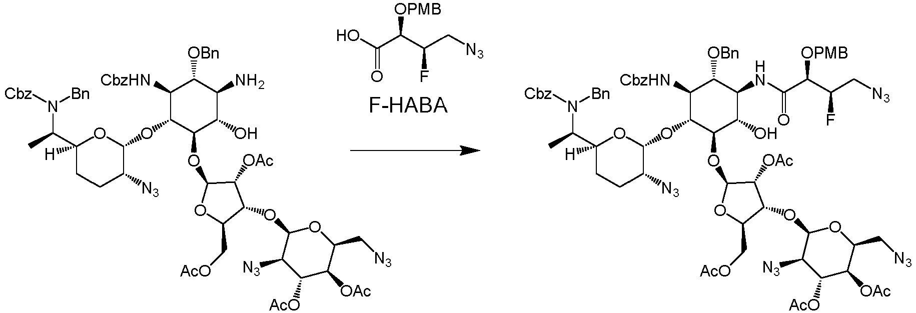

To a solution of benzyl ((1*R*)-1-((2*S*,5*R*)-5-azido-6-(((3a*R*,4*S*,5*S*,6*R*,7*S*,7a*S*)-4-(benzyloxy)-5-(((benzyloxy)carbonyl)amino)-7-hydroxy-2-oxooctahydrobenzo[*d*]oxazol-6-yl)oxy)tetrahydro-2*H*-pyran-2-yl)ethyl)(benzyl)carbamate (104 mg, 0.091 mmol) and (2R,3R)-4-azido-3-fluoro-2-[(4-methoxyphenyl)methoxy]butanoic acid (F-HABA, Example 5; 44 mg, 0.157 mmol) in anhydrous THF (5 mL) was added DMTMM chloride (44 mg, 0.160 mmol). The mixture was stirred at rt for 18 h, then diluted with a saturated aqueous NH₄Cl solution (3 mL) and EtOAc (5 mL). The separated aqueous layer was extracted with EtOAc (3 x 4 mL). The combined organic layers were washed with brine (5 mL), dried (MgSO₄), filtered and concentrated under reduced pressure. The material was purified by prep-HPLC (ACCQ, BEH C18 column) using a MeCN in Ammonium formate (10 mM, pH 3.8) and lyophilized to provide the title compound (103 mg, 80%) as a white solid. m/z (ESI) [M+H]⁺: M+H+ : 1404.6.

### Step I: Synthesis of ((2R,3R)-4-amino-N-((1R,2S,3S,4R,5R,6S)-3-amino-4-(((2R,3R,6S)-3-amino-6-((R)-1-aminoethyl)tetrahydro-2H-pyran-2-yl)oxy)-5-(((2S,3R,4S,5R)-4-(((2R,3R,4R,5S,6S)-3-amino-6-(aminomethyl)-4,5-dihydroxytetrahydro-2H-pyran-2-yl)oxy)-3-hydroxy-5-(hydroxymethyl)tetrahydrofuran-2-yl)oxy)-2,6-dihydroxycyclohexyl)-3-fluoro-2-hydroxybutanamide (Compound 4)

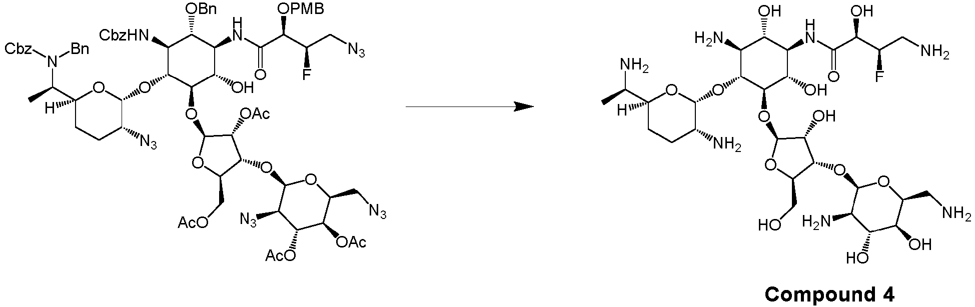

A solution of 2*S*,3*R*,4*R*,5*R*,6*R*)-6-(((2*R*,3*R*,4*R*,5*S*)-4-acetoxy-2-(acetoxymethyl)-5-(((1*R*,2*S*,3*S*,4*R,*5*S*,6*R*)-3-((2*R*,3*R*)-4-azido-3-fluoro-2-((4-methoxybenzyl)oxy)butanamido)-6-(((2*R*,3*R*,6*S*)-3-azido-6-((*R*)-1-(benzyl((benzyloxy)carbonyl)amino)ethyl)tetrahydro-2*H*-pyran-2-yl)oxy)-4-(benzyloxy)-5-(((benzyloxy)carbonyl)amino)-2-hydroxycyclohexyl)oxy)tetrahydrofuran-3-yl)oxy)-5-azido-2-(azidomethyl)tetrahydro-2*H*-pyran-3,4-diyl diacetate (104 mg, 0.074 mmol) in MeOH (9.0 mL) was added to a suspension of Pd(OH)₂ (359 mg, 0.51 mmol, 20% wet) in a mixture of AcOH (7.50 mL) and water (3.0 mL), degassed by 3 x vacuum-H₂ cycle, then bubbled with hydrogen for 10 min. The mixture was stirred under hydrogen atmosphere (1 atm) for 2.5 h, degassed with N₂ for 5 min, then filtered on 0.22 µM PVDF filter syringe which was washed with MeOH (3 x 3 mL) and water (2 x 2 mL), and the volatiles were evaporated under reduced pressure (rotavapor bath temperature <35 °C). The material was purified by filtration using 5% MeCN in 10 mM NH₄HCO₃ (pH 10) and lyophilized to give a solid. H₂SO₄ (0.05 M in H₂O) was added dropwise to a solution of the above solid in H₂O (3 mL) at 0 °C until pH 4, then lyophilized to give a solid, which dissolved in water (2 mL), followed by MeOH (10 mL) to give a precipitate. This precipitate was filtered through 0.22 uM PVDF syringe filter, rinsed with MeOH (2 x 5 mL), then washed with water (5 mL). The solid was dissolved in water (10 mL) and lyophilized to give the title **Compound 4** (30 mg, 40%) as a white solid. ¹H NMR (500 MHz, D₂O) δ 5.95 - 5.88 (m, 1H), 5.47 - 5.24 (m, 3H), 4.57 (d, J = 10.2 Hz, 1H), 4.51 - 4.34 (m, 3H), 4.27 (d, J = 3.7 Hz, 2H), 4.18 (d, J = 12.1 Hz, 1H), 4.04 - 3.88 (m, 5H), 3.82 - 3.59 (m, 5H), 3.58 - 3.37 (m, 5H), 3.33 - 3.22 (m, 2H), 2.12-2.00 (m, 2H), 1.96 - 1.89 (m, 1H), 1.69 - 1.58 (m, 1H), 1.31 (d, J = 6.9 Hz, 3H).

### Example 10. Synthesis of (S)-4-amino-N-((1R,2S,3S,4R,5R,6S)-3-amino-4-(((2R,3R,6S)-3-amino-6-((R)-1-aminoethyl)tetrahydro-2H-pyran-2-yl)oxy)-5-(((2S,3R,4S,5R)-4-(((2R,3R,4R,5S,6S)-3-amino-6-(aminomethyl)-4,5-dihydroxytetrahydro-2H-pyran-2-yl)oxy)-3-hydroxy-5-(hydroxymethyl)tetrahydrofuran-2-yl)oxy)-2,6-dihydroxycyclohexyl)-2-hydroxybutanamide (Compound 5)

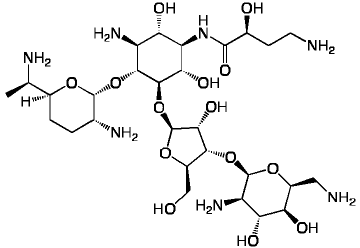

### Step A: Synthesis of (2S,3R,4R,5R,6R)-6-(((2R,3R,4R,5S)-4-acetoxy-2-(acetoxymethyl)-5-(((1R,2R,3S,4R,5S,6S)-2-(((2R,3R,6S)-3-azido-6-((R)-1-(benzyl((benzyloxy)carbonyl)amino)ethyl)tetrahydro-2H-pyran-2-yl)oxy)-4-(benzyloxy)-5-((S)-2-(benzyloxy)-4-(((benzyloxy)carbonyl)amino)butanamido)-3-(((benzyloxy)carbonyl)amino)-6-hydroxycyclohexyl)oxy)tetrahydrofuran-3-yl)oxy)-5-azido-2-(azidomethyl)tetrahydro-2H-pyran-3,4-diyl diacetate

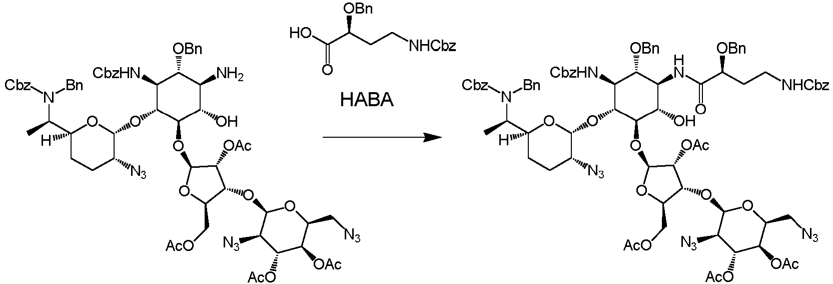

To a solution of benzyl N-[(1R)-1-[(2S,5R,6R)-6-[(1R,2R,3S,4S,5R,6S)-4-amino-2-[(2S,3R,4S,5R)-4-[(2R,3R,4R,5S,6S)-3-azido-6-(azidomethyl)-4,5-dihydroxy-tetrahydropyran-2-yl]oxy-3-hydroxy-5-(hydroxymethyl)tetrahydrofuran-2-yl]oxy-5-benzyloxy-6-(benzyloxy carbonylamino)-3-hydroxy-cyclohexoxy]-5-azido-tetrahydropyran-2-yl]ethyl]-N-benzyl-carbamate (90 mg, 0.079 mmol), (2S)-2-benzyloxy-4-(benzyloxycarbonylamino)butanoic acid (HABA; 40.7 mg, 0.119 mmol) and DIPEA (0.04 mL, 0.237 mmol) in anhydrous DMF (3 mL) was added PyBOP (49.3 mg, 0.0948 mol) at 0 °C. The mixture warmed to rt and stirred for 18 h. The mixture was poured into water (5 mL). The pH was adjusted to 4 using 1N HCl and then EtOAc (40 mL) was added. The separated aqueous layer was extracted with EtOAc (5 mL x 3). The combined organic layers were washed with brine (5 mL), dried (MgSO₄), filtered and concentrated under reduced pressure. The material was purified by reversed phase column (ACCQ) using a gradient of 60-80% ACN in ammonium formate (10 mM, pH 3.8) and concentrated under reduced pressure. The material was then dissolved in EtOAc (30 mL) and water (5 mL). The separated aqueous layer was extracted with EtOAc (3 x 5 mL). The combined organic layers were washed by brine (5 mL), dried (MgSO₄), filtered and concentrated under reduced pressure to provide the titled compound (79.3 mg, 69%) as a white solid. m/z (ESI) [M+H]⁺: 1464.5, HPLC A(05) tR=2.65 min.

### Step B: Synthesis of (S)-4-amino-N-((1R,2S,3S,4R,5R,6S)-3-amino-4-(((2R,3R,6S)-3-amino-6-((R)-1-aminoethyl)tetrahydro-2H-pyran-2-yl)oxy)-5-(((2S,3R,4S,5R)-4-(((2R,3R,4R,5S,6S)-3-amin-6-(aminomethyl)-4,5-dihydroxytetrahydro-2H-pyran-2-yl)oxy)-3-hydroxy-5-(hydroxymethyl)tetrahydrofuran-2-yl)oxy)-2,6-dihydroxycyclohexyl)-2-hydroxybutanamide (Compound 5)

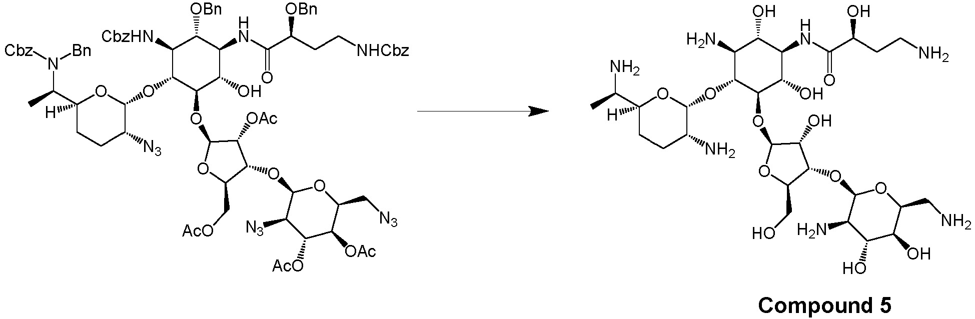

A solution of (2*S*,3*R*,4*R*,5*R*,6*R*)-6-(((2*R*,3*R*,4*R*,5*S*)-4-acetoxy-2-(acetoxymethyl)-5-(((1*R*,2*R*,3*S*,4*R*,5*S*,6*S*)-2-(((2*R*,3*R*,6*S*)-3-azido-6-((*R*)-1-(benzyl((benzyloxy)carbonyl)amino)ethyl)tetrahydro-2*H*-pyran-2-yl)oxy)-4-(benzyloxy)-5-((S)-2-(benzyloxy)-4-(((benzyloxy)carbonyl)amino)butanamido)-3-(((benzyloxy)carbonyl)amino)-6-hydroxycyclohexyl)oxy)tetrahydrofuran-3-yl)oxy)-5-azido-2-(azidomethyl)tetrahydro-2*H*-pyran-3,4-diyl diacetate (79 mg, 0.0539 mmol) in MeOH (6.5 mL) was added to a suspension of Pd(OH)₂ (20% wet, 261 mg, 0.372 mmol) in a mixture of AcOH (5.5 mL) and water (2.2 mL), degassed by 3 x vacuum-H₂ cycle, then bubbled with hydrogen for 10 min. The mixture was stirred with hydrogen bubbled (1 atm, refilling the balloon with H₂ when necessary) for 4 h. The suspension was degassed with N₂ for 15 min, then filtered on 0.22 µM PVDF filter syringe which was washed with MeOH (3 x 5 mL) and water (2 x 5 mL), and the volatiles were evaporated under reduced pressure (rotavapor bath temperature < 35 °C). The material was purified by filtration using 5% MeCN in 10 mM NH₄HCO₃ (pH 10) and lyophilized to give a solid. H₂SO₄ (0.05 M in H₂O) was added dropwise to a solution of the above solid in H₂O (3 mL) at 0 °C until pH 4, then lyophilized. The solid was dissolved in water (2 mL), followed by MeOH (10 mL). The suspension was filtered through 0.22 uM PVDF syringe filter, rinsed with MeOH (2 x 5 mL), then washed with water (5 mL). The solid was dissolved in water (10 mL) and lyophilized to provide the title product **Compound 5** (26 mg, 48%) as a solid. ¹H NMR (400 MHz, D₂O) δ 5.76 (dd, J = 10.0, 3.7 Hz, 1H), 5.27 (dd, J = 6.2, 2.8 Hz, 1H), 5.16 (d, J = 1.8 Hz, 1H), 4.40 (dd, J = 7.2, 4.7 Hz, 1H), 4.35 - 4.28 (m, 1H), 4.26 - 4.18 (m, 2H), 4.10 (t, J = 3.3 Hz, 2H), 4.03 - 3.95 (m, 1H), 3.93 - 3.67 (m, 5H), 3.64 - 3.19 (m, 8H), 3.18 - 3.08 (m, 1H), 3.08 - 2.98 (m, 2H), 2.13 - 1.99 (m, 1H), 1.98 - 1.84 (m, 3H), 1.81 - 1.70 (m, 1H),1.55 - 1.40 (m, 1H), 1.14 (dd, J = 6.9, 1.2 Hz, 3H). m/z (ESI) [M+H]⁺: 714.4.

### Example 11. Synthesis of (S)-4-amino-N-((1R,2S,3S,4R,5R,6S)-3-amino-4-(((2R,3R,6S)-3-amino-6-((S)-1-aminoethyl)tetrahydro-2H-pyran-2-yl)oxy)-5-(((2S,3R,4S,5R)-4-(((2R,3R,4R,5S,6S)-3-amino-6-(aminomethyl)-4,5-dihydroxytetrahydro-2H-pyran-2-yl)oxy)-3-hydroxy-5-(hydroxymethyl)tetrahydrofuran-2-yl)oxy)-2,6-dihydroxycyclohexyl)-2-hydroxybutanamide (Compound 6)

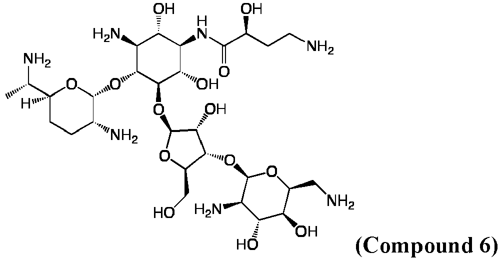

### Step A: Synthesis of (2S,3R,4R,5R,6R)-6-(((2R,3R,4R,5S)-4-acetoxy-2-(acetoxymethyl)-5-(((1R,2R,3S,4R,5S,6S)-2-(((2R,3R,6S)-3-azido-6-((S)-1-(benzyl((benzyloxy)carbonyl)amino)ethyl)tetrahydro-2H-pyran-2-yl)oxy)-4-(benzyloxy)-5-((S)-2-(benzyloxy)-4-(((benzyloxy)carbonyl)amino)butanamido)-3-(((benzyloxy)carbonyl)amino)-6-hydroxycyclohexyl)oxy)tetrahydrofuran-3-yl)oxy)-5-azido-2-(azidomethyl)tetrahydro-2H-pyran-3,4-diyl diacetate

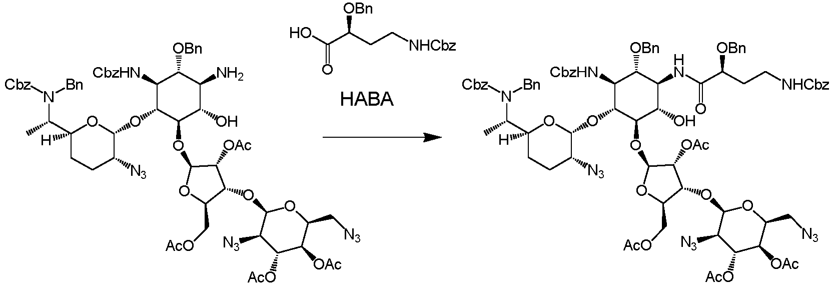

To a solution of benzyl N-[(1S)-1-[(2S,5R,6R)-6-[(1R,2R,3S,4S,5R,6S)-4-amino-2-[(2S,3R,4S,5R)-4-[(2R,3R,4R,5S,6S)-3-azido-6-(azidomethyl)-4,5-dihydroxy-tetrahydropyran-2-yl]oxy-3-hydroxy-5-(hydroxymethyl)tetrahydrofuran-2-yl]oxy-5-benzyloxy-6-(benzyloxy carbonylamino)-3-hydroxy-cyclohexoxy]-5-azido-tetrahydropyran-2-yl]ethyl]-N-benzyl-carbamate (Example 8, Step J; 133 mg, 0.117 mmol), (2S)-2-benzyloxy-4-(benzyloxycarbonylamino)butanoic acid (HABA; 60.1 mg, 0.175 mmol) and DIPEA (0.061 mL, 0.350 mmol) in anhydrous DMF (4.0 mL) was added PyBOP (73.0 mg, 0.140 mol) at 0 °C. The mixture warmed to rt and stirred for 18 h. The mixture was poured into water (5.0 mL) then EtOAc (5.0 mL) was added. The separated aqueous layer was extracted with EtOAc (3 x 5.0 mL). The combined organic layers were washed with brine (5.0 mL), dried (MgSO₄), filtered and concentrated under reduced pressure. The material was purified by reversed phase column (C18, 40 g) using a gradient of 0-100% ACN in Ammonium formate (10 mM, pH 3.8) and concentrated under reduced pressure and lyophilized to provide the title compound (66 mg, 38%) as a white solid. m/z (ESI) [M+H]⁺: 1464.5, HPLC A(05) tR=2.94 min.

### Step B: Synthesis of (S)-4-amino-N-((1R,2S,3S,4R,5R,6S)-3-amino-4-(((2R,3R,6S)-3-amino-6-((S)-1-aminoethyl)tetrahydro-2H-pyran-2-yl)oxy)-5-(((2S,3R,4S,5R)-4-(((2R,3R,4R,5S,6S)-3-amino-6-(aminomethyl)-4,5-dihydroxytetrahydro-2H-pyran-2-yl)oxy)-3-hydroxy-5-(hydroxymethyl)tetrahydrofuran-2-yl)oxy)-2,6-dihydroxycyclohexyl)-2-hydroxybutanamide (Compound 6)

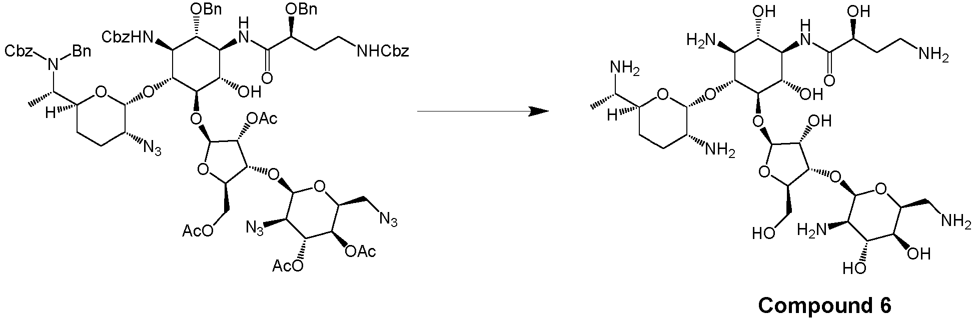

A solution of (2*S*,3*R*,4*R*,5*R*,6*R*)-6-(((2*R*,3*R*,4*R*,5*S*)-4-acetoxy-2-(acetoxymethyl)-5-(((1*R*,2*R*,3*S*,4*R*,5*S*,6*S*)-2-(((2*R*,3*R*,6*S*)-3-azido-6-((*S*)-1-(benzyl((benzyloxy)carbonyl)amino)ethyl)tetrahydro-2H-pyran-2-yl)oxy)-4-(benzyloxy)-5-((S)-2-(benzyloxy)-4-(((benzyloxy)carbonyl)amino)butanamido)-3-(((benzyloxy)carbonyl)amino)-6-hydroxycyclohexyl)oxy)tetrahydrofuran-3-yl)oxy)-5-azido-2-(azidomethyl)tetrahydro-2*H*-pyran-3,4-diyl diacetate (66 mg, 0.0451 mmol) in MeOH (7 mL) was added to a suspension of Pd(OH)₂ (20% wet, 218 mg, 0.311 mmol) in a mixture of AcOH (5.0 mL) and water (3.0 mL). Hydrogen was bubbled for 3 h, then degassed with N₂ for 15 min. The suspension was filtered on 0.22 µM PVDF filter syringe which was washed with MeOH (8.0 mL), water (8.0 mL), and DCM (8.0 mL), then the volatiles were evaporated under reduced pressure (rotary evaporator; bath temperature <35 °C). The crude material was purified by filtration using 5% MeCN in 10 mM NH₄HCO₃ (pH 10) and lyophilized to give a solid. H₂SO₄ (0.05 M in H₂O) was added dropwise to a solution of the above solid in H₂O (3 mL) at 0 °C until pH 4, then lyophilized. The solid was dissolved in water (2 mL), followed by MeOH (10 mL). The suspension was filtered through 0.22 uM PVDF syringe filter, rinsed with MeOH (2 x 5 mL), then washed with water (5 mL). The solid was dissolved in water (10 mL) and lyophilized to provide the title product Compound 6 (30 mg, 66%) as a solid. ¹H NMR (500 MHz, D₂O) *δ* 6.04 (d, *J* = 3 Hz, 1H), 5.47 (m, 1H), 5.33 (m, 2H), 4.58 - 4.55 (m, 1H), 4.48 - 4.34 (m, 3H), 4.30 - 4.21 (m, 3H), 4.05 - 3.85 (m, 6H), 3.81 - 3.66 (m, 2H), 3.63 (m, 1H), 3.61- 3.14 (m, 7H), 2.26 - 2.16 (m, 1H), 2.11 - 2.01 (m, 4H), 1.63 - 1.50 (m, 1H), 1.35 (d, *J* = 6.0 Hz, 3H). Note: 20 exchangeable protons were not observed. m/z (ESI) [M+H]⁺: 714.3, HPLC A(05) tR=0.33 min.

### Example 12: Synthesis of (2R,3R)-4-amino-N-((1R,2S,3S,4R,5R,6S)-3-amino-4-(((2R,3R,6S)-3-amino-6-((S)-1-(methylamino)ethyl)tetrahydro-2H-pyran-2-yl)oxy)-5-(((2S,3R,4S,5R)-4-(((2R,3R,4R,5S,6S)-3-amino-6-(aminomethyl)-4,5-dihydroxytetrahydro-2H-pyran-2-yl)oxy)-3-hydroxy-5-(hydroxymethyl)tetrahydrofuran-2-yl)oxy)-2,6-dihydroxycyclohexyl)-3-fluoro-2-hydroxybutanamide (Compound 7)

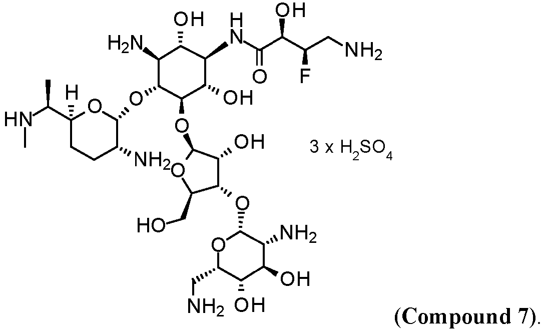

### Step A: Synthesis of (S)-N-[(1S)-1-[(2S)-3,4-dihydro-2H-pyran-2-yl]ethyl]-N,2-dimethylpropane-2-sulfinamide

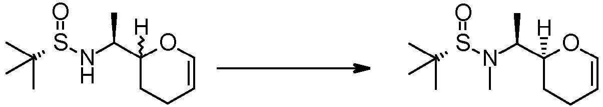

Sodium hydride (60% in mineral oil, 19.2 g, 500 mmol) was added to a mixture of (*S*)-*N*-[(1*S*)-1-[(2*S*)-3,4-dihydro-2*H*-pyran-2-yl]ethyl]-2-methyl-propane-2-sulfinamide (83.0 g, 334 mmol) in THF (1.0 L) at 0°C under nitrogen. The mixture was stirred at 0 °C for 15 min. Iodomethane (41.5 mL, 667 mmol) was added and the mixture was stirred at rt for 3 h. The mixture was diluted with aqueous saturated NH₄Cl (500.0 mL), water (200.0 mL) and the aqueous phase was extracted with ethyl acetate (3 x 250 mL). The combined organic phases were washed with brine (250 mL), dried (MgSO₄), filtered, and concentrated under reduced pressure. The crude residue (94.3 g) was split in two portions, and each fraction was purified by silica gel chromatography (350 g, Biotage HC, liquid loading) using a gradient of 0 to 30% EtOAc in hexanes to provide title compound, and mixed fractions. A third round of purification was performed on mixed fractions that were purified by silica gel chromatography (350 g HC, liquid loading) using a gradient of 0 to 20% EtOAc in hexanes. All the pure fractions from the three purifications were combined to provide the title compound (30.2 g, 31%, ~85% purity). m/z: ES+ [M+H]+ 246.2; HPLC (A05); tR = 2.29 m. (desired title product). m/z: ES+ [M+H]+ 246.2; HPLC (A05); tR = 2.34 min. (unwanted diastereomer).

### Step B: Synthesis of (S)-N-[(1S)-1-[(2S)-6-hydroxy-5-iodo-tetrahydropyran-2-yl]ethyl]-N,2-dimethyl-propane-2-sulfinamide

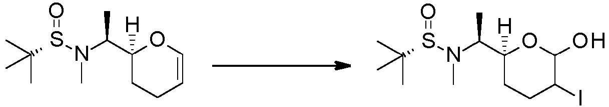

Iodine (31.4 g, 124 mmol) was added portion wise to a suspension of (*S*)-*N*-[(1*S*)-1-[(2*S*)-3,4-dihydro-2H-pyran-2-yl]ethyl]-N,2-dimethyl-propane-2-sulfinamide (30.4 g, 124 mmol) and NaHCO₃ (31.2 g, 372 mmol) in ACN (500.0 mL) and H₂O (500.0 mL) at 0 °C. The mixture was stirred at 0 °C for 30 min. The mixture was stirred at rt for 2 h 30. The mixture was diluted with sat. aq. Na₂S₂O₃ (500.0 mL) and the aqueous layer was extracted with EtOAc (3 x 500.0 mL). The combined organic phases were washed with brine (300.0 mL), dried (MgSO₄), filtered and concentrated to provide the title compound as an oil (47.7 g, 99% crude). The compound was used without any further purification. m/z (ESI) [M+Na]⁺: 412.0, HPLC tR= 2.29 min and 2.40 min (desired product and diastereoisomers).

### Step C: Synthesis of benzyl-N-[(1S)-1-[(2S)-6-hydroxy-5-iodo-tetrahydropyran-2-yl]ethyl]-N-methyl-carbamate

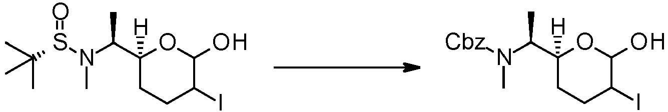

Aqueous 1M HCl (147.0 mL, 147 mmol) was added to a solution of (*S*)-*N*-[(1*S*)-1-[(2*S*)-6-hydroxy-5-iodo-tetrahydropyran-2-yl]ethyl]-N,2-dimethyl-propane-2-sulfinamide (47.7 g, 123 mmol) in dioxane (750.0 mL) at 5-10 °C. The mixture was stirred at 5-10 °C for 45 min, then PBS 10x buffer (~2.0 L) was added until pH reached ~6. The mixture was warmed to rt, then CbzCl (35.0 mL, 245 mmol) was added. The mixture was stirred at rt for 3 h, then EtOAc (500.0 mL) was added. The separated aqueous layer was extracted with EtOAc (3 x 400.0 mL). The combined organic layers were washed with brine, dried (MgSO₄), filtered and concentrated under reduced pressure. The residue was split in two portions (2 x 45 g of crude), then purified on silica (330 g, liquid loading) using EtOAc and hexanes (0 to 50%) to provide the title compound (15.0 g, 27%) as an oil. m/z (ESI) [M+Na]+: 442.0, HPLC, A(05) tR= 2.49 m, 2.54 min. (title product).

### Step D: Synthesis of benzyl-N-[(1S)-1-[(2S)-5-azido-6-oxo-tetrahydropyran-2-yl]ethyl]-N-methylcarbamate

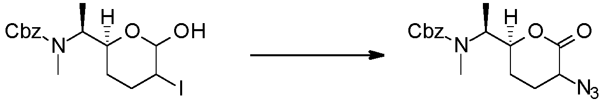

### Oxidation:

To a mixture of benzyl-*N*-[(1*S*)-1-[(2*S*)-6-hydroxy-5-iodo-tetrahydropyran-2-yl]ethyl]-*N*-methylcarbamate (15.0 g, 35.8 mmol) and 4 Å sieves (5.0 g) in DCM (250.0 mL) was added PDC (33.6 g, 89.4 mmol). The mixture was stirred at rt for 18 h, then filtered on a pad of silica gel, rinsed with EtOAc (3 x 350.0 mL) and concentrated under reduced pressure. m/z (ESI) [M+H]⁺: 418.0, HPLC (A05); tR = 2.44 min (oxidized intermediate).

### Azidation:

The residue was taken in DMF (150.0 mL) and cooled to 0°C. NaN₃ (2.21 g, 34.0 mmol) was added, and the mixture was stirred for 1 h at 0°C. Brine (500.0 mL) and Et₂O (150.0 mL) were added. The separated aqueous layer was extracted with Et₂O (2 x 150.0 mL). The combined organic layers were dried (MgSO₄) and concentrated under reduced pressure. The material was purified by MPLC on silica (220 g, Biotage HC) using a gradient of 0-60% EtOAc in hexane to provide the title compound (8.0 g, 67% over 2 steps). m/z (ESI) [M+K]⁺: 371.4, HPLC (A05); tR = 2.33 min (title product).

### Step E: Synthesis of benzyl-N-[(1S)-1-[(2S,5R)-5-azido-6-hydroxy-tetrahydropyran-2-yl]ethyl]-N-methyl-carbamate

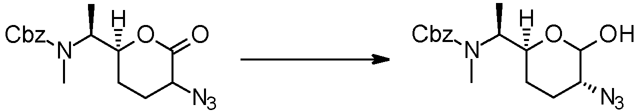

DIBAL-H (1M in Toluene, 43.3 mL, 43.3 mmol) was added dropwise over 20 min to a solution of benzyl-*N*-[(1*S*)-1-[(2*S*)-5-azido-6-oxo-tetrahydropyran-2-yl]ethyl]-N-methyl-carbamate (8.0 g, 24.1 mmol) in DCM (250.0 mL) at -78 °C. After 1 h at -78 °C, EtOH (4.2 mL) was added dropwise. The mixture was poured into a saturated aqueous solution of Rochelle's salt (350.0 mL, conc. 730 g/L in H₂O). The mixture was stirred for 18 h. The separated aqueous layer was extracted with DCM (3 x 150.0 mL). The combined organic layer was washed with brine, dried over MgSO₄, and concentrated under reduced pressure. The residue was purified on silica gel (330 g, Biotage HC, dry loading) by MPLC using 0 to 30% EtOAc in hexane, (gradient for 50 min) to provide the title compound (two diastereoisomers) (3.3 g, 41% desired diastereomer pair, and 1.9 g, 24% of undesired diastereomer pair) as an oil. UPLC: ESI+ [M+H+]⁺: 335.2, UPLC A(05) tR=3.89, 4.15 min (first pair of diastereomers); 4.00, 4.22 min (second pair of diastereomers).

### Step F: Synthesis of [(3R,6S)-3-azido-6-[(1S)-1-[benzyloxycarbonyl(methyl)amino]ethyl]tetrahydropyran-2-yl] (1E)-2,2,2-trifluoro-N-phenyl-ethanimidate

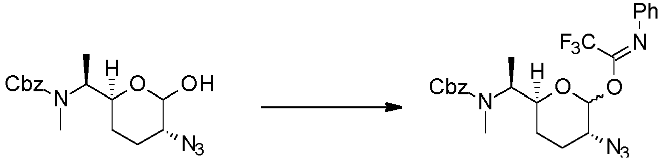

Cesium carbonate (10.9 g, 33.5 mmol) was added to a solution of 2,2,2-trifluoro-N-phenylacetimidoyl chloride (4.28 mL, 26.8 mmol) and benzyl N-[(1S)-1-[(2S,5R)-5-azido-6-hydroxytetrahydropyran-2-yl]ethyl]-N-methyl-carbamate (2.80 g, 8.37 mmol) and in DCM (150.0 mL) at rt under nitrogen. The mixture was stirred at rt for 2 h, then filtered on Celite, rinsed with DCM (40.0 mL) and concentrated. The residue was purified by silica gel chromatography (50 g, Biotage HC, dry pack) with hexane and EtOAc (0-30%) to provide the title compound (2.50 g, 59%) as an oil. m/z (ESI) [M+Na]⁺: 528.2 ; HPLC (A05) tR = 2.96 min.

### Step G: Synthesis of [(1S,2R,3S,4S,5R,6S)-3,4-diacetoxy-5-[(3R,6S)-3-azido-6-[(1S)-1-[benzyloxycarbonyl(methyl)amino]ethyl]tetrahydropyran-2-yl]oxy-2,6-bis(benzyloxycarbonylamino)cyclohexyl] acetate

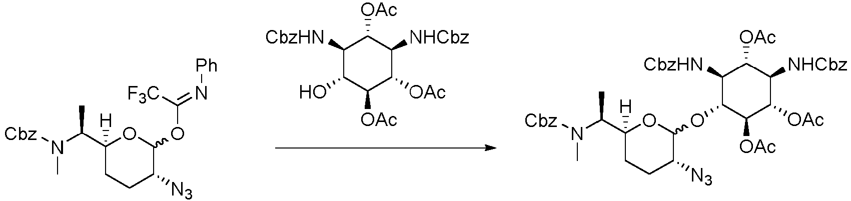

A solution of [(1*S*,2*R*,3*S*,4*S*,5*R*,6*R*)-3,4-diacetoxy-2,6-bis(benzyloxycarbonylamino)-5-hydroxycyclohexyl] acetate (885 mg, 1.55 mmol) and [(3*R*,6*S*)-3-azido-6-[(1*S*)-1-[benzyloxycarbonyl (methyl)amino]ethyl]tetrahydropyran-2-yl]-(1*E*)-2,2,2-trifluoro-*N*-phenyl-ethanimidate (521 mg, 1.03 mmol) were co-evaporated from anhydrous toluene (2 x 15.0 mL). The mixture was dried under reduced pressure for 30 min. The residue was taken in anhydrous DCM (16.0 mL) and Et₂O (8.0 mL), then transferred via a syringe to a RBF charged with pre-activated 3Å and 4Å sieves. The mixture was stirred at rt for 10 min, then Bi(OTf)₃ (3.57 g, 5.44 mmol) was added in one portion at 0 °C. The mixture was stirred at rt for 18h, then diluted by saturated aq. NH₄Cl solution (100.0 mL), and DCM (100.0 mL). The mixture was filtered (Celite), and partitioned. The aqueous was extracted with DCM (3 x 50.0 mL). The combined organic layers were washed brine (100.0 mL), dried (MgSO₄), filtered and concentrated under reduced pressure to give the crude product (6.2 g, crude, ~70% purity) as solid. The material was used directly to the next step without further purification. m/z: ES+ [M+H]+ 889.4 ; LCMS (A05); tR = 2.80 min, 2.85 min (anomeric mixture).

### Step H: Synthesis of benzyl-N-[(1S)-1-[(2S,5R,6R)-5-azido-6-[(1R,2S,3R,4R,5S,6R)-2,4-bis(benzyloxycarbonylamino)-3,5,6-trihydroxy-cyclohexoxy]tetrahydropyran-2-yl]ethyl]-N-methylcarbamate

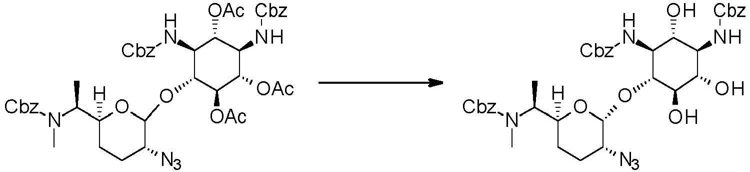

Sodium methoxide (1.70 mL, 7.81 mmol, 4.60 M in MeOH) was added to a solution of [(1*S*,2*R*,3*S*,4*S*,5*R*,6*S*)-3,4-diacetoxy-5-[(3*R*,6*S*)-3-azido-6-[(1*S*)-1-[benzyloxycarbonyl(methyl)amino]ethyl]tetrahydro pyran-2-yl]oxy-2,6-bis(benzyloxycarbonylamino)cyclohexyl] acetate (6.20 g, 4.88 mmol, crude) in MeOH (75.0 mL) at rt. The mixture was stirred at rt for 2 h, diluted with AcOH (~1.70 mL) until pH ~5, then concentrated under reduced pressure. The material was purified by reversed phase preparative HPLC (Xbridge^{™} C18, 50 x 250 mm, 10 µM) using a gradient of 22-63% ACN in ammonium formate (10 mM, pH 3.8). The fractions were pooled, concentrated under reduced pressure to remove ACN. The aqueous was extracted with EtOAc (3 x 50.0 mL). The combined organic phases were dried (MgSO₄), filtered and concentrated under reduced pressure to provide the title compound (1.9 g, 51%) as a solid. m/z (ESI) [M+H]⁺; 763.99

### Step I: Synthesis of benzyl-N-[(1S)-1-[(2S,5R,6R)-6-[(3aS,4R,5S,6S,7R,7aS)-5,7-bis(benzyloxycarbonylamino)-6-hydroxy-spiro[3a,4,5,6,7,7a-hexahydro-1,3-benzodioxole-2,1'-cyclohexane]-4-yl]oxy-5-azido-tetrahydropyran-2-yl]ethyl]-N-methyl-carbamate

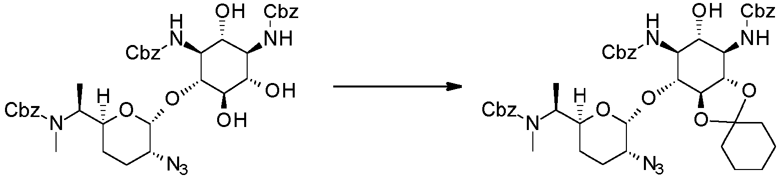

To a solution of benzyl-*N*-[(1*S*)-1-[(2*S*,5*R*,6*R*)-5-azido-6-[(1*R*,2*S*,3*R*,4*R*,5*S*,6*R*)-2,4-bis(benzyloxycarbonylamino)-3,5,6-trihydroxy-cyclohexoxy]tetrahydropyran-2-yl]ethyl]-N-methylcarbamate (1.9 g, 2.49 mmol) and 1,1-dimethoxycyclohexane (3.21 mL, 21.1 mmol) in DME (40.0 mL) was added dried PPTS (259 mg, 1.03 mmol). The mixture was stirred at 90 °C under nitrogen for 2 h. The mixture was cooled to rt and concentrated under reduced pressure. The material was purified by silica gel chromatography (50, Biotage HC, dry loading) with EtOAc and hexanes (0-100%), the fractions were combined and concentrated under reduced pressure to provide the title compound (1.7 g, 81%) as a solid. m/z (ESI) [M+H]⁺: 843.4 ; HPLC (A05) tR = 2.97 min.

### Step J: Synthesis of benzyl-N-[(1S)-1-[(2S,5R,6R)-6-[(3aS,4R,5S,6S,7R,7aS)-6-benzyloxy-5,7-bis(benzyloxycarbonylamino)spiro[3a,4,5,6,7,7a-hexahydro-1,3-benzodioxole-2,1'-cyclohexane]-4-yl]oxy-5-azido-tetrahydropyran-2-yl]ethyl]-N-methyl-carbamate

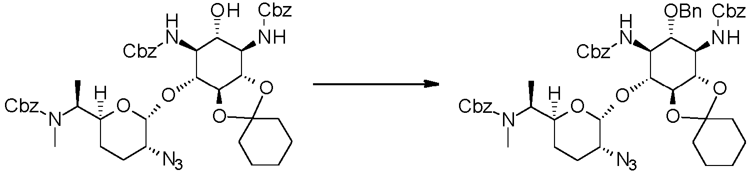

Sodium hydride (60% dispersion in mineral oil, 186 mg, 4.64 mmol) was added to a mixture of benzyl-*N*-[(1*S*)-1-[(2*S*,5*R*,6*R*)-6-[(3a*S*,4*R*,5*S*,6*S*,7*R*,7a*S*)-5,7-bis(benzyloxycarbonylamino)-6-hydroxy-spiro[3a,4,5,6,7,7a-hexahydro-1,3-benzodioxole-2, 1'-cyclohexane]-4-yl]oxy-5-azido-tetrahydro pyran-2-yl]ethyl]-N-methyl-carbamate (1.70 g, 2.02 mmol) and BnBr (2.7 mL, 22.6 mmol) in THF (60.0 mL) at 0 °C under nitrogen. The mixture was stirred at rt for 1h30, then diluted by AcOH (227 µL, 3.97 mmol), followed by silica gel and EtOAc (50.0 mL). The mixture was concentrated under reduced pressure. The residue was purified by silica gel chromatography (120 g, Biotage HC, dry loading) using a gradient of 0-30% EtOAc in hexanes to provide the title compound (1.2 g, 64%) as an oil. m/z: ES+ [M+H]⁺ 933.4; LCMS (A05); tR = 3.12 min.

### Step K: Synthesis of benzyl-N-[(1S)-1-[(2S,5R,6R)-5-azido-6-[(1R,2S,3R,4S,5S,6R)-3-benzyloxy-2,4-bis(benzyloxycarbonylamino)-5,6-dihydroxy-cyclohexoxy]tetrahydropyran-2-yl]ethyl]-N-methylcarbamate

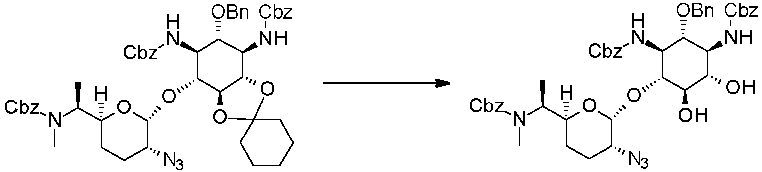

A solution of benzyl-*N*-[(1*S*)-1-[(2*S*,5*R*,6*R*)-6-[(3a*S*,4*R*,5*S*,6*S*,7*R*,7a*S*)-6-benzyloxy-5,7-bis(benzyloxycarbonylamino)spiro[3a,4,5,6,7,7a-hexahydro-1,3-benzodioxole-2,1'-cyclohexane]-4yl]oxy-5-azido-tetrahydropyran-2-yl]ethyl]-*N*-methyl-carbamate (1.20 g, 1.29 mmol) in THF (15.0 mL), water (5.0 mL) and AcOH (12.5 mL) was heated at 80 °C for 12 h, then cooled to rt. The mixture was diluted by slow addition of sat aq. NaHCO₃ (~50 mL) at 0 °C and stirred for 20 min. The separated aqueous layer was extracted with EtOAc (3 x 20.0 mL). The combined organic layer was washed with brine (2 x 10.0 mL), dried (MgSO₄), filtered and concentrated under reduced pressure to provide the title product (1.36 g, 80% purity) as a solid and was used directly without further purification. m/z (ESI) [M+H]⁺: 853.4 HPLC (A05) tR = 2.82 min.

### Step L: Synthesis of benzyl-N-[(1S)-1-[(2S,5R,6R)-5-azido-6-[(1R,2S,3R,4S,5S,6R)-3-benzyloxy-2,4-bis(benzyloxycarbonylamino)-5,6-dihydroxy-cyclohexoxy]tetrahydropyran-2-yl]ethyl]-N-methylcarbamate

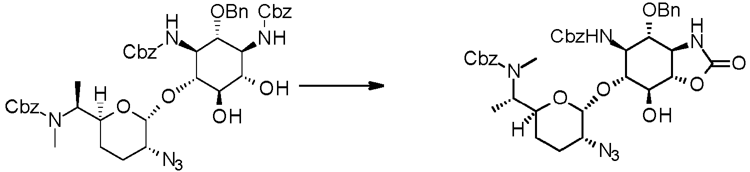

Sodium hydride (223 mg, 5.57 mmol, 60% oil dispersion) was added to a solution of benzyl-*N-*[(1*S*)-1-[(2*S*,5*R*,6*R*)-5-azido-6-[(1*R*,2*S*,3*R*,4*S*,5*S*,6*R*)-3-benzyloxy-2,4-bis(benzyloxycarbonylamino)-5,6-dihydroxy-cyclohexoxy]tetrahydropyran-2-yl]ethyl]-*N*-methyl-carbamate (950 mg, 1.11 mmol) in dry DMF (12 mL) at 0 °C. The mixture was stirred at 0 °C for 1h, diluted by AcOH (0.54 mL, 9.47 mmol), and then slowly poured into stirring water (200 mL). The precipitation was filtered and the solid was washed with water (3 x 150 mL) and lyophilized. The solid was further purified by silica gel column (25 g cartridge, dry loading) using a EtOAc and hexanes (0-100%) to provide the title product (731 mg, 88%) as a solid. m/z (ESI) [M+H]⁺: 745.5, HPLC (A05) tR = 2.60 min.

### Step M; Synthesis of (2S,3R,4R,5R,6R)-6-(((2R,3R,4R,5S)-4-acetoxy-2-(acetoxymethyl)-5-(((3aR,4S,5S,6R,7S,7aS)-6-(((2R,3R,6S)-3-azido-6-((S)-1-(((benzyloxy)carbonyl)(methyl)amino)ethyl)tetrahydro-2H-pyran-2-yl)oxy)-4-(benzyloxy)-5-(((benzyloxy)carbonyl)amino)-2-oxooctahydrobenzo[d]oxazol-7-yl)oxy)tetrahydrofuran-3-yl)oxy)-5-azido-2-(azidomethyl)tetrahydro-2H-pyran-3,4-diyl diacetate

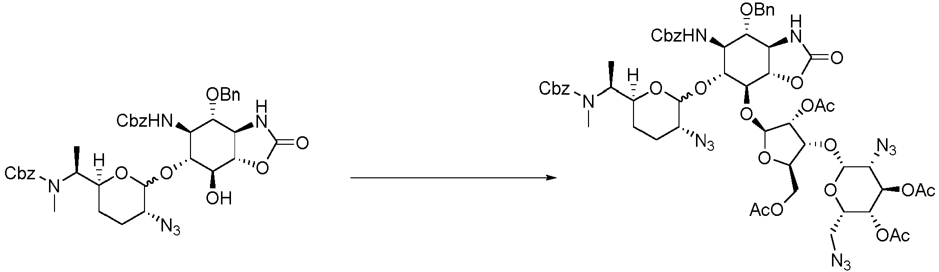

Potassium carbonate (814 mg, 5.89 mmol) was added to a mixture of [(2*R*,3*R*,4*R*)-4-acetoxy-3-[(2*R*,3*R*,4*R*,5*R*,6*S*)-4,5-diacetoxy-3-azido-6-(azidomethyl)tetrahydropyran-2-yl]oxy-5-hydroxy-tetrahydrofuran-2-yl]methyl acetate (2.08 g, 3.93 mmol) and CCl₃CN (0.6 mL, 5.89 mmol) in dry DCM (20 mL). The mixture was stirred at rt for 18h, then filtered through syringe filter, rinsed with DCM, and concentrated under reduced pressure. The residue and benzyl-*N*-[(1*S*)-1-[(2*S*,5*R*,6*R*)-6-[[(3a*R*,4*S*,5*S*,6R*,*7*S*,7a*S*)-4-benzyloxy-5-(benzyloxycarbonylamino)-7-hydroxy-2-oxo-3a,4,5,6,7,7a-hexahydro-3H-1,3-benzoxazol-6-yl]oxy]-5-azido-tetrahydropyran-2-yl]ethyl]-N-methyl-carbamate (731 mg, 0.981 mmol) was co-evaporated with dry toluene (10 mL) under reduced pressure. The residue was dissolved in dry DCM (45 mL), then cannulated to a RBF charged with pre-activated 4Å sieves. The mixture was stirred at -78 °C for 10 min, then BF₃·OEt₂ (2.03 mL, 16.4 mmol) was added at -78 °C over 3 min. Upon completion of the addition, the dry ice bath was removed immediately. The mixture was stirred at rt for 1.5h, then diluted by a saturated aqueous solution of NaHCO₃ (30 mL) at 0 °C. The mixture was filtered through Celite, and the filter cake was washed with DCM (3 x 60 mL). The mixture was partitioned, and the aqueous was extracted with DCM (3 x 20 mL). The combined organic layers were washed with brine (20 mL), dried (MgSO₄), filtered and concentrated under reduced pressure. The material was purified by preparative HPLC (Xbridge^{™} C18, 50 x 250 mm, 10 µM) using 72-86% ACN in ammonium formate (10 mM, pH3.8) and concentrated under reduced to remove ACN. The aqueous was extracted with EtOAc (3 x 50 mL). The combined organic phase was washed with brine (2 x 20 mL), dried (MgSO₄), filtered and concentrated under reduced pressure to provide the title compound (839 mg, 68%) as a solid. m/z (ESI) [M+NH4]⁺: 1274.8; HPLC (A05) tR= 2.83 min.

### Step N: Synthesis of benzyl-((S)-1-((2S,5S,6R)-6-(((1R,2R,3S,4S,5R,6S)-4-amino-2-(((2S,3R,4S,5R)-4-(((2R,3R,4R,5S,6S)-3-azido-6-(azidomethyl)-4,5-dihydroxytetrahydro-2H-pyran-2-yl)oxy)-3-hydroxy-5-(hydroxymethyl)tetrahydrofuran-2-yl)oxy)-5-(benzyloxy)-6-(((benzyloxy)carbonyl)amino)-3-hydroxycyclohexyl)oxy)-5-azidotetrahydro-2H-pyran-2-yl)ethyl)(methyl)carbamate

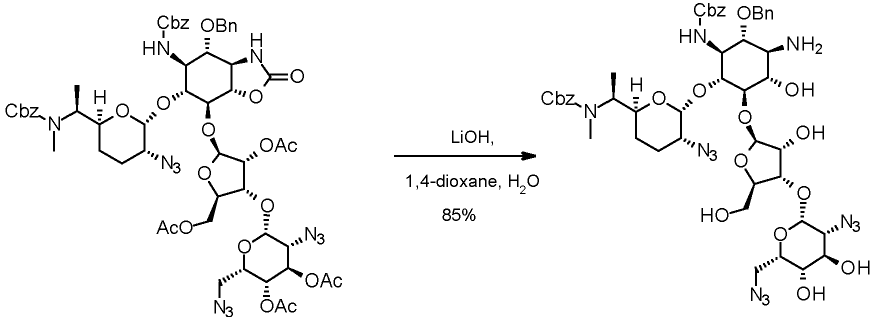

Lithium hydroxide monohydrate (840 mg, 20 mmol) was added to a solution of (2S,3R,4R,5R,6R)-6-(((2R,3R,4R,5S)-4-acetoxy-2-(acetoxymethyl)-5-(((3aR,4S,5S,6R,7S,7aS)-6-(((2R,3R,6S)-3-azido-6-((S)-1-(((benzyloxy)carbonyl)(methyl)amino)ethyl)tetrahydro-2H-pyran-2-yl)oxy)-4-(benzyloxy)-5-(((benzyloxy)carbonyl)amino)-2-oxooctahydrobenzo[d]oxazol-7-yl)oxy)tetrahydrofuran-3-yl)oxy)-5-azido-2-(azidomethyl)tetrahydro-2H-pyran-3,4-diyl diacetate (839 mg, 0.667 mmol) in 1,4-dioxane (10 mL) and water (5 mL). The mixture was stirred at 40 °C for 18 h, cooled to rt, diluted by EtOAc (50 mL) and water (20 mL) and then partitioned. The separated aqueous layer was extracted with EtOAc (3 x 50 mL). The combined organic layers were washed with water (3 x 5 mL), brine (5 mL), dried (MgSO₄), filtered, concentrated under reduced pressure. The material was purified by preparative HPLC (ACCQ) using 50-60% ACN in AmBic buffer (10 mM, pH 10) and concentrated under reduced to remove ACN. The aqueous was extracted in EtOAc (3 x 50 mL). The combined organic phases were washed with water (3 x 20 mL), brine (2 x 10 mL), dried (MgSO₄), filtered, concentrated under reduced pressure to provide the title compound (602 mg, 85%) as a solid. m/z (ESI) [M+H]⁺: 1063.3, HPLC (A05) tR=2.35 min.

### Step O: Synthesis of benzyl-((S)-1-((2S,5S,6R)-5-azido-6-(((1R,2R,3S,4S,5R,6S)-4-((2R,3R)-4-azido-3-fluoro-2-((4-methoxybenzyl)oxy)butanamido)-2-(((2S,3R,4S,5R)-4-(((2R,3R,4R,5S,6S)-3-azido-6-(azidomethyl)-4,5-dihydroxytetrahydro-2H-pyran-2-yl)oxy)-3-hydroxy-5-(hydroxymethyl)tetrahydrofuran-2-yl)oxy)-5-(benzyloxy)-6-(((benzyloxy)carbonyl)amino)-3-hydroxycyclohexyl)oxy)tetrahydro-2H-pyran-2-yl)ethyl)(methyl)carbamate

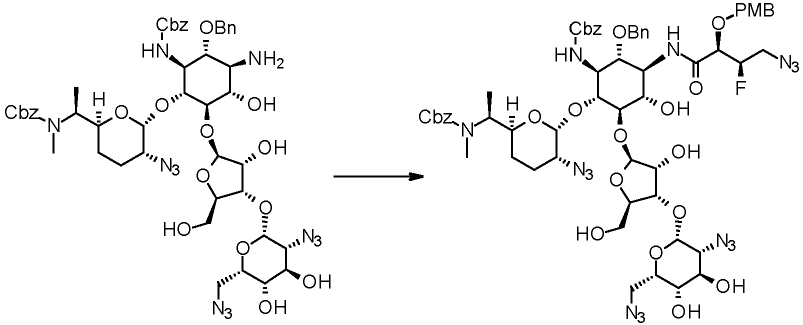

DMTMM chloride (66.4 mg, 0.24 mmol) was added to a solution of (2R,3R)-4-azido-3-fluoro-2-[(4-methoxyphenyl)methoxy]butanoic acid (65.9 mg, 0.233 mmol) and benzyl-((S)-1-((2S,5S,6R)-6-(((1R,2R,3S,4S,5R,6S)-4-amino-2-(((2S,3R,4S,5R)-4-(((2R,3R,4R,5S,6S)-3-azido-6-(azidomethyl)-4,5-dihydroxytetrahydro-2H-pyran-2-yl)oxy)-3-hydroxy-5-(hydroxymethyl)tetrahydrofuran-2-yl)oxy)-5-(benzyloxy)-6-(((benzyloxy)carbonyl)amino)-3-hydroxycyclohexyl)oxy)-5-azidotetrahydro-2H-pyran-2-yl)ethyl)(methyl)carbamate (150 mg, 0.141 mmol) in anhydrous THF (6 mL) at rt. The mixture was stirred at rt for 18 h, then diluted by sat NH₄Cl solution (10 mL) and EtOAc (20 mL), and then partitioned. The aqueous was extracted with EtOAc (2 x 5 mL). The combined organic layers were washed with water (2 x 5 mL), brine (2 x 5 mL), dried (MgSO₄), filtered and concentrated under reduced pressure to give a foam. The material was purified by preparative HPLC (ACCQ) using 58-69% ACN in Ammonium formatee (10 mM, pH 3.8) and concentrated under reduced to remove ACN. The aqueous was extracted in EtOAc (3 x 20 mL). The combined organic phase was washed with water (2 x 10 mL), brine (2 x 5 mL), dried (MgSO₄), filtered, concentrated under reduced pressure to provide the title compound (167.2 mg, 89%) as a solid. m/z (ESI) [M+H]+: 1328.2, HPLC A(05) tR = 2.70 min.

### Step P: Synthesis of (2R,3R)-4-amino-N-((1R,2S,3S,4R,5R,6S)-3-amino-4-(((2R,3R,6S)-3-amino-6-((S)-1-(methylamino)ethyl)tetrahydro-2H-pyran-2-yl)oxy)-5-(((2S,3R,4S,SR)-4-(((2R,3R,4R,SS,6S)-3-amino-6-(aminomethyl)-4,5-dihydroxytetrahydro-2H-pyran-2-yl)oxy)-3-hydroxy-5-(hydroxymethyl)tetrahydrofuran-2-yl)oxy)-2,6-dihydroxycyclohexyl)-3-fluoro-2-hydroxybutanamide (Compound 7)

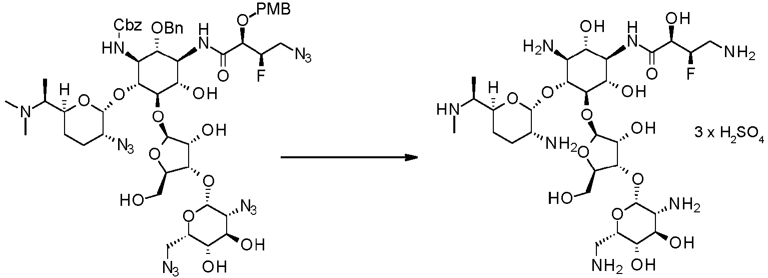

A solution of benzyl-((S)-1-((2S,5S,6R)-5-azido-6-(((1R,2R,3S,4S,5R,6S)-4-((2R,3R)-4-azido-3-fluoro-2-((4-methoxybenzyl)oxy)butanamido)-2-(((2S,3R,4S,5R)-4-(((2R,3R,4R,5S,6S)-3-azido-6-(azidomethyl)-4,5-dihydroxytetrahydro-2H-pyran-2-yl)oxy)-3-hydroxy-5-(hydroxymethyl)tetrahydrofuran-2-yl)oxy)-5-(benzyloxy)-6-(((benzyloxy)carbonyl)amino)-3-hydroxycyclohexyl)oxy)tetrahydro-2H-pyran-2-yl)ethyl)(methyl)carbamate (167 mg, 0.126 mmol) in MeOH (3.6 mL) was added to a suspension of Pd(OH)₂ (530 mg, 0.754 mmol, 20% wet) in AcOH (3 mL) and water (1.2 mL). Hydrogen was bubbled continuously for 4 h. then nitrogen was bubbled for 15 min. The suspension was filtered on 0.45 µM PVDF filter syringe, which was washed with MeOH (20 mL) and water (2 x 12 mL). The volatiles were evaporated under reduced pressure (rotavapor bath temperature < 35 °C). The material was purified by preparative HPLC using 5-15% of MeCN in 10 mM ammonium bicarbonate pH 10, then lyophilized to give a solid. H₂SO₄ (0.05 M in H₂O) was added dropwise to a solution of the above solid in H₂O (3 mL) at 0 °C until pH 4-5, then lyophilized to give a solid. The solid was dissolved in water (2 mL), followed by MeOH (10 mL) to give a precipitate. This suspension was filtered through 0.45 uM PVDF syringe filter, rinsed with MeOH (2 x 10 mL). The solid was dissolved in water (5 mL) and then lyophilized to give the title product (70 mg, 54%) as a white solid. ¹H NMR (500 MHz, D₂O) δ 5.98 (d, J = 3.8 Hz, 1H), 5.43 (d, J = 2.0 Hz, 1H), 5.39 - 5.20 (m, 2H), 4.59 - 4.46 (m, 2H), 4.43 (dd, J = 4.7, 2.2 Hz, 1H), 4.35 (ddt, J = 5.5, 3.9, 1.7 Hz, 1H), 4.30 - 4.22 (m, 2H), 4.08 (t, J = 9.3 Hz, 1H), 4.02 - 3.91 (m, 4H), 3.88 - 3.82 (m, 1H), 3.80 - 3.72 (m, 2H), 3.66 (dd, J = 10.6, 9.1 Hz, 1H), 3.62 - 3.58 (m, 1H), 3.58 - 3.36 (m, 5H), 3.31 - 3.17 (m, 2H), 2.75 (s, 3H), 2.14 - 2.00 (m, 3H), 1.64 - 1.49 (m, 1H), 1.32 (d, J = 6.8 Hz, 3H. Note: 25 exchangeable protons are not observed. m/z (ESI) [M+H]+: 746.2; HPLC A (05) tR= 0.32 min.

### Example 13: Synthesis of (2S,3S,4R,5R,6R)-5-amino-2-(aminomethyl)-6-(((2R,3S,4R,5S)-5-(((1R,2R,3S,4R,5R,6S)-3,5-diamino-2-(((2R,3R,6S)-3-amino-6-((S)-1-aminoethyl)tetrahydro-2H-pyran-2-yl)oxy)-4,6-dihydroxycyclohexyl)oxy)-4-hydroxy-2-(hydroxymethyl)tetrahydrofuran-3-yl)oxy)tetrahydro-2H-pyran-3,4-diol (Compound 8)

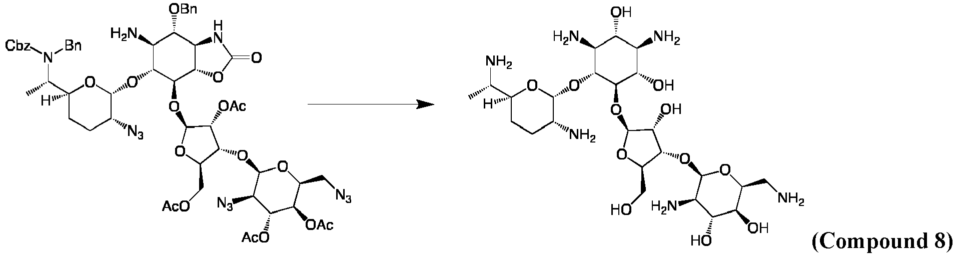

A solution of (2S,3R,4R,5R,6R)-6-(((2R,3R,4R,5S)-4-acetoxy-2-(acetoxymethyl)-5-(((3aR,4S,5S,6R,7S,7aS)-6-(((2R,3R,6S)-3-azido-6-((S)-1-(((benzyloxy)carbonyl)(methyl)amino)ethyl)tetrahydro-2*H*-pyran-2-yl)oxy)-4-(benzyloxy)-5-(((benzyloxy)carbonyl)amino)-2-oxooctahydrobenzo[d]oxazol-7-yl)oxy)tetrahydrofuran-3-yl)oxy)-5-azido-2-(azidomethyl)tetrahydro-2H-pyran-3,4-diyl diacetate **(Example 12, Step N;** 62 mg, 0.0544 mmol) in MeOH (3.6 mL) was added to a suspension of Pd(OH)₂ (229 mg, 0.327 mmol, 20% wet) in a mixture of AcOH (3 mL) and water (1.2 mL). The mixture was evacuated with H₂ for three times, then bubbled with hydrogen (balloon) continuously for 4 h. The mixture was bubbled with N₂ for 15 min. The suspension was filtered on 0.45 µM PVDF filter syringe which was washed with MeOH (2 x 10 mL) and water (2 x 12 mL), and the volatiles were evaporated under reduced pressure (rotavapor bath temperature <35 °C). The material was purified by filtration using 5% MeCN in 10 mM ammonium bicarbonate (pH 10) and lyophilized to give a solid. H₂SO₄ (0.05 M in H₂O) was added dropwise to a solution of the above solid in H₂O (3 mL) at 0 °C until pH 4-5, then lyophilized to give a solid. The solid was dissolved in water (2 mL), and MeOH (10 mL) was added. The suspension was filtered through 0.45 uM PVDF syringe filter and rinsed with MeOH (2 x 5 mL). The solid was dissolved with water (10 mL) and lyophilized to give the title (18 mg, 18%, 50% purity) as a white solid. m/z (ESI) [M+H]⁺: 613.2; HPLC A (05) tR= 0.33 min.

### Example 14: Minimum Inhibitory Concentration Assays

Minimum inhibitory concentrations for representative example compounds herein were determined by a broth microdilution method in accordance with the Clinical and Laboratory Standards Institute (CLSI) guidelines. In brief, organism suspensions were adjusted to a 0.5 McFarland standard to yield a final inoculum between 3×10⁵ and 7×10⁵ colony-forming units (CFU)/mL. The inoculum was prepared by suspension of a colony from an agar plate that was prepared the previous day. Compound dilutions and inoculum were prepared in sterile, cation adjusted Mueller-Hinton Broth (Beckton Dickinson).

Bacteria were suspended in sterile saline and added to each assay plate to obtain a final concentration of 5×10⁵ CFU/mL. An inoculum volume of 100 µL was added to wells containing 100 µL of broth with 2-fold serial dilutions of the test compound. All inoculated microdilution trays were incubated in ambient air at 35°C for 18-24 hours. Following incubation, the lowest concentration of the compound that prevented visible growth (OD 600 nm < 0.05) is recorded was the MIC. The MIC was determined to be the lowest concentration of the test compound that resulted in no visible bacterial growth as compared to an untreated control. Performance of the assay was monitored by the use of laboratory quality-control strains and a standard compound with a defined MIC spectrum (neomycin), in accordance with CLSI guidelines.

Compounds of the disclosure **(Compounds 1-6)** were evaluated against multiple strains of *A. baumannii, E. coli,* and *P. aeruginosa.* Results are provided in Table 1 below, which demonstrated that Compounds 1-6 were active against the majority of the strains evaluated with MIC values generally equal to or less than 16 µg/ml. Importantly, all of the compounds retain antimicrobial activity against strains of *P. aeruginosa* and *E*. *coli* that express aminoglycoside modifying enzymes such as Aph(3')-IIb or Aph(3')-Ia. Additionally, **Compounds 1-6** retain potent activity against an *E. coli* strain (NECO0245) that expresses the ribosomal methyl transferase ArmA, which is known to confer resistance to all clinically relevant parenteral aminoglycoside antibiotics.

**Table 1. Microbiology data - Primary Panel**

| **Strain Code** | **Phenotype** | **Species** | **Compound # and Activity*** | | | | | | | | |
|---|---|---|---|---|---|---|---|---|---|---|---|
| | | | **NEO** | **1** | **2** | **3** | **4** | **5** | **6** | **7** | **8** |
| NABA0006 | WT parent | *A. baumannii* | + | + | + | + | + | + | + | + | + |
| NABA0007 | ΔadeJK efflux | *A. baumannii* | + | + | + | + | + | + | + | + | + |
| NABA0020 | AME negative strain | *A. baumannii* | + | + | + | + | + | + | + | + | - |
| NECO0001 | WT QC | *E. coli* | + | + | + | + | + | + | + | + | + |
| NECO0002 | WT | *E. coli* | + | + | + | + | + | + | + | + | + |
| NECO0003 | ΔtolC | *E. coli* | + | + | + | + | + | + | + | + | + |
| NPAE0001 | WT Aph(3')-IIb, QC | *P. aeruginosa* | - | + | + | + | + | + | + | + | + |
| NPAE0002 | WT Aph(3')-IIb | *P. aeruginosa* | + | + | + | + | + | + | + | + | + |
| NPAE0006 | Efflux KO, Aph(3')-IIb | *P. aeruginosa* | + | + | + | + | + | + | + | + | + |
| NPAE0010 | MexXY overexpress ed, Aph(3°)-IIb | *P. aeruginosa* | - | + | + | + | + | + | + | - | + |
| NPAE0359 | aph(3')-IIb KO (tetR) | *P. aeruginosa* | - | + | + | + | + | + | + | + | + |
| NECO0243 | empty vector | *E. coli* | + | + | + | + | + | + | + | + | + |
| NECO0244 | AAC(6')-Ib | *E. coli* | + | + | + | + | + | + | + | + | + |
| NECO0350 | AAC(2')-I | *E. coli* | + | + | + | + | + | + | + | + | + |
| NECO0289 | APH(3')-Ia | *E. coli* | - | + | + | + | + | + | + | - | - |
| NECO0359 | ANT(4')-Ia | *E. coli* | + | + | + | + | + | + | + | + | + |
| NECO0302 | AAC(3)-IIa | *E. coli* | + | + | + | + | + | + | + | + | + |
| NECO0245 | ArmA | *E. coli* | + | + | + | + | + | + | + | + | + |

| | | | | | | | | | | | |
|---|---|---|---|---|---|---|---|---|---|---|---|
| *MIC threshold for positive activity (+) is ≤ 16 µg/mL, and for inactive (-) is > 16 µg/mL); NEO = neomycin | | | | | | | | | | | |

Several compounds of the disclosure **(Compounds 1, 2, 3,** and **6)** were further evaluated against a secondary panel of clinical isolates of multi-drug resistant bacterial pathogens including *A. baumannii,* methicillin-resistant *Staphylococcus aureus* (MRSA), *P. aeruginosa,* and carbapenem-resistant Enterobacteriaceae (CRE). Results are provided in **Table 2** below, which demonstrated that **Compounds 1, 2, 3,** and **6** were active against most of the strains evaluated, with minimum inhibitory concentration (MIC) values generally equal to or less than 16 µg/ml.

**Table 2. Microbiology Data - Secondary Panel**

| **Strain Code** | **Phenotype** | **Species** | **Aka** | **Compound # and Activity*** | | | | |
|---|---|---|---|---|---|---|---|---|
| | | | | **1** | **2** | **3** | **6** | **Neomycin** |
| NABA1427 | MDR Clinical isolate | *A. baumannii* | | + | + | + | + | - |
| NABA1433 | MDR Clinical isolate | *A. baumannii* | | + | + | + | + | - |
| NABA1434 | MDR Clinical isolate | *A. baumannii* | | + | + | + | + | + |
| NABA1436 | MDR Clinical isolate | *A. baumannii* | | + | + | + | + | - |
| NABA1437 | MDR Clinical isolate | *A. baumannii* | | + | + | + | + | + |
| NABA1438 | MDR Clinical isolate | *A. baumannii* | | + | + | + | + | - |
| NABA1439 | MDR Clinical isolate | *A. baumannii* | | + | + | + | + | - |
| NABA1448 | MDR Clinical isolate | *A. baumannii* | | + | + | + | + | - |
| NABA1449 | MDR Clinical isolate | *A. baumannii* | | + | + | + | + | + |
| NABA1450 | MDR Clinical isolate | *A. baumannii* | | + | + | + | + | - |
| NSAU001 | wild-type; QC | *S. aureus* | ATCC 29213 | + | + | + | + | + |
| NSAU1004 | MDR Clinical isolate | *S. aureus* | | + | + | + | + | + |
| NSAU1015 | MDR Clinical isolate | *S. aureus* | | + | + | + | + | + |
| NSAU1023 | MDR Clinical isolate | *S. aureus* | | + | + | + | + | - |
| NSAU1063 | MDR Clinical isolate | *S. aureus* | | + | + | + | + | - |
| NPAE1288 | MDR Clinical isolate | *P. aeruginosa* | | + | + | + | + | - |
| NPAE1293 | MDR Clinical isolate | *P. aeruginosa* | | + | + | + | + | - |
| NPAE1299 | MDR Clinical isolate | *P. aeruginosa* | | + | + | + | + | - |
| NPAE1302 | MDR Clinical isolate | *P. aeruginosa* | | + | + | + | + | - |
| NPAE1303 | MDR Clinical isolate | *P. aeruginosa* | | + | + | + | + | - |
| NPAE1308 | MDR Clinical isolate | *P. aeruginosa* | | + | + | + | - | - |
| NPAE13 10 | MDR Clinical isolate | *P. aeruginosa* | | + | + | + | + | + |
| NPAE1313 | MDR Clinical isolate | *P. aeruginosa* | | - | + | - | + | - |
| NPAE1316 | MDR Clinical isolate | *P. aeruginosa* | | + | + | + | + | + |
| NPAE1323 | MDR Clinical isolate | *P. aeruginosa* | | + | + | + | + | - |
| AECO1225 | MDR Clinical isolate (CDC CRE panel) | *E. coli* | 0118** | + | + | + | + | + |
| AECO1226 | MDR Clinical isolate (CDC CRE panel) | *E. coli* | 0119** | + | + | + | + | + |
| ASSE1001 | MDR Clinical isolate (CDC CRE panel) | *S. enterica* | 0127** | + | + | + | + | + |
| AECO 1227 | MDR Clinical isolate (CDC CRE panel) | *E. coli* | 0128** | + | + | + | + | + |
| AKPN1246 | MDR Clinical isolate (CDC CRE panel) | *K. pneumoniae* | 0139** | + | + | + | + | + |
| AKPN1250 | MDR Clinical isolate (CDC CRE panel) | *K. pneumoniae* | 0143** | + | + | + | + | + |
| AKPN1253 | MDR Clinical isolate (CDC CRE panel) | *K. pneumoniae* | 0148** | + | + | + | + | + |
| AKPN1255 | MDR Clinical isolate (CDC CRE panel) | *K. pneumoniae* | 0153** | + | + | + | + | + |
| ACSP1001 | MDR Clinical isolate (CDC CRE panel) | *C. freundii* | 0157** | + | + | + | + | - |
| APMI1048 | MDR Clinical isolate (CDC CRE panel) | *P. mirabilis* | 0159** | + | + | + | + | + |
| AEAE1042 | MDR Clinical isolate (CDC CRE panel) | *K. aerogenes* | 0161** | + | + | + | + | + |
| AECL1089 | MDR Clinical isolate (CDC CRE panel) | *E. cloacae* | 0154** | + | + | + | + | + |

| | | | | | | | | |
|---|---|---|---|---|---|---|---|---|
| * MIC threshold for positive activity (+) is ≤ 16 µg/mL, and for inactive (-) is > 16 µg/mL) **CDC Antibiotic Resistance (AR) Isolate Bank Strain Code | | | | | | | | |

Several compounds of the disclosure **(Compound 1, 2,** and **3)** were further evaluated against several hundred bacterial isolates (isolated in the United States and Europe, 1:1) selected from the SENTRY Antimicrobial Surveillance Program (JMI Laboratories, North Liberty, Iowa). The set of clinical isolates included 300 randomly selected *Enterobacterales,* 103 randomly selected *P. aeruginosa,* 100 randomly selected *A. baumannii-calcoaceticus,* 20 randomly selected Methicillin-sensitive *S*. *aureus* (MSSA), and 30 randomly selected Methicillin-resistant *S*. *aureus* (MRSA). In addition, 101 *Enterobacterales* and 50 *P. aeruginosa* isolates with genetically defined mechanisms of aminoglycoside resistance were included. The MIC values at which ≥ 50% (MIC₅₀) and ≥ 90% (MIC₉₀) of isolates within a given set are inhibited were determined for each compound against each set of organisms. The results are provided in **Table 3** and demonstrate that **Compounds 1, 2** and **3** are active against all sets of organisms tested with MIC_{50/90} values ≤ 16 µg/mL.

**Table 3 Microbiology Surveillance of Clinical Isolates (USA & Europe)**

| **Antibacterial Surveillance (MIC_{50/90})** | | | | | |
|---|---|---|---|---|---|
| | | Compound | | | |
| **Organism Group** | **# of Isolates** | **GEN** | **1** | **2** | **3** |
| Enterobacterales | 300 | +/+ | +/+ | +/+ | +/+ |
| AG-resistant Enterobacterales | 101 | -/- | +/+ | +/+ | +/+ |
| *P. aeruginosa* | 103 | +/+ | +/+ | +/+ | +/+ |
| AG-resistant *P. aeruginosa* | 50 | +/- | +/+ | +/+ | +/+ |
| *A. baumannii-calcoaceticus* | 100 | +/- | +/+ | +/+ | +/+ |
| MSSA | 20 | +/+ | +/+ | +/+ | +/+ |
| MRSA | 30 | +/+ | +/+ | +/+ | +/+ |

| | | | | | |
|---|---|---|---|---|---|
| MIC₅₀= MIC value at which ≥ 50% of isolates are inhibited; MIC₉₀= MIC value at which ≥90% of isolates are inhibited; + = MIC₅₀ or MIC₉₀ ≤ 16 µg/mL; - = MIC₅₀ or MIC₉₀ >16 µg/mL; AG-resistant = aminoglycoside resistant; MSSA = Methicillin-sensitive *S*. *aureus;* MRSA = Methicillin-resistant S. *aureus;* GEN = gentamicin | | | | | |

### Example 15. In vivo kidney accumulation studies

Compounds are evaluated for potential to accumulate in kidney tissue as a surrogate measure of nephrotoxicity. The measured drug concentrations in rat kidneys, normalized to the plasma pharmacokinetics area under the curve, indicates the extent of accumulation in the kidneys. Without wishing to be bound by theory, it is believed that aminoglycosides with lower nephrotoxic potential are generally less likely to demonstrate high kidney accumulation, and lower accumulation is therefore believed to correlate with a better safety profile with respect to nephrotoxicity.

### Example 16. In vivo repeat-dose nephrotoxicity studies

Compounds are evaluated for nephrotoxic potential via a repeat-dose study in rats. Individual drugs are administered to rats via subcutaneous or intravenous injection once daily for multiple sequential days. Blood samples are analyzed for biomarkers of nephrotoxicity such as serum creatinine, urea, and BUN. At terminal time-points, the rat kidneys are resected, fixed, and tissues analyzed by histopathology and immunochemistry to determine No Observed Adverse Effect Level (NOAEL) value for each compound. Without wishing to be bound by theory, it is believed that seemingly small structural modifications on the aminoglycoside ABCD ring system (e.g., removing or altering substituents) may result in significant differences in nephrotoxicity independent of any potential changes in antibacterial activity.

### Example 17. In vivo antibacterial efficacy studies

Compounds of the disclosure were evaluated for *in vivo* antibacterial activity in a neutropenic mouse lung infection model. CD-1 male mice (6 - 8 weeks old) were rendered neutropenic by a 0.2 mL IP injection of cyclophosphamide (Cytoxan) at 150 mg/kg at day -4 and 100 mg/kg at day -1 pre-infection. Mice were anesthetized by IP injection of 0.2 mL of ketamine HCl (100 mg/kg) and xylazine (10 mg/kg), followed by intranasal inoculation with 0.05 mL of a target bacterial inoculum of approximately 1 x 10⁶ colony forming units (CFU). Drug treatment was initiated by subcutaneous injection 2 hours post-infection and continued q6hr. After 26 hours post-infections, the mice were euthanized, lungs homogenized, and the homogenized lungs serially diluted, spread onto BHI/Charcoal agar and incubated at 37°C. After incubation overnight, the colonies were counted manually.

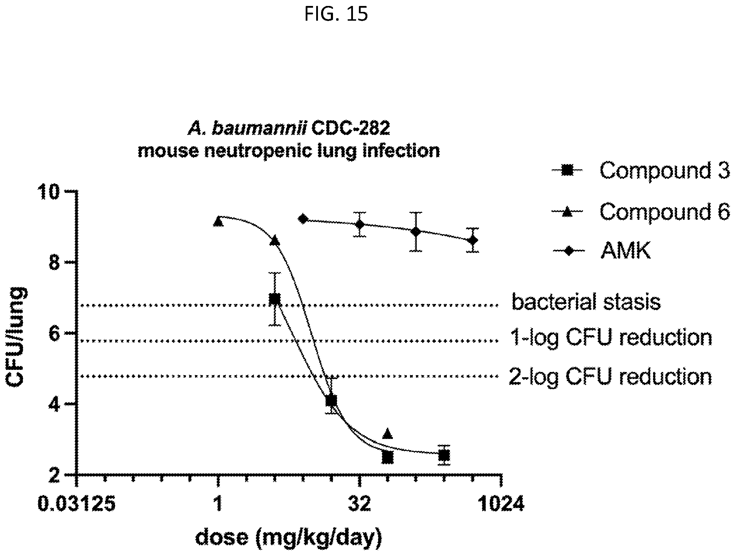

Two compounds of the disclosure, **Compounds 3** and **6,** were evaluated in the model against the *A. baumannii* strain CDC-313 from the CDC and FDA Antimicrobial Resistance Isolate Bank. Results from the study are shown in **FIG. 15****.** With reference to **FIG. 15****,** the data demonstrate that **Compounds 3** and 6 have antibacterial activity *in vivo* against the highly resistant *A. baumannii* CDC-313 strain, as evidenced by greater than 2-log reduction in bacterial CFUs relative to CFUs present at the initiation of therapy. In contrast, amikacin (AMK, an aminoglycoside antibiotic) showed minimal activity against the strain.

## Claims

1. A compound having a structure: 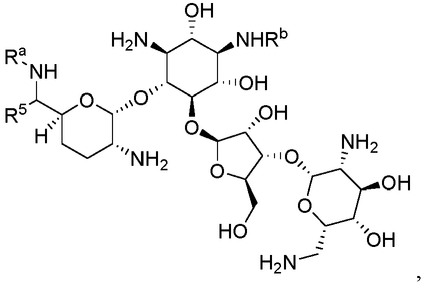
or a pharmaceutically acceptable salt or solvate thereof,
wherein:
R^{a} is H or methyl;
R^{b} has a structure according to Formula (II): 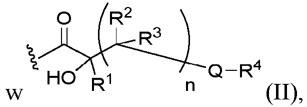 wherein:
Q is NH, O, or S;
n is an integer from 0 to 4;
R¹ is hydrogen or C₁₋₃ alkyl;
R² and R³ are each selected independently for each occurrence from the group consisting of hydrogen, alkyl, halogen, and -OH;
R⁴ is H, C₁₋₃ alkyl, or -C(=NH)NR^{4a}R^{ab}, wherein R^{4a} and R^{4b} are each independently selected from the group consisting of hydrogen and C₁₋₃ alkyl; or
R¹ and R⁴, together with the atoms to which they are attached, form a heterocycloalkyl ring system comprising at least one N; and
R⁵ is H or methyl

2. The compound of claim 1, wherein R^{a} is H.

3. The compound of claim 1, wherein R^{a} is methyl.

4. The compound of any one of claims 1-3, wherein R^{b} is selected from the group consisting of:

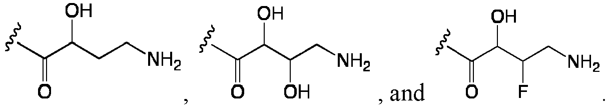

5. The compound of claim 4, wherein R^{b} is selected from the group consisting of:

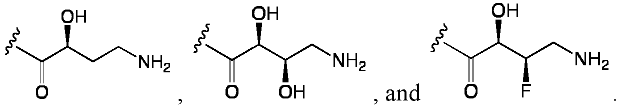

6. The compound of any one of claims 1-5, wherein R⁵ is methyl.

7. The compound of claim 6, having a structure selected from the group consisting of:

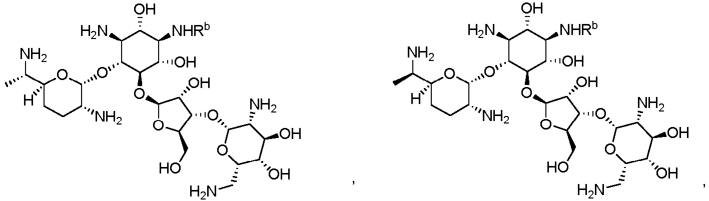

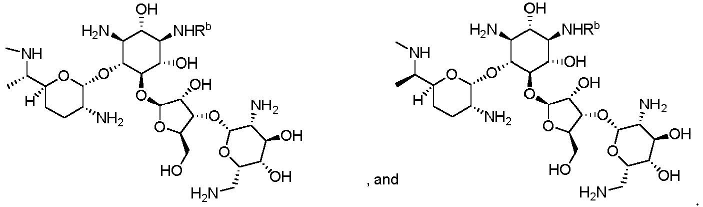

8. The compound of claim 7, having a structure selected from the group consisting of:

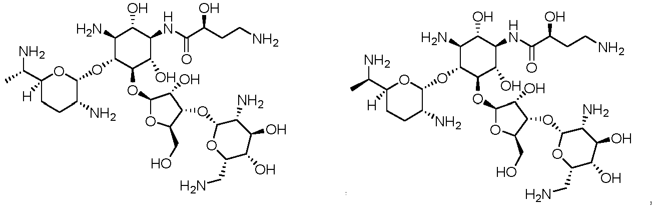

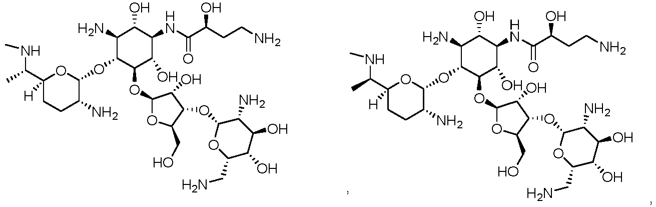

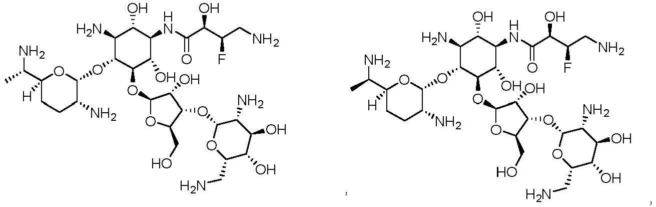

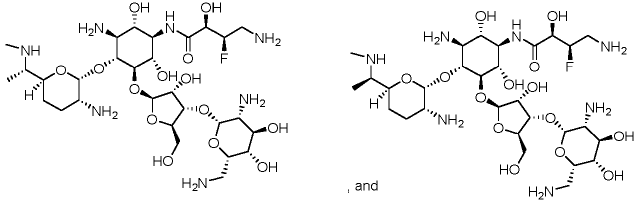

9. The compound of any one of claims 1-5, wherein R⁵ is H.

10. The compound of claim 9, having a structure selected from the group consisting of:

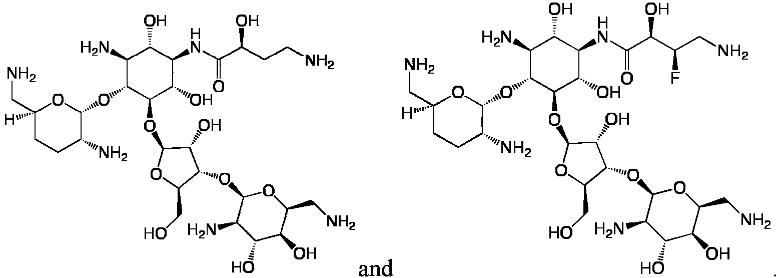

11. A pharmaceutical composition comprising the compound of any one of claims 1-10, or a pharmaceutically acceptable salt, solvate, tautomer, or stereoisomer thereof, and at least one pharmaceutically acceptable excipient.

12. The compound of any one of claims 1-10, or a pharmaceutically acceptable salt, solvate, tautomer, or stereoisomer thereof, or the pharmaceutical composition of claim 11, for use in treating a bacterial infection in a subject in need thereof.

13. The compound or pharmaceutical composition for use according to claim 12, wherein the bacterial infection is with Gram-positive, Gram-negative, aerobic, or facultative anaerobic bacteria.

14. The compound or pharmaceutical composition for use according to claim 13, wherein one or more of the following apply:
the bacterial infection is with an *Escherichia* sp., a *Klebsiella* sp. *a Proteus* sp., a *Citrobacter* sp., a *Morganella* sp., a *Providencia* sp., *a Yersinia* sp., an *Enterobacter* sp., a *Salmonella* sp., or a *Serratia* sp.;
the bacterial infection is with a *Moraxella* sp., a *Pseudomonas* sp., an *Acinetobacter* sp., a *Mycobacterium* sp., a *Staphylococcus* sp., a *Bacillus* sp., a *Francisella* sp., or a *Burkholderia* sp.

## Patentansprüche

1. Eine Verbindung mit einer Struktur: 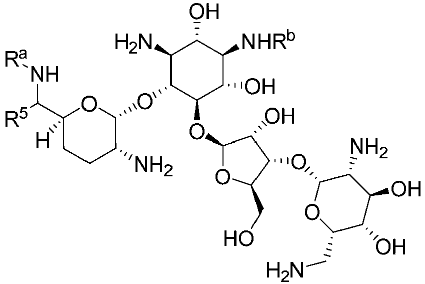
oder ein pharmazeutisch annehmbares Salz oder Solvat davon,
wobei:
R^{a} H oder Methyl ist;
R^{b} eine Struktur gemäß Formel (II) aufweist: 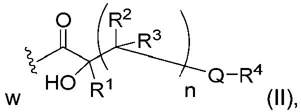 wobei:
Q NH, O oder S ist;
n eine ganze Zahl von 0 bis 4 ist;
R¹ Wasserstoff oder C₁₋₃-Alkyl ist;
R² und R³ jeweils unabhängig voneinander bei jedem Auftreten aus der Gruppe ausgewählt sind, die aus Wasserstoff, Alkyl, Halogen und -OH besteht;
R⁴ H, C₁₋₃-Alkyl oder -C(=NH)NR^{4a}R^{4b} ist, wobei R^{4a} und R^{4b} jeweils unabhängig voneinander aus der Gruppe ausgewählt sind, die aus Wasserstoff und C₁₋₃-Alkyl besteht; oder
R¹ und R⁴ zusammen mit den Atomen, an die sie gebunden sind, ein Heterocycloalkyl-Ringsystem bilden, das mindestens ein N beinhaltet; und
R⁵ H oder Methyl ist.

2. Verbindung gemäß Anspruch 1, wobei R^{a} H ist.

3. Verbindung gemäß Anspruch 1, wobei R^{a} Methyl ist.

4. Verbindung gemäß einem der Ansprüche 1-3, wobei R^{b} aus der Gruppe ausgewählt ist, die aus Folgendem besteht:

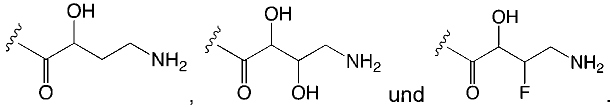

5. Verbindung gemäß Anspruch 4, wobei R^{b} aus der Gruppe ausgewählt ist, die aus Folgendem besteht:

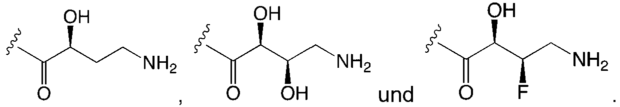

6. Verbindung gemäß einem der Ansprüche 1-5, wobei R⁵ Methyl ist.

7. Verbindung gemäß Anspruch 6, die eine Struktur aufweist, die aus der Gruppe ausgewählt ist, die aus Folgendem besteht:

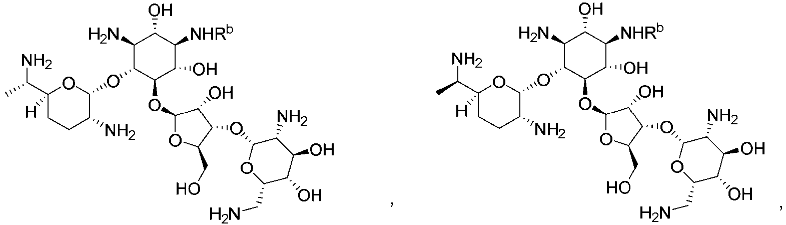

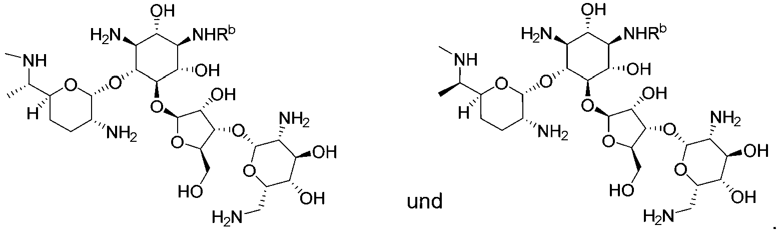

8. Verbindung gemäß Anspruch 7, die eine Struktur aufweist, die aus der Gruppe ausgewählt ist, die aus Folgendem besteht:

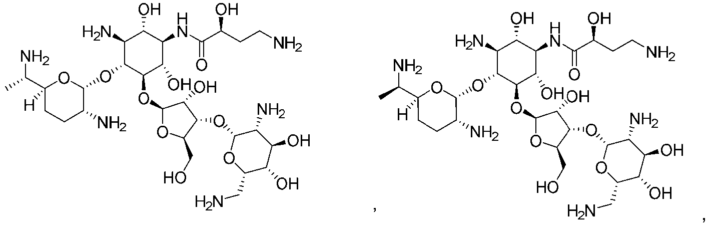

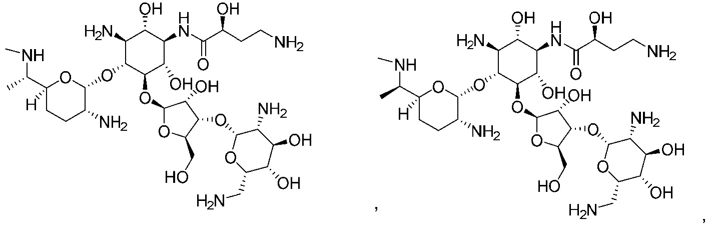

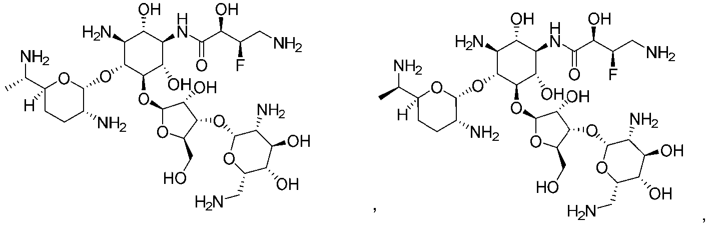

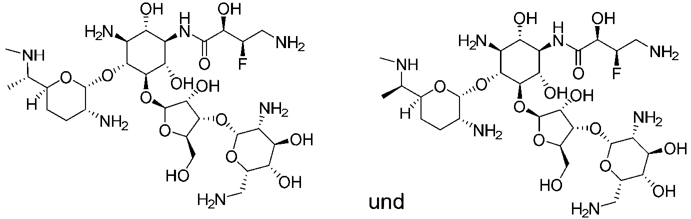

9. Verbindung gemäß einem der Ansprüche 1-5, wobei R⁵ H ist.

10. Verbindung gemäß Anspruch 9, die eine Struktur aufweist, die aus der Gruppe ausgewählt ist, die aus Folgendem besteht:

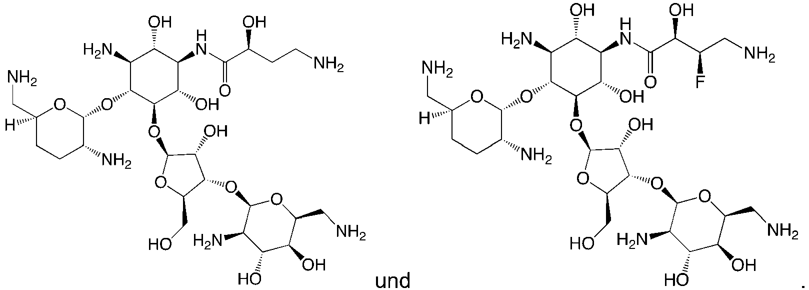

11. Eine pharmazeutische Zusammensetzung, die die Verbindung gemäß einem der Ansprüche 1-10 oder ein pharmazeutisch annehmbares Salz, Solvat, Tautomer oder Stereoisomer davon und mindestens einen pharmazeutisch annehmbaren Exzipienten beinhaltet.

12. Verbindung gemäß einem der Ansprüche 1-10 oder ein pharmazeutisch annehmbares Salz, Solvat, Tautomer oder Stereoisomer davon oder die pharmazeutische Zusammensetzung gemäß Anspruch 11 zur Verwendung bei der Behandlung einer bakteriellen Infektion bei einem Subjekt, das dessen bedarf.

13. Verbindung oder pharmazeutische Zusammensetzung zur Verwendung gemäß Anspruch 12, wobei die bakterielle Infektion mit grampositiven, gramnegativen, aeroben oder fakultativen anaeroben Bakterien ist.

14. Verbindung oder pharmazeutische Zusammensetzung zur Verwendung gemäß Anspruch 13, wobei eines oder mehrere der Folgenden zutreffen:
die bakterielle Infektion mit einer *Escherichia* sp., einer Klebsiella sp., einer *Proteus* sp., einer *Citrobacter* sp., einer *Morganella* sp., einer *Providencia* sp., einer *Yersinia* sp., einer *Enterobacter* sp.*,* einer *Salmonella* sp. oder einer *Serratia* sp. ist;
die bakterielle Infektion mit einer *Moraxella* sp., einer *Pseudomonas* sp., einer *Acinetobacter* sp., einer *Mycobacterium* sp., einer *Staphylococcus* sp., einer *Bacillus* sp., einer *Francisella* sp. oder einer *Burkholderia* sp. ist.

## Revendications

1. Un composé ayant une structure : 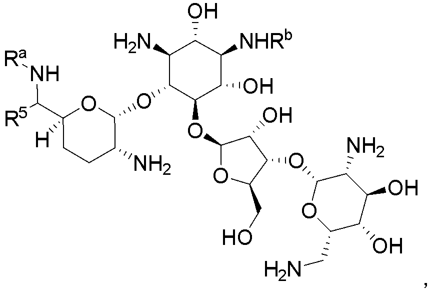
ou un sel ou solvate pharmaceutiquement acceptable de celui-ci,
dans lequel :
R^{a} est H ou un méthyle ;
R^{b} a une structure selon la Formule (II) : 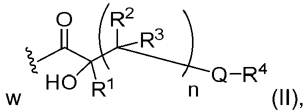 dans lequel :
Q est NH, O, ou S ;
n est un entier valant de 0 à 4 ;
R¹ est l'hydrogène ou un alkyle en C₁₋₃ ;
R² et R³ sont chacun sélectionnés indépendamment pour chaque occurrence dans le groupe constitué de l'hydrogène, un alkyle, un halogène, et -OH ;
R⁴ est H, un alkyle en C₁₋₃, ou -C(=NH)NR^{4a}R^{4b}, dans lequel R^{4a} et R^{4b} sont chacun sélectionnés indépendamment dans le groupe constitué de l'hydrogène et d'un alkyle en C₁₋₃ ; ou
R¹ et R⁴, conjointement avec les atomes auxquels ils sont attachés, forment un système cyclique hétérocycloalkyle comprenant au moins un N ; et
R⁵ est H ou un méthyle.

2. Le composé de la revendication 1, dans lequel R^{a} est H.

3. Le composé de la revendication 1, dans lequel R^{a} est un méthyle.

4. Le composé de l'une quelconque des revendications 1 à 3, dans lequel R^{b} est sélectionné dans le groupe constitué de :

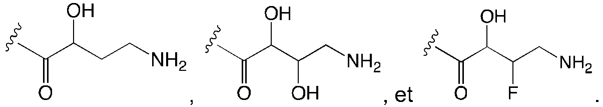

5. Le composé de la revendication 4, dans lequel R^{b} est sélectionné dans le groupe constitué de :

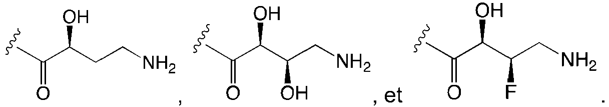

6. Le composé de l'une quelconque des revendications 1 à 5, dans lequel R⁵ est un méthyle.

7. Le composé de la revendication 6, ayant une structure sélectionnée dans le groupe constitué de :

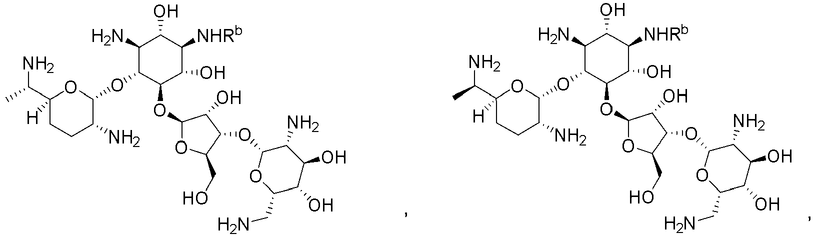

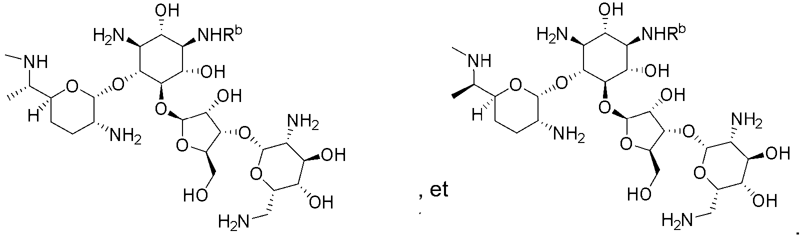

8. Le composé de la revendication 7, ayant une structure sélectionnée dans le groupe constitué de :

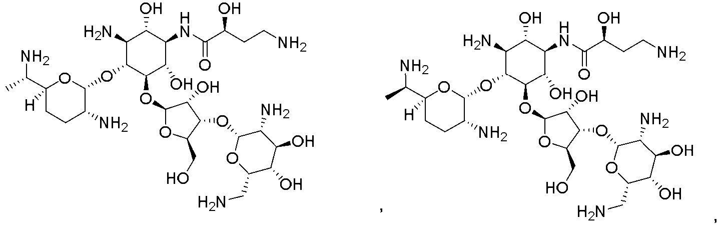

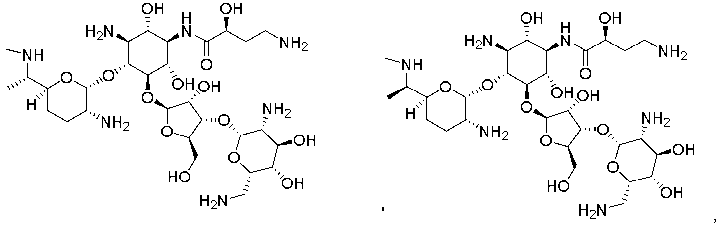

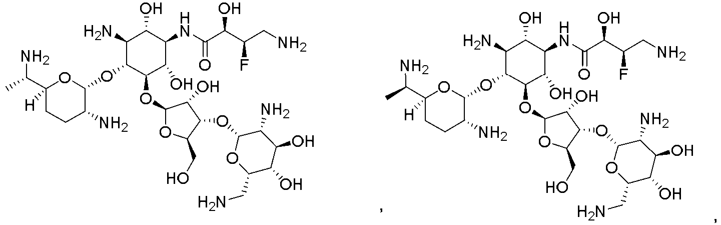

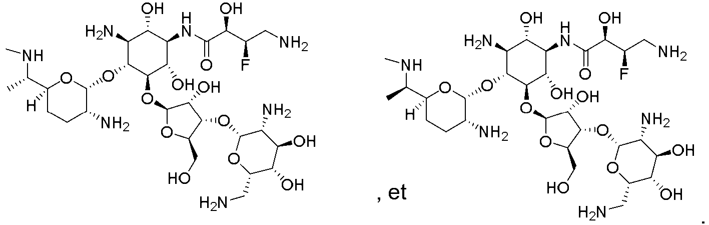

9. Le composé de l'une quelconque des revendications 1 à 5, dans lequel R⁵ est H.

10. Le composé de la revendication 9, ayant une structure sélectionnée dans le groupe constitué de :

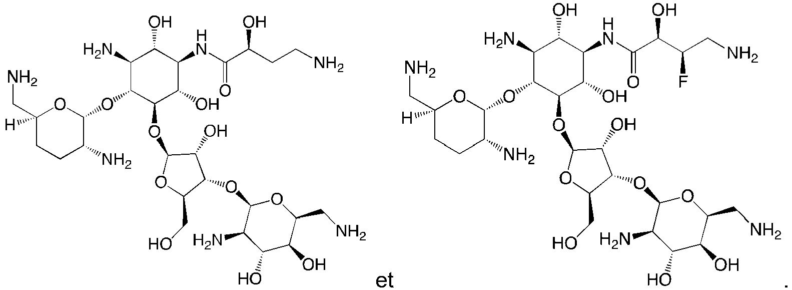

11. Une composition pharmaceutique comprenant le composé de l'une quelconque des revendications 1 à 10, ou un sel, solvate, tautomère, ou stéréoisomère pharmaceutiquement acceptable de celui-ci, et au moins un excipient pharmaceutiquement acceptable.

12. Le composé de l'une quelconque des revendications 1 à 10, ou un sel, solvate, tautomère, ou stéréoisomère pharmaceutiquement acceptable de celui-ci, ou la composition pharmaceutique de la revendication 11, pour leur utilisation dans le traitement d'une infection bactérienne chez un sujet en ayant besoin.

13. Le composé ou la composition pharmaceutique pour leur utilisation selon la revendication 12, dans lesquels l'infection bactérienne est à bactéries Gram-positives, Gram-négatives, aérobies, ou anaérobies facultatives.

14. Le composé ou la composition pharmaceutique pour leur utilisation selon la revendication 13, dans lesquels un ou plusieurs des suivants s'appliquent :
l'infection bactérienne est à *Escherichia* sp., *Klebsiella sp., Proteus sp., Citrobacter sp., Morganella sp., Providencia sp., Yersinia sp., Enterobacter sp., Salmonella sp.,* ou *Serratia sp. ;*
l'infection bactérienne est à *Moraxella sp., Pseudomonas sp., Acinetobacter sp., Mycobacterium sp., Staphylococcus sp., Bacillus sp., Francisella sp.,* ou *Burkholderia sp.*
